# EUROPEAN PATENT APPLICATION

(11) **EP 4 659 811 A1**
(43) Date of publication of application: **10.12.2025**
(21) Application number: 24180879.9
(22) Date of filing: 07.06.2024
(51) Int. Cl.: A61P 31/20

(54) **BK VIRUS NEUTRALIZING ANTIBODIES**

(71) Applicant: Université de Strasbourg, 67000 Strasbourg (FR); Institut National de la Santé et de la Recherche Médicale, 75013 Paris (FR); Centre National de la Recherche Scientifique, 75016 Paris (FR); LES HOPITAUX UNIVERSITAIRES DE STRASBOURG, 67000 Strasbourg (FR); Université Grenoble Alpes, 38400 Saint-Martin-d'Hères (FR); Commissariat à l'Energie Atomique et aux Energies Alternatives, 75015 Paris (FR); Centre Hospitalier Universitaire Grenoble Alpes, 38043 Grenoble Cedex 9 (FR)
(72) Inventor: POIGNARD, Pascal, 38044 GRENOBLE (FR); DERGAN-DYLON, Leonardo Sebastián, 38044 GRENOBLE (FR); BUISSON, Marlyse, 38044 GRENOBLE (FR); SCHOEHN, Guy, 38044 GRENOBLE (FR); PEDENKO, Borys, 38044 GRENOBLE (FR); FAFI-KREMER, Samira, 67000 STRASBOURG (FR); SOLIS, Morgane, 67000 STRASBOURG (FR); CAILLARD-OHLMANN, Sophie, 67000 STRASBOURG (FR)
(74) Representative: Ipsilon

(57) **Abstract**

The present patent application relates to the fields of antibodies and polyomaviruses. More specifically, the present patent application relates to a human monoclonal antibody, or antigen-binding fragment thereof, directed against a polyomavirus, in particular BKV, and that is useful in the treatment of an infection by said polyomavirus or associated-disorder thereof.

## Description

### FIELD OF THE INVENTION

The present patent application relates to the fields of antibodies and polyomaviruses. More specifically, the present patent application relates to a human monoclonal antibody, or antigen-binding fragment thereof, directed against a polyomavirus, in particular BKV, and that is useful in the treatment of an infection by said polyomavirus or associated-disorder thereof.

### INTRODUCTION

Human polyomaviruses, such as BK virus (BKV) or JC virus (JCV), are responsible for a common childhood infection highly prevalent worldwide, which elicits a neutralizing antibody and cellular immune response, while establishing a dormant and persistent infection in the kidney with minimal clinical manifestations. Despite its minimal impact among healthy individuals, polyomaviruses can reactivate and can cause severe pathology in transplant recipients and other immunocompromised individuals. It is estimated that up to 30% of the 100,000 kidney transplants performed annually worldwide are potentially compromised by BKV replication or, in its progressive form, by BKV-associated nephropathy (BKVAN) (Barth et al., Crit Rev Microbiol. 2017; 43: 178-195; Dharnidharka et al., Transplantation 2009; 87: 1019-1026). Early BKV replication after transplantation increases the risk of late acute rejection, which can expose recipients to graft loss when BKVAN develops (Seifert et al., J Am Soc Nephrol. 2017; 28: 1314-1325). Prolonged BKV replication also increases the risk of urothelial carcinoma in kidney transplant recipients (Gupta et al., Am J Transplant. 2018; 18(1): 245-252). In addition, BKV infection has been reported to cause symptomatic haemorrhagic cystitis in approximately 5% of the 50,000 hematopoietic stem cell transplants performed annually worldwide, as well as in 5% of cancer patients treated with high dose cyclophosphamide (Barth et al., Crit Rev Microbiol. 2017; 43: 178-195; Dharnidharka et al., Transplantation 2009; 87: 1019-1026), which leads to prolonged hospitalization and, in severe cases, can be fatal. As for the related JCV, its reactivation can typically impact the central 25 nervous system (CNS), which can result in progressive multifocal leukoencephalopathy (PML), a condition that is also frequently fatal.

Current guidelines recommend regular screening to detect BKV viruria or viremia after transplantation. Patients exhibiting persistent high BKV DNA loads usually undergo an immunosuppression reduction to lessen BKV replication and prevent BKVAN occurrence. However, because of its delayed nature, this pre-emptive strategy is not always successful and can actually increase the risk of graft rejection and death (Seifert et al., J Am Soc Nephrol. 2017; 28: 1314-1325; J Am Soc Nephrol. 2015; 26(4): 966-75). Besides, some individuals who are infected with one BKV subtype may remain vulnerable to other BKV subtypes after implementation of T cell immunosuppression, due to the absence of protective cross-reactive antibody responses.

There are at present no available BKV-specific antiviral therapies on the market.

Human intravenous immunoglobulin preparations (i.v. Ig, also referred as IVIG) have previously been shown to be effective in the prevention and treatment of many human viral infections in transplant patients. Randhawa et al. demonstrated that IVIG preparations commercialized under the names Privigen^{®} and Cytogam^{®} (both from CSL Behring GmbH, Marburg, Germany) contain antibodies capable of neutralizing all major BKPV genotypes *in vitro (*Am J Transplant. 2015; 15: 1014-1020), albeit much less efficiently for genotype IV These preparations however contain a mixture of uncharacterized human IgGs that are pooled from human plasma; they are thus susceptible to batch-to-batch variations and are not specific to BKV Some case reports even mentioned the presence of anti-blood group antibodies (ant-A or anti-B) in commercial IVIG products, which raises the risk of hemolysis in non-group O recipient patients.

Given the incomplete success of pre-emptive and supportive strategies for BKV-associated diseases, there is thus an urgent need to develop new anti-BKV preventive and therapeutic strategies, that are specific, safe, and highly effective against a wide range of BKV subtypes.

The present invention addresses the above discussed need in the art.

### SUMMARY OF THE INVENTION

The present patent invention is based on the development of novel human monoclonal antibodies, or antigen-binding fragments thereof, capable of binding and neutralizing BKV virus, with desirable pharmacokinetic properties and other desirable attributes, including binding and neutralizing of BKV subtype I, II and IV Targeted therapy with those antibodies can accordingly provide a novel therapeutic approach for BKV infection and address the large unmet medical needs associated with BKV related diseases.

The development of those antibodies is based on a novel approach which enabled the selection of a particular PBMCs (peripheral blood mononuclear cells) human donor with high anti-BKV antibody neutralization titer in serum, across various BKV subtypes. Memory B cells of this elite BKV neutralizer were then isolated for rescue of those monoclonal antibodies. Out of the 53 monoclonal antibodies isolated from this donor, 47 were selected for their affinity for BKV and their neutralization capacity and epitopes were characterized.

Accordingly, in a first aspect, the present invention relates to a monoclonal antibody, preferably human, or antigen-binding fragment or chain thereof, that binds and optionally neutralizes at least one BK virus subtype, said antibody comprising a heavy chain variable domain (VH) and/or a light chain variable domain (VL) with six complementary-determining regions (CDRs) selected from the following combinations:
a)
   - a VH-CDR1 comprising, or consisting of, amino acid sequence GFTFRSYXₐ₁ (SEQ ID NO: 1), wherein Xₐ₁ is T or S;
   - a VH-CDR2 comprising, or consisting of, amino acid sequence ISGSSNXₐ₂K (SEQ ID NO: 2), wherein Xₐ₂ is H or Q;
   - a VH-CDR3 comprising, or consisting of, amino acid sequence AREDDII,TPLYGMDV (SEQ ID NO: 3);
   - a VL-CDR1 comprising, or consisting of, amino acid sequence QSVRSNY (SEQ ID NO: 4);
   - a VL-CDR2 comprising, or consisting of, amino acid sequence GAS; and
   - a VL-CDR3 comprising, or consisting of, amino acid sequence HQYXₐ₃SSPRT (SEQ ID NO: 5), wherein Xₐ₃ is G or A; or
b)
   - a VH-CDR1 comprising, or consisting of, amino acid sequence GGSIRNSY (SEQ ID NO: 23);
   - a VH-CDR2 comprising, or consisting of, amino acid sequence IYYTGX_{c1}T (SEQ ID NO: 24), wherein X_{c1} is T or S;
   - a VH-CDR3 comprising, or consisting of, amino acid sequence AQGGGSGDLHY (SEQ ID NO: 25);
   - a VL-CDR1 comprising, or consisting of, amino acid sequence RSDVGGYNY (SEQ ID NO: 26);
   - a VL-CDR2 comprising, or consisting of, amino acid sequence DIS; and
   - a VL-CDR3 comprising, or consisting of, amino acid sequence ISFATGYTWV (SEQ ID NO: 27); or
c)
   - a VH-CDR1 comprising, or consisting of, amino acid sequence GGSISPYY (SEQ ID NO: 30);
   - a VH-CDR2 comprising, or consisting of, amino acid sequence IYYSGNT (SEQ ID NO: 31);
   - a VH-CDR3 comprising, or consisting of, the amino acid sequence ARDSCSGTCYLGX_{d1}NWFDP (SEQ ID NO: 32), wherein X_{d1} is D or G;
   - a VL-CDR1 comprising, or consisting of, amino acid sequence SSNIGANND (SEQ ID NO: 33);
   - a VL-CDR2 comprising, or consisting of, amino acid sequence GNN; and
   - a VL-CDR3 comprising, or consisting of, amino acid sequence QSYDSSLSTSKV (SEQ ID NO: 34); or
d)
   - a VH-CDR1 comprising, or consisting of, amino acid sequence GGSMTSGSYY (SEQ ID NO: 140);
   - a VH-CDR2 comprising, or consisting of, amino acid sequence IYTSGRT (SEQ ID NO: 141);
   - a VH-CDR3 comprising, or consisting of, amino acid sequence ARWGDSSAYYYVGDP (SEQ ID NO: 142);
   - a VL-CDR1 comprising, or consisting of, amino acid sequence QNINTY (SEQ ID NO: 143);
   - a VL-CDR2 comprising, or consisting of, amino acid sequence AAS; and
   - a VL-CDR3 comprising, or consisting of, amino acid sequence QQSFSLPHT (SEQ ID NO: 144); or
e)
   - a VH-CDR1 comprising, or consisting of, amino acid sequence GYTFTTHI (SEQ ID NO: 62);
   - a VH-CDR2 comprising, or consisting of, amino acid sequence INAGNGNT (SEQ ID NO: 63);
   - a VH-CDR3 comprising, or consisting of, amino acid sequence AREDQWLGRSPLEY (SEQ ID NO: 64);
   - a VL-CDR1 comprising, or consisting of, amino acid sequence QSVSSSY (SEQ ID NO: 65);
   - a VL-CDR2 comprising, or consisting of, amino acid sequence GAS; and
   - a VL-CDR3 comprising, or consisting of, amino acid sequence QQYGNSYPWT (SEQ ID NO: 66); or
f)
   - a VH-CDR1 comprising, or consisting of, amino acid sequence GFTFTNYW (SEQ ID NO: 57);
   - a VH-CDR2 comprising, or consisting of, amino acid sequence IYPDDSDA (SEQ ID NO: 58);
   - a VH-CDR3 comprising, or consisting of, amino acid sequence ARGGFCSGSVCYNPAWFDL (SEQ ID NO: 59);
   - a VL-CDR1 comprising, or consisting of, amino acid sequence QTINTY (SEQ ID NO: 60);
   - a VL-CDR2 comprising, or consisting of, amino acid sequence DAS; and
   - a VL-CDR3 comprising, or consisting of, amino acid sequence QQSYSTPLT (SEQ ID NO: 61); or
g)
   - a VH-CDR1 comprising, or consisting of, amino acid sequence GFTFDDHA (SEQ ID NO: 47);
   - a VH-CDR2 comprising, or consisting of, amino acid sequence INWNSASI (SEQ ID NO: 48);
   - a VH-CDR3 comprising, or consisting of, amino acid sequence AKDFGLWFGESRGIDA (SEQ ID NO: 49);
   - a VL-CDR1 comprising, or consisting of, amino acid sequence QSLLHSNGYNY (SEQ ID NO: 50);
   - a VL-CDR2 comprising, or consisting of, amino acid sequence LGS; and
   - a VL-CDR3 comprising, or consisting of, amino acid sequence MQALQTPIT (SEQ ID NO: 51); or
h)
   - a VH-CDR1 comprising, or consisting of, amino acid sequence GFXₒ₁FGXₒ₂YS (SEQ ID NO: 97), wherein Xₒ₁ is S or R, Xₒ₂ is S or T;
   - a VH-CDR2 comprising, or consisting of, amino acid sequence ISSGSSSI (SEQ ID NO: 98);
   - a VH-CDR3 comprising, or consisting of, amino acid sequence ARGWTGYDAFDI (SEQ ID NO: 99);
   - a VL-CDR1 comprising, or consisting of, amino acid sequence QVISNY (SEQ ID NO: 100);
   - a VL-CDR2 comprising, or consisting of, amino acid sequence AAS; and
   - a VL-CDR3 comprising, or consisting of, amino acid sequence QKYNNAPLT (SEQ ID NO: 101); or
i)
   - a VH-CDR1 comprising, or consisting of, amino acid sequence GFTFSNFG (SEQ ID NO: 82);
   - a VH-CDR2 comprising, or consisting of, amino acid sequence ISYDGSDK (SEQ ID NO: 83);
   - a VH-CDR3 comprising, or consisting of, amino acid sequence AKDRVRTKYYYDHSGPFDY (SEQ ID NO: 84);
   - a VL-CDR1 comprising, or consisting of, amino acid sequence NIGSKS (SEQ ID NO: 85);
   - a VL-CDR2 comprising, or consisting of, amino acid sequence DDD; and
   - a VL-CDR3 comprising, or consisting of, amino acid sequence QVWMTNSDRPV (SEQ ID NO: 86); or
j)
   - a VH-CDR1 comprising, or consisting of, amino acid sequence GGSISSGNYY (SEQ ID NO: 135);
   - a VH-CDR2 comprising, or consisting of, amino acid sequence IYTSGNT (SEQ ID NO: 136);
   - a VH-CDR3 comprising, or consisting of, amino acid sequence ARVRRDGYNAFDY (SEQ ID NO: 137);
   - a VL-CDR1 comprising, or consisting of, amino acid sequence NLGDKF (SEQ ID NO: 138);
   - a VL-CDR2 comprising, or consisting of, amino acid sequence QDS; and
   - a VL-CDR3 comprising, or consisting of, amino acid sequence QAWDSDTLYV (SEQ ID NO: 139); or
k)
   - a VH-CDR1 comprising, or consisting of, amino acid sequence GFXₖ₁FSSYA (SEQ ID NO: 67), wherein Xₖ₁ is T or P;
   - a VH-CDR2 comprising, or consisting of, amino acid sequence Xₖ₂TGSXₖ₃Xₖ₄Xₖ₅T (SEQ ID NO: 68), wherein Xₖ₂ is L or I, Xₖ₃ is G or A, Xₖ₄ is G or S, Xₖ₅ is S or G;
   - a VH-CDR3 comprising, or consisting of, amino acid sequence AKEGAYSYDXₖ₆WYFDL (SEQ ID NO: 69), wherein Xₖ₆ is F or Y;
   - a VL-CDR1 comprising, or consisting of, amino acid sequence QXₖ₇VSGXₖ₈Y (SEQ ID NO: 70), wherein Xₖ₇ is T or S and Xₖ₈ is N or S;
   - a VL-CDR2 comprising, or consisting of, amino acid sequence GAS; and
   - a VL-CDR3 comprising, or consisting of, amino acid sequence QQYGXₖ₉Xₖ₁₀Xₖ₁₁T (SEQ ID NO: 71), wherein Xₖ₉ is R or S, Xₖ₁₀ is P or L, Xₖ₁₁ is L or V; or
1)
   - a VH-CDR1 comprising, or consisting of, amino acid sequence GGSISSGGYY (SEQ ID NO: 52);
   - a VH-CDR2 comprising, or consisting of, amino acid sequence MYYSGST (SEQ ID NO: 53);
   - a VH-CDR3 comprising, or consisting of, amino acid sequence ARDRRDYGDYYYFGMDV (SEQ ID NO: 54);
   - a VL-CDR1 comprising, or consisting of, amino acid sequence SSNIGAGYD (SEQ ID NO: 55);
   - a VL-CDR2 comprising, or consisting of, amino acid sequence ANS; and
   - a VL-CDR3 comprising, or consisting of, amino acid sequence QSYDSSLSVYVV (SEQ ID NO: 56); or
m)
   - a VH-CDR1 comprising, or consisting of, amino acid sequence GYTFTSYS (SEQ ID NO: 150);
   - a VH-CDR2 comprising, or consisting of, amino acid sequence INAGNGDT (SEQ ID NO: 151);
   - a VH-CDR3 comprising, or consisting of, amino acid sequence ARSNSPTVTYNYYYYAMDV (SEQ ID NO: 152);
   - a VL-CDR1 comprising, or consisting of, amino acid sequence QSVRSSY (SEQ ID NO: 153);
   - a VL-CDR2 comprising, or consisting of, amino acid sequence DAS; and
   - a VL-CDR3 comprising, or consisting of, amino acid sequence QQYGNSPLT (SEQ ID NO: 154); or
n)
   - a VH-CDR1 comprising, or consisting of, amino acid sequence GFTFRSYT (SEQ ID NO: 6);
   - a VH-CDR2 comprising, or consisting of, amino acid sequence ISGSSNHK (SEQ ID NO: 7);
   - a VH-CDR3 comprising, or consisting of, amino acid sequence AREDDILTPLYGMDV (SEQ ID NO: 3);
   - a VL-CDR1 comprising, or consisting of, amino acid sequence SSDVGAYNY (SEQ ID NO: 111);
   - a VL-CDR2 comprising, or consisting of, amino acid sequence DVS; and
   - a VL-CDR3 comprising, or consisting of, amino acid sequence CSYAHRGTPL (SEQ ID NO: 112); or
o)
   - a VH-CDR1 comprising, or consisting of, amino acid sequence AYTFTNSA (SEQ ID NO: 12);
   - a VH-CDR2 comprising, or consisting of, amino acid sequence INTNTGNP (SEQ ID NO: 13);
   - a VH-CDR3 comprising, or consisting of, amino acid sequence X_{b1}VPYNFGGDHNSX_{b2}X_{b3}FFGX_{b4}DX_{b5} (SEQ ID NO: 14), wherein X_{b1} is T or A, X_{b2} is Y or F, X_{b3} is Y or H, X_{b4} is V or M and X_{b5} is I or V;
   - a VL-CDR1 comprising, or consisting of, amino acid sequence SSDVGX_{b6}YNX_{b7} (SEQ ID NO: 15), wherein X_{b6} is S or T and X_{b7} is L or F;
   - a VL-CDR2 comprising, or consisting of, amino acid sequence EGT; and
   - a VL-CDR3 comprising, or consisting of, amino acid sequence X_{b8}SYAGX_{b9}NX_{b10}FV (SEQ ID NO: 16), wherein X_{b8} is C or S, X_{b9} is S or G and X_{b10} is T or A;
P)
   - a VH-CDR1 comprising, or consisting of, amino acid sequence GFSXₚ₁SXₚ₂SE (SEQ ID NO: 104), wherein Xₚ₁ is L or S, Xₚ₂ is N or G;
   - a VH-CDR2 comprising, or consisting of, amino acid sequence VRSXₒ₃ANRHAXₒ₄ (SEQ ID NO: 105), wherein Xₚ₃ is R or T, Xₚ₄ is T or P;
   - a VH-CDR3 comprising, or consisting of, amino acid sequence TGVRGVISRYFAMDV (SEQ ID NO: 106);
   - a VL-CDR1 comprising, or consisting of, amino acid sequence Xₚ₅SDXₚ₆GXₚ₇YNY (SEQ ID NO: 107), wherein Xₚ₅ is S or N, Xₚ₆ is V or L, Xₚ₇ is A or S;
   - a VL-CDR2 comprising, or consisting of, amino acid sequence DVXₚ₈, wherein Xₚ₈ is S or T; and
   - a VL-CDR3 comprising, or consisting of, amino acid sequence CSYAXₚ₉Xₚ₁₀GXₚ₁₁PXₚ₁₂ (SEQ ID NO: 108), wherein Xₚ₉ is H or S, Xₚ₁₀ is R or V, Xₚ₁₁ is T or S, Xₚ₁₂ is L or I; or
q)
   - a VH-CDR1 comprising, or consisting of, amino acid sequence GFTFSGSE (SEQ ID NO: 117);
   - a VH-CDR2 comprising, or consisting of, amino acid sequence VRSRANRHAT (SEQ ID NO: 110);
   - a VH-CDR3 comprising, or consisting of, amino acid sequence TGVRGVISRYFAMDV (SEQ ID NO: 106);
   - a VL-CDR1 comprising, or consisting of, amino acid sequence NIGSNS (SEQ ID NO: 118);
   - a VL-CDR2 comprising, or consisting of, amino acid sequence DDS; and
   - a VL-CDR3 comprising, or consisting of, amino acid sequence QVWDRRGDLLV (SEQ ID NO: 119); or
r)
   - a VH-CDR1 comprising, or consisting of, amino acid sequence GETYVGHS (SEQ ID NO: 145);
   - a VH-CDR2 comprising, or consisting of, amino acid sequence ISLHNGNV (SEQ ID NO: 146);
   - a VH-CDR3 comprising, or consisting of, the amino acid sequence ARVPMKIYGVIVFGEYYYDQLDV (SEQ ID NO: 147);
   - a VL-CDR1 comprising, or consisting of, amino acid sequence GSDVGGYNY (SEQ ID NO: 148);
   - a VL-CDR2 comprising, or consisting of, amino acid sequence EVS; and
   - a VL-CDR3 comprising, or consisting of, amino acid sequence SSYAGSKVV (SEQ ID NO: 149); or
s)
   - a VH-CDR1 comprising, or consisting of, amino acid sequence GFTFRSYA (SEQ ID NO: 160);
   - a VH-CDR2 comprising, or consisting of, amino acid sequence LSSSGTTT (SEQ ID NO: 161);
   - a VH-CDR3 comprising, or consisting of, amino acid sequence AKDRNAQWLGSEPLDP (SEQ ID NO: 162);
   - a VL-CDR1 comprising, or consisting of, amino acid sequence QSVSSSY (SEQ ID NO: 65);
   - a VL-CDR2 comprising, or consisting of, amino acid sequence GAS; and
   - a VL-CDR3 comprising, or consisting of, amino acid sequence QQAYTFPWT (SEQ ID NO: 163); or
t)
   - a VH-CDR1 comprising, or consisting of, amino acid sequence GFTFSSYG (SEQ ID NO: 87);
   - a VH-CDR2 comprising, or consisting of, amino acid sequence ISKDGSNK (SEQ ID NO: 88);
   - a VH-CDR3 comprising, or consisting of, amino acid sequence AKIPLRRYSDSGDYSSGDF (SEQ ID NO: 89);
   - a VL-CDR1 comprising, or consisting of, amino acid sequence ENLVYSDGNTY (SEQ ID NO: 90);
   - a VL-CDR2 comprising, or consisting of, amino acid sequence KVS; and
   - a VL-CDR3 comprising, or consisting of, amino acid sequence MQTTHWPPLT (SEQ ID NO: 91); or
u)
   - a VH-CDR1 comprising, or consisting of, amino acid sequence GGSISSGRYY (SEQ ID NO: 172);
   - a VH-CDR2 comprising, or consisting of, amino acid sequence VYFSGST (SEQ ID NO: 173);
   - a VH-CDR3 comprising, or consisting of, amino acid sequence ARAVRDYGDSYSFDY (SEQ ID NO: 174);
   - a VL-CDR1 comprising, or consisting of, amino acid sequence SSDIGGYNY (SEQ ID NO: 175;
   - a VL-CDR2 comprising, or consisting of, amino acid sequence DVV; and
   - a VL-CDR3 comprising, or consisting of, amino acid sequence CSYSIRGTPV (SEQ ID NO: 176);
v)
   - a VH-CDR1 comprising, or consisting of, amino acid sequence GXᵣ₁YISTXᵣ₂SYY (SEQ ID NO: 120), wherein Xᵣ₁ is G or T, Xᵣ₂ is S or N;
   - a VH-CDR2 comprising, or consisting of, amino acid sequence IEYXᵣ₃GST (SEQ ID NO: 121), wherein Xᵣ₃ is S or G;
   - a VH-CDR3 comprising, or consisting of, amino acid sequence ASANSXᵣ₄YYDSGGYXᵣ₅APXᵣ₆ALDXᵣ₇ (SEQ ID NO: 122), wherein Xᵣ₄ is K or H, Xᵣ₅ is Y or C, Xᵣ₆ is G or S, Xᵣ₇ is L or I;
   - a VL-CDR1 comprising, or consisting of, amino acid sequence QSXᵣ₈SSH (SEQ ID NO: 123), wherein Xᵣ₈ is V or A;
w)
   - a VH-CDR1 comprising, or consisting of, amino acid sequence GGRFSRYG (SEQ ID NO: 155);
   - a VH-CDR2 comprising, or consisting of, amino acid sequence IIPLLGTA (SEQ ID NO: 156);
   - a VH-CDR3 comprising, or consisting of, amino acid sequence ATARREYDRPGAEYFIQ (SEQ ID NO: 157);
   - a VL-CDR1 comprising, or consisting of, amino acid sequence QSVSTN (SEQ ID NO: 158);
   - a VL-CDR2 comprising, or consisting of, amino acid sequence DAF; and
   - a VL-CDR3 comprising, or consisting of, amino acid sequence QQYNYWPPLT (SEQ ID NO: 159); or
x)
   - a VH-CDR1 comprising, or consisting of, amino acid sequence GFTFESYG (SEQ ID NO: 37);
   - a VH-CDR2 comprising, or consisting of, amino acid sequence ISNDGSIE (SEQ ID NO: 38);
   - a VH-CDR3 comprising, or consisting of, amino acid sequence AKDAVGITGGSLYYSYGMDV (SEQ ID NO: 39);
   - a VL-CDR1 comprising, or consisting of, amino acid sequence QTIKNY (SEQ ID NO: 40);
   - a VL-CDR2 comprising, or consisting of, amino acid sequence TAS; and
   - a VL-CDR3 comprising, or consisting of, amino acid sequence QQSYSAPYS (SEQ ID NO: 41);
y) • a VH-CDR1 comprising, or consisting of, amino acid sequence GFIFNNHG (SEQ ID NO: 92);
   - a VH-CDR2 comprising, or consisting of, amino acid sequence ISYDGKKE (SEQ ID NO: 93);
   - a VH-CDR3 comprising, or consisting of, amino acid sequence AKEAYGSGSPCDY (SEQ ID NO: 94);
   - a VL-CDR1 comprising, or consisting of, amino acid sequence QRVSSNY (SEQ ID NO: 95);
   - a VL-CDR2 comprising, or consisting of, amino acid sequence GAS; and
   - a VL-CDR3 comprising, or consisting of, amino acid sequence QQYGTSPRS (SEQ ID NO: 96);
   - a VL-CDR2 comprising, or consisting of, amino acid sequence AAS; and
   - a VL-CDR3 comprising, or consisting of, amino acid sequence QQYNNWPPGXᵣ₉Xᵣ₁₀ (SEQ ID NO: 124), wherein Xᵣ₉ is Y or T, Xᵣ₁₀ is S or absent; or
z)
   - a VH-CDR1 comprising, or consisting of, amino acid sequence GFTFSSYS (SEQ ID NO: 164);
   - a VH-CDR2 comprising, or consisting of, amino acid sequence ISSSSRTI (SEQ ID NO: 165);
   - a VH-CDR3 comprising, or consisting of, amino acid sequence ARQAVAHFDY (SEQ ID NO: 166);
   - a VL-CDR1 comprising, or consisting of, amino acid sequence QRVSSNY (SEQ ID NO: 95);
   - a VL-CDR2 comprising, or consisting of, amino acid sequence GAS; and
   - a VL-CDR3 comprising, or consisting of, amino acid sequence QQYGTSPRS (SEQ ID NO: 96); or
a')
   - a VH-CDR1 comprising, or consisting of, amino acid sequence GFTFSTYT (SEQ ID NO: 167);
   - a VH-CDR2 comprising, or consisting of, amino acid sequence ISSRSTSI (SEQ ID NO: 168);
   - a VH-CDR3 comprising, or consisting of, amino acid sequence VRDRGYYGSNWFDS (SEQ ID NO: 169);
   - a VL-CDR1 comprising, or consisting of, amino acid sequence SSDVGNYNY (SEQ ID NO: 170);
   - a VL-CDR2 comprising, or consisting of, amino acid sequence DVT; and
   - a VL-CDR3 comprising, or consisting of, amino acid sequence CAYAGSYTLV (SEQ ID NO: 171); or
b')
   - a VH-CDR1 comprising, or consisting of, amino acid sequence GFTVSTNC (SEQ ID NO: 42);
   - a VH-CDR2 comprising, or consisting of, amino acid sequence IYSGGTT (SEQ ID NO: 43);
   - a VH-CDR3 comprising, or consisting of, amino acid sequence ARSVDTYDIYALDV (SEQ ID NO: 44);
   - a VL-CDR1 comprising, or consisting of, amino acid sequence QSISRW (SEQ ID NO: 45);
   - a VL-CDR2 comprising, or consisting of, amino acid sequence KAS; and
   - a VL-CDR3 comprising, or consisting of, amino acid sequence QQYNSYPWT (SEQ ID NO: 46); or
c')
   - a VH-CDR1 comprising, or consisting of, amino acid sequence GGSIRNSY (SEQ ID NO: 23);
   - a VH-CDR2 comprising, or consisting of, amino acid sequence IYYTGTT (SEQ ID NO: 28);
   - a VH-CDR3 comprising, or consisting of, amino acid sequence AQGGGSGDLHY (SEQ ID NO: 25);
   - a VL-CDR1 comprising, or consisting of, amino acid sequence QSVRSNY (SEQ ID NO: 4);
   - a VL-CDR2 comprising, or consisting of, amino acid sequence GAS; and
   - a VL-CDR3 comprising, or consisting of, amino acid sequence HQYASSPRT (SEQ ID NO: 11).

In a preferred aspect, the monoclonal antibody of the invention comprises a heavy chain variable domain (VH) and/or a light chain variable domain (VL) with six complementary-determining regions (CDRs) selected from the following combinations:
i)
   - a VH-CDR1 comprising, or consisting of, amino acid sequence SEQ ID NO: 6 or 9;
   - a VH-CDR2 comprising, or consisting of, amino acid sequence SEQ ID NO: 7 or 10;
   - a VH-CDR3 comprising, or consisting of, amino acid sequence SEQ ID NO: 3;
   - a VL-CDR1 comprising, or consisting of, amino acid sequence SEQ ID NO: 4;
   - a VL-CDR2 comprising, or consisting of, amino acid sequence GAS; and
   - a VL-CDR3 comprising, or consisting of, amino acid sequence SEQ ID NO: 8 or 11; or
ii)
   - a VH-CDR1 comprising, or consisting of, amino acid sequence SEQ ID NO: 125 or 130;
   - a VH-CDR2 comprising, or consisting of, amino acid sequence SEQ ID NO: 126 or 131;
   - a VH-CDR3 comprising, or consisting of, amino acid sequence SEQ ID NO: 127 or 132;
   - a VL-CDR1 comprising, or consisting of, amino acid sequence SEQ ID NO: 128 or 133;
   - a VL-CDR2 comprising, or consisting of, amino acid sequence AAS; and
   - a VL-CDR3 comprising, or consisting of, amino acid sequence SEQ ID NO: 129 or 134; or
iii)
   - a VH-CDR1 comprising, or consisting of, amino acid sequence SEQ ID NO: 72 or 77;
   - a VH-CDR2 comprising, or consisting of, amino acid sequence SEQ ID NO: 73 or 78;
   - a VH-CDR3 comprising, or consisting of, amino acid sequence SEQ ID NO: 74 or 79;
   - a VL-CDR1 comprising, or consisting of, amino acid sequence SEQ ID NO: 75 or 80;
   - a VL-CDR2 comprising, or consisting of, amino acid sequence GAS; and
   - a VL-CDR3 comprising, or consisting of, amino acid sequence SEQ ID NO: 76 or 81; or
iv)
   - a VH-CDR1 comprising, or consisting of, amino acid sequence SEQ ID NO: 12;
   - a VH-CDR2 comprising, or consisting of, amino acid sequence SEQ ID NO: 13;
   - a VH-CDR3 comprising, or consisting of, amino acid sequence SEQ ID NO: 17 or 20;
   - a VL-CDR1 comprising, or consisting of, amino acid sequence SEQ ID NO: 18 or 21;
   - a VL-CDR2 comprising, or consisting of, amino acid sequence EGT; and
   - a VL-CDR3 comprising, or consisting of, amino acid sequence SEQ ID NO: 19 or 22; or
v)
   - a VH-CDR1 comprising, or consisting of, amino acid sequence SEQ ID NO: 109 or 113;
   - a VH-CDR2 comprising, or consisting of, amino acid sequence SEQ ID NO: 110 or 114;
   - a VH-CDR3 comprising, or consisting of, amino acid sequence SEQ ID NO: 106;
   - a VL-CDR1 comprising, or consisting of, amino acid sequence SEQ ID NO: 111 or 115;
   - a VL-CDR2 comprising, or consisting of, amino acid sequence DVS or DVT; and
   - a VL-CDR3 comprising, or consisting of, amino acid sequence SEQ ID NO: 112 or 116.

In another preferred aspect, the six complementary determining regions (CDRs) of the antibody or antigen-binding fragment according to the invention are selected from one of the following combinations:
1) VH-CDR1, VH-CDR2 and VH-CDR3 comprising, or consisting of, amino acid sequence SEQ ID NO: 6, 7 and 3, respectively; and VL-CDR1 and VL-CDR3 comprising, or consisting of, amino acid sequence SEQ ID NO: 4 and 8, respectively, and VL-CDR2 comprising, or consisting of, amino acid sequence GAS; or
2) VH-CDR1, VH-CDR2 and VH-CDR3 comprising, or consisting of, amino acid sequence SEQ ID NO: 9, 10 and 3, respectively; and VL-CDR1 and VL-CDR3 comprising, or consisting of, amino acid sequence SEQ ID NO: 4 and 11, respectively, and VL-CDR2 comprising, or consisting of, amino acid sequence GAS; or
3) VH-CDR1, VH-CDR2 and VH-CDR3 comprising, or consisting of, amino acid sequence SEQ ID NO: 23, 28 and 25, respectively; and VL-CDR1 and VL-CDR3 comprising, or consisting of, amino acid sequence SEQ ID NO: 26 and 27, respectively and VL-CDR2 comprising, or consisting of, amino acid sequence DIS; or
4) VH-CDR1, VH-CDR2 and VH-CDR3 comprising, or consisting of, amino acid sequence SEQ ID NO: 23, 29 and 25, respectively; and VL-CDR1 and VL-CDR3 comprising, or consisting of, amino acid sequence SEQ ID NO: 26 and 27, respectively and VL-CDR2 comprising, or consisting of, amino acid sequence DIS; or
5) VH-CDR1, VH-CDR2 and VH-CDR3 comprising, or consisting of, amino acid sequence SEQ ID NO: 30, 31 and 35, respectively; and VL-CDR1 and VL-CDR3 comprising, or consisting of, amino acid sequence SEQ ID NO: 33 and 34, respectively, and VL-CDR2 comprising, or consisting of, amino acid sequence GNN; or
6) VH-CDR1, VH-CDR2 and VH-CDR3 comprising, or consisting of, amino acid sequence SEQ ID NO: 30, 31 and 36, respectively; and VL-CDR1 and VL-CDR3 comprising, or consisting of, amino acid sequence SEQ ID NO: 33 and 34, respectively, and VL-CDR2 comprising, or consisting of, amino acid sequence GNN; or
7) VH-CDR1, VH-CDR2 and VH-CDR3 comprising, or consisting of, amino acid sequence SEQ ID NO: 140, 141 and 142, respectively; and VL-CDR1 and VL-CDR3 comprising, or consisting of, amino acid sequence SEQ ID NO: 143 and 144, respectively, and VL-CDR2 comprising, or consisting of, amino acid sequence AAS; or
8) VH-CDR1, VH-CDR2 and VH-CDR3 comprising, or consisting of, amino acid sequence SEQ ID NO: 62, 63 and 64, respectively; and VL-CDR1 and VL-CDR3 comprising, or consisting of, amino acid sequence SEQ ID NO: 65 and 66, respectively, and VL-CDR2 comprising, or consisting of, amino acid sequence GAS; or
9) VH-CDR1, VH-CDR2 and VH-CDR3 comprising, or consisting of, amino acid sequence SEQ ID NO: 57, 58 and 59, respectively; and VL-CDR1 and VL-CDR3 comprising, or consisting of, amino acid sequence SEQ ID NO: 60 and 61, respectively, and VL-CDR2 comprising, or consisting of, amino acid sequence DAS; or
10) VH-CDR1, VH-CDR2 and VH-CDR3 comprising, or consisting of, amino acid sequence SEQ ID NO: 47, 48 and 49, respectively; and VL-CDR1 and VL-CDR3 comprising, or consisting of, amino acid sequence SEQ ID NO: 50 and 51, respectively, and VL-CDR2 comprising, or consisting of, amino acid sequence LGS; or
11) VH-CDR1, VH-CDR2 and VH-CDR3 comprising, or consisting of, amino acid sequence SEQ ID NO: 102, 98 and 99, respectively; and VL-CDR1 and VL-CDR3 comprising, or consisting of, amino acid sequence SEQ ID NO: 100 and 101, respectively, and VL-CDR2 comprising, or consisting of, amino acid sequence AAS; or
12) VH-CDR1, VH-CDR2 and VH-CDR3 comprising, or consisting of, amino acid sequence SEQ ID NO: 103, 98 and 99, respectively; and VL-CDR1 and VL-CDR3 comprising, or consisting of, amino acid sequence SEQ ID NO: 100 and 101, respectively, and VL-CDR2 comprising, or consisting of, amino acid sequence AAS; or
13) VH-CDR1, VH-CDR2 and VH-CDR3 comprising, or consisting of, amino acid sequence SEQ ID NO: 82, 83 and 84, respectively; and VL-CDR1, VL-CDR2 and VL-CDR3 comprising, or consisting of, amino acid sequence SEQ ID NO: 85 and 86, respectively, and VL-CDR2 comprising, or consisting of, amino acid sequence DDD; or
14) VH-CDR1, VH-CDR2 and VH-CDR3 comprising, or consisting of, amino acid sequence SEQ ID NO: 135, 136 and 137, respectively; and VL-CDR1 and VL-CDR3 comprising, or consisting of, amino acid sequence SEQ ID NO: 138 and 139, respectively, and VL-CDR2 comprising, or consisting of, amino acid sequence QDS; or
15) VH-CDR1, VH-CDR2 and VH-CDR3 comprising, or consisting of, amino acid sequence SEQ ID NO: 72, 73 and 74, respectively; and VL-CDR1, VL-CDR2 and VL-CDR3 comprising, or consisting of, amino acid sequence SEQ ID NO: 75 and 76, respectively, and VL-CDR2 comprising, or consisting of, amino acid sequence GAS; or
16) VH-CDR1, VH-CDR2 and VH-CDR3 comprising, or consisting of, amino acid sequence SEQ ID NO: 77, 78 and 79, respectively; and VL-CDR1, VL-CDR2 and VL-CDR3 comprising, or consisting of, amino acid sequence SEQ ID NO:80 and 81, respectively, and VL-CDR2 comprising, or consisting of, amino acid sequence GAS; or
17) VH-CDR1, VH-CDR2 and VH-CDR3 comprising, or consisting of, amino acid sequence SEQ ID NO: 52, 53 and 54, respectively; and VL-CDR1 and VL-CDR3 comprising, or consisting of, amino acid sequence SEQ ID NO: 55 and 56, respectively, and VL-CDR2 comprising, or consisting of, amino acid sequence ANS; or
18) VH-CDR1, VH-CDR2 and VH-CDR3 comprising, or consisting of, amino acid sequence SEQ ID NO: 150, 151 and 152, respectively; and VL-CDR1 and VL-CDR3 comprising, or consisting of, amino acid sequence SEQ ID NO: 153 and 154, respectively, and VL-CDR2 comprising, or consisting of, amino acid sequence DAS; or
19) VH-CDR1, VH-CDR2 and VH-CDR3 comprising, or consisting of, amino acid sequence SEQ ID NO: 6, 7 and 3, respectively; and VL-CDR1 and VL-CDR3 comprising, or consisting of, amino acid sequence SEQ ID NO: 111 and 112, respectively, and VL-CDR2 comprising, or consisting of, amino acid sequence DVS; or
20) VH-CDR1, VH-CDR2 and VH-CDR3 comprising, or consisting of, amino acid sequence SEQ ID NO: 12, 13 and 17, respectively; and VL-CDR1 and VL-CDR3 comprising, or consisting of, amino acid sequence SEQ ID NO: 18 and 19, respectively and VL-CDR2 comprising, or consisting of, amino acid sequence EGT; or
21) VH-CDR1, VH-CDR2 and VH-CDR3 comprising, or consisting of, amino acid sequence SEQ ID NO: 12, 13 and 20, respectively; and VL-CDR1 and VL-CDR3 comprising, or consisting of, amino acid sequence SEQ ID NO: 21 and 22, respectively and VL-CDR2 comprising, or consisting of, amino acid sequence EGT; or
22) VH-CDR1, VH-CDR2 and VH-CDR3 comprising, or consisting of, amino acid sequence SEQ ID NO: 109, 110 and 106, respectively; and VL-CDR1 and VL-CDR3 comprising, or consisting of, amino acid sequence SEQ ID NO: 111 and 112, respectively, and VL-CDR2 comprising, or consisting of, amino acid sequence DVS; or
23) VH-CDR1, VH-CDR2 and VH-CDR3 comprising, or consisting of, amino acid sequence SEQ ID NO: 113, 114 and 106, respectively; and VL-CDR1 and VL-CDR3 comprising, or consisting of, amino acid sequence SEQ ID NO: 115 and 116, respectively, and VL-CDR2 comprising, or consisting of, amino acid sequence DVT; or
24) VH-CDR1, VH-CDR2 and VH-CDR3 comprising, or consisting of, amino acid sequence SEQ ID NO: 117, 110 and 106, respectively; and VL-CDR1 and VL-CDR3 comprising, or consisting of, amino acid sequence SEQ ID NO: 118 and 119, respectively, and VL-CDR2 comprising, or consisting of, amino acid sequence DDS; or
25) VH-CDR1, VH-CDR2 and VH-CDR3 comprising, or consisting of, amino acid sequence SEQ ID NO: 145, 146 and 147, respectively; and VL-CDR1 and VL-CDR3 comprising, or consisting of, amino acid sequence SEQ ID NO: 148 and 149, respectively, and VL-CDR2 comprising, or consisting of, amino acid sequence EVS; or
26) VH-CDR1, VH-CDR2 and VH-CDR3 comprising, or consisting of, amino acid sequence SEQ ID NO: 160, 161 and 162, respectively; and VL-CDR1 and VL-CDR3 comprising, or consisting of, amino acid sequence SEQ ID NO: 65 and 163, respectively, and VL-CDR2 comprising, or consisting of, amino acid sequence GAS; or
27) VH-CDR1, VH-CDR2 and VH-CDR3 comprising, or consisting of, amino acid sequence SEQ ID NO: 87, 88 and 89, respectively; and VL-CDR1, VL-CDR2 and VL-CDR3 comprising, or consisting of, amino acid sequence SEQ ID NO: 90 and 91, respectively, and VL-CDR2 comprising, or consisting of, amino acid sequence KVS; or
28) VH-CDR1, VH-CDR2 and VH-CDR3 comprising, or consisting of, amino acid sequence SEQ ID NO: 172, 173 and 174, respectively; and VL-CDR1 and VL-CDR3 comprising, or consisting of, amino acid sequence SEQ ID NO: 175 and 176, respectively, and VL-CDR2 comprising, or consisting of, amino acid sequence DVV; or
29) VH-CDR1, VH-CDR2 and VH-CDR3 comprising, or consisting of, amino acid sequence SEQ ID NO: 125, 126 and 127, respectively; and VL-CDR1 and VL-CDR3 comprising, or consisting of, amino acid sequence SEQ ID NO: 128 and 129, respectively, and VL-CDR2 comprising, or consisting of, amino acid sequence AAS; or
30) VH-CDR1, VH-CDR2 and VH-CDR3 comprising, or consisting of, amino acid sequence SEQ ID NO: 130, 131 and 132, respectively; and VL-CDR1 and VL-CDR3 comprising, or consisting of, amino acid sequence SEQ ID NO: 133 and 134, respectively, and VL-CDR2 comprising, or consisting of, amino acid sequence AAS; or
31) VH-CDR1, VH-CDR2 and VH-CDR3 comprising, or consisting of, amino acid sequence SEQ ID NO: 155, 156 and 157, respectively; and VL-CDR1 and VL-CDR3 comprising, or consisting of, amino acid sequence SEQ ID NO: 158 and 159, respectively, and VL-CDR2 comprising, or consisting of, amino acid sequence DAF; or
32) VH-CDR1, VH-CDR2 and VH-CDR3 comprising, or consisting of, amino acid sequence SEQ ID NO: 37, 38 and 39, respectively; and VL-CDR1 and VL-CDR3 comprising, or consisting of, amino acid sequence SEQ ID NO: 40 and 41, respectively, and VL-CDR2 comprising, or consisting of, amino acid sequence TAS; or
33) VH-CDR1, VH-CDR2 and VH-CDR3 comprising, or consisting of, amino acid sequence SEQ ID NO: 92, 93 and 94, respectively; and VL-CDR1, VL-CDR2 and VL-CDR3 comprising, or consisting of, amino acid sequence SEQ ID NO: 95 and 96, respectively, and VL-CDR2 comprising, or consisting of, amino acid sequence GAS; or
34) VH-CDR1, VH-CDR2 and VH-CDR3 comprising, or consisting of, amino acid sequence SEQ ID NO: 164, 165 and 166, respectively; and VL-CDR1 and VL-CDR3 comprising, or consisting of, amino acid sequence SEQ ID NO: 95 and 96, respectively, and VL-CDR2 comprising, or consisting of, amino acid sequence GAS; or
35) VH-CDR1, VH-CDR2 and VH-CDR3 comprising, or consisting of, amino acid sequence SEQ ID NO: 167, 168 and 169, respectively; and VL-CDR1 and VL-CDR3 comprising, or consisting of, amino acid sequence SEQ ID NO: 170 and 171, respectively, and VL-CDR2 comprising, or consisting of, amino acid sequence DVT; or
36) VH-CDR1, VH-CDR2 and VH-CDR3 comprising, or consisting of, amino acid sequence SEQ ID NO: 42, 43 and 44, respectively; and VL-CDR1 and VL-CDR3 comprising, or consisting of, amino acid sequence SEQ ID NO: 45 and 46, respectively, and VL-CDR2 comprising, or consisting of, amino acid sequence KAS; or
37) VH-CDR1, VH-CDR2 and VH-CDR3 comprising, or consisting of, amino acid sequence SEQ ID NO: 23, 28 and 25, respectively; and VL-CDR1 and VL-CDR3 comprising, or consisting of, amino acid sequence SEQ ID NO: 4 and 11, respectively, and VL-CDR2 comprising, or consisting of, amino acid sequence GAS.

In another aspect of the invention, the variable domains of the antibody or antigen-binding fragment thereof according to the invention are selected from one of the following combinations:
I)
   - the heavy chain variable domain (VH) comprises, or consists of, an amino acid sequence having at least 80% sequence identity to SEQ ID NO: 177, 179, 181 or 183; and
   - the light chain variable domain (VL) comprises, or consists of, an amino acid sequence having at least 80 % sequence identity to SEQ ID NO: 178, 180, 182 or 184,
   with the proviso that the CDRs are as described herein; or
II)
   - the heavy chain variable domain (VH) comprises, or consists of, an amino acid sequence having at least 80% sequence identity to SEQ ID NO: 189, 191, 193, or 195; and
   - the light chain variable domain (VL) comprises, or consists of, an amino acid sequence having at least 80 % sequence identity to SEQ ID NO: 190, 192, 194 or 196,
   with the proviso that the CDRs are as described herein; or
III)
   - the heavy chain variable domain (VH) comprises, or consists of, an amino acid sequence having at least 80% sequence identity to SEQ ID NO: 197, 199 or 201; and
   - the light chain variable domain (VL) comprises, or consists of, an amino acid sequence having at least 80 % sequence identity to SEQ ID NO: 198, 200 or 202,
   with the proviso that the CDRs are as described herein; or
IV)
   - the heavy chain variable domain (VH) comprises, or consists of, an amino acid sequence having at least 80% sequence identity to SEQ ID NO: 249, 251 or 243; and
   - the light chain variable domain (VL) comprises, or consists of, an amino acid sequence having at least 80 % sequence identity to SEQ ID NO: 250, 252 or 254,
   with the proviso that the CDRs are as described herein; or
V)
   - the heavy chain variable domain (VH) comprises, or consists of, an amino acid sequence having at least 80% sequence identity to SEQ ID NO: 213 or 215; and
   - the light chain variable domain (VL) comprises, or consists of, an amino acid sequence having at least 80 % sequence identity to SEQ ID NO: 214 or 216,
   with the proviso that the CDRs are as described herein; or
VI)
   - the heavy chain variable domain (VH) comprises, or consists of, an amino acid sequence having at least 80% sequence identity to SEQ ID NO: 211; and
   - the light chain variable domain (VL) comprises, or consists of, an amino acid sequence having at least 80 % sequence identity to SEQ ID NO: 212,
   with the proviso that the CDRs are as described herein; or
VII)
   - the heavy chain variable domain (VH) comprises, or consists of, an amino acid sequence having at least 80% sequence identity to SEQ ID NO: 207; and
   - the light chain variable domain (VL) comprises, or consists of, an amino acid sequence having at least 80 % sequence identity to SEQ ID NO: 208,
   with the proviso that the CDRs are as described herein; or
VIII)
   - the heavy chain variable domain (VH) comprises, or consists of, an amino acid sequence having at least 80% sequence identity to SEQ ID NO: 233 or 235; and
   - the light chain variable domain (VL) comprises, or consists of, an amino acid sequence having at least 80 % sequence identity to SEQ ID NO: 234 or 236,
   with the proviso that the CDRs are as described herein; or
IX)
   - the heavy chain variable domain (VH) comprises, or consists of, an amino acid sequence having at least 80% sequence identity to SEQ ID NO: 225 or 227; and
   - the light chain variable domain (VL) comprises, or consists of, an amino acid sequence having at least 80 % sequence identity to SEQ ID NO: 226 or 228,
   with the proviso that the CDRs are as described herein; or
X)
   - the heavy chain variable domain (VH) comprises, or consists of, an amino acid sequence having at least 80% sequence identity to SEQ ID NO: 247; and
   - the light chain variable domain (VL) comprises, or consists of, an amino acid sequence having at least 80 % sequence identity to SEQ ID NO: 248,
   with the proviso that the CDRs are as described herein; or
XI)
   - the heavy chain variable domain (VH) comprises, or consists of, an amino acid sequence having at least 80% sequence identity to SEQ ID NO: 217, 219, 221 or 223; and
   - the light chain variable domain (VL) comprises, or consists of, an amino acid sequence having at least 80 % sequence identity to SEQ ID NO: 218, 220, 222 or 224,
   with the proviso that the CDRs are as described herein; or
XII)
   - the heavy chain variable domain (VH) comprises, or consists of, an amino acid sequence having at least 80% sequence identity to SEQ ID NO: 209; and
   - the light chain variable domain (VL) comprises, or consists of, an amino acid sequence having at least 80 % sequence identity to SEQ ID NO: 210,
   with the proviso that the CDRs are as described herein; or
XIII)
   - the heavy chain variable domain (VH) comprises, or consists of, an amino acid sequence having at least 80% sequence identity to SEQ ID NO: 257; and
   - the light chain variable domain (VL) comprises, or consists of, an amino acid sequence having at least 80 % sequence identity to SEQ ID NO: 258,
   with the proviso that the CDRs are as described herein; or
XIV)
   - the heavy chain variable domain (VH) comprises, or consists of, an amino acid sequence having at least 80% sequence identity to SEQ ID NO: 261; and
   - the light chain variable domain (VL) comprises, or consists of, an amino acid sequence having at least 80 % sequence identity to SEQ ID NO: 262,
   with the proviso that the CDRs are as described herein; or
XV)
   - the heavy chain variable domain (VH) comprises, or consists of, an amino acid sequence having at least 80% sequence identity to SEQ ID NO: 185 or 187; and
   - the light chain variable domain (VL) comprises, or consists of, an amino acid sequence having at least 80 % sequence identity to SEQ ID NO: 186 or 188,
   with the proviso that the CDRs are as described herein; or
XVI)
   - the heavy chain variable domain (VH) comprises, or consists of, an amino acid sequence having at least 80% sequence identity to SEQ ID NO: 237, 239 or 241; and
   - the light chain variable domain (VL) comprises, or consists of, an amino acid sequence having at least 80 % sequence identity to SEQ ID NO: 238, 240 or 242,
   with the proviso that the CDRs are as described herein; or
XVII)
   - the heavy chain variable domain (VH) comprises, or consists of, an amino acid sequence having at least 80% sequence identity to SEQ ID NO: 237, 239 or 241; and
   - the light chain variable domain (VL) comprises, or consists of, an amino acid sequence having at least 80 % sequence identity to SEQ ID NO: 238, 240 or 242,
   with the proviso that the CDRs are as described herein; or
XVIII)
   - the heavy chain variable domain (VH) comprises, or consists of, an amino acid sequence having at least 80% sequence identity to SEQ ID NO: 255; and
   - the light chain variable domain (VL) comprises, or consists of, an amino acid sequence having at least 80 % sequence identity to SEQ ID NO: 256,
   with the proviso that the CDRs are as described herein; or
XIX)
   - the heavy chain variable domain (VH) comprises, or consists of, an amino acid sequence having at least 80% sequence identity to SEQ ID NO: 263; and
   - the light chain variable domain (VL) comprises, or consists of, an amino acid sequence having at least 80 % sequence identity to SEQ ID NO: 264,
   with the proviso that the CDRs are as described herein; or
XX)
   - the heavy chain variable domain (VH) comprises, or consists of, an amino acid sequence having at least 80% sequence identity to SEQ ID NO: 229; and
   - the light chain variable domain (VL) comprises, or consists of, an amino acid sequence having at least 80 % sequence identity to SEQ ID NO: 230,
   with the proviso that the CDRs are as described herein; or
XXI)
   - the heavy chain variable domain (VH) comprises, or consists of, an amino acid sequence having at least 80% sequence identity to SEQ ID NO: 269; and
   - the light chain variable domain (VL) comprises, or consists of, an amino acid sequence having at least 80 % sequence identity to SEQ ID NO: 270,
   with the proviso that the CDRs are as described herein; or
XXII)
   - the heavy chain variable domain (VH) comprises, or consists of, an amino acid sequence having at least 80% sequence identity to SEQ ID NO: 243 or 245; and
   - the light chain variable domain (VL) comprises, or consists of, an amino acid sequence having at least 80 % sequence identity to SEQ ID NO: 244 or 246,
   with the proviso that the CDRs are as described herein; or
XXIII)
   - the heavy chain variable domain (VH) comprises, or consists of, an amino acid sequence having at least 80% sequence identity to SEQ ID NO: 259; and
   - the light chain variable domain (VL) comprises, or consists of, an amino acid sequence having at least 80 % sequence identity to SEQ ID NO: 260,
   with the proviso that the CDRs are as described herein; or
XXIV)
   - the heavy chain variable domain (VH) comprises, or consists of, an amino acid sequence having at least 80% sequence identity to SEQ ID NO: 203; and
   - the light chain variable domain (VL) comprises, or consists of, an amino acid sequence having at least 80 % sequence identity to SEQ ID NO: 204,
   with the proviso that the CDRs are as described herein; or
XXV)
   - the heavy chain variable domain (VH) comprises, or consists of, an amino acid sequence having at least 80% sequence identity to SEQ ID NO: 231; and
   - the light chain variable domain (VL) comprises, or consists of, an amino acid sequence having at least 80 % sequence identity to SEQ ID NO: 232,
   with the proviso that the CDRs are as described herein; or
XXVI)
   - the heavy chain variable domain (VH) comprises, or consists of, an amino acid sequence having at least 80% sequence identity to SEQ ID NO: 265; and
   - the light chain variable domain (VL) comprises, or consists of, an amino acid sequence having at least 80 % sequence identity to SEQ ID NO: 266,
   with the proviso that the CDRs are as described herein; or
XXVII)
   - the heavy chain variable domain (VH) comprises, or consists of, an amino acid sequence having at least 80% sequence identity to SEQ ID NO: 267; and
   - the light chain variable domain (VL) comprises, or consists of, an amino acid sequence having at least 80 % sequence identity to SEQ ID NO: 268,
   with the proviso that the CDRs are as described herein; or
XXVIII)
   - the heavy chain variable domain (VH) comprises, or consists of, an amino acid sequence having at least 80% sequence identity to SEQ ID NO: 205; and
   - the light chain variable domain (VL) comprises, or consists of, an amino acid sequence having at least 80 % sequence identity to SEQ ID NO: 206,
   with the proviso that the CDRs are as described herein.

In a preferred aspect, the variable domains of the antibody or antigen-binding fragment thereof according to the invention are selected from one of the following combinations:
- the heavy chain variable domain (VH) comprises, or consists of, an amino acid sequence having at least 80% sequence identity to SEQ ID NO: 177; and
   the light chain variable domain (VL) comprises, or consists of, an amino acid sequence having at least 80 % sequence identity to SEQ ID NO: 178,
   with the proviso that the CDRs as described herein; or
- the heavy chain variable domain (VH) comprises, or consists of, an amino acid sequence having at least 80% sequence identity to SEQ ID NO: 179; and
   the light chain variable domain (VL) comprises, or consists of, an amino acid sequence having at least 80 % sequence identity to SEQ ID NO: 180,
   with the proviso that the CDRs as described herein; or
- the heavy chain variable domain (VH) comprises, or consists of, an amino acid sequence having at least 80% sequence identity to SEQ ID NO: 181; and
   the light chain variable domain (VL) comprises, or consists of, an amino acid sequence having at least 80 % sequence identity to SEQ ID NO: 182,
   with the proviso that the CDRs are as described herein; or
- the heavy chain variable domain (VH) comprises, or consists of, an amino acid sequence having at least 80% sequence identity to SEQ ID NO: 183; and
   the light chain variable domain (VL) comprises, or consists of, an amino acid sequence having at least 80 % sequence identity to SEQ ID NO: 184,
   with the proviso that the CDRs are as described herein; or
- the heavy chain variable domain (VH) comprises, or consists of, an amino acid sequence having at least 80% sequence identity to SEQ ID NO: 189; and
   the light chain variable domain (VL) comprises, or consists of, an amino acid sequence having at least 80 % sequence identity to SEQ ID NO: 190,
   with the proviso that the CDRs are as described herein; or
- the heavy chain variable domain (VH) comprises, or consists of, an amino acid sequence having at least 80% sequence identity to SEQ ID NO: 191; and
   the light chain variable domain (VL) comprises, or consists of, an amino acid sequence having at least 80 % sequence identity to SEQ ID NO: 192,
   with the proviso that the CDRs are as described herein; or
- the heavy chain variable domain (VH) comprises, or consists of, an amino acid sequence having at least 80% sequence identity to SEQ ID NO: 193; and
   the light chain variable domain (VL) comprises, or consists of, an amino acid sequence having at least 80 % sequence identity to SEQ ID NO: 194,
   with the proviso that the CDRs are as described herein; or
- the heavy chain variable domain (VH) comprises, or consists of, an amino acid sequence having at least 80% sequence identity to SEQ ID NO: 197; and
   the light chain variable domain (VL) comprises, or consists of, an amino acid sequence having at least 80 % sequence identity to SEQ ID NO: 198,
   with the proviso that the CDRs are as described herein; or
- the heavy chain variable domain (VH) comprises, or consists of, an amino acid sequence having at least 80% sequence identity to SEQ ID NO: 199; and
   the light chain variable domain (VL) comprises, or consists of, an amino acid sequence having at least 80 % sequence identity to SEQ ID NO: 200,
   with the proviso that the CDRs are as described herein; or
- the heavy chain variable domain (VH) comprises, or consists of, an amino acid sequence having at least 80% sequence identity to SEQ ID NO: 201; and
   the light chain variable domain (VL) comprises, or consists of, an amino acid sequence having at least 80 % sequence identity to SEQ ID NO: 202,
   with the proviso that the CDRs are as described herein; or
- ) the heavy chain variable domain (VH) comprises, or consists of, an amino acid sequence having at least 80% sequence identity to SEQ ID NO: 249; and
   the light chain variable domain (VL) comprises, or consists of, an amino acid sequence having at least 80 % sequence identity to SEQ ID NO: 250,
   with the proviso that the CDRs are as described herein; or
- the heavy chain variable domain (VH) comprises, or consists of, an amino acid sequence having at least 80% sequence identity to SEQ ID NO: 251; and
   the light chain variable domain (VL) comprises, or consists of, an amino acid sequence having at least 80 % sequence identity to SEQ ID NO: 252,
   with the proviso that the CDRs are as described herein; or
- the heavy chain variable domain (VH) comprises, or consists of, an amino acid sequence having at least 80% sequence identity to SEQ ID NO: 253; and
   the light chain variable domain (VL) comprises, or consists of, an amino acid sequence having at least 80 % sequence identity to SEQ ID NO: 254,
   with the proviso that the CDRs are as described herein; or
- the heavy chain variable domain (VH) comprises, or consists of, an amino acid sequence having at least 80% sequence identity to SEQ ID NO: 213; and
   the light chain variable domain (VL) comprises, or consists of, an amino acid sequence having at least 80 % sequence identity to SEQ ID NO: 214,
   with the proviso that the CDRs are as described herein; or
- the heavy chain variable domain (VH) comprises, or consists of, an amino acid sequence having at least 80% sequence identity to SEQ ID NO: 215; and
   the light chain variable domain (VL) comprises, or consists of, an amino acid sequence having at least 80 % sequence identity to SEQ ID NO: 216,
   with the proviso that the CDRs are as described herein; or
- the heavy chain variable domain (VH) comprises, or consists of, an amino acid sequence having at least 80% sequence identity to SEQ ID NO: 211; and
   the light chain variable domain (VL) comprises, or consists of, an amino acid sequence having at least 80 % sequence identity to SEQ ID NO: 212,
   with the proviso that the CDRs are as described herein; or
- the heavy chain variable domain (VH) comprises, or consists of, an amino acid sequence having at least 80% sequence identity to SEQ ID NO: 207; and
   the light chain variable domain (VL) comprises, or consists of, an amino acid sequence having at least 80 % sequence identity to SEQ ID NO: 208,
   with the proviso that the CDRs are as described herein; or
- ) the heavy chain variable domain (VH) comprises, or consists of, an amino acid sequence having at least 80% sequence identity to SEQ ID NO: 233; and
   the light chain variable domain (VL) comprises, or consists of, an amino acid sequence having at least 80 % sequence identity to SEQ ID NO: 234,
   with the proviso that the CDRs are as described herein; or
- the heavy chain variable domain (VH) comprises, or consists of, an amino acid sequence having at least 80% sequence identity to SEQ ID NO: 235; and
   the light chain variable domain (VL) comprises, or consists of, an amino acid sequence having at least 80 % sequence identity to SEQ ID NO: 236,
   with the proviso that the CDRs are as described herein; or
- the heavy chain variable domain (VH) comprises, or consists of, an amino acid sequence having at least 80% sequence identity to SEQ ID NO: 225; and
   the light chain variable domain (VL) comprises, or consists of, an amino acid sequence having at least 80 % sequence identity to SEQ ID NO: 226,
   with the proviso that the CDRs are as described herein; or
- the heavy chain variable domain (VH) comprises, or consists of, an amino acid sequence having at least 80% sequence identity to SEQ ID NO: 227; and
   the light chain variable domain (VL) comprises, or consists of, an amino acid sequence having at least 80 % sequence identity to SEQ ID NO: 228,
   with the proviso that the CDRs are as described herein; or
- ) the heavy chain variable domain (VH) comprises, or consists of, an amino acid sequence having at least 80% sequence identity to SEQ ID NO: 247; and
   the light chain variable domain (VL) comprises, or consists of, an amino acid sequence having at least 80 % sequence identity to SEQ ID NO: 248,
   with the proviso that the CDRs are as described herein; or
- the heavy chain variable domain (VH) comprises, or consists of, an amino acid sequence having at least 80% sequence identity to SEQ ID NO: 217; and
   the light chain variable domain (VL) comprises, or consists of, an amino acid sequence having at least 80 % sequence identity to SEQ ID NO: 218,
   with the proviso that the CDRs are as described herein; or
- the heavy chain variable domain (VH) comprises, or consists of, an amino acid sequence having at least 80% sequence identity to SEQ ID NO: 219; and
   the light chain variable domain (VL) comprises, or consists of, an amino acid sequence having at least 80 % sequence identity to SEQ ID NO: 220,
   with the proviso that the CDRs are as described herein; or
- the heavy chain variable domain (VH) comprises, or consists of, an amino acid sequence having at least 80% sequence identity to SEQ ID NO: 221; and
   the light chain variable domain (VL) comprises, or consists of, an amino acid sequence having at least 80 % sequence identity to SEQ ID NO: 222,
   with the proviso that the CDRs are as described herein; or
- the heavy chain variable domain (VH) comprises, or consists of, an amino acid sequence having at least 80% sequence identity to SEQ ID NO: 223; and
   the light chain variable domain (VL) comprises, or consists of, an amino acid sequence having at least 80 % sequence identity to SEQ ID NO: 224,
   with the proviso that the CDRs are as described herein; or
- the heavy chain variable domain (VH) comprises, or consists of, an amino acid sequence having at least 80% sequence identity to SEQ ID NO: 209; and
   the light chain variable domain (VL) comprises, or consists of, an amino acid sequence having at least 80 % sequence identity to SEQ ID NO: 210,
   with the proviso that the CDRs are as described herein; or
- the heavy chain variable domain (VH) comprises, or consists of, an amino acid sequence having at least 80% sequence identity to SEQ ID NO: 257; and
   the light chain variable domain (VL) comprises, or consists of, an amino acid sequence having at least 80 % sequence identity to SEQ ID NO: 258,
   with the proviso that the CDRs are as described herein; or
- the heavy chain variable domain (VH) comprises, or consists of, an amino acid sequence having at least 80% sequence identity to SEQ ID NO: 261; and
   the light chain variable domain (VL) comprises, or consists of, an amino acid sequence having at least 80 % sequence identity to SEQ ID NO: 262,
   with the proviso that the CDRs are as described herein; or
- the heavy chain variable domain (VH) comprises, or consists of, an amino acid sequence having at least 80% sequence identity to SEQ ID NO: 185; and
   the light chain variable domain (VL) comprises, or consists of, an amino acid sequence having at least 80 % sequence identity to SEQ ID NO: 186,
   with the proviso that the CDRs are as described herein; or
- the heavy chain variable domain (VH) comprises, or consists of, an amino acid sequence having at least 80% sequence identity to SEQ ID NO: 187; and
   the light chain variable domain (VL) comprises, or consists of, an amino acid sequence having at least 80 % sequence identity to SEQ ID NO: 188,
   with the proviso that the CDRs are as described herein; or
- the heavy chain variable domain (VH) comprises, or consists of, an amino acid sequence having at least 80% sequence identity to SEQ ID NO: 237; and
   the light chain variable domain (VL) comprises, or consists of, an amino acid sequence having at least 80 % sequence identity to SEQ ID NO: 238,
   with the proviso that the CDRs are as described herein; or
- the heavy chain variable domain (VH) comprises, or consists of, an amino acid sequence having at least 80% sequence identity to SEQ ID NO: 239; and
   the light chain variable domain (VL) comprises, or consists of, an amino acid sequence having at least 80 % sequence identity to SEQ ID NO: 240,
   with the proviso that the CDRs are as described herein; or
- the heavy chain variable domain (VH) comprises, or consists of, an amino acid sequence having at least 80% sequence identity to SEQ ID NO: 241; and
   the light chain variable domain (VL) comprises, or consists of, an amino acid sequence having at least 80 % sequence identity to SEQ ID NO: 242,
   with the proviso that the CDRs are as described herein; or
- ) the heavy chain variable domain (VH) comprises, or consists of, an amino acid sequence having at least 80% sequence identity to SEQ ID NO: 255; and
   the light chain variable domain (VL) comprises, or consists of, an amino acid sequence having at least 80 % sequence identity to SEQ ID NO: 256,
   with the proviso that the CDRs are as described herein; or
- the heavy chain variable domain (VH) comprises, or consists of, an amino acid sequence having at least 80% sequence identity to SEQ ID NO: 263; and
   the light chain variable domain (VL) comprises, or consists of, an amino acid sequence having at least 80 % sequence identity to SEQ ID NO: 264,
   with the proviso that the CDRs are as described herein; or
- ) the heavy chain variable domain (VH) comprises, or consists of, an amino acid sequence having at least 80% sequence identity to SEQ ID NO: 229; and
   the light chain variable domain (VL) comprises, or consists of, an amino acid sequence having at least 80 % sequence identity to SEQ ID NO: 230,
   with the proviso that the CDRs are as described herein; or
- the heavy chain variable domain (VH) comprises, or consists of, an amino acid sequence having at least 80% sequence identity to SEQ ID NO: 269; and
   the light chain variable domain (VL) comprises, or consists of, an amino acid sequence having at least 80 % sequence identity to SEQ ID NO: 270,
   with the proviso that the CDRs are as described herein; or
- the heavy chain variable domain (VH) comprises, or consists of, an amino acid sequence having at least 80% sequence identity to SEQ ID NO: 243; and
   the light chain variable domain (VL) comprises, or consists of, an amino acid sequence having at least 80 % sequence identity to SEQ ID NO: 244,
   with the proviso that the CDRs are as described herein; or
- the heavy chain variable domain (VH) comprises, or consists of, an amino acid sequence having at least 80% sequence identity to SEQ ID NO: 245; and
   the light chain variable domain (VL) comprises, or consists of, an amino acid sequence having at least 80 % sequence identity to SEQ ID NO: 246,
   with the proviso that the CDRs are as described herein; or
- the heavy chain variable domain (VH) comprises, or consists of, an amino acid sequence having at least 80% sequence identity to SEQ ID NO: 259; and
   the light chain variable domain (VL) comprises, or consists of, an amino acid sequence having at least 80 % sequence identity to SEQ ID NO: 260,
   with the proviso that the CDRs are as described herein; or
- the heavy chain variable domain (VH) comprises, or consists of, an amino acid sequence having at least 80% sequence identity to SEQ ID NO: 203; and
   the light chain variable domain (VL) comprises, or consists of, an amino acid sequence having at least 80 % sequence identity to SEQ ID NO: 204,
   with the proviso that the CDRs are as described herein; or
- the heavy chain variable domain (VH) comprises, or consists of, an amino acid sequence having at least 80% sequence identity to SEQ ID NO: 231; and
   the light chain variable domain (VL) comprises, or consists of, an amino acid sequence having at least 80 % sequence identity to SEQ ID NO: 232,
   with the proviso that the CDRs are as described herein; or
- the heavy chain variable domain (VH) comprises, or consists of, an amino acid sequence having at least 80% sequence identity to SEQ ID NO: 265; and
   the light chain variable domain (VL) comprises, or consists of, an amino acid sequence having at least 80 % sequence identity to SEQ ID NO: 266,
   with the proviso that the CDRs are as described herein; or
- the heavy chain variable domain (VH) comprises, or consists of, an amino acid sequence having at least 80% sequence identity to SEQ ID NO: 267; and
   the light chain variable domain (VL) comprises, or consists of, an amino acid sequence having at least 80 % sequence identity to SEQ ID NO: 268,
   with the proviso that the CDRs are as described herein; or
- the heavy chain variable domain (VH) comprises, or consists of, an amino acid sequence having at least 80% sequence identity to SEQ ID NO: 205; and
   the light chain variable domain (VL) comprises, or consists of, an amino acid sequence having at least 80 % sequence identity to SEQ ID NO: 206,
   with the proviso that the CDRs are as defined in claim 3.36); or
- the heavy chain variable domain (VH) comprises, or consists of, an amino acid sequence having at least 80% sequence identity to SEQ ID NO: 195; and
   the light chain variable domain (VL) comprises, or consists of, an amino acid sequence having at least 80 % sequence identity to SEQ ID NO: 196,
   with the proviso that the CDRs are as described herein.

In a preferred aspect of the invention, the antibody, or antigen-binding fragment thereof, according to the invention, is an IgG, an IgA, an sIgA, an IgM or a nanobody, preferably an IgG, said IgG being preferably an IgG1, IgG2, IgG3 or IgG4.

In a preferred aspect, the antibody, or antigen-binding fragment thereof, according to the invention, further comprises, especially in the Fc region, modifications which modulate Fc/FcRn binding, complement binding, complement-dependent cytotoxicity (CDC), antibody-dependent cellular toxicity (ADCC), antibody-dependent cell phagocytosis (ADCP), glycosylation and/or pharmacokinetics such as half-life.

In a preferred aspect, the antibody, or antigen-binding fragment thereof, according to the invention, comprises one or more non-natural amino acids.

In a preferred aspect, the antibody, or antigen-binding fragment thereof, according to the invention, is comprised in an immunoconjugate, such as an immunoconjugate wherein the antibody, or antigen-binding fragment thereof, is conjugated with another moiety, such as another moiety selected from another antibody, including another anti-BKV antibody, a cytotoxic moiety (to form an ADC), a cell-penetrating compound or a tissue-penetrating compound.

In a preferred aspect, the antibody, or antigen-binding fragment thereof, according to the invention, is labeled with a detectable molecule.

In another aspect, the invention is directed to a combination of two or more antibodies, or antigen-binding fragments thereof, as described herein.

In another aspect, the invention relates to a host cell expressing the antibody, or antigen-binding fragment thereof according to the invention, or comprising the set of nucleic acids or the vector according to the invention.

In another aspect, the invention pertains to a pharmaceutical or diagnostic composition comprising: (1) at least one of the antibodies, or antigen-binding fragment thereof, according to the invention, or any combination thereof; and (2) optionally at least one pharmaceutically or diagnostically acceptable excipient.

In a preferred embodiment, the pharmaceutical composition is in a form suitable for a sustainable release, and/or for intravenous, intramuscular, subcutaneous intranasal, or intrathecal administration, or for administration by infusion or by aerosol.

In a further aspect, the invention is directed to a pharmaceutical composition as described herein, for use as a medicament.

In a preferred embodiment, the pharmaceutical composition is for use in the treatment of a BK virus (BKV) infection or associated disorder thereof, in a subject in need thereof.

In a preferred embodiment, the subject is an immunocompromised subject, especially a graft-recipient such as a kidney-graft or bone-marrow-graft recipient.

In a preferred embodiment, the disorder associated with a BK virus (BKV) infection is a BKV-related leukodystrophy.

In another aspect, the invention relates to a pharmaceutical composition as described herein and at least one antibody reacting with a non-BKV virus, as a combined preparation for simultaneous, separate or sequential use as a medicament, the non-BKV virus being selected from the group consisting of a SARS-CoV-2 virus and other coronaviruses, a Respiratory Syncytial Virus (RSV), a Herpes Virus Simplex (HSV), an Epstein-Barr virus (EBV), a cytomegalovirus (CMV), an influenza virus, a parainfluenza virus, a metapneumovirus, and a JC virus.

In another aspect, the invention pertains to two or more antibodies or antigen-binding fragments according to the invention, as a combined preparation for simultaneous, separate or sequential use as a medicament, preferably in the treatment of a BK virus (BKV) infection or associated disorder thereof, in a subject in need thereof.

In another aspect, the invention relates to an *in vitro* use of the diagnostic composition as described herein, for detecting the presence of BKV in a sample, preferably in a biological sample.

In another aspect, the invention is directed to an *in vitro* use of an anti-idiotypic antibody that binds to the antibody or antigen-binding fragment thereof according to the invention, for measuring the concentration of said antibody or fragment in a sample, preferably in a biological sample.

### LEGENDS OF THE FIGURES

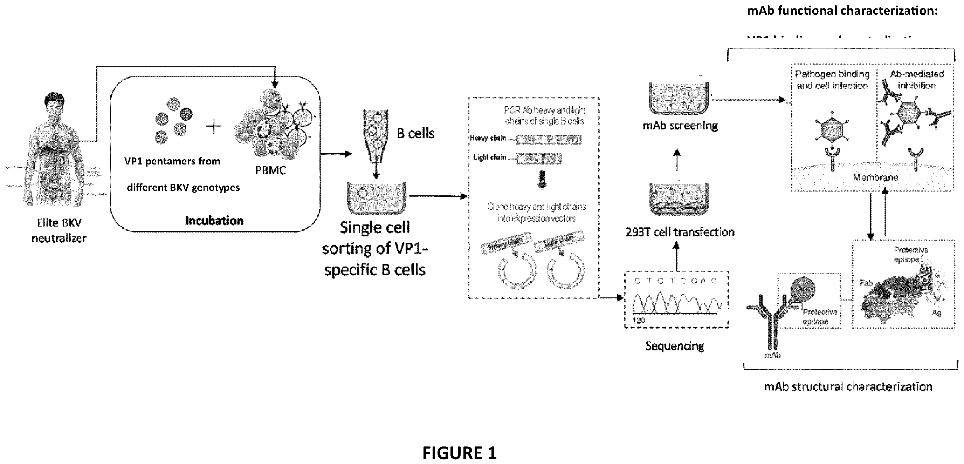

**Figure 1****.** Workflow representation of the generation of monoclonal antibodies by analysis of the memory B-cell repertoire of elite BKV neutralizers.

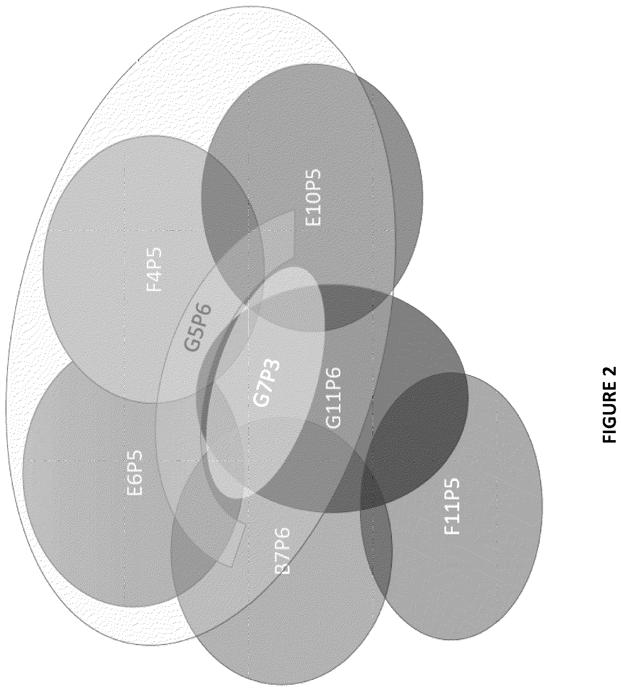

**Figure 2****.** Epitope mapping by cross-competition ELISA. Representation of overlap of monoclonal antibody epitopes. Epitope binning was performed with antibodies displaying neutralizing activity against at least one genotype. Except for antibody F 11P5, which competed only against two antibodies, the neutralizing antibodies largely competed with each other. This can be explained by the relatively small accessible surface area on the outer face of the VP1 pentamer at the viral surface and the large footprint occupied by each antibody (see epitope determination). Considering the potential stoichiometry of five antibodies per VP1 pentamer, it is expected that neutralizing antibodies would extensively compete.
**Figure 3****.** Cryo-EM structure of BKV VP1 pentamers complexed with Fabs for E11P6, E6P5 and G7P7. The interfacing area is based on the occupancy by the heavy and light chains.
**Figure 4****.** Epitope mapping by cryo-EM: Interactions of Fab G7P7 with VP1 pentamers.
**Figure 5****.** Epitope mapping by cryo-EM: Interactions of Fab E6P5 with VP1 pentamers.
**Figure 6****.** Epitope mapping by cryo-EM: Interactions of Fab E11P6 with VP1 pentamers.

### DETAILED DESCRIPTION OF THE INVENTION

The present invention may be understood more readily by reference to the following detailed description, including preferred embodiments of the invention and examples described below.

### Definitions

Unless otherwise defined herein or required by context, terms used in connection with the present invention, such as scientific and technical terms, shall have the meanings that are commonly known and understood in the art. Additional definitions may be provided throughout the detailed description.

The term *"antibody"* as used herein refers to a polypeptide of the immunoglobulin family that is capable of specific binding to a given antigen (herein, the Viral Protein 1, or VP1 of the BK or JC virus). Basic immunoglobulin structures in vertebrate systems are well understood (Harlow et al., Antibodies: A Laboratory Manual, Cold Spring Harbor Laboratory Press, ed. 1988). For example, antibodies that are naturally-occurring in humans and many mammals comprise four polypeptides - two full-length light chains (abbreviated herein as LC) and two full-length heavy chains (abbreviated herein as HC) - which are joined to one another with disulfide bonds to form a Y-shaped protein. Each of those chains, whether light or heavy, contains a *"constant"* domain (or region), as well as a *"variable"* domain (or region): constant domains usually confer important biological properties such as secretion, transplacental mobility, Fc receptor binding, complement binding, and the like; while variable domains determine antigen recognition and specificity. Because these variable domains are essential for antigen binding, it will be appreciated that an antibody as disclosed herein comprises at least the variable domain of a heavy chain (abbreviated herein as VH) and at least the variable domain of a light chain (abbreviated herein as VL). These VH and VL domains can be further subdivided into regions of hypervariability, termed complementarity determining regions (CDR), which are interspersed with regions that are more conserved, termed framework regions (FR). Each VH and VL of an antibody is typically composed of three CDRs and four FRs arranged (i.e. operably linked) from N- to C-terminus in the following preferred order: FR1, CDR1, FR2, CDR2, FR3, CDR3, and FR4. This is these regions of hypervariability of the heavy and light chains, especially the CDRs, that contain a binding domain (paratope) that interacts with a particular region of the antigen (epitope). As for the constant domains, the heavy chain may contain three constant domains, referred as CH1, CH2 and CH3, while the light chain may contain one constant domain, referred as CL. The two constant domains CH2 and CH3 of the heavy chains essentially form the Fc region (the trunk of the Y shape), which is capable of modulating immune cell activity once the antibody binds to the antigen. Antibodies are typically divided into five main immunoglobulin isotypes or classes (e.g., IgG, IgE, IgM, IgD, IgA and IgY), which are themselves divided into discrete subclasses (e.g., IgG1, IgG2, IgG3, IgG4, IgA1 and IgA2). This classification is based on the nature of heavy chain they contain, which includes alpha, delta, epsilon, gamma, and mu heavy chains, and essentially defines the function triggered by the antibody. In IgG, which make up the vast majority of antibodies in humans, heavy chains are as described above (VH and CH1, CH2, CH3). IgA and IgD also have three constant domains per heavy chain, whereas IgM and IgE each have four constant domains per heavy chain. IgG are herein particularly preferred. Antibody light chains can be classified in mammals either as kappa light chains or lambda light chains. The term antibody also encompasses, without limitation, antibodies that are polyclonal or monoclonal, human/ humanized or non-human/non-humanized, chimeric or non-chimeric.

An *"epitope"* or *"antigenic determinant"* refers herein to a region of an antigen to which an antibody binds to. The particular domain of the antibody which binds an epitope is named *"paratope".* Epitopes of a protein antigen can be formed from contiguous amino acids (i.e. it is a linear epitope) or noncontiguous amino acids that are juxtaposed by tertiary folding of the antigen (i.e. it is a conformational epitope). Linear epitopes are typically retained when exposed to denaturing solvents whereas conformational epitopes are typically lost on treatment with denaturing solvents. An epitope can typically include at least 3, preferably at least 4, 5, 6, 7, 8, 9 or 10 amino acids in a unique spatial conformation. Methods for determining spatial conformation of epitopes include, for example, x-ray crystallography, cryo-electron microscopy (cryo-EM), or nuclear magnetic resonance (NMR). Epitope mapping, which aims at identifying the specific amino acids of the antigen involved in the binding with an antibody, can be determined e.g. by array-based oligo-peptide scanning, site-directed mutagenesis mapping, high-throughput shotgun mutagenesis epitope mapping, or cross-linking-coupled mass spectrometry (See e.g. Epitope Mapping Protocols, Methods in Molecular Biology, Vol. 66, Glenn E. Morris, Ed. 1996). One specific example of mutagenesis mapping relies on the arginine/glutamic scanning protocol: briefly, amino acids at all or some specific positions of the antigen are substituted with either arginine or glutamic acid (or arginine with glutamic acid and vice-versa); binding to the mutants is then assessed to determine which residues affect binding. A similar approach relies on alanine scanning; substitution with alanine indeed eliminates side-chain interactions without altering main-chain conformation or introducing steric or electrostatic effects. Antibodies that recognize the same epitope can be identified in a simple immunoassay showing the ability of one antibody to block the binding of another antibody to a target antigen.

By contrast to polyclonal antibodies which recognize different epitopes of the same antigen, *"monoclonal antibodies"* or *"mAbs"* are antibodies which recognize a unique epitope on an antigen. That is because monoclonal antibodies typically derive from a single parent cell, such as a single B cell parent clone (i.e. single cell lineage), while polyclonal antibodies would stem from different parent cells, such as different B cell parent clones (i.e. from a different cell lineage).

A *"human antibody"* refers herein to an antibody, as defined above, that is naturally occurring in a human or that is produced in a suitable host cell, such as a human cell, as long as it contains antibody amino acid sequences that are characteristics of the human species. By contrast, humanized antibodies originate from a non-human species and have been modified, especially in the framework regions and/or constant regions, to resemble human antibodies so as to avoid any undesired immunogenic reactions when administered to humans.

The term "antigen-binding fragment", "antibody functional fragment" or "antibody fragment" designates herein an immunologically active fragment or portion of an antibody as defined above. This means that said fragment retains the same, or substantially the same, ability to bind to the same the antigen, more particularly to the same epitope, as the antibody from which it is derived. To do so, the antigen-binding fragment will typically retain at least the antigen-binding region of a traditional four-chain antibody, such as the CDR(s). Examples of antigen-binding fragments are well-known in the art, and include, to name a few, Fab (i.e. fragment antigen-binding region, which is comprised of one constant and one variable domain of each of the heavy chain and light chain i.e. VL, CL, VH and CH1), F(ab') (corresponding to a Fab, on which a few amino acids have been added at the C-terminus of the CH1 domain of the heavy chain, including one or more cysteines from the hinge region), F(ab')2 (corresponding to two Fabs, or a bivalent Fab, connected at a hinge region), scFv (single chain variable fragment, which is comprised of a VL and a VH), or HCAb (heavy chain antibody, which is comprised of a single heavy chain with one variable domain (VHH)). Antigen-binding fragments may also include single domain antibodies, maxibodies, minibodies, nanobodies, intrabodies, diabodies, triabodies, tetrabodies, or v-NAR. If needed, an Fc region may be added to the antigen-binding fragment.

As used herein, the terms *"Fc region", "Fc domain"* and *"fragment crystallizable region"* are interchangeable and refer to the tail region of an antibody that interacts with cell surface receptors called Fc receptors. The Fc region is typically composed of two domains, optionally identical, derived from the second and third constant domains of the antibody two heavy chains (i.e., CH2 and CH3 domains). A portion of the Fc region may refer to the CH2 or the CH3 domain. Optionally, the Fc region may further comprise all or a portion of the antibody hinge region, which is the flexible region generally comprised between the CH1 and CH2 domains. Accordingly, the Fc domain may comprise the hinge, the CH2 domain and the CH3 domain. Optionally, the Fc domain is that from IgG1, IgG2, IgG3 or IgG4, optionally with IgG1 hinge-CH2-CH3 or IgG4 hinge-CH2-CH3.

As explained above, each VH and VL of an antibody typically comprises regions of hypervariability termed *"complementarity determining regions", "complementarity determining domains"* or *"CDRs".* The CDRs are the antigen-binding site of the antibody variable domains that harbors specificity for and confer binding affinity to said antigen. In other words, the CDRs are structurally complementary to the epitope of the antigen and are thus directly responsible for the binding specificity. There are typically three CDRs (CDR1 to 3, numbered sequentially starting from the N-terminus) in each human VH or VL, constituting in total about 15 to 20% of the variable domain therein. This means that a conventional antibody generally contains six CDRs. These CDRs can be referred to by their region and sequential order. For example, "*VH-CDR1*" refers to the first CDR of the heavy chain variable domain; *"VH-CDR2 "* to the second CDR of this domain; *"VH-CDR3 "* to the third CDR of this domain. Likewise, "*VL-CDR1*" refers to the first CDR of the light chain variable domain; "*VL-CDR2*" to the second CDR of this domain; *"VL-CDR3"* to the third CDR of this domain. Given the particular structure of an antibody, their CDRs are not contiguous to each other.

The term *"framework regions"* or *"FRs"* refer to regions of the VH or VL that are interposed between the CDRs of an antibody. In general, these regions act as a scaffold that provides for positioning the CDRs in correct orientation, so as to support the binding of these CDRs to its cognate epitope. Framework regions are also known to exhibit far less variation in amino acid sequence than CDRs (Kuby, Immunology, 4th ed., Chapter 4. W.H. Freeman & Co., New York, 2000), and as such, are relatively conserved within a species, especially within an immunoglobulin class /subclass. Mutations of framework regions may nevertheless occur in cells and/or during affinity maturing of an antibody; such mutations are encompassed in the scope of the present invention as long as the antibody, or antigen-binding fragment thereof, retains at least its ability to bind its antigen. There are typically 4 FRs (FR1 to 4, numbered sequentially starting from the N-terminus) in each human VH or VL, constituting in total about 80 to 85 % of the variable domain therein. The framework regions of an antibody heavy chain may be referred to as "*HFR1*"*, "HFR2", "HFR3"* and *"HFR4",* while the framework regions of an antibody light chain may be referred to as "*LFRI*", *"LFR2", "LFR3"* and "*LFR4*"*.*

The precise amino acid sequence boundaries of each antibody region or domain can be readily determined using any of a number of techniques and/or schemes that are well-known in the art. Examples of numbering schemes include the EU numbering scheme (Edelman et al. Proc. Natl. Acad. Sci. USA 1969; 63; 78-85), Kabat numbering scheme (Kabat et al., Sequences of Proteins of Immunological Interest, NIH Publication No. 91-3242, 5th ed. 1991), Chothia numbering scheme (Al-Lazikani et al., J. Mol. Biol. 1997; 273:927-948), Contact numbering scheme (MacCallum et al., J. Mol. Biol. 1996; 262:732-745), IMGT numbering scheme (Lefranc et al., Immunol Today. 1997; 18(11):509 ; Lefranc et al., Dev. Comp. Immunol. 2003; 27:55-77), AHo numbering scheme (Honegge and Pluckthun, J. Mol. Biol. 2001; 309:657-70), Martin numbering scheme (enhanced Chotia or AbM; Abhinandan et al. Mol Immunol. 2008; 45:3832-9), and Wolfguy numbering scheme (Bujotzek et al., Proteins 2015; 83(4):681-95). Unless otherwise specified, the numbering scheme used herein for identification of a particular antibody region or domain is the IMGT numbering scheme. Under IMGT unique numbering for the variable and the constant domains of an antibody, conserved amino acids from framework regions always have the same number whatever the immunoglobulin chain type, whatever the domain (variable or constant), and whatever the species they come from. For correspondence with other numbering schemes, see the IMGT Scientific charts as provided on the IMGT website (e.g. https://www.imgt.org/IMGTScientificChart/).

As used herein, the term *"binding"* or "*bind*" refers to the capacity to recognize and contact (or interact with) another entity, herein the antibody is capable recognize and contact (or interact with) an antigen. This binding can entail some complementarity between the antigen binding domain (paratope) of the antibody and its epitope on the antigen, whether this epitope is linear or conformational. According to this definition, an antibody specifically or selectively binds to an antigen when it binds to that antigen via its antigen binding domain more readily than it would to a random, unrelated antigen. In other words, an antibody highly specific to an antigen would typically lack binding, or substantially lack binding, to a random, unrelated antigen. This *"specificity"* or *"selectivity"* in binding can be assessed for example by measuring the relative affinity by which a certain antibody (or antigen-binding fragment thereof) binds to a certain antigen.

The term *"compete with"* with regard to binding can be used herein interchangeably with *"competing", "cross-compete with", "competitively inhibit"* or *"is a competitive inhibitor of*"*.* An antibody that competes with a reference antibody for a common antigen typically shares the same epitope on that antigen or has an adjacent epitope sufficiently proximal to the epitope of the reference antibody for steric hindrance to occur. The antibody is said to be competing when it inhibits the binding of the reference antibody to a common antigen. This competition is also an indicator of the relative affinity by which the tested antibody binds to the antigen.

By "*affinity*" or *"binding affinity*", it is meant herein the strength of the binding between an antibody and an antigen, such as between the paratope and the epitope. It is well within the skill of the person in the art to assess the affinity of an antibody to an antigen, by methods well-known in the art, such as ELISA (Enzyme-Linked Immunosorbent Assay), BLI (Biolayer Interferometry), RIA (radioimmunoassay), SPR (Surface Plasmon Resonance) (see, e.g., Harlow et al., Using Antibodies, A Laboratory Manual, Cold Spring Harbor Press, ed. 1988), and/or as described in below Example's section 1.10 with regard to BKV In a conventional antibody/antigen binding assay, binding affinity is typically represented by the equilibrium dissociated constant (K_{D}), which is a ratio of the rate at which the antibody binds its target antigen (Kₒₙ) and of the rate at which the antibody-antigen complex dissociates (K_{off}). K_{D} and affinity are inversely related: a high affinity interaction is characterized by a low K_{D}, a fast recognizing (high Kₒₙ) and a strong stability of formed complexes (low K_{off}). Antibodies with a higher affinity for a particular antigen would be expected to bind more strongly and stably to said antigen. As an alternative, an apparent estimate of binding affinity can be provided by measuring the half-maximal effective concentration (EC₅₀), which, in a binding assay, is the concentration of antibody at which half (50%) of its target antigen is bound thereto. In the context of the present invention, the EC₅₀ of antibodies with a high affinity for the antigen is preferably equal or lower than about 3 µg/ml, more preferably equal or lower than about 2.5 µg/ml, 2 µg/ml, 1.5 µg/ml or 1 µg/ml. Competitive binding assays can also be performed between antibodies. Typically, such an assay involves the use of purified antigen bound to a solid surface or cells bearing either of these, an unlabeled test antibody at varying concentration and a labeled reference antibody at a fixed concentration. Competitive inhibition is then measured by determining the amount of label bound to the solid surface or cells in the presence of the test antibody.

By *"neutralizing"* or *"neutralization"* of an antigen, it is meant herein the capacity to inhibit or reduce the activity of said antigen, which, for an infectious or pathogenic antigen, means the capacity to inhibit or reduce the infectious or pathogenic activity, such as viral replication. This is an important property to assess since not all antibodies that bind to an infectious or pathogenic antigen are neutralizing. It is well within the skill of the person in the art to assess the neutralization activity of an antibody against such antigen, by methods well-known in the art named neutralization assays, such as pseudotype virus assay, microneutralization assay, plaque reduction assay, and/or as described in below Example's section 1.9 with regard to BKV Antibody neutralization can be represented by the IC₅₀ (half-maximal inhibitory concentration), which, in a neutralization assay, is the concentration of antibody at which half (50%) of the antigen activity is inhibited. In the context of the present invention, the IC₅₀ of antibodies with a high neutralization potency against the antigen is preferably equal or lower than about 10 µg/ml, more preferably equal or lower than about 1 µg/ml, 0.9 µg/ml, 0.5 µg/ml, 0.05 µg/ml or 0.001 µg/ml. As an alternative, antibody neutralization can be provided by measuring the IC₉₀, which, in a neutralization assay, is the concentration of antibody at which 90% of the antigen activity is inhibited.

"*BKV*" or *"BK virus"* (also known as *"human polyomavirus 1*", or "*BK polyomavirus*") and "*JCV*" or *"JC virus"* (also known as *"human polyomavirus 2", "JC polyomavirus"* or *"John Cunningham virus"*) are related members of the *Polyomaviridae* family, belonging more specifically to the *betapolyomavirus* genus. These polyomaviruses are icosahedral, non-enveloped, viruses measuring approximately 40-45 nM in diameter with a circular doublestranded DNA genome of approximately 5,000 base pairs (Bennett et al., Microbes and Infection 2012; 14(9): 672-683; Johne et al., Arch. Virol. 2011; 156(9): 1627-1634). These polyomaviruses were both originally isolated from immunosuppressed patients in 1971 (Gardner et al., Lancet. 1971; 1 (7712): 1253-7; Padgett et al., Lancet. 1971; 1 (7712): 1257-60 ) and share 75% sequence similarity in their genome. Both of these viruses can be easily identified and differentiated from each other by carrying out serological tests using specific commercial antibodies or a PCR-based genotyping approach. Genetic heterogeneity in the viral protein 1 (VP1) has allowed to further classify BKV isolates into four main subtypes or genotypes (I-IV). BKV subtype I is the most prevalent in the world (80%) followed by subtype IV (15%), subtypes II and III being rather rare. Subgroups within BKV subtypes have also been identified (Morel et al., J Clin Microbiol 2017; 55(4): 1177-1185), such as subgroup Ia, Ib2, Ic, etc. When referring to a plurality of BKV subtypes, it is meant herein at least two BKV subtypes, preferably at least three BKV subtypes.

"*VP1*" or *"viral protein* 1" refers herein to the major BKV or JCV capsid protein that is exposed on the virion surface when the virus enters a host cell, and that forms a pentamer having affinity for the ganglioside receptor on a host cell. It is the binding of VP1 pentamers (i.e. of five VP1 monomers) to ganglioside receptors on the cell surface which initiates internalization through a caveolae-mediated endocytic pathway, followed by trafficking of the virus to the ER and finally to the nucleus where it would replicate. In some assays that may be used in the present invention, VP1 may be provided in the form of VP1 pentamers, pseudovirions, or virus-like particles (VLPs). A protocol for preparing pseudovirions or VP1 pentamers is provided in Example's section 1.4 or 1.3, respectively. Examples of sequences of a VP1 monomer is provided in Table 1 below for information.

**Table 1. VP1 sequence and naturally-occurring variants thereof**

| **Name** | **Sequence** | **SEQ ID NO:** |
|---|---|---|
| **VP1 BKV subtype I** | MAPTKRKGECPGAAPKKPKEPVQVPKLLIKGGVEVLE VKTGVDAITEVECFLNPEMGDPDENLRGFSLKLSAEND FSSDSPERKMLPCYSTARIPLPNLNEDLTCGNLLMWEA VTVQTEVIGITSMLNLHAGSQKVHEHGGGKPIQGSNFH FFAVGGEPLEMQGVLMNYRSKYPDGTITPKNPTAQSQV MNTDHKAYLDKNNAYPVECWVPDPSRNENARYFGTFT GGENVPPVLHVTNTATTVLLDEQGVGPLCKADSLYVSA ADICGLFTNSSGTQQWRGLARYFKIRLRKRSVKNPYPIS FLLSDLINRRTQRVDGQPMYGMESQVEEVRVFDGTERL PGDPDMIRYIDKQGQLQTKML | SEQ ID NO: 271 |
| | Amino acids may be mutated at position 60 (D60N), 68 (L68Q), 72 (A72E), 73 (E73Q), 75 (D75N), and/or 82 (E82D) | |
| **VP1 BKV subtype II** | MAPTKRKGECPGAAPKKPKEPVQVPKLLIKGGVEVLE VKTGVDAITEVECFLNPEMGDPDDNLRGYSLKLTAENA FDSDSPDKKMLPCYSTARIPLPNLNEDLTCGNLLMWEA VTVKTEVIGITSMLNLHAGSQKVHENGGGKPVQGSNF HFFAVGGDPLEMQGVLMNYRTKYPQGTITPKNPTAQSQ VMNTDHKAYLDKNNAYPVECWIPDPSRNENTRYFGTY TGGENVPPVLHVTNTATTVLLDEQGVGPLCKADSLYVS AADICGLFTNSSGTQQWRGLARYFKIRLRKRSVKNPYP ISFLLSDLINRRTQKVDGQPMYGMESQVEEVRVFDGTE QLPGDPDMIRYIDRQGQLQTKMV | SEQ ID NO: 272 |
| **VP1 BKV subtype IV** | MAPTKRKGECPGAAPKKPKEPVQVPKLLIKGGVEVLE VKTGVDAITEVECFLNPEMGDPDNDLRGYSLRLTAETA FDSDSPDRKMLPCYSTARIPLPNLNEDLTCGNLLMWEA VTVKTEVIGITSMLNLHAGSQKVHENGGGKPIQGSNFH FFAVGGDPLEMQGVLMNYRTKYPEGTVTPKNPTAQSQ VMNTDHKAYLDKNNAYPVECWIPDPSRNENTRYFGTY TGGENVPPVLHVTNTATTVLLDEQGVGPLCKADSLYVS AADICGLFTNSSGTQQWRGLPRYFKIRLRKRSVKNPYPI SFLLSDLINRRTQRVDGQPMYGMESQVEEVRVFDGTEQ LPGDPDMIRYIDRQGQLQTKMV | SEQ ID NO: 273 |
| | Amino acids may be mutated at position 77(D77Q) | |
| **VP1 JCV** | MAPTKRKGERKDPVQVPKLLIRGGVEVLEVKTGVDSIT EVECFLTPEMGDPDEHLRGFSKSISISDTFESDSPNRDML PCYSVARIPLPNLNEDLTCGNILMWEAVTLKTEVIGVTS LMNVHSNGQATHDNGAGKPVQGTSFHFFSVGGEALEL QGVLFNYRTKYPDGTIFPKNATVQSQVMNTEHKAYLD KNKAYPVECWVPDPTRNENTRYFGTLTGGENVPPVLHI TNTATTVLLDEFGVGPLCKGDNLYLSAVDVCGMFTNRS GSQQWRGLSRYFKVQLRKRRVKNPYPISFLLTDLINRRT PRVDGQPMYGMDAQVEEVRVFEGTEELPGDPDMMRY VDKYGQLQTKML | SEQ ID NO: 274 |

The term "*amino acid*" or *"amino acid residue"* refers to a molecule containing both an amino group and a carboxyl group, with no peptide bond within the molecule. In other words, an amino acid is a molecule that has a carboxylic acid functionality and an amine nitrogen having at least one free hydrogen, preferably in alpha position thereto, but no amide bond in the molecule structure. This term encompasses not only naturally-occurring amino acids but also, synthetic, and unnatural amino acids, as well as amino acid analogs and amino acid mimetics that function in a manner similar to naturally occurring amino acids. Naturally occurring amino acids are those encoded by the genetic code (e.g. Alanine (Ala, A), Arginine (Arg, R), Asparagine (Asn, N), Aspartic acid (Asp, D), Cysteine (Cys, C), Glutamine (Gln, Q), Glutamic acid (Glu, E), Glycine (Gly, G), Histidine (His, H), Isoleucine (Ile, I), Leucine (Leu, L), Lysine (Lys, K), Methionine (Met, M), Phenylalanine (Phe, F), Proline (Pro, P), Serine (Ser, S), Threonine (Thr, T), Tryptophan (Trp, W), Tyrosine (Tyr, Y) and Valine (Val, V)) in D or L form, preferably in L form, as well as those amino acids that are later modified, e.g., hydroxyproline, g-carboxyglutamate, and O-phosphoserine. Amino acid analogs refer to compounds that have the same basic chemical structure as a naturally occurring amino acid, i.e., an a-carbon that is bound to a hydrogen, a carboxyl group, an amino group, and a side chain (R group), e.g., homoserine, norleucine, methionine sulfoxide, methionine methyl sulfonium. Such analogs have modified side chains (see e.g., norleucine) or modified peptide backbones, but retain the same basic chemical structure as a naturally occurring amino acid. Amino acid mimetics refers to chemical compounds that have a structure that is different from the general chemical structure of an amino acid, but that functions in a manner similar to a naturally occurring amino acid. In the context of the present invention, amino acids are herein preferably those that are naturally-occurring.

Amino acids are the building blocks of proteins, such as of antibodies or VP 1 as disclosed herein. The term *"polypeptide"* or *"protein"* refers herein to a precise succession of amino acid residues, also referred as amino acid sequence. As such, these terms include polypeptides of any size, preferably of at least 200, 300, 400, 500, 600, 700 amino acids or more, and/or polypeptides that have undergone post-translational modifications. In the context of the present invention, human monoclonal antibodies disclosed herein, or the VP1 protein they target, may be defined by sequence identity to a reference sequence, and/or contain amino acid mutation(s) which retain, or substantially retain, their biological activity. Herein, it will be understood that the biological activity of the human monoclonal antibodies according to the invention includes at least the binding and/or neutralizing of BK virus, while the biological activity of VP1 includes at least the binding of VP1 to ganglioside receptors on the host cell surface.

*"Sequence identity"* between amino acid sequences can be determined by comparing a position in each of the sequences which may be aligned for the purposes of comparison. When a position in the compared sequences is occupied by the same amino acid, then the sequences are identical at that position. A degree of identity between amino acid sequences is a function of the number of identical amino acid sequences that are shared between these sequences. To determine the *"percentage of sequence identity"* between two amino acid sequences, the sequences can be aligned for optimal comparison. For example, gaps can be introduced in the sequence of a first amino acid sequence for optimal alignment with the second amino acid sequence. The amino acid residues at corresponding amino acid positions can then compared. When a position in the first sequence is occupied by the same amino acid residue as the corresponding position in the second sequence, the molecules are identical at that position. The percentage (%) of identity between the two sequences is a function of the number of identical positions shared by the sequences. Hence, the percentage of identity can be calculated by multiplying the number of identical positions by 100 and dividing by the length of the aligned region (overlapping positions), including gaps (only internal gaps, not the gaps at the sequence ends). In this comparison, the sequences can be of the same length, or may be of different lengths. Identity scoring only counts perfect matches, and does not consider the degree of similarity of amino acids to one another. The percentage of identity can be calculated over the entire length of the sequence of reference, or over the entire relevant section thereof (if, for example, the percentage of sequence identity does not apply to one or more sections of the sequence of reference). Optimal alignment of sequences may be conducted by a global homology alignment algorithm, such as by the one described by Needleman and Wunsch (Journal of Molecular Biology 1970, 48(3): 443-53), by a local homology alignment algorithm such as the one described by Smith-Waterman et al. (Journal of Molecular Biology 1981; 147 (1): 195-197), by computerized implementations of either of these algorithms (for a global alignment, see e.g. the EMBOSS Needle program available online from EMBL-EBI; for a local alignment, see e.g. the BLAST program available online from NCBI), or by mere visual inspection. A global homology alignment may be preferred if the sequences to be aligned have the same or similar length. Alternatively, one may favor a local alignment if the sequences to be aligned are substantially different in their length.

The term *"modification"* or *"modified"* with regard to a polypeptide is intended to refer to an alteration or change in or to the polypeptide. Such modification may be desired, for example, to optimize the properties of the polypeptide or eliminate undesired properties of the polypeptide. Examples of such modification include those to remove glycosylation sites, GPI anchors, and/or solvent exposed lysines, to name a few. These modifications may be achieved by engineering a mutation of the relevant amino acid in the polypeptide, or by chemical or enzymatic treatment of the relevant amino acid residue such as acetylation, amidation, hydroxylation, methylation, phosphorylation, derivatization by known protecting/blocking groups, and/or attachment of a carbohydrate or lipid moieties, cofactors and the like.

The term *"mutated"* or *"mutation"* refers to an alteration or change of an amino acid in a reference polypeptide, such as by amino acid substitution, insertion and/or deletion. A *"substitution"* refers to the replacement of an amino acid at a particular sequence position in a reference polypeptide by another amino acid; an *"insertion"* to the addition of an amino acid at a particular sequence position in a reference polypeptide; and a *"deletion"* to the removal an amino acid at a particular sequence position in a reference polypeptide. Silent mutations, conservative mutations, or minor deletions are herein particularly preferred.

*"Conservative substitutions"* are a particularly preferred type of conservative mutations or modifications. This term refers to an amino acid substitution which does not alter the overall conformation and function of a polypeptide of reference, including, preferably the replacement of an amino acid with one having similar properties (such as, for example, polarity, hydrogen bonding potential, acidic, basic, shape, hydrophobic, aromatic, and the like). Amino acids with similar properties are well known in the art. For example, arginine, histidine and lysine are hydrophilic-basic amino acids and may be interchangeable. Similarly, isoleucine, a hydrophobic amino acid, may be replaced with leucine, methionine or valine. Neutral hydrophilic amino acids, which can be substituted for one another, include asparagine, glutamine, serine and threonine.

Examples of conservative substitutions are set out in Table 2 below.

**Table 2. Conservative substitutions I**

| **Side chain characteristic** | **Amino Acid** |
|---|---|
| Non-polar | G, A, P, I, L, V |
| Polar uncharged | C, S, T, M, N, Q |
| Polar-charged | D, E, K, R |
| Aromatic | H, F, W, Y |
| Other | N, Q, D, E |

Alternatively, conservative amino acids can be grouped as described in Lehninger, 1975, as set out in Table 3 below.

**Table 3. Conservative substitutions II**

| **Side chain characteristic** | **Amino Acid** |
|---|---|
| *Non-polar (hydrophobic)* | |
| Aliphatic | A, L, I, V, P |
| Aromatic | F, W |
| Sulfur-containing | M |
| Borderline | G |

| *Polar uncharged* | |
|---|---|
| Hydroxyl | S, T, Y |
| Amides | N, Q |
| Sulfhydryl | C |
| Borderline | G |

| *Polar-charged* | |
|---|---|
| Positively charged (basic) | K, R, H |
| Negatively charged (acidic) | D, E |

As a further alternative, exemplary conservative substitutions are set out in Table 4 below.

**Table 4. Conservative substitutions III**

| **Original amino acid residue** | **Amino acid substitution** |
|---|---|
| Ala (A) | Val (V), Leu (L), Ile (I) |
| Arg (R) | Lys (K), Gln (Q), Asn (N) |
| Asn (N) | Gln (Q), His (H), Lys(K), Arg (R) |
| Asp (D) | Glu (E) |
| Cys (C) | Ser (S) |
| Gln (Q) | Asn (N) |
| Glu (E) | Asp (D) |
| His (H) | Asn (N), Gln (Q), Lys (K), Arg (R) |
| Ile (I) | Leu (L), Val (V), Met (M), Ala (A), Phe (F) |
| Leu (L) | Ile (I), Val (V), Met (M), Ala (A), Phe (F) |
| Lys (K) | Arg (R), Gln (Q), Asn (N) |
| Met (M) | Leu (L), Phe (F), Ile (I) |
| Phe (F) | Leu (L), Val (V), Ile (I), Ala (A) |
| Pro (P) | Gly (G) |
| Ser (S) | Thr (S) |
| Thr (T) | Ser (S) |
| Trp (W) | Tyr (T) |
| Tyr (Y) | Trp (W), Phe (F), Thr (T), Ser (S) |
| Val (V) | Ile (I), Leu (L), Met (M), Phe (F), Ala (A) |

The term "*isolated*" means herein that a material has been removed or separated from its natural environment or otherwise subjected to human manipulation. The isolated material may be substantially or essentially free from components that normal accompany it in its natural states (e.g. having a degree of purity greater than 90%, 95% or 99%). This term may apply to any biological material disclosed herein, such as the antibody according to the invention. For example, the antibody according to the invention, can be substantially free of cellular material or other contaminating proteins from the cell or tissue source from which it was derived, or substantially free of chemical precursors or other chemicals when chemically synthesized.

Generally speaking, the term *"treatment"* or *"treating"* means obtaining a desired physiological or pharmacological effect depending on the degree of severity of the symptom or disorder of interest, or risks thereof, i.e. herein, depending on the degree of severity of the BKV or JCV infection or associated disorder thereof, or risks of developing such infection or associated disorder. The effect may thus be prophylactic in terms of a partial or complete prevention of the symptom or disorder and/or may be therapeutic in terms of a partial or complete cure of the symptom or disorder. The term *"prophylactic"* characterizes the capacity to avoid, or minimize the onset or development of a symptom or disorder before its onset (for example, after exposure to BKV or JCV, but before the onset of associated symptom). The term *"therapeutic"* refers to the capacity to inhibit the symptom or disorder (i.e. arresting the development thereof), and/or to relieve said symptom or disorder (i.e. regression leading to an improvement).

"*BKV or JCV infection*" refers herein either to the passive or active entry of the BKV or JCV virus into a host cell and subsequent increase in viral load (i.e. viral replication) within the host. This term encompasses a primary BKV or JCV infection, as well as a secondary BKV or JCV infection (i.e. after reactivation of the virus). Such infection may be non-pathogenic, or subclinical in its manifestation, at least initially.

"*BKV- of JCV-associated disorder"* refers herein to a disorder in a host that has been infected by BKV or JCV Typical examples of BKV- of JCV-associated disorders include, without limitation, graft dysfunction or rejection (such as kidney graft), BKV-related leukodystrophy, nephropathy, BK virus-associated nephritis (BKVAN), hemorrhagic cystitis, Progressive Multifocal Leukoencephalopathy (PML), granule cell neuronopathy (GCN), interstitial kidney disease, ureteral stenosis, urothelial carcinoma, vasculitis, colitis, retinitis, meningitis, pneumonitis, liver disease, and immune reconstitution inflammatory syndrome (IRIS).

The term "*about*" means that the value of reference can vary within a certain range depending on the margin of error allowed, which may be easily determined by one skilled in the art. Preferably, this margin of error is of 10%, more preferably of 5%, even more preferably of 1%.

### Antibodies

The present invention relates to a set of monoclonal antibodies, preferably human monoclonal antibodies, and antigen-binding fragments or chains thereof, the antibodies being able to bind and optionally neutralize at least one BK virus subtype. They bind more particularly to the VP1 protein, and efficiently neutralize viral infection from a BK virus. Optionally, the BK virus is selected in the group consisting of BK virus subtype I, BK virus subtype II and BK virus subtype IV The antibody or the antigen-binding fragments thereof can bind and optionally neutralize one, two or three BK virus subtypes, especially selected from the group consisting of BK virus subtype I, BK virus subtype II and BK virus subtype IV For instance, it binds and optionally neutralizes:
- BK virus subtype I; or
- BK virus subtype II; or
- BK virus subtype IV; or
- BK virus subtypes I and II; or
- BK virus subtypes I and IV; or
- BK virus subtypes II and IV; or
- BK virus subtypes I, II and IV

Optionally, the antibody or the antigen-binding fragments thereof further binds and optionally neutralize JC virus. Optionally, the antibody or the antigen-binding fragments thereof does not bind and neutralize JC virus.

The antibodies disclosed herein are defined by the complementary-determining regions (CDRs), which are key determinants in antigen binding, the CDRs being defined by using the IMGT numbering scheme. The antibodies can also be defined by their heavy chain variable domain (VH) and light chain variable domain (VL), also determined according to the IMGT numbering scheme. In a very specific aspect, the sequences of CDRs and variable domains of the specific antibodies are disclosed in Tables 5 and 6 below.

More generally, some antibodies can be classified in subgroups and consensus sequences of the CDRs sequences can be defined.

It will be appreciated that mutants of the above-described CDRs may also be used herein, which retain or substantially retain the capacity for binding to VP1, and accordingly retain or substantially retain the capacity for neutralizing BKV and optionally JCV For example, 1, 2 or 3 amino acids may be mutated in one or more of those CDRs, such as by substitution, deletion and/or insertion, preferably by substitution, more preferably by conservative substitution.

Because CDRs are responsible for most antibody-antigen interactions, the CDRs described herein may be grafted onto any suitable antibody framework regions, without impacting, or without substantially impacting, the biological activity of the antibody or antigen-binding fragment thereof according to the invention.

In the context of the present invention, it will be understood that human antibody frameworks are herein preferred. Human framework sequences can be easily obtained from e.g. publicly available databases such as http://www2.mrc-imb.cam.ac.uk/vbase/list2.php, or https://vdjbase.org/. If need be, those framework regions may also be mutated. For example, 1, 2, 3, 4, 5, 6, 7, 8, 9, or 10 amino acids may be mutated in one or more framework regions such as by substitution, deletion and/or insertion, preferably by substitution, more preferably by conservative substitution. Protocols for selecting amino acid residues in framework regions which can mutated are well-known in the art.

### Family A

According to an aspect of the invention, the human monoclonal antibody, or antigen-binding fragment thereof, that binds and optionally neutralizes at least one BK virus subtype, comprises a heavy chain variable domain (VH) and a light chain variable domain (VL) with six complementary-determining regions (CDRs) selected from the following combinations:
- a VH-CDR1 comprising, or consisting of, amino acid sequence GFTFRSYXₐ₁ (SEQ ID NO: 1), wherein Xₐ₁ is T or S;
- a VH-CDR2 comprising, or consisting of, amino acid sequence ISGSSNXₐ₂K (SEQ ID NO: 2), wherein Xₐ₂ is H or Q;
- a VH-CDR3 comprising, or consisting of, amino acid sequence AREDDII,TPLYGMDV (SEQ ID NO: 3);
- a VL-CDR1 comprising, or consisting of, amino acid sequence QSVRSNY (SEQ ID NO: 4);
- a VL-CDR2 comprising, or consisting of, amino acid sequence GAS; and
- a VL-CDR3 comprising, or consisting of, amino acid sequence HQYXₐ₃SSPRT (SEQ ID NO: 5), wherein Xₐ₃ is G or A.

Particularly, in this aspect, the antibody, or antigen-binding fragment thereof, may comprise six CDRs selected from the following combinations:
- a VH-CDR1 comprising, or consisting of, amino acid sequence SEQ ID NO: 6 or 9;
- a VH-CDR2 comprising, or consisting of, amino acid sequence SEQ ID NO: 7 or 10;
- a VH-CDR3 comprising, or consisting of, amino acid sequence SEQ ID NO: 3;
- a VL-CDR1 comprising, or consisting of, amino acid sequence SEQ ID NO: 4;
- a VL-CDR2 comprising, or consisting of, amino acid sequence GAS; and
- a VL-CDR3 comprising, or consisting of, amino acid sequence SEQ ID NO: 8 or 11.

Preferably, in this aspect, the antibody, or antigen-binding fragment thereof, comprises the following combination:
- VH-CDR1, VH-CDR2 and VH-CDR3 comprising, or consisting of, amino acid sequence SEQ ID NO: 6, 7 and 3, respectively; and VL-CDR1 and VL-CDR3 comprising, or consisting of, amino acid sequence SEQ ID NO: 4 and 8, respectively, and VL-CDR2 comprising, or consisting of, amino acid sequence GAS; or
- VH-CDR1, VH-CDR2 and VH-CDR3 comprising, or consisting of, amino acid sequence SEQ ID NO: 9, 10 and 3, respectively; and VL-CDR1 and VL-CDR3 comprising, or consisting of, amino acid sequence SEQ ID NO: 4 and 11, respectively, and VL-CDR2 comprising, or consisting of, amino acid sequence GAS.

In a preferred aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 177, 179, 181 or 183; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 178, 180, 182 or 184.
A total of 1 to 10 amino acids may be optionally mutated in any one of SEQ ID NO: 177, 179, 181, 183, 178, 180, 182 or 184, such as by substitution, deletion and/or insertion, preferably by substitution, more preferably by conservative substitution. Said mutations, if any, preferably occur in regions outside the CDRs (underlined in Table 6). Accordingly, the amino acid sequence has at least 80%, 85%, 90%, 91%, 92%, 93%, 94%, 95%, 96%, 97%, 98%, or 99% identity to the recited sequence with the proviso that the CDRs are not mutated.

Accordingly, in a particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 177, 179, 181 or 183; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 178, 180, 182 or 184,
- with the proviso that the CDRs are selected from the following combinations:
   VH-CDR1, VH-CDR2 and VH-CDR3 comprising, or consisting of, amino acid sequence SEQ ID NO: 1, 2 and 3, respectively; and VL-CDR1 and VL-CDR3 comprising, or consisting of, amino acid sequence SEQ ID NO: 4 and 5, respectively, and VL-CDR2 comprising, or consisting of, amino acid sequence GAS.

In another particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 177, 179, 181 or 183; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 178, 180, 182 or 184,
- with the proviso that the CDRs are selected from the following combinations:
   - a VH-CDR1 comprising, or consisting of, amino acid sequence SEQ ID NO: 6 or 9;
   - a VH-CDR2 comprising, or consisting of, amino acid sequence SEQ ID NO: 7 or 10;
   - a VH-CDR3 comprising, or consisting of, amino acid sequence SEQ ID NO: 3;
   - a VL-CDR1 comprising, or consisting of, amino acid sequence SEQ ID NO: 4;
   - a VL-CDR2 comprising, or consisting of, amino acid sequence GAS; and
   - a VL-CDR3 comprising, or consisting of, amino acid sequence SEQ ID NO: 8 or 11.

Preferred antibodies or antigen-binding fragments thereof according to this aspect of the invention are selected from the following group:
∘ a human monoclonal antibody, or antigen-binding fragment thereof, comprising:
   - a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 177; and
   - a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 178,
   - with the proviso that the CDRs are as follow:
   VH-CDR1, VH-CDR2 and VH-CDR3 comprising, or consisting of, amino acid sequence SEQ ID NO: 6, 7 and 3, respectively; and VL-CDR1 and VL-CDR3 comprising, or consisting of, amino acid sequence SEQ ID NO: 4 and 8, respectively, and VL-CDR2 comprising, or consisting of, amino acid sequence GAS;
   or
∘ a human monoclonal antibody, or antigen-binding fragment thereof, comprising:
   - a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 179; and
   - a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 180,
   - with the proviso that the CDRs are as follow:
      VH-CDR1, VH-CDR2 and VH-CDR3 comprising, or consisting of, amino acid sequence SEQ ID NO: 6, 7 and 3, respectively; and VL-CDR1 and VL-CDR3 comprising, or consisting of, amino acid sequence SEQ ID NO: 4 and 8, respectively, and VL-CDR2 comprising, or consisting of, amino acid sequence GAS;
   or
∘ a human monoclonal antibody, or antigen-binding fragment thereof, comprising:
   - a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 181; and
   - a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 182,
   - with the proviso that the CDRs are as follow:
      VH-CDR1, VH-CDR2 and VH-CDR3 comprising, or consisting of, amino acid sequence SEQ ID NO: 9, 10 and 3, respectively; and VL-CDR1 and VL-CDR3 comprising, or consisting of, amino acid sequence SEQ ID NO: 4 and 11, respectively, and VL-CDR2 comprising, or consisting of, amino acid sequence GAS;
   or
∘ a human monoclonal antibody, or antigen-binding fragment thereof, comprising:
   - a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 183; and
   - a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 184,
   - with the proviso that the CDRs are as follow:
      VH-CDR1, VH-CDR2 and VH-CDR3 comprising, or consisting of, amino acid sequence SEQ ID NO: 9, 10 and 3, respectively; and VL-CDR1 and VL-CDR3 comprising, or consisting of, amino acid sequence SEQ ID NO: 4 and 11, respectively, and VL-CDR2 comprising, or consisting of, amino acid sequence GAS.

Even more preferred antibodies or antigen-binding fragments thereof according to this aspect of the invention are selected from the following group:
∘ a human monoclonal antibody, or antigen-binding fragment thereof, comprising:
   - a heavy chain variable domain (VH) comprising, or consisting of, amino acid sequence SEQ ID NO: 177; and
   - a light chain variable domain (VL) comprising, or consisting of, amino acid sequence SEQ ID NO: 178, (antibody B9P6);
   or
∘ a human monoclonal antibody, or antigen-binding fragment thereof, comprising:
   - a heavy chain variable domain (VH) comprising, or consisting of, amino acid sequence SEQ ID NO: 179; and
   - a light chain variable domain (VL) comprising, or consisting of, amino acid sequence SEQ ID NO: 180 (antibody E9P7);
   or
∘ a human monoclonal antibody, or antigen-binding fragment thereof, comprising:
   - a heavy chain variable domain (VH) comprising, or consisting of, amino acid sequence SEQ ID NO: 181; and
   - a light chain variable domain (VL) comprising, or consisting of, amino acid sequence SEQ ID NO: 182 (antibody G11P 1);
   or
∘ a human monoclonal antibody, or antigen-binding fragment thereof, comprising:
   - a heavy chain variable domain (VH) comprising, or consisting of, amino acid sequence SEQ ID NO: 183; and
   - a light chain variable domain (VL) comprising, or consisting of, amino acid sequence SEQ ID NO: 184 (antibody E11P6).

The human monoclonal antibodies, or antigen-binding fragments thereof, according to this aspect of the invention, exhibit a particularly good neutralization potency on several BKV subtypes, in particular on subtypes I, II and IV They also show a good neutralization potency on a number of naturally-occurring variants of BKV subtypes, in particular on subtypes I, II and IV

### Family B

According to another aspect of the invention, the human monoclonal antibody, or antigen-binding fragment thereof, that binds and optionally neutralizes at least one BK virus subtype, comprises a heavy chain variable domain (VH) and a light chain variable domain (VL) with six complementary-determining regions (CDRs) selected from the following combinations:
- a VH-CDR1 comprising, or consisting of, amino acid sequence GGSIRNSY (SEQ ID NO: 23);
- a VH-CDR2 comprising, or consisting of, amino acid sequence IYYTGX_{c1}T (SEQ ID NO: 24), wherein X_{c1} is T or S;
- a VH-CDR3 comprising, or consisting of, amino acid sequence AQGGGSGDLHY (SEQ ID NO: 25);
- a VL-CDR1 comprising, or consisting of, amino acid sequence RSDVGGYNY (SEQ ID NO: 26);
- a VL-CDR2 comprising, or consisting of, amino acid sequence DIS; and
- a VL-CDR3 comprising, or consisting of, amino acid sequence ISFATGYTWV (SEQ ID NO: 27).

Optionally, the antibody, or antigen-binding fragment thereof, comprises the following combination:
- a VH-CDR1 comprising, or consisting of, amino acid sequence SEQ ID NO: 23;
- a VH-CDR2 comprising, or consisting of, amino acid sequence SEQ ID NO: 28 or 29;
- a VH-CDR3 comprising, or consisting of, amino acid sequence SEQ ID NO: 25;
- a VL-CDR1 comprising, or consisting of, amino acid sequence SEQ ID NO: 5 or 26, preferably 26;
- a VL-CDR2 comprising, or consisting of, amino acid sequence EGT; and
- a VL-CDR3 comprising, or consisting of, amino acid sequence SEQ ID NO: 11 or 27, preferably 27.

Preferably, in this aspect, the antibody, or antigen-binding fragment thereof, comprises the following combination:
- VH-CDR1, VH-CDR2 and VH-CDR3 comprising, or consisting of, amino acid sequence SEQ ID NO: 23, 28 and 25, respectively; and VL-CDR1 and VL-CDR3 comprising, or consisting of, amino acid sequence SEQ ID NO: 26 and 27, respectively, and VL-CDR2 comprising, or consisting of, amino acid sequence DIS; or
- VH-CDR1, VH-CDR2 and VH-CDR3 comprising, or consisting of, amino acid sequence SEQ ID NO: 23, 29 and 25, respectively; and VL-CDR1 and VL-CDR3 comprising, or consisting of, amino acid sequence SEQ ID NO: 26 and 27, respectively, and VL-CDR2 comprising, or consisting of, amino acid sequence DIS.

In a preferred aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 189, 191, 193 or 195; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 190, 192, 194 or 196.
A total of 1 to 10 amino acids may be optionally mutated in any one of SEQ ID NO: 189, 191, 193, 195, 190, 192, 194 or 196, such as by substitution, deletion and/or insertion, preferably by substitution, more preferably by conservative substitution. Said mutations, if any, preferably occur in regions outside the CDRs (underlined in Table 6). Accordingly, the amino acid sequence has at least 80%, 85%, 90%, 91%, 92%, 93%, 94%, 95%, 96%, 97%, 98%, or 99% identity to the recited sequence with the proviso that the CDRs are not mutated.

Accordingly, in a particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 189, 191, 193 or 195; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 190, 192, 194 or 196,
- with the proviso that the CDRs are selected from the following combinations:
   VH-CDR1, VH-CDR2 and VH-CDR3 comprising, or consisting of, amino acid sequence SEQ ID NO: 23, 24 and 25, respectively; and VL-CDR1 and VL-CDR3 comprising, or consisting of, amino acid sequence SEQ ID NO: 26 and 27, respectively, and VL-CDR2 comprising, or consisting of, amino acid sequence DIS.

Optionally, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 189, 191, 193 or 195; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 190, 192, 194 or 196,
- with the proviso that the CDRs are selected from the following combinations:
   - a VH-CDR1 comprising, or consisting of, amino acid sequence SEQ ID NO: 23;
   - a VH-CDR2 comprising, or consisting of, amino acid sequence SEQ ID NO: 28 or 29;
   - a VH-CDR3 comprising, or consisting of, amino acid sequence SEQ ID NO: 25;
   - a VL-CDR1 comprising, or consisting of, amino acid sequence SEQ ID NO: 5 or 26, preferably 26;
   - a VL-CDR2 comprising, or consisting of, amino acid sequence EGT; and
   - a VL-CDR3 comprising, or consisting of, amino acid sequence SEQ ID NO: 11 or 27, preferably 27.

Preferred antibodies or antigen-binding fragments thereof according to this aspect of the invention are selected from the following group:
∘ a human monoclonal antibody, or antigen-binding fragment thereof, comprising:
   - a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 189; and
   - a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 190,
   - with the proviso that the CDRs are as follow:
      VH-CDR1, VH-CDR2 and VH-CDR3 comprising, or consisting of, amino acid sequence SEQ ID NO: 23, 28 and 25, respectively; and VL-CDR1 and VL-CDR3 comprising, or consisting of, amino acid sequence SEQ ID NO: 26 and 27, respectively, and VL-CDR2 comprising, or consisting of, amino acid sequence DIS;
   or
∘ a human monoclonal antibody, or antigen-binding fragment thereof, comprising:
   - a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 191; and
   - a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 192,
   - with the proviso that the CDRs are as follow:
      VH-CDR1, VH-CDR2 and VH-CDR3 comprising, or consisting of, amino acid sequence SEQ ID NO: 23, 28 and 25, respectively; and VL-CDR1 and VL-CDR3 comprising, or consisting of, amino acid sequence SEQ ID NO: 26 and 27, respectively, and VL-CDR2 comprising, or consisting of, amino acid sequence DIS;
   or
∘ a human monoclonal antibody, or antigen-binding fragment thereof, comprising:
   - a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 193; and
   - a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 194,
   - with the proviso that the CDRs are as follow:
      VH-CDR1, VH-CDR2 and VH-CDR3 comprising, or consisting of, amino acid sequence SEQ ID NO: 23, 29 and 25, respectively; and VL-CDR1 and VL-CDR3 comprising, or consisting of, amino acid sequence SEQ ID NO: 26 and 27, respectively, and VL-CDR2 comprising, or consisting of, amino acid sequence DIS.

Even more preferred antibodies or antigen-binding fragments thereof according to this aspect of the invention are selected from the following group:
∘ a human monoclonal antibody, or antigen-binding fragment thereof, comprising:
   - a heavy chain variable domain (VH) comprising, or consisting of, amino acid sequence SEQ ID NO: 189; and
   - a light chain variable domain (VL) comprising, or consisting of, amino acid sequence SEQ ID NO: 190 (antibody C11P1);
   or
∘ a human monoclonal antibody, or antigen-binding fragment thereof, comprising:
   - a heavy chain variable domain (VH) comprising, or consisting of, amino acid sequence SEQ ID NO: 191; and
   - a light chain variable domain (VL) comprising, or consisting of, amino acid sequence SEQ ID NO: 192 (antibody G5P1);
   or
∘ a human monoclonal antibody, or antigen-binding fragment thereof, comprising:
   - a heavy chain variable domain (VH) comprising, or consisting of, amino acid sequence SEQ ID NO: 193; and
   - a light chain variable domain (VL) comprising, or consisting of, amino acid sequence SEQ ID NO: 194 (antibody E7P5).

The human monoclonal antibodies, or antigen-binding fragments thereof, according to this aspect of the invention, exhibit a particularly good neutralization potency on several BKV subtypes, in particular on subtypes I, II and IV

They also show a good neutralization potency on a number of naturally-occurring variants of BKV subtypes, in particular on subtypes I, II and IV

### Family C

According to another aspect of the invention, the human monoclonal antibody, or antigen-binding fragment thereof, that binds and optionally neutralizes at least one BK virus subtype, comprises a heavy chain variable domain (VH) and a light chain variable domain (VL) with six complementary-determining regions (CDRs) selected from the following combinations:
- a VH-CDR1 comprising, or consisting of, amino acid sequence GGSISPYY (SEQ ID NO: 30);
- a VH-CDR2 comprising, or consisting of, amino acid sequence IYYSGNT (SEQ ID NO: 31);
- a VH-CDR3 comprising, or consisting of, amino acid sequence ARDSCSGTCYLGX_{d1}NWFDP (SEQ ID NO: 32), wherein X_{d1} is D or G;
- a VL-CDR1 comprising, or consisting of, amino acid sequence SSNIGANND (SEQ ID NO: 33);
- a VL-CDR2 comprising, or consisting of, amino acid sequence GNN; and
- a VL-CDR3 comprising, or consisting of, amino acid sequence QSYDSSLSTSKV (SEQ ID NO: 34).

Preferably, in this aspect, the antibody, or antigen-binding fragment thereof, comprises the following combination:
- VH-CDR1, VH-CDR2 and VH-CDR3 comprising, or consisting of, amino acid sequence SEQ ID NO: 30, 31 and 35, respectively; and VL-CDR1 and VL-CDR3 comprising, or consisting of, amino acid sequence SEQ ID NO: 33 and 35, respectively, and VL-CDR2 comprising, or consisting of, amino acid sequence GNN; or
- VH-CDR1, VH-CDR2 and VH-CDR3 comprising, or consisting of, amino acid sequence SEQ ID NO: 30, 31 and 36, respectively; and VL-CDR1 and VL-CDR3 comprising, or consisting of, amino acid sequence SEQ ID NO: 33 and 34, respectively, and VL-CDR2 comprising, or consisting of, amino acid sequence GNN.

In a preferred aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 197, 199 or 201; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 198, 200 or 202.

A total of 1 to 10 amino acids may be optionally mutated in any one of SEQ ID NO: 197, 199, 201, 198, 200 or 202, such as by substitution, deletion and/or insertion, preferably by substitution, more preferably by conservative substitution. Said mutations, if any, preferably occur in regions outside the CDRs (underlined in Table 6). Accordingly, the amino acid sequence has at least 80%, 85%, 90%, 91%, 92%, 93%, 94%, 95%, 96%, 97%, 98%, or 99% identity to the recited sequence with the proviso that the CDRs are not mutated.

Accordingly, in a particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 197, 199 or 201; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 198, 200 or 202,
- with the proviso that the CDRs are selected from the following combinations:
   VH-CDR1, VH-CDR2 and VH-CDR3 comprising, or consisting of, amino acid sequence SEQ ID NO: 30, 31 and 32, respectively; and VL-CDR1 and VL-CDR3 comprising, or consisting of, amino acid sequence SEQ ID NO: 33 and 34, respectively, and VL-CDR2 comprising, or consisting of, amino acid sequence GNN.

Optionally, antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 197, 199 or 201; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 198, 200 or 202,
- with the proviso that the CDRs are selected from the following combinations:
   VH-CDR1, VH-CDR2 and VH-CDR3 comprising, or consisting of, amino acid sequence SEQ ID NO: 30, 31 and 35 or 36, respectively; and VL-CDR1 and VL-CDR3 comprising, or consisting of, amino acid sequence SEQ ID NO: 33 and 34, respectively, and VL-CDR2 comprising, or consisting of, amino acid sequence GNN.

Preferred antibodies or antigen-binding fragments thereof according to this aspect of the invention are selected from the following group:
∘ a human monoclonal antibody, or antigen-binding fragment thereof, comprising:
   - a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 197; and
   - a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 198,
   - with the proviso that the CDRs are as follow:
      VH-CDR1, VH-CDR2 and VH-CDR3 comprising, or consisting of, amino acid sequence SEQ ID NO: 30, 31 and 35, respectively; and VL-CDR1 and VL-CDR3 comprising, or consisting of, amino acid sequence SEQ ID NO: 33 and 34, respectively, and VL-CDR2 comprising, or consisting of, amino acid sequence GNN;
   or
∘ a human monoclonal antibody, or antigen-binding fragment thereof, comprising:
   - a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 199; and
   - a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 200,
   - with the proviso that the CDRs are as follow:
      VH-CDR1, VH-CDR2 and VH-CDR3 comprising, or consisting of, amino acid sequence SEQ ID NO: 30, 31 and 35, respectively; and VL-CDR1 and VL-CDR3 comprising, or consisting of, amino acid sequence SEQ ID NO: 33 and 34, respectively, and VL-CDR2 comprising, or consisting of, amino acid sequence GNN;
   or
∘ a human monoclonal antibody, or antigen-binding fragment thereof, comprising:
   - a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 201; and
   - a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 202,
   - with the proviso that the CDRs are as follow:
      VH-CDR1, VH-CDR2 and VH-CDR3 comprising, or consisting of, amino acid sequence SEQ ID NO: 30, 31 and 36, respectively; and VL-CDR1 and VL-CDR3 comprising, or consisting of, amino acid sequence SEQ ID NO: 33 and 34, respectively, and VL-CDR2 comprising, or consisting of, amino acid sequence GNN.

Even more preferred antibodies or antigen-binding fragments thereof according to this aspect of the invention are selected from the following group:
∘ a human monoclonal antibody, or antigen-binding fragment thereof, comprising:
   - a heavy chain variable domain (VH) comprising, or consisting of, amino acid sequence SEQ ID NO: 197; and
   - a light chain variable domain (VL) comprising, or consisting of, amino acid sequence SEQ ID NO: 198 (antibody B8P7);
   or
∘ a human monoclonal antibody, or antigen-binding fragment thereof, comprising:
   - a heavy chain variable domain (VH) comprising, or consisting of, amino acid sequence SEQ ID NO: 199; and
   - a light chain variable domain (VL) comprising, or consisting of, amino acid sequence SEQ ID NO: 200 (antibody G6P7);
   or
∘ a human monoclonal antibody, or antigen-binding fragment thereof, comprising:
   - a heavy chain variable domain (VH) comprising, or consisting of, amino acid sequence SEQ ID NO: 201; and
   - a light chain variable domain (VL) comprising, or consisting of, amino acid sequence SEQ ID NO: 202 (antibody C4P6).

The human monoclonal antibodies, or antigen-binding fragments thereof, according to this aspect of the invention, exhibit a particularly good neutralization potency on several BKV subtypes, in particular on subtypes I, II and IV

They also show good a neutralization potency on a number of naturally-occurring variants of BKV subtypes, in particular on subtypes I, II and IV

### Family D

According to another aspect of the invention, the human monoclonal antibody, or antigen-binding fragment thereof, that binds and optionally neutralizes at least one BK virus subtype, comprises a heavy chain variable domain (VH) and a light chain variable domain (VL) with the six following complementary-determining regions (CDRs):
- a VH-CDR1 comprising, or consisting of, amino acid sequence GGSMTSGSYY (SEQ ID NO: 140);
- a VH-CDR2 comprising, or consisting of, amino acid sequence IYTSGRT (SEQ ID NO: 141);
- a VH-CDR3 comprising, or consisting of, amino acid sequence ARWGDSSAYYYVGDP (SEQ ID NO: 142);
- a VL-CDR1 comprising, or consisting of, amino acid sequence QNINTY (SEQ ID NO: 143);
- a VL-CDR2 comprising, or consisting of, amino acid sequence AAS; and
- a VL-CDR3 comprising, or consisting of, amino acid sequence QQSFSLPHT (SEQ ID NO: 144).

In a preferred aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 249, 251 or 253; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 250, 252 or 254.

A total of 1 to 10 amino acids may be optionally mutated in any one of SEQ ID NO: 249, 251, 253, 250, 252 or 254, such as by substitution, deletion and/or insertion, preferably by substitution, more preferably by conservative substitution. Said mutations, if any, preferably occur in regions outside the CDRs (underlined in Table 6). Accordingly, the amino acid sequence has at least 80%, 85%, 90%, 91%, 92%, 93%, 94%, 95%, 96%, 97%, 98%, or 99% identity to the recited sequence with the proviso that the CDRs are not mutated.

Accordingly, in a particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 249, 251 or 253; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 250, 252 or 254,
- with the proviso that the CDRs are selected from the following combinations:
   VH-CDR1, VH-CDR2 and VH-CDR3 comprising, or consisting of, amino acid sequence SEQ ID NO: 140, 141 and 142, respectively; and VL-CDR1 and VL-CDR3 comprising, or consisting of, amino acid sequence SEQ ID NO: 143 and 144, respectively, and VL-CDR2 comprising, or consisting of, amino acid sequence AAS.

Preferred antibodies or antigen-binding fragments thereof according to this aspect of the invention are selected from the following group:
∘ a human monoclonal antibody, or antigen-binding fragment thereof, comprising:
   - a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 249; and
   - a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 250,
   - with the proviso that the CDRs are as follow:
      VH-CDR1, VH-CDR2 and VH-CDR3 comprising, or consisting of, amino acid sequence SEQ ID NO: 140, 141 and 142, respectively; and VL-CDR1 and VL-CDR3 comprising, or consisting of, amino acid sequence SEQ ID NO: 143 and 144, respectively, and VL-CDR2 comprising, or consisting of, amino acid sequence AAS;
   or
∘ a human monoclonal antibody, or antigen-binding fragment thereof, comprising:
   - a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 251; and
   - a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 252,
   - with the proviso that the CDRs are as follow:
      VH-CDR1, VH-CDR2 and VH-CDR3 comprising, or consisting of, amino acid sequence SEQ ID NO: 140, 141 and 142, respectively; and VL-CDR1 and VL-CDR3 comprising, or consisting of, amino acid sequence SEQ ID NO: 143 and 144, respectively, and VL-CDR2 comprising, or consisting of, amino acid sequence AAS;
   or
∘ a human monoclonal antibody, or antigen-binding fragment thereof, comprising:
   - a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 253; and
   - a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 254,
   - with the proviso that the CDRs are as follow:
      VH-CDR1, VH-CDR2 and VH-CDR3 comprising, or consisting of, amino acid sequence SEQ ID NO: 140, 141 and 142, respectively; and VL-CDR1 and VL-CDR3 comprising, or consisting of, amino acid sequence SEQ ID NO: 143 and 144, respectively, and VL-CDR2 comprising, or consisting of, amino acid sequence AAS.

Even more preferred antibodies or antigen-binding fragments thereof according to this aspect of the invention are selected from the following group:
∘ a human monoclonal antibody, or antigen-binding fragment thereof, comprising:
   - a heavy chain variable domain (VH) comprising, or consisting of, amino acid sequence SEQ ID NO: 249; and
   - a light chain variable domain (VL) comprising, or consisting of, amino acid sequence SEQ ID NO: 250 (antibody G8P6);
   or
∘ a human monoclonal antibody, or antigen-binding fragment thereof, comprising:
   - a heavy chain variable domain (VH) comprising, or consisting of, amino acid sequence SEQ ID NO: 251; and
   - a light chain variable domain (VL) comprising, or consisting of, amino acid sequence SEQ ID NO: 252 (antibody C6P7);
   or
∘ a human monoclonal antibody, or antigen-binding fragment thereof, comprising:
   - a heavy chain variable domain (VH) comprising, or consisting of, amino acid sequence SEQ ID NO: 253; and
   - a light chain variable domain (VL) comprising, or consisting of, amino acid sequence SEQ ID NO: 254 (antibody G7P7).

The human monoclonal antibodies, or antigen-binding fragments thereof, according to this aspect of the invention, show a particularly good neutralization potency on several BKV subtypes, in particular on subtypes II and IV

### Family E

According to another aspect of the invention, the human monoclonal antibody, or antigen-binding fragment thereof, that binds and optionally neutralizes at least one BK virus subtype, comprises a heavy chain variable domain (VH) and a light chain variable domain (VL) with the six following complementary-determining regions (CDRs):
- a VH-CDR1 comprising, or consisting of, amino acid sequence GYTFTTHI (SEQ ID NO: 62);
- a VH-CDR2 comprising, or consisting of, amino acid sequence INAGNGNT (SEQ ID NO: 63);
- a VH-CDR3 comprising, or consisting of, amino acid sequence AREDQWLGRSPLEY (SEQ ID NO: 64);
- a VL-CDR1 comprising, or consisting of, amino acid sequence QSVSSSY (SEQ ID NO: 65);
- a VL-CDR2 comprising, or consisting of, amino acid sequence GAS; and
- a VL-CDR3 comprising, or consisting of, amino acid sequence QQYGNSYPWT (SEQ ID NO: 66).

In a preferred aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 213 or 215; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 214 or 216.

A total of 1 to 10 amino acids may be optionally mutated in any one of SEQ ID NO: 213, 215, 214 or 216, such as by substitution, deletion and/or insertion, preferably by substitution, more preferably by conservative substitution. Said mutations, if any, preferably occur in regions outside the CDRs (underlined in Table 6). Accordingly, the amino acid sequence has at least 80%, 85%, 90%, 91%, 92%, 93%, 94%, 95%, 96%, 97%, 98%, or 99% identity to the recited sequence with the proviso that the CDRs are not mutated.

Accordingly, in a particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 213 or 215; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 214 or 216,
with the proviso that the CDRs are selected from the following combinations:
VH-CDR1, VH-CDR2 and VH-CDR3 comprising, or consisting of, amino acid sequence SEQ ID NO: 62, 63 and 64, respectively; and VL-CDR1 and VL-CDR3 comprising, or consisting of, amino acid sequence SEQ ID NO: 65 and 66, respectively, and VL-CDR2 comprising, or consisting of, amino acid sequence GAS.

Preferred antibodies or antigen-binding fragments thereof according to this aspect of the invention are selected from the following group:
∘ a human monoclonal antibody, or antigen-binding fragment thereof, comprising:
   - a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 213; and
   - a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 214,
   - with the proviso that the CDRs are as follow:
      VH-CDR1, VH-CDR2 and VH-CDR3 comprising, or consisting of, amino acid sequence SEQ ID NO: 62, 63 and 64, respectively; and VL-CDR1 and VL-CDR3 comprising, or consisting of, amino acid sequence SEQ ID NO: 65 and 66, respectively, and VL-CDR2 comprising, or consisting of, amino acid sequence GAS;
   or
∘ a human monoclonal antibody, or antigen-binding fragment thereof, comprising:
   - a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 215; and
   - a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 216,
   - with the proviso that the CDRs are as follow:
      VH-CDR1, VH-CDR2 and VH-CDR3 comprising, or consisting of, amino acid sequence SEQ ID NO: 62, 63 and 64, respectively; and VL-CDR1 and VL-CDR3 comprising, or consisting of, amino acid sequence SEQ ID NO: 65 and 66, respectively, and VL-CDR2 comprising, or consisting of, amino acid sequence GAS.

Even more preferred antibodies or antigen-binding fragments thereof according to this aspect of the invention are selected from the following group:
∘ a human monoclonal antibody, or antigen-binding fragment thereof, comprising:
   - a heavy chain variable domain (VH) comprising, or consisting of, amino acid sequence SEQ ID NO: 213; and
   - a light chain variable domain (VL) comprising, or consisting of, amino acid sequence SEQ ID NO: 214 (antibody F3P6);
   or
∘ a human monoclonal antibody, or antigen-binding fragment thereof, comprising:
   - a heavy chain variable domain (VH) comprising, or consisting of, amino acid sequence SEQ ID NO: 215; and
   - a light chain variable domain (VL) comprising, or consisting of, amino acid sequence SEQ ID NO: 216 (antibody G5P6).

The human monoclonal antibodies, or antigen-binding fragments thereof, according to this aspect of the invention, exhibit a particularly good neutralization potency on several BKV subtypes, in particular on subtypes I, II and IV

They also show a good neutralization potency on a number of naturally-occurring variants of BKV subtypes.

### Family F

According to another aspect of the invention, the human monoclonal antibody, or antigen-binding fragment thereof, that binds and optionally neutralizes at least one BK virus subtype, comprises a heavy chain variable domain (VH) and a light chain variable domain (VL) with the six following complementary-determining regions (CDRs):
- a VH-CDR1 comprising, or consisting of, amino acid sequence GFTFTNYW (SEQ ID NO: 57);
- a VH-CDR2 comprising, or consisting of, amino acid sequence IYPDDSDA (SEQ ID NO: 58);
- a VH-CDR3 comprising, or consisting of, amino acid sequence ARGGFCSGSVCYNPAWFDL (SEQ ID NO: 59);
- a VL-CDR1 comprising, or consisting of, amino acid sequence QTINTY (SEQ ID NO: 60);
- a VL-CDR2 comprising, or consisting of, amino acid sequence DAS; and
- a VL-CDR3 comprising, or consisting of, amino acid sequence QQSYSTPLT (SEQ ID NO: 61).

In a preferred aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 211; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 212.

A total of 1 to 10 amino acids may be optionally mutated in any one of SEQ ID NO: 211 or 212, such as by substitution, deletion and/or insertion, preferably by substitution, more preferably by conservative substitution. Said mutations, if any, preferably occur in regions outside the CDRs (underlined in Table 6). Accordingly, the amino acid sequence has at least 80%, 85%, 90%, 91%, 92%, 93%, 94%, 95%, 96%, 97%, 98%, or 99% identity to the recited sequence with the proviso that CDRs are not mutated.

Accordingly, in a particular aspect, the antibody, or antigen-binding fragment thereof comprises:
- a heavy chain ariable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 211; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 212,
- with the proviso that the CDRs are selected from the following combinations:
   VH-CDR1, VH-CDR2 and VH-CDR3 comprising, or consisting of, amino acid sequence SEQ ID NO: 57, 58 and 59, respectively; and VL-CDR1 and VL-CDR3 comprising, or consisting of, amino acid sequence SEQ ID NO: 60 and 61, respectively, and VL-CDR2 comprising, or consisting of, amino acid sequence DAS.

Preferably, the antibody, or antigen-binding fragment thereof comprises:
- a heavy chain (HC) variable domain (VH) comprising, or consisting of, amino acid sequence SEQ ID NO: 211; and
- a light chain (LC) variable domain (VL) comprising, or consisting of, amino acid sequence SEQ ID NO: 212.

This preferred antibody has been named antibody B7P6.

The human monoclonal antibodies, or antigen-binding fragments thereof, according to this aspect of the invention, exhibit a particularly good neutralization potency on several BKV subtypes, in particular on subtypes I, II and IV

### Family G

According to another aspect of the invention, the human monoclonal antibody, or antigen-binding fragment thereof, that binds and optionally neutralizes at least one BK virus subtype, comprises a heavy chain variable domain (VH) and a light chain variable domain (VL) with the six following complementary-determining regions (CDRs):
- a VH-CDR1 comprising, or consisting of, amino acid sequence GFTFDDHA (SEQ ID NO: 47);
- a VH-CDR2 comprising, or consisting of, amino acid sequence INWNSASI (SEQ ID NO: 48);
- a VH-CDR3 comprising, or consisting of, amino acid sequence AKDFGLWFGESRGIDA (SEQ ID NO: 49);
- a VL-CDR1 comprising, or consisting of, amino acid sequence QSLLHSNGYNY (SEQ ID NO: 50);
- a VL-CDR2 comprising, or consisting of, amino acid sequence LGS; and
- a VL-CDR3 comprising, or consisting of, amino acid sequence MQALQTPIT (SEQ ID NO: 51).

In a preferred aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 207; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 208.

A total of 1 to 10 amino acids may be optionally mutated in any one of SEQ ID NO: 207 or 208, such as by substitution, deletion and/or insertion, preferably by substitution, more preferably by conservative substitution. Said mutations, if any, preferably occur in regions outside the CDRs (underlined in Table 6). Accordingly, the amino acid sequence has at least 80%, 85%, 90%, 91%, 92%, 93%, 94%, 95%, 96%, 97%, 98%, or 99% identity to the recited sequence with the proviso that the CDRs are not mutated.

Accordingly, in a particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 207; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 208,
- with the proviso that the CDRs are selected from the following combinations:
   VH-CDR1, VH-CDR2 and VH-CDR3 comprising, or consisting of, amino acid sequence SEQ ID NO: 47, 48 and 49, respectively; and VL-CDR1 and VL-CDR3 comprising, or consisting of, amino acid sequence SEQ ID NO: 50 and 51, respectively, and VL-CDR2 comprising, or consisting of, amino acid sequence LGS.

Preferably, the antibody, or antigen-binding fragment thereof comprises:
- a heavy chain variable domain (VH) comprising, or consisting of, amino acid sequence SEQ ID NO: 207; and
- a light chain variable domain (VL) comprising, or consisting of, amino acid sequence SEQ ID NO: 208.

This preferred antibody has been named antibody E6P5.

### Family H

According to another aspect of the invention, the human monoclonal antibody, or antigen-binding fragment thereof, that binds and optionally neutralizes at least one BK virus subtype, comprises a heavy chain variable domain (VH) and a light chain variable domain (VL) with six complementary-determining regions (CDRs) selected from the following combinations:
- a VH-CDR1 comprising, or consisting of, amino acid sequence GFXₒ₁FGXₒ₂YS (SEQ ID NO: 97), wherein Xₒ₁ is S or R, Xₒ₂ is S or T;
- a VH-CDR2 comprising, or consisting of, amino acid sequence ISSGSSSI (SEQ ID NO: 98);
- a VH-CDR3 comprising, or consisting of, amino acid sequence ARGWTGYDAFDI (SEQ ID NO: 99);
- a VL-CDR1 comprising, or consisting of, amino acid sequence QVISNY (SEQ ID NO: 100);
- a VL-CDR2 comprising, or consisting of, amino acid sequence AAS; and
- a VL-CDR3 comprising, or consisting of, amino acid sequence QKYNNAPLT (SEQ ID NO: 101).

Preferably, in this aspect, the antibody, or antigen-binding fragment thereof, comprises the following combination:
- VH-CDR1, VH-CDR2 and VH-CDR3 comprising, or consisting of, amino acid sequence SEQ ID NO: 102, 98 and 99, respectively; and VL-CDR1 and VL-CDR3 comprising, or consisting of, amino acid sequence SEQ ID NO: 100 and 101, respectively, and VL-CDR2 comprising, or consisting of, amino acid sequence AAS; or
- VH-CDR1, VH-CDR2 and VH-CDR3 comprising, or consisting of, amino acid sequence SEQ ID NO: 103, 98 and 99, respectively; and VL-CDR1 and VL-CDR3 comprising, or consisting of, amino acid sequence SEQ ID NO: 100 and 101, respectively, and VL-CDR2 comprising, or consisting of, amino acid sequence AAS.

In a preferred aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 233 or 235; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 234 or 236.

A total of 1 to 10 amino acids may be optionally mutated in any one of SEQ ID NO: 233, 235, 234 or 236, such as by substitution, deletion and/or insertion, preferably by substitution, more preferably by conservative substitution. Said mutations, if any, preferably occur in regions outside the CDRs (underlined in Table 6). Accordingly, the amino acid sequence has at least 80%, 85%, 90%, 91%, 92%, 93%, 94%, 95%, 96%, 97%, 98%, or 99% identity to the recited sequence with the proviso that the CDRs are not mutated.

Accordingly, in a particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 233 or 235; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 234 or 236,
- with the proviso that the CDRs are selected from the following combinations:
   VH-CDR1, VH-CDR2 and VH-CDR3 comprising, or consisting of, amino acid sequence SEQ ID NO: 97, 98 and 99, respectively; and VL-CDR1 and VL-CDR3 comprising, or consisting of, amino acid sequence SEQ ID NO: 100 and 101, respectively, and VL-CDR2 comprising, or consisting of, amino acid sequence AAS.

Preferred antibodies or antigen-binding fragments thereof according to this aspect of the invention are selected from the following group:
∘ a human monoclonal antibody, or antigen-binding fragment thereof, comprising:
   - a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 233; and
   - a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 234,
   - with the proviso that the CDRs are as follow:
      VH-CDR1, VH-CDR2 and VH-CDR3 comprising, or consisting of, amino acid sequence SEQ ID NO: 102, 98 and 99, respectively; and VL-CDR1 and VL-CDR3 comprising, or consisting of, amino acid sequence SEQ ID NO: 100 and 101, respectively, and VL-CDR2 comprising, or consisting of, amino acid sequence AAS;
   or
∘ a human monoclonal antibody, or antigen-binding fragment thereof, comprising:
   - a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 235; and
   - a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 236,
   - with the proviso that the CDRs are as follow:
      VH-CDR1, VH-CDR2 and VH-CDR3 comprising, or consisting of, amino acid sequence SEQ ID NO: 103, 98 and 99, respectively; and VL-CDR1 and VL-CDR3 comprising, or consisting of, amino acid sequence SEQ ID NO: 100 and 101, respectively, and VL-CDR2 comprising, or consisting of, amino acid sequence AAS.

Even more preferred antibodies or antigen-binding fragments thereof according to this aspect of the invention are selected from the following group:
∘ a human monoclonal antibody, or antigen-binding fragment thereof, comprising:
   - a heavy chain variable domain (VH) comprising, or consisting of, amino acid sequence SEQ ID NO: 233; and
   - a light chain variable domain (VL) comprising, or consisting of, amino acid sequence SEQ ID NO: 234 (antibody G7P3);
   or
∘ a human monoclonal antibody, or antigen-binding fragment thereof, comprising:
   - a heavy chain variable domain (VH) comprising, or consisting of, amino acid sequence SEQ ID NO: 235; and
   - a light chain variable domain (VL) comprising, or consisting of, amino acid sequence SEQ ID NO: 236 (antibody E8P5).

### Family I

According to another aspect of the invention, the human monoclonal antibody, or antigen-binding fragment thereof, that binds and optionally neutralizes at least one BK virus subtype, comprises a heavy chain variable domain (VH) and a light chain variable domain (VL) with the six following complementary-determining regions (CDRs):
- a VH-CDR1 comprising, or consisting of, amino acid sequence GFTFSNFG (SEQ ID NO: 82);
- a VH-CDR2 comprising, or consisting of, amino acid sequence ISYDGSDK (SEQ ID NO: 83);
- a VH-CDR3 comprising, or consisting of, amino acid sequence AKDRVRTKYYYDHSGPFDY (SEQ ID NO: 84);
- a VL-CDR1 comprising, or consisting of, amino acid sequence NIGSKS (SEQ ID NO: 85);
- a VL-CDR2 comprising, or consisting of, amino acid sequence DDD; and
- a VL-CDR3 comprising, or consisting of, amino acid sequence QVWMTNSDRPV (SEQ ID NO: 86).

In a preferred aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 225 or 227; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 226 or 228.

A total of 1 to 10 amino acids may be optionally mutated in any one of SEQ ID NO: 225, 227, 226 or 228, such as by substitution, deletion and/or insertion, preferably by substitution, more preferably by conservative substitution. Said mutations, if any, preferably occur in regions outside the CDRs (underlined in Table 6). Accordingly, the amino acid sequence has at least 80%, 85%, 90%, 91%, 92%, 93%, 94%, 95%, 96%, 97%, 98%, or 99% identity to the recited sequence with the proviso that the CDRs are not mutated.

Accordingly, in a particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 225 or 227; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 226 or 228,
- with the proviso that the CDRs are selected from the following combinations:
   VH-CDR1, VH-CDR2 and VH-CDR3 comprising, or consisting of, amino acid sequence SEQ ID NO: 82, 83 and 84, respectively; and VL-CDR1 and VL-CDR3 comprising, or consisting of, amino acid sequence SEQ ID NO: 85 and 86, respectively, and VL-CDR2 comprising, or consisting of, amino acid sequence DDD.

Preferred antibodies or antigen-binding fragments thereof according to this aspect of the invention are selected from the following group:
∘ a human monoclonal antibody, or antigen-binding fragment thereof, comprising:
   - a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 225; and
   - a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 226,
   - with the proviso that the CDRs are as follow:
      VH-CDR1, VH-CDR2 and VH-CDR3 comprising, or consisting of, amino acid sequence SEQ ID NO: 82, 83 and 84, respectively; and VL-CDR1 and VL-CDR3 comprising, or consisting of, amino acid sequence SEQ ID NO: 85 and 86, respectively, and VL-CDR2 comprising, or consisting of, amino acid sequence DDD;
   or
∘ a human monoclonal antibody, or antigen-binding fragment thereof, comprising:
   - a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 227; and
   - a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 228,
   - with the proviso that the CDRs are as follow:
      VH-CDR1, VH-CDR2 and VH-CDR3 comprising, or consisting of, amino acid sequence SEQ ID NO: 82, 83 and 84, respectively; and VL-CDR1 and VL-CDR3 comprising, or consisting of, amino acid sequence SEQ ID NO: 85 and 86, respectively, and VL-CDR2 comprising, or consisting of, amino acid sequence DDD.

Even more preferred antibodies or antigen-binding fragments thereof according to this aspect of the invention are selected from the following group:
∘ a human monoclonal antibody, or antigen-binding fragment thereof, comprising:
   - a heavy chain variable domain (VH) comprising, or consisting of, amino acid sequence SEQ ID NO: 225; and
   - a light chain variable domain (VL) comprising, or consisting of, amino acid sequence SEQ ID NO: 226 (antibody B7P3);
   or
∘ a human monoclonal antibody, or antigen-binding fragment thereof, comprising:
   - a heavy chain variable domain (VH) comprising, or consisting of, amino acid sequence SEQ ID NO: 227; and
   - a light chain variable domain (VL) comprising, or consisting of, amino acid sequence SEQ ID NO: 228 (antibody F4P5).

### Family J

According to another aspect of the invention, the human monoclonal antibody, or antigen-binding fragment thereof, that binds and optionally neutralizes at least one BK virus subtype, comprises a heavy chain variable domain (VH) and a light chain variable domain (VL) with the six following complementary-determining regions (CDRs):
- a VH-CDR1 comprising, or consisting of, amino acid sequence GGSISSGNYY (SEQ ID NO: 13 5);
- a VH-CDR2 comprising, or consisting of, amino acid sequence IYTSGNT (SEQ ID NO: 136);
- a VH-CDR3 comprising, or consisting of, amino acid sequence ARVRRDGYNAFDY (SEQ ID NO: 137);
- a VL-CDR1 comprising, or consisting of, amino acid sequence NLGDKF (SEQ ID NO: 138);
- a VL-CDR2 comprising, or consisting of, amino acid sequence QDS; and
- a VL-CDR3 comprising, or consisting of, amino acid sequence QAWDSDTLYV (SEQ ID NO: 139).

In a preferred aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 247; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 248.

A total of 1 to 10 amino acids may be optionally mutated in any one of SEQ ID NO: 247 or 248, such as by substitution, deletion and/or insertion, preferably by substitution, more preferably by conservative substitution. Said mutations, if any, preferably occur in regions outside the CDRs (underlined in Table 6). Accordingly, the amino acid sequence has at least 80%, 85%, 90%, 91%, 92%, 93%, 94%, 95%, 96%, 97%, 98%, or 99% identity to the recited sequence with the proviso that the CDRs are not mutated.

Accordingly, in a particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 247; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 248,
- with the proviso that the CDRs are selected from the following combinations:
   VH-CDR1, VH-CDR2 and VH-CDR3 comprising, or consisting of, amino acid sequence SEQ ID NO: 135, 136 and 137, respectively; and VL-CDR1 and VL-CDR3 comprising, or consisting of, amino acid sequence SEQ ID NO: 138 and 139, respectively, and VL-CDR2 comprising, or consisting of, amino acid sequence QDS.

Preferably, the antibody, or antigen-binding fragment thereof comprises:
- a heavy chain variable domain (VH) comprising, or consisting of, amino acid sequence SEQ ID NO: 247; and
- a light chain variable domain (VL) comprising, or consisting of, amino acid sequence SEQ ID NO: 248.

This preferred antibody has been named antibody E10P5.

### Family K

According to another aspect of the invention, the human monoclonal antibody, or antigen-binding fragment thereof, that binds and optionally neutralizes at least one BK virus subtype, comprises a heavy chain variable domain (VH) and a light chain variable domain (VL) with six complementary-determining regions (CDRs) selected from the following combinations:
- a VH-CDR1 comprising, or consisting of, amino acid sequence GFXₖ₁FSSYA (SEQ ID NO: 67), wherein Xₖ₁ is T or P;
- a VH-CDR2 comprising, or consisting of, amino acid sequence Xₖ₂TGSXₖ₃Xₖ₄Xₖ₅T (SEQ ID NO: 68), wherein Xₖ₂ is L or I, Xₖ₃ is G or A, Xₖ₄ is G or S, Xₖ₅ is S or G;
- a VH-CDR3 comprising, or consisting of, amino acid sequence AKEGAYSYDXₖ₆WYFDL (SEQ ID NO: 69), wherein Xₖ₆ is F or Y;
- a VL-CDR1 comprising, or consisting of, amino acid sequence QXₖ₇VSGXₖ₈Y (SEQ ID NO: 70), wherein Xₖ₇ is T or S and Xₖ₈ is N or S;
- a VL-CDR2 comprising, or consisting of, amino acid sequence GAS; and
- a VL-CDR3 comprising, or consisting of, amino acid sequence QQYGXₖ₉Xₖ₁₀Xₖ₁₁T (SEQ ID NO: 71), wherein Xₖ₉ is R or S, Xₖ₁₀ is P or L, Xₖ₁₁ is L or V

Particularly, in this aspect, the antibody, or antigen-binding fragment thereof, may comprise six CDRs selected from the following combinations:
- a VH-CDR1 comprising, or consisting of, amino acid sequence SEQ ID NO: 72 or 77;
- a VH-CDR2 comprising, or consisting of, amino acid sequence SEQ ID NO: 73 or 78;
- a VH-CDR3 comprising, or consisting of, amino acid sequence SEQ ID NO: 74 or 79;
- a VL-CDR1 comprising, or consisting of, amino acid sequence SEQ ID NO: 75 or 80;
- a VL-CDR2 comprising, or consisting of, amino acid sequence GAS; and
- a VL-CDR3 comprising, or consisting of, amino acid sequence SEQ ID NO: 76 or 81.

Preferably, in this aspect, the antibody, or antigen-binding fragment thereof, comprises the following combination:
- VH-CDR1, VH-CDR2 and VH-CDR3 comprising, or consisting of, amino acid sequence SEQ ID NO: 72, 73 and 74, respectively; and VL-CDR1 and VL-CDR3 comprising, or consisting of, amino acid sequence SEQ ID NO: 75 and 76, respectively, and VL-CDR2 comprising, or consisting of, amino acid sequence GAS; or
- VH-CDR1, VH-CDR2 and VH-CDR3 comprising, or consisting of, amino acid sequence SEQ ID NO: 77, 78 and 79, respectively; and VL-CDR1 and VL-CDR3 comprising, or consisting of, amino acid sequence SEQ ID NO: 80 and 81, respectively, and VL-CDR2 comprising, or consisting of, amino acid sequence GAS.

In a preferred aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 217, 219, 221 or 223; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 218, 220, 222 or 224.
A total of 1 to 10 amino acids may be optionally mutated in any one of SEQ ID NO: 217, 219, 221, 223, 218, 220, 222 or 224, such as by substitution, deletion and/or insertion, preferably by substitution, more preferably by conservative substitution. Said mutations, if any, preferably occur in regions outside the CDRs (underlined in Table 6). Accordingly, the amino acid sequence has at least 80%, 85%, 90%, 91%, 92%, 93%, 94%, 95%, 96%, 97%, 98%, or 99% identity to the recited sequence with the proviso that the CDRs are not mutated.

Accordingly, in a particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 217, 219, 221 or 223; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 218, 220, 222 or 224,
- with the proviso that the CDRs are selected from the following combinations:
   VH-CDR1, VH-CDR2 and VH-CDR3 comprising, or consisting of, amino acid sequence SEQ ID NO: 67, 68 and 69, respectively; and VL-CDR1 and VL-CDR3 comprising, or consisting of, amino acid sequence SEQ ID NO: 70 and 71, respectively, and VL-CDR2 comprising, or consisting of, amino acid sequence GAS.

Optionally, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 217, 219, 221 or 223; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 218, 220, 222 or 224,
with the proviso that the CDRs are selected from the following combinations:
- a VH-CDR1 comprising, or consisting of, amino acid sequence SEQ ID NO: 72 or 77;
- a VH-CDR2 comprising, or consisting of, amino acid sequence SEQ ID NO: 73 or 78;
- a VH-CDR3 comprising, or consisting of, amino acid sequence SEQ ID NO: 74 or 79;
- a VL-CDR1 comprising, or consisting of, amino acid sequence SEQ ID NO: 75 or 80;
- a VL-CDR2 comprising, or consisting of, amino acid sequence GAS; and
- a VL-CDR3 comprising, or consisting of, amino acid sequence SEQ ID NO: 76 or 81.

Preferred antibodies or antigen-binding fragments thereof according to this aspect of the invention are selected from the following group:
∘ a human monoclonal antibody, or antigen-binding fragment thereof, comprising:
   - a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 217; and
   - a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 218,
   - with the proviso that the CDRs are as follow:
      VH-CDR1, VH-CDR2 and VH-CDR3 comprising, or consisting of, amino acid sequence SEQ ID NO: 72, 73 and 74, respectively; and VL-CDR1 and VL-CDR3 comprising, or consisting of, amino acid sequence SEQ ID NO: 75 and 76, respectively, and VL-CDR2 comprising, or consisting of, amino acid sequence GAS;
   or
∘ a human monoclonal antibody, or antigen-binding fragment thereof, comprising:
   - a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 219; and
   - a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 220,
   - with the proviso that the CDRs are as follow:
      VH-CDR1, VH-CDR2 and VH-CDR3 comprising, or consisting of, amino acid sequence SEQ ID NO: 72, 73 and 74, respectively; and VL-CDR1 and VL-CDR3 comprising, or consisting of, amino acid sequence SEQ ID NO: 75 and 76, respectively, and VL-CDR2 comprising, or consisting of, amino acid sequence GAS;
   or
∘ a human monoclonal antibody, or antigen-binding fragment thereof, comprising:
   - a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 221; and
   - a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 222,
   - with the proviso that the CDRs are as follow:
      VH-CDR1, VH-CDR2 and VH-CDR3 comprising, or consisting of, amino acid sequence SEQ ID NO: 72, 73 and 74, respectively; and VL-CDR1 and VL-CDR3 comprising, or consisting of, amino acid sequence SEQ ID NO: 75 and 76, respectively, and VL-CDR2 comprising, or consisting of, amino acid sequence GAS;
   or
∘ a human monoclonal antibody, or antigen-binding fragment thereof, comprising:
   - a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 223; and
   - a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 224,
   - with the proviso that the CDRs are as follow:
      VH-CDR1, VH-CDR2 and VH-CDR3 comprising, or consisting of, amino acid sequence SEQ ID NO: 77, 78 and 79, respectively; and VL-CDR1 and VL-CDR3 comprising, or consisting of, amino acid sequence SEQ ID NO: 80 and 81, respectively, and VL-CDR2 comprising, or consisting of, amino acid sequence GAS.

Even more preferred antibodies or antigen-binding fragments thereof according to this aspect of the invention are selected from the following group:
∘ a human monoclonal antibody, or antigen-binding fragment thereof, comprising:
   - a heavy chain variable domain (VH) comprising, or consisting of, amino acid sequence SEQ ID NO: 217; and
   - a light chain variable domain (VL) comprising, or consisting of, amino acid sequence SEQ ID NO: 218 (antibody B10P1);
   or
∘ a human monoclonal antibody, or antigen-binding fragment thereof, comprising:
   - a heavy chain variable domain (VH) comprising, or consisting of, amino acid sequence SEQ ID NO: 219; and
   - a light chain variable domain (VL) comprising, or consisting of, an amino acid sequence SEQ ID NO: 220 (antibody G9P2);
   or
∘ a human monoclonal antibody, or antigen-binding fragment thereof, comprising:
   - a heavy chain variable domain (VH) comprising, or consisting of, amino acid sequence SEQ ID NO: 221; and
   - a light chain variable domain (VL) comprising, or consisting of, amino acid sequence SEQ ID NO: 222 (antibody C4P7);
   or
∘ a human monoclonal antibody, or antigen-binding fragment thereof, comprising:
   - a heavy chain variable domain (VH) comprising, or consisting of, amino acid sequence SEQ ID NO: 223; and
   - a light chain variable domain (VL) comprising, or consisting of, amino acid sequence SEQ ID NO: 224 (antibody D7P6).

### Family L

According to another aspect of the invention, the human monoclonal antibody, or antigen-binding fragment thereof, that binds and optionally neutralizes at least one BK virus subtype, comprises a heavy chain variable domain (VH) and a light chain variable domain (VL) with the six following complementary-determining regions (CDRs):
- a VH-CDR1 comprising, or consisting of, amino acid sequence GGSISSGGYY (SEQ ID NO: 52);
- a VH-CDR2 comprising, or consisting of, amino acid sequence MYYSGST (SEQ ID NO: 53);
- a VH-CDR3 comprising, or consisting of, amino acid sequence ARDRRDYGDYYYFGMDV (SEQ ID NO: 54);
- a VL-CDR1 comprising, or consisting of, amino acid sequence SSNIGAGYD (SEQ ID NO: 55);
- a VL-CDR2 comprising, or consisting of, amino acid sequence ANS; and
- a VL-CDR3 comprising, or consisting of, amino acid sequence QSYDSSLSVYVV (SEQ ID NO: 56).

In a preferred aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 209; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 210.

A total of 1 to 10 amino acids may be optionally mutated in any one of SEQ ID NO: 209 or 210, such as by substitution, deletion and/or insertion, preferably by substitution, more preferably by conservative substitution. Said mutations, if any, preferably occur in regions outside the CDRs (underlined in Table 6). Accordingly, the amino acid sequence has at least 80%, 85%, 90%, 91%, 92%, 93%, 94%, 95%, 96%, 97%, 98%, or 99% identity to the recited sequence with the proviso that the CDRs are not mutated.

Accordingly, in a particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 209; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 210,
- with the proviso that the CDRs are selected from the following combinations:
   VH-CDR1, VH-CDR2 and VH-CDR3 comprising, or consisting of, amino acid sequence SEQ ID NO: 52, 53 and 54, respectively; and VL-CDR1 and VL-CDR3 comprising, or consisting of, amino acid sequence SEQ ID NO: 55 and 56, respectively, and VL-CDR2 comprising, or consisting of, amino acid sequence ANS.

Preferably, the antibody, or antigen-binding fragment thereof comprises:
- a heavy chain variable domain (VH) comprising, or consisting of, amino acid sequence SEQ ID NO: 209; and
- a light chain variable domain (VL) comprising, or consisting of, amino acid sequence SEQ ID NO: 210.

This preferred antibody has been named antibody E4P2.

### Family M

According to another aspect of the invention, the human monoclonal antibody, or antigen-binding fragment thereof, that binds and optionally neutralizes at least one BK virus subtype, comprises a heavy chain variable domain (VH) and a light chain variable domain (VL) with the six following complementary-determining regions (CDRs):
- a VH-CDR1 comprising, or consisting of, amino acid sequence GYTFTSYS (SEQ ID NO: 150);
- a VH-CDR2 comprising, or consisting of, amino acid sequence INAGNGDT (SEQ ID NO: 151);
- a VH-CDR3 comprising, or consisting of, amino acid sequence ARSNSPTVTYNYYYYAMDV (SEQ ID NO: 152);
- a VL-CDR1 comprising, or consisting of, amino acid sequence QSVRSSY (SEQ ID NO: 153);
- a VL-CDR2 comprising, or consisting of, amino acid sequence DAS; and
- a VL-CDR3 comprising, or consisting of, amino acid sequence QQYGNSPLT (SEQ ID NO: 154).

In a preferred aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 257; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 258.

A total of 1 to 10 amino acids may be optionally mutated in any one of SEQ ID NO: 257 or 258, such as by substitution, deletion and/or insertion, preferably by substitution, more preferably by conservative substitution. Said mutations, if any, preferably occur in regions outside the CDRs (underlined in Table 6). Accordingly, the amino acid sequence has at least 80%, 85%, 90%, 91%, 92%, 93%, 94%, 95%, 96%, 97%, 98%, or 99% identity to the recited sequence with the proviso that the CDRs re not mutated.

Accordingly, in a particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 257; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 258,
- with the proviso that the CDRs are selected from the following combinations:
   VH-CDR1, VH-CDR2 and VH-CDR3 comprising, or consisting of, amino acid sequence SEQ ID NO: 150, 151 and 152, respectively; and VL-CDR1 and VL-CDR3 comprising, or consisting of, amino acid sequence SEQ ID NO: 153 and 154, respectively, and VL-CDR2 comprising, or consisting of, amino acid sequence DAS.

Preferably, the antibody, or antigen-binding fragment thereof comprises:
- a heavy chain variable domain (VH) comprising, or consisting of, amino acid sequence SEQ ID NO: 257; and
- a light chain variable domain (VL) comprising, or consisting of, amino acid sequence SEQ ID NO: 258.

This preferred antibody has been named antibody F3P7.

### Family N

According to another aspect of the invention, the human monoclonal antibody, or antigen-binding fragment thereof, that binds and optionally neutralizes at least one BK virus subtype, comprises a heavy chain variable domain (VH) and a light chain variable domain (VL) with the six following complementary-determining regions (CDRs):
- a VH-CDR1 comprising, or consisting of, amino acid sequence GFTFRSYT (SEQ ID NO: 6);
- a VH-CDR2 comprising, or consisting of, amino acid sequence ISGSSNHK (SEQ ID NO: 7);
- a VH-CDR3 comprising, or consisting of, amino acid sequence AREDDILTPLYGMDV (SEQ ID NO: 3);
- a VL-CDR1 comprising, or consisting of, amino acid sequence SSDVGAYNY (SEQ ID NO: 111);
- a VL-CDR2 comprising, or consisting of, amino acid sequence DVS; and
- a VL-CDR3 comprising, or consisting of, amino acid sequence CSYAHRGTPL (SEQ ID NO: 112).

In a preferred aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 261; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 262.

A total of 1 to 10 amino acids may be optionally mutated in any one of SEQ ID NO: 261 or 262, such as by substitution, deletion and/or insertion, preferably by substitution, more preferably by conservative substitution. Said mutations, if any, preferably occur in regions outside the CDRs (underlined in Table 6). Accordingly, the amino acid sequence has at least 80%, 85%, 90%, 91%, 92%, 93%, 94%, 95%, 96%, 97%, 98%, or 99% identity to the recited sequence with the proviso that the CDRs are not mutated.

Accordingly, in a particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 261; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 262,
- with the proviso that the CDRs are selected from the following combinations:
   VH-CDR1, VH-CDR2 and VH-CDR3 comprising, or consisting of, amino acid sequence SEQ ID NO: 6, 7 and 3, respectively; and VL-CDR1 and VL-CDR3 comprising, or consisting of, amino acid sequence SEQ ID NO: 111 and 112, respectively, and VL-CDR2 comprising, or consisting of, amino acid sequence DVS.

Preferably, the antibody, or antigen-binding fragment thereof comprises:
- a heavy chain variable domain (VH) comprising, or consisting of, amino acid sequence SEQ ID NO: 261; and
- a light chain variable domain (VL) comprising, or consisting of, amino acid sequence SEQ ID NO: 262.

This preferred antibody has been named antibody D11P7.

### Family O

According to another aspect of the invention, the human monoclonal antibody, or antigen-binding fragment thereof, that binds and optionally neutralizes at least one BK virus subtype, comprises a heavy chain variable domain (VH) and a light chain variable domain (VL) with six complementary-determining regions (CDRs) selected from the following combinations:
- a VH-CDR1 comprising, or consisting of, amino acid sequence AYTFTNSA (SEQ ID NO: 12);
- a VH-CDR2 comprising, or consisting of, amino acid sequence INTNTGNP (SEQ ID NO: 13);
- a VH-CDR3 comprising, or consisting of, amino acid sequence X_{b1}VPYNFGGDHNSX_{b2}X_{b3}FFGX_{b4}DX_{b5} (SEQ ID NO: 14), wherein X_{b1} is T or A, X_{b2} is Y or F, X_{b3} is Y or H, X_{b4} is V or M and X_{b5} is I or V;
- a VL-CDR1 comprising, or consisting of, amino acid sequence SSDVGX_{b6}YNX_{b7} (SEQ ID NO: 15), wherein X_{b6} is S or T and X_{b7} is L or F;
- a VL-CDR2 comprising, or consisting of, amino acid sequence EGT; and
- a VL-CDR3 comprising, or consisting of, amino acid sequence X_{b8}SYAGX_{b9}NX_{b10}FV (SEQ ID NO: 16), wherein X_{b8} is C or S, X_{b9} is S or G and X_{b10} is T or A.

Particularly, in this aspect, the antibody, or antigen-binding fragment thereof, may comprise six CDRs selected from the following combinations:
- a VH-CDR1 comprising, or consisting of, amino acid sequence SEQ ID NO: 12;
- a VH-CDR2 comprising, or consisting of, amino acid sequence SEQ ID NO: 13;
- a VH-CDR3 comprising, or consisting of, amino acid sequence SEQ ID NO: 17 or 20;
- a VL-CDR1 comprising, or consisting of, amino acid sequence SEQ ID NO: 18 or 21;
- a VL-CDR2 comprising, or consisting of, amino acid sequence EGT; and
- a VL-CDR3 comprising, or consisting of, amino acid sequence SEQ ID NO: 19 or 22.

Preferably, in this aspect, the antibody, or antigen-binding fragment thereof, comprises the following combination:
- VH-CDR1, VH-CDR2 and VH-CDR3 comprising, or consisting of, amino acid sequence SEQ ID NO: 12, 13 and 17, respectively; and VL-CDR1 and VL-CDR3 comprising, or consisting of, amino acid sequence SEQ ID NO: 18 and 19, respectively and VL-CDR2 comprising, or consisting of, amino acid sequence EGT; or
- VH-CDR1, VH-CDR2 and VH-CDR3 comprising, or consisting of, amino acid sequence SEQ ID NO: 12, 13 and 20, respectively; and VL-CDR1 and VL-CDR3 comprising, or consisting of, amino acid sequence SEQ ID NO: 21 and 22, respectively and VL-CDR2 comprising, or consisting of, amino acid sequence EGT.

In a preferred aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 185 or 187; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 186 or 188.

A total of 1 to 10 amino acids may be optionally mutated in any one of SEQ ID NO: 185, 187, 186 or 188, such as by substitution, deletion and/or insertion, preferably by substitution, more preferably by conservative substitution. Said mutations, if any, preferably occur in regions outside the CDRs (underlined in Table 6). Accordingly, the amino acid sequence has at least 80%, 85%, 90%, 91%, 92%, 93%, 94%, 95%, 96%, 97%, 98%, or 99% identity to the recited sequence with the proviso that the CDRs are not mutated.

Accordingly, in a particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 185 or 187; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 186 or 188,
- with the proviso that the CDRs are selected from the following combinations:
   VH-CDR1, VH-CDR2 and VH-CDR3 comprising, or consisting of, amino acid sequence SEQ ID NO: 12, 13 and 14, respectively; and VL-CDR1 and VL-CDR3 comprising, or consisting of, amino acid sequence SEQ ID NO: 15 and 16, respectively, and VL-CDR2 comprising, or consisting of, amino acid sequence EGT.

In a particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 185 or 187; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 186 or 188,
- with the proviso that the CDRs are selected from the following combinations:
   - a VH-CDR1 comprising, or consisting of, amino acid sequence SEQ ID NO: 12;
   - a VH-CDR2 comprising, or consisting of, amino acid sequence SEQ ID NO: 13;
   - a VH-CDR3 comprising, or consisting of, amino acid sequence SEQ ID NO: 17 or 20;
   - a VL-CDR1 comprising, or consisting of, amino acid sequence SEQ ID NO: 18 or 21;
   - a VL-CDR2 comprising, or consisting of, amino acid sequence EGT; and
   - a VL-CDR3 comprising, or consisting of, amino acid sequence SEQ ID NO: 19 or 22.

Preferred antibodies or antigen-binding fragments thereof according to this aspect of the invention are selected from the following group:
∘ a human monoclonal antibody, or antigen-binding fragment thereof, comprising:
   - a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 185; and
   - a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 186,
   - with the proviso that the CDRs are as follow:
      VH-CDR1, VH-CDR2 and VH-CDR3 comprising, or consisting of, amino acid sequence SEQ ID NO: 12, 13 and 17, respectively; and VL-CDR1 and VL-CDR3 comprising, or consisting of, amino acid sequence SEQ ID NO: 18 and 19, respectively, and VL-CDR2 comprising, or consisting of, amino acid sequence EGT;
   or
∘ a human monoclonal antibody, or antigen-binding fragment thereof, comprising:
   - a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 187; and
   - a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 188,
   - with the proviso that the CDRs are as follow:
      VH-CDR1, VH-CDR2 and VH-CDR3 comprising, or consisting of, amino acid sequence SEQ ID NO: 12, 13 and 20, respectively; and VL-CDR1 and VL-CDR3 comprising, or consisting of, amino acid sequence SEQ ID NO: 21 and 22, respectively, and VL-CDR2 comprising, or consisting of, amino acid sequence EGT.

Even more preferred antibodies or antigen-binding fragments thereof according to this aspect of the invention are selected from the following group:
∘ a human monoclonal antibody, or antigen-binding fragment thereof, comprising:
   - a heavy chain variable domain (VH) comprising, or consisting of, amino acid sequence SEQ ID NO: 185; and
   - a light chain variable domain (VL) comprising, or consisting of, amino acid sequence SEQ ID NO: 186 (antibody F11P5);
   or
∘ a human monoclonal antibody, or antigen-binding fragment thereof, comprising:
   - a heavy chain variable domain (VH) comprising, or consisting of, amino acid sequence SEQ ID NO: 187; and
   - a light chain variable domain (VL) comprising, or consisting of, amino acid sequence SEQ ID NO: 188 (antibody D3P7).

### Family P

According to another aspect of the invention, the human monoclonal antibody, or antigen-binding fragment thereof, that binds and optionally neutralizes at least one BK virus subtype, comprises a heavy chain variable domain (VH) and a light chain variable domain (VL) with six complementary-determining regions (CDRs) selected from the following combinations:
- a VH-CDR1 comprising, or consisting of, amino acid sequence GFSXₚ₁SXₚ₂SE (SEQ ID NO: 104), wherein Xₚ₁ is L or S, Xₚ₂ is N or G;
- a VH-CDR2 comprising, or consisting of, amino acid sequence VRSXₒ₃ANRHAXₒ₄ (SEQ ID NO: 105), wherein Xₚ₃ is R or T, Xₚ₄ is T or P;
- a VH-CDR3 comprising, or consisting of, amino acid sequence TGVRGVISRYFAMDV (SEQ ID NO: 106);
- a VL-CDR1 comprising, or consisting of, amino acid sequence Xₚ₅SDXₚ₆GXₚ₇YNY (SEQ ID NO: 107), wherein Xₚ₅ is S or N, Xₚ₆ is V or L, Xₚ₇ is A or S;
- a VL-CDR2 comprising, or consisting of, amino acid sequence DVXₚ₈, wherein Xₚ₈ is S or T; and
- a VL-CDR3 comprising, or consisting of, amino acid sequence CSYAXₚ₉Xₚ₁₀GXₚ₁₁PXₚ₁₂ (SEQ ID NO: 108), wherein Xₚ₉ is H or S, Xₚ₁₀ is R or V, Xₚ₁₁ is T or S, Xₚ₁₂ is L or I.

Particularly, in this aspect, the antibody, or antigen-binding fragment thereof, may comprise six CDRs selected from the following combinations:
- a VH-CDR1 comprising, or consisting of, amino acid sequence SEQ ID NO: 109 or 113;
- a VH-CDR2 comprising, or consisting of, amino acid sequence SEQ ID NO: 110 or 114;
- a VH-CDR3 comprising, or consisting of, amino acid sequence SEQ ID NO: 106;
- a VL-CDR1 comprising, or consisting of, amino acid sequence SEQ ID NO: 111 or 115;
- a VL-CDR2 comprising, or consisting of, amino acid sequence DVS or DVT; and
- a VL-CDR3 comprising, or consisting of, amino acid sequence SEQ ID NO: 112 or 116.

Preferably, in this aspect, the antibody, or antigen-binding fragment thereof, comprises the following combination:
- VH-CDR1, VH-CDR2 and VH-CDR3 comprising, or consisting of, amino acid sequence SEQ ID NO: 109, 110 and 106, respectively; and VL-CDR1 and VL-CDR3 comprising, or consisting of, amino acid sequence SEQ ID NO: 111 and 112, respectively, and VL-CDR2 comprising, or consisting of, amino acid sequence DVS; or
- VH-CDR1, VH-CDR2 and VH-CDR3 comprising, or consisting of, amino acid sequence SEQ ID NO: 113, 114 and 106, respectively; and VL-CDR1 and VL-CDR3 comprising, or consisting of, amino acid sequence SEQ ID NO: 115 and 116, respectively, and VL-CDR2 comprising, or consisting of, amino acid sequence DVT.

In a preferred aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 237 or 239; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 238 or 240.

A total of 1 to 10 amino acids may be optionally mutated in any one of SEQ ID NO: 237, 239, 238 or 240, such as by substitution, deletion and/or insertion, preferably by substitution, more preferably by conservative substitution. Said mutations, if any, preferably occur in regions outside the CDRs (underlined in Table 6). Accordingly, the amino acid sequence has at least 80%, 85%, 90%, 91%, 92%, 93%, 94%, 95%, 96%, 97%, 98%, or 99% identity to the recited sequence with the proviso that the CDRs are not mutated.

Accordingly, in a particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 237 or 239; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 238 or 240,
- with the proviso that the CDRs are selected from the following combinations:
   VH-CDR1, VH-CDR2 and VH-CDR3 comprising, or consisting of, amino acid sequence SEQ ID NO: 104, 105 and 106, respectively; and VL-CDR1 and VL-CDR3 comprising, or consisting of, amino acid sequence SEQ ID NO: 107 and 108, respectively, and VL-CDR2 comprising, or consisting of, amino acid sequence DVXₚ₈, wherein Xₚ₈ is S or T.

Optionally, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 237 or 239; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 238 or 240,
- with the proviso that the CDRs are selected from the following combinations:
   - a VH-CDR1 comprising, or consisting of, amino acid sequence SEQ ID NO: 109 or 113;
   - a VH-CDR2 comprising, or consisting of, amino acid sequence SEQ ID NO: 110 or 114;
   - a VH-CDR3 comprising, or consisting of, amino acid sequence SEQ ID NO: 106;
   - a VL-CDR1 comprising, or consisting of, amino acid sequence SEQ ID NO: 111 or 115;
   - a VL-CDR2 comprising, or consisting of, amino acid sequence DVS or DVT; and
   - a VL-CDR3 comprising, or consisting of, amino acid sequence SEQ ID NO: 112 or 116.

Preferred antibodies or antigen-binding fragments thereof according to this aspect of the invention are selected from the following group:
∘ a human monoclonal antibody, or antigen-binding fragment thereof, comprising:
   - a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 237; and
   - a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 238,
   - with the proviso that the CDRs are as follow:
      VH-CDR1, VH-CDR2 and VH-CDR3 comprising, or consisting of, amino acid sequence SEQ ID NO: 109, 110 and 106, respectively; and VL-CDR1 and VL-CDR3 comprising, or consisting of, amino acid sequence SEQ ID NO: 111 and 112, respectively, and VL-CDR2 comprising, or consisting of, amino acid sequence DVS;
   or
∘ a human monoclonal antibody, or antigen-binding fragment thereof, comprising:
   - a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 239; and
   - a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 240,
   - with the proviso that the CDRs are as follow:
      VH-CDR1, VH-CDR2 and VH-CDR3 comprising, or consisting of, amino acid sequence SEQ ID NO: 113, 114 and 106, respectively; and VL-CDR1 and VL-CDR3 comprising, or consisting of, amino acid sequence SEQ ID NO: 115 and 116, respectively, and VL-CDR2 comprising, or consisting of, amino acid sequence DVT.

Even more preferred antibodies or antigen-binding fragments thereof according to this aspect of the invention are selected from the following group:
∘ a human monoclonal antibody, or antigen-binding fragment thereof, comprising:
   - a heavy chain variable domain (VH) comprising, or consisting of, amino acid sequence SEQ ID NO: 237; and
   - a light chain variable domain (VL) comprising, or consisting of, amino acid sequence SEQ ID NO: 238 (antibody C11P7);
   or
∘ a human monoclonal antibody, or antigen-binding fragment thereof, comprising:
   - a heavy chain variable domain (VH) comprising, or consisting of, amino acid sequence SEQ ID NO: 239; and
   - a light chain variable domain (VL) comprising, or consisting of, amino acid sequence SEQ ID NO: 240 (antibody C6P8).

The human monoclonal antibodies, or antigen-binding fragments thereof, according to this aspect of the invention, show a particularly good neutralization potency on several BKV subtypes, in particular on subtypes I, II and IV

### Family Q

According to another aspect of the invention, the human monoclonal antibody, or antigen-binding fragment thereof, that binds and optionally neutralizes at least one BK virus subtype, comprises a heavy chain variable domain (VH) and a light chain variable domain (VL) with the six following complementary-determining regions (CDRs):
- a VH-CDR1 comprising, or consisting of, amino acid sequence GFTFSGSE (SEQ ID NO: 117);
- a VH-CDR2 comprising, or consisting of, amino acid sequence VRSRANRHAT (SEQ ID NO: 110);
- a VH-CDR3 comprising, or consisting of, amino acid sequence TGVRGVISRYFAMDV (SEQ ID NO: 106);
- a VL-CDR1 comprising, or consisting of, amino acid sequence NIGSNS (SEQ ID NO: 118);
- a VL-CDR2 comprising, or consisting of, amino acid sequence DDS; and
- a VL-CDR3 comprising, or consisting of, amino acid sequence QVWDRRGDLLV (SEQ ID NO: 119).

In a preferred aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 241; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 242.

A total of 1 to 10 amino acids may be optionally mutated in any one of SEQ ID NO: 241 or 242, such as by substitution, deletion and/or insertion, preferably by substitution, more preferably by conservative substitution. Said mutations, if any, preferably occur in regions outside the CDRs (underlined in Table 6). Accordingly, the amino acid sequence has at least 80%, 85%, 90%, 91%, 92%, 93%, 94%, 95%, 96%, 97%, 98%, or 99% identity to the recited sequence with the proviso that the CDRs are not mutated.

Accordingly, in a particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 241; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 242,
- with the proviso that the CDRs are selected from the following combinations:
   VH-CDR1, VH-CDR2 and VH-CDR3 comprising, or consisting of, amino acid sequence SEQ ID NO: 117, 110 and 106, respectively; and VL-CDR1 and VL-CDR3 comprising, or consisting of, amino acid sequence SEQ ID NO: 118 and 119, respectively, and VL-CDR2 comprising, or consisting of, amino acid sequence DDS.

Preferably, the antibody, or antigen-binding fragment thereof comprises:
- a heavy chain (HC) variable domain (VH) comprising, or consisting of, amino acid sequence SEQ ID NO: 241; and
- a light chain (LC) variable domain (VL) comprising, or consisting of, amino acid sequence SEQ ID NO: 242.

This preferred antibody has been named antibody C6P8.

### Family R

According to another aspect of the invention, the human monoclonal antibody, or antigen-binding fragment thereof, that binds and optionally neutralizes at least one BK virus subtype, comprises a heavy chain variable domain (VH) and a light chain variable domain (VL) with the six following complementary-determining regions (CDRs):
- a VH-CDR1 comprising, or consisting of, amino acid sequence GETYVGHS (SEQ ID NO: 145);
- a VH-CDR2 comprising, or consisting of, amino acid sequence ISLHNGNV (SEQ ID NO: 146);
- a VH-CDR3 comprising, or consisting of, amino acid sequence ARVPMKIYGVIVFGEYYYDQLDV (SEQ ID NO: 147);
- a VL-CDR1 comprising, or consisting of, amino acid sequence GSDVGGYNY (SEQ ID NO: 148);
- a VL-CDR2 comprising, or consisting of, amino acid sequence EVS; and
- a VL-CDR3 comprising, or consisting of, amino acid sequence SSYAGSKVV (SEQ ID NO: 149).

In a preferred aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 255; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 256.

A total of 1 to 10 amino acids may be optionally mutated in any one of SEQ ID NO: 255 or 256, such as by substitution, deletion and/or insertion, preferably by substitution, more preferably by conservative substitution. Said mutations, if any, preferably occur in regions outside the CDRs (underlined in Table 6). Accordingly, the amino acid sequence has at least 80%, 85%, 90%, 91%, 92%, 93%, 94%, 95%, 96%, 97%, 98%, or 99% identity to the recited sequence with the proviso that the CDRs are not mutated.

Accordingly, in a particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 255; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 256,
- with the proviso that the CDRs are selected from the following combinations:
   VH-CDR1, VH-CDR2 and VH-CDR3 comprising, or consisting of, amino acid sequence SEQ ID NO: 145, 146 and 147, respectively; and VL-CDR1 and VL-CDR3 comprising, or consisting of, amino acid sequence SEQ ID NO: 148 and 149, respectively, and VL-CDR2 comprising, or consisting of, amino acid sequence EVS.

Preferably, the antibody, or antigen-binding fragment thereof comprises:
- a heavy chain variable domain (VH) comprising, or consisting of, amino acid sequence SEQ ID NO: 255; and
- a light chain variable domain (VL) comprising, or consisting of, amino acid sequence SEQ ID NO: 256.

This preferred antibody has been named antibody C10P7.

### Family S

According to another aspect of the invention, the human monoclonal antibody, or antigen-binding fragment thereof, that binds and optionally neutralizes at least one BK virus subtype, comprises a heavy chain variable domain (VH) and a light chain variable domain (VL) with the six following complementary-determining regions (CDRs):
- a VH-CDR1 comprising, or consisting of, amino acid sequence GFTFRSYA (SEQ ID NO: 160);
- a VH-CDR2 comprising, or consisting of, amino acid sequence LSSSGTTT (SEQ ID NO: 161);
- a VH-CDR3 comprising, or consisting of, amino acid sequence AKDRNAQWLGSEPLDP (SEQ ID NO: 162);
- a VL-CDR1 comprising, or consisting of, amino acid sequence QSVSSSY (SEQ ID NO: 65);
- a VL-CDR2 comprising, or consisting of, amino acid sequence GAS; and
- a VL-CDR3 comprising, or consisting of, amino acid sequence QQAYTFPWT (SEQ ID NO: 163).

In a preferred aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 263; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 264.

A total of 1 to 10 amino acids may be optionally mutated in any one of SEQ ID NO: 263 or 264, such as by substitution, deletion and/or insertion, preferably by substitution, more preferably by conservative substitution. Said mutations, if any, i preferably occur in regions outside the CDRs (underlined in Table 6). Accordingly, the amino acid sequence has at least 80%, 85%, 90%, 91%, 92%, 93%, 94%, 95%, 96%, 97%, 98%, or 99% identity to the recited sequence with the proviso that the CDRs are not mutated.

Accordingly, in a particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 263; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 264,
- with the proviso that the CDRs are selected from the following combinations:
   VH-CDR1, VH-CDR2 and VH-CDR3 comprising, or consisting of, amino acid sequence SEQ ID NO: 160, 161 and 162, respectively; and VL-CDR1 and VL-CDR3 comprising, or consisting of, amino acid sequence SEQ ID NO: 65 and 163, respectively, and VL-CDR2 comprising, or consisting of, amino acid sequence GAS.

Preferably, the antibody, or antigen-binding fragment thereof comprises:
- a heavy chain variable domain (VH) comprising, or consisting of, amino acid sequence SEQ ID NO: 263; and
- a light chain variable domain (VL) comprising, or consisting of, amino acid sequence SEQ ID NO: 264.

This preferred antibody has been named antibody E10P2.

### Family T

According to another aspect of the invention, the human monoclonal antibody, or antigen-binding fragment thereof, that binds and optionally neutralizes at least one BK virus subtype, comprises a heavy chain variable domain (VH) and a light chain variable domain (VL) with the six following complementary-determining regions (CDRs):
- a VH-CDR1 comprising, or consisting of, amino acid sequence GFTFSSYG (SEQ ID NO: 87);
- a VH-CDR2 comprising, or consisting of, amino acid sequence ISKDGSNK (SEQ ID NO: 88);
- a VH-CDR3 comprising, or consisting of, amino acid sequence AKIPLRRYSDSGDYSSGDF (SEQ ID NO: 89);
- a VL-CDR1 comprising, or consisting of, amino acid sequence ENLVYSDGNTY (SEQ ID NO: 90);
- a VL-CDR2 comprising, or consisting of, amino acid sequence KVS; and
- a VL-CDR3 comprising, or consisting of, amino acid sequence MQTTHWPPLT (SEQ ID NO: 91).

In a preferred aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 229; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 230.

A total of 1 to 10 amino acids may be optionally mutated in any one of SEQ ID NO: 229 or 230, such as by substitution, deletion and/or insertion, preferably by substitution, more preferably by conservative substitution. Said mutations, if any, preferably occur in regions outside the CDRs (underlined in Table 6). Accordingly, the amino acid sequence has at least 80%, 85%, 90%, 91%, 92%, 93%, 94%, 95%, 96%, 97%, 98%, or 99% identity to the recited sequence with the proviso that the CDRs are not mutated.

Accordingly, in a particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 229; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 230,
- with the proviso that the CDRs are selected from the following combinations:
   VH-CDR1, VH-CDR2 and VH-CDR3 comprising, or consisting of, amino acid sequence SEQ ID NO: 87, 88 and 89, respectively; and VL-CDR1 and VL-CDR3 comprising, or consisting of, amino acid sequence SEQ ID NO: 90 and 91, respectively, and VL-CDR2 comprising, or consisting of, amino acid sequence KVS.

Preferably, the antibody, or antigen-binding fragment thereof comprises:
- a heavy chain ariable domain (VH) comprising, or consisting of, amino acid sequence SEQ ID NO: 229; and
- a light chain variable domain (VL) comprising, or consisting of, amino acid sequence SEQ ID NO: 230.

This preferred antibody has been named antibody B9P4.

### Family U

According to another aspect of the invention, the human monoclonal antibody, or antigen-binding fragment thereof, that binds and optionally neutralizes at least one BK virus subtype, comprises a heavy chain variable domain (VH) and a light chain variable domain (VL) with the six following complementary-determining regions (CDRs):
- a VH-CDR1 comprising, or consisting of, amino acid sequence GGSISSGRYY (SEQ ID NO: 172);
- a VH-CDR2 comprising, or consisting of, amino acid sequence VYFSGST (SEQ ID NO: 173);
- a VH-CDR3 comprising, or consisting of, amino acid sequence ARAVRDYGDSYSFDY (SEQ ID NO: 174);
- a VL-CDR1 comprising, or consisting of, amino acid sequence SSDIGGYNY (SEQ ID NO: 175;
- a VL-CDR2 comprising, or consisting of, amino acid sequence DVV; and
- a VL-CDR3 comprising, or consisting of, amino acid sequence CSYSIRGTPV (SEQ ID NO: 176).

In a preferred aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 269; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 270.

A total of 1 to 10 amino acids may be optionally mutated in any one of SEQ ID NO: 269 or 270, such as by substitution, deletion and/or insertion, preferably by substitution, more preferably by conservative substitution. Said mutations, if any, preferably occur in regions outside the CDRs (underlined in Table 6). Accordingly, the amino acid sequence has at least 80%, 85%, 90%, 91%, 92%, 93%, 94%, 95%, 96%, 97%, 98%, or 99% identity to the recited sequence with the proviso that the CDRs are not mutated.

Accordingly, in a particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 269; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 270,
- with the proviso that the CDRs are selected from the following combinations:
   VH-CDR1, VH-CDR2 and VH-CDR3 comprising, or consisting of, amino acid sequence SEQ ID NO: 172, 173 and 174, respectively; and VL-CDR1 and VL-CDR3 comprising, or consisting of, amino acid sequence SEQ ID NO: 175 and 176, respectively, and VL-CDR2 comprising, or consisting of, amino acid sequence DVV.

Preferably, the antibody, or antigen-binding fragment thereof comprises:
- a heavy chain variable domain (VH) comprising, or consisting of, amino acid sequence SEQ ID NO: 269; and
- a light chain variable domain (VL) comprising, or consisting of, amino acid sequence SEQ ID NO: 270.

This preferred antibody has been named antibody C10P8.

### Family V

According to another aspect of the invention, the human monoclonal antibody, or antigen-binding fragment thereof, that binds and optionally neutralizes at least one BK virus subtype, comprises a heavy chain variable domain (VH) and a light chain variable domain (VL) with six complementary-determining regions (CDRs) selected from the following combinations:
- a VH-CDR1 comprising, or consisting of, amino acid sequence GXᵣ₁YISTXᵣ₂SYY (SEQ ID NO: 120), wherein Xᵣ₁ is G or T, Xᵣ₂ is S or N;
- a VH-CDR2 comprising, or consisting of, amino acid sequence IEYXᵣ₃GST (SEQ ID NO: 121), wherein Xᵣ₃ is S or G;
- a VH-CDR3 comprising, or consisting of, amino acid sequence ASANSXᵣ₄YYDSGGYXᵣ₅APXᵣ₆ALDXᵣ₇ (SEQ ID NO: 122), wherein Xᵣ₄ is K or H, Xᵣ₅ is Y or C, Xᵣ₆ is G or S, Xᵣ₇ is L or I;
- a VL-CDR1 comprising, or consisting of, amino acid sequence QSXᵣ₈SSH (SEQ ID NO: 123), wherein Xᵣ₈ is V or A;
- a VL-CDR2 comprising, or consisting of, amino acid sequence AAS; and
- a VL-CDR3 comprising, or consisting of, amino acid sequence QQYNNWPPGXᵣ₉Xᵣ₁₀ (SEQ ID NO: 124), wherein Xᵣ₉ is Y or T, Xᵣ₁₀ is S or absent.

Particularly, in this aspect, the antibody, or antigen-binding fragment thereof, may comprise six CDRs selected from the following combinations:
- a VH-CDR1 comprising, or consisting of, amino acid sequence SEQ ID NO: 125 or 130;
- a VH-CDR2 comprising, or consisting of, amino acid sequence SEQ ID NO: 126 or 131;
- a VH-CDR3 comprising, or consisting of, amino acid sequence SEQ ID NO: 127 or 132;
- a VL-CDR1 comprising, or consisting of, amino acid sequence SEQ ID NO: 128 or 133;
- a VL-CDR2 comprising, or consisting of, amino acid sequence AAS; and
- a VL-CDR3 comprising, or consisting of, amino acid sequence SEQ ID NO: 129 or 134.

Preferably, in this aspect, the antibody, or antigen-binding fragment thereof, comprises the following combination:
- VH-CDR1, VH-CDR2 and VH-CDR3 comprising, or consisting of, amino acid sequence SEQ ID NO: 125, 126 and 127, respectively; and VL-CDR1 and VL-CDR3 comprising, or consisting of, amino acid sequence SEQ ID NO: 128 and 129, respectively, and VL-CDR2 comprising, or consisting of, amino acid sequence AAS; or
- VH-CDR1, VH-CDR2 and VH-CDR3 comprising, or consisting of, amino acid sequence SEQ ID NO: 130, 131 and 132, respectively; and VL-CDR1 and VL-CDR3 comprising, or consisting of, amino acid sequence SEQ ID NO: 133 and 134, respectively, and VL-CDR2 comprising, or consisting of, amino acid sequence AAS.

In a preferred aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 243 or 245; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 244 or 246.

A total of 1 to 10 amino acids may be optionally mutated in any one of SEQ ID NO: 243, 245, 244 or 246, such as by substitution, deletion and/or insertion, preferably by substitution, more preferably by conservative substitution. Said mutations, if any, i preferably occur in regions outside the CDRs (underlined in Table 6). Accordingly, the amino acid sequence has at least 80%, 85%, 90%, 91%, 92%, 93%, 94%, 95%, 96%, 97%, 98%, or 99% identity to the recited sequence with the proviso that the CDRs are not mutated.

Accordingly, in a particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 243 or 245; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 244 or 246,
- with the proviso that the CDRs are selected from the following combinations:
   VH-CDR1, VH-CDR2 and VH-CDR3 comprising, or consisting of, amino acid sequence SEQ ID NO: 120, 121 and 122, respectively; and VL-CDR1 and VL-CDR3 comprising, or consisting of, amino acid sequence SEQ ID NO: 123 and 124, respectively, and VL-CDR2 comprising, or consisting of, amino acid sequence AAS.

Optionally, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 243 or 245; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 244 or 246,
- with the proviso that the CDRs are selected from the following combinations:
   - a VH-CDR1 comprising, or consisting of, amino acid sequence SEQ ID NO: 125 or 130;
   - a VH-CDR2 comprising, or consisting of, amino acid sequence SEQ ID NO: 126 or 131;
   - a VH-CDR3 comprising, or consisting of, amino acid sequence SEQ ID NO: 127 or 132;
   - a VL-CDR1 comprising, or consisting of, amino acid sequence SEQ ID NO: 128 or 133;
   - a VL-CDR2 comprising, or consisting of, amino acid sequence AAS; and
   - a VL-CDR3 comprising, or consisting of, amino acid sequence SEQ ID NO: 129 or 134.

Preferred antibodies or antigen-binding fragments thereof according to this aspect of the invention are selected from the following group:
∘ a human monoclonal antibody, or antigen-binding fragment thereof, comprising:
   - a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 243; and
   - a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 244,
   - with the proviso that the CDRs are as follow:
      VH-CDR1, VH-CDR2 and VH-CDR3 comprising, or consisting of, amino acid sequence SEQ ID NO: 125, 126 and 127, respectively; and VL-CDR1 and VL-CDR3 comprising, or consisting of, amino acid sequence SEQ ID NO: 128 and 129, respectively, and VL-CDR2 comprising, or consisting of, amino acid sequence AAS;
   or
∘ a human monoclonal antibody, or antigen-binding fragment thereof, comprising:
   - a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 245; and
   - a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 246,
   - with the proviso that the CDRs are as follow:
      VH-CDR1, VH-CDR2 and VH-CDR3 comprising, or consisting of, amino acid sequence SEQ ID NO: 130, 131 and 132, respectively; and VL-CDR1 and VL-CDR3 comprising, or consisting of, amino acid sequence SEQ ID NO: 133 and 134, respectively, and VL-CDR2 comprising, or consisting of, amino acid sequence AAS.

Even more preferred antibodies or antigen-binding fragments thereof according to this aspect of the invention are selected from the following group:
∘ a human monoclonal antibody, or antigen-binding fragment thereof, comprising:
   - a heavy chain variable domain (VH) comprising, or consisting of, amino acid sequence SEQ ID NO: 243; and
   - a light chain variable domain (VL) comprising, or consisting of, amino acid sequence SEQ ID NO: 244 (antibody G8P7);
   or
∘ a human monoclonal antibody, or antigen-binding fragment thereof, comprising:
   - a heavy chain variable domain (VH) comprising, or consisting of, amino acid sequence SEQ ID NO: 245; and
   - a light chain variable domain (VL) comprising, or consisting of, amino acid sequence SEQ ID NO: 246 (antibody D9P6).

### Family W

According to another aspect of the invention, the human monoclonal antibody, or antigen-binding fragment thereof, that binds and optionally neutralizes at least one BK virus subtype, comprises a heavy chain variable domain (VH) and a light chain variable domain (VL) with the six following complementary-determining regions (CDRs):
- a VH-CDR1 comprising, or consisting of, amino acid sequence GGRFSRYG (SEQ ID NO: 155);
- a VH-CDR2 comprising, or consisting of, amino acid sequence IIPLLGTA (SEQ ID NO: 156);
- a VH-CDR3 comprising, or consisting of, amino acid sequence ATARREYDRPGAEYFIQ (SEQ ID NO: 157);
- a VL-CDR1 comprising, or consisting of, amino acid sequence QSVSTN (SEQ ID NO: 158);
- a VL-CDR2 comprising, or consisting of, amino acid sequence DAF; and
- a VL-CDR3 comprising, or consisting of, amino acid sequence QQYNYWPPLT (SEQ ID NO: 159).

In a preferred aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 259; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 260.

A total of 1 to 10 amino acids may be optionally mutated in any one of SEQ ID NO: 259 or 260, such as by substitution, deletion and/or insertion, preferably by substitution, more preferably by conservative substitution. Said mutations, if any, preferably occur in regions outside the CDRs (underlined in Table 6). Accordingly, the amino acid sequence has at least 80%, 85%, 90%, 91%, 92%, 93%, 94%, 95%, 96%, 97%, 98%, or 99% identity to the recited sequence with the proviso that the CDRs are not mutated.

Accordingly, in a particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 259; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 260,
- with the proviso that the CDRs are selected from the following combinations:
   VH-CDR1, VH-CDR2 and VH-CDR3 comprising, or consisting of, amino acid sequence SEQ ID NO: 155, 156 and 157, respectively; and VL-CDR1 and VL-CDR3 comprising, or consisting of, amino acid sequence SEQ ID NO: 158 and 159, respectively, and VL-CDR2 comprising, or consisting of, amino acid sequence DAF.

Preferably, the antibody, or antigen-binding fragment thereof comprises:
- a heavy chain variable domain (VH) comprising, or consisting of, amino acid sequence SEQ ID NO: 259; and
- a light chain variable domain (VL) comprising, or consisting of, amino acid sequence SEQ ID NO: 260.

This preferred antibody has been named antibody B5P4.

### Family X

According to another aspect of the invention, the human monoclonal antibody, or antigen-binding fragment thereof, that binds and optionally neutralizes at least one BK virus subtype, comprises a heavy chain variable domain (VH) and a light chain variable domain (VL) with the six following complementary-determining regions (CDRs):
- a VH-CDR1 comprising, or consisting of, amino acid sequence GFTFESYG (SEQ ID NO: 37);
- a VH-CDR2 comprising, or consisting of, amino acid sequence ISNDGSIE (SEQ ID NO: 38);
- a VH-CDR3 comprising, or consisting of, amino acid sequence AKDAVGITGGSLYYSYGMDV (SEQ ID NO: 39);
- a VL-CDR1 comprising, or consisting of, amino acid sequence QTIKNY (SEQ ID NO: 40);
- a VL-CDR2 comprising, or consisting of, amino acid sequence TAS; and
- a VL-CDR3 comprising, or consisting of, amino acid sequence QQSYSAPYS (SEQ ID NO: 41).

In a preferred aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 203; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 204.

A total of 1 to 10 amino acids may be optionally mutated in any one of SEQ ID NO: 203 or 204, such as by substitution, deletion and/or insertion, preferably by substitution, more preferably by conservative substitution. Said mutations, if any, inpreferably occur in regions outside the CDRs (underlined in Table 6). Accordingly, the amino acid sequence has at least 80%, 85%, 90%, 91%, 92%, 93%, 94%, 95%, 96%, 97%, 98%, or 99% identity to the recited sequence with the proviso that the CDRs are not mutated.

Accordingly, in a particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 203; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 204,
- with the proviso that the CDRs are selected from the following combinations:
   VH-CDR1, VH-CDR2 and VH-CDR3 comprising, or consisting of, amino acid sequence SEQ ID NO: 37, 38 and 39, respectively; and VL-CDR1 and VL-CDR3 comprising, or consisting of, amino acid sequence SEQ ID NO: 40 and 41, respectively, and VL-CDR2 comprising, or consisting of, amino acid sequence TAS.

Preferably, the antibody, or antigen-binding fragment thereof comprises:
- a heavy chain variable domain (VH) comprising, or consisting of, amino acid sequence SEQ ID NO: 203; and
- a light chain variable domain (VL) comprising, or consisting of, amino acid sequence SEQ ID NO: 204.

This preferred antibody has been named antibody B3P3.

### Family Y

According to another aspect of the invention, the human monoclonal antibody, or antigen-binding fragment thereof, that binds and optionally neutralizes at least one BK virus subtype, comprises a heavy chain variable domain (VH) and a light chain variable domain (VL) with the six following complementary-determining regions (CDRs):
- a VH-CDR1 comprising, or consisting of, amino acid sequence GFIFNNHG (SEQ ID NO: 92);
- a VH-CDR2 comprising, or consisting of, amino acid sequence ISYDGKKE (SEQ ID NO: 93);
- a VH-CDR3 comprising, or consisting of, amino acid sequence AKEAYGSGSPCDY (SEQ ID NO: 94);
- a VL-CDR1 comprising, or consisting of, amino acid sequence QRVSSNY (SEQ ID NO: 95);
- a VL-CDR2 comprising, or consisting of, amino acid sequence GAS; and
- a VL-CDR3 comprising, or consisting of, amino acid sequence QQYGTSPRS (SEQ ID NO: 96).

In a preferred aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 231; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 232.

A total of 1 to 10 amino acids may be optionally mutated in any one of SEQ ID NO: 231 or 232, such as by substitution, deletion and/or insertion, preferably by substitution, more preferably by conservative substitution. Said mutations, if any, preferably occur in regions outside the CDRs (underlined in Table 6). Accordingly, the amino acid sequence has at least 80%, 85%, 90%, 91%, 92%, 93%, 94%, 95%, 96%, 97%, 98%, or 99% identity to the recited sequence with the proviso that the CDRs are not mutated.

Accordingly, in a particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 231; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 232,
- with the proviso that the CDRs are selected from the following combinations:
   VH-CDR1, VH-CDR2 and VH-CDR3 comprising, or consisting of, amino acid sequence SEQ ID NO: 92, 93 and 94, respectively; and VL-CDR1 and VL-CDR3 comprising, or consisting of, amino acid sequence SEQ ID NO: 95 and 96, respectively, and VL-CDR2 comprising, or consisting of, amino acid sequence GAS.

Preferably, the antibody, or antigen-binding fragment thereof comprises:
- a heavy chain variable domain (VH) comprising, or consisting of, amino acid sequence SEQ ID NO: 231; and
- a light chain variable domain (VL) comprising, or consisting of, amino acid sequence SEQ ID NO: 232.

This preferred antibody has been named antibody E5P6.

### Family Z

According to another aspect of the invention, the human monoclonal antibody, or antigen-binding fragment thereof, that binds and optionally neutralizes at least one BK virus subtype, comprises a heavy chain variable domain (VH) and a light chain variable domain (VL) with the six following complementary-determining regions (CDRs):
- a VH-CDR1 comprising, or consisting of, amino acid sequence GFTFSSYS (SEQ ID NO: 164);
- a VH-CDR2 comprising, or consisting of, amino acid sequence ISSSSRTI (SEQ ID NO: 165);
- a VH-CDR3 comprising, or consisting of, amino acid sequence ARQAVAHFDY (SEQ ID NO: 166);
- a VL-CDR1 comprising, or consisting of, amino acid sequence QRVSSNY (SEQ ID NO: 95);
- a VL-CDR2 comprising, or consisting of, amino acid sequence GAS; and
- a VL-CDR3 comprising, or consisting of, amino acid sequence QQYGTSPRS (SEQ ID NO: 96).

In a preferred aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 265; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 266.

A total of 1 to 10 amino acids may be optionally mutated in any one of SEQ ID NO: 265 or 266, such as by substitution, deletion and/or insertion, preferably by substitution, more preferably by conservative substitution. Said mutations, if any, preferably occur in regions outside the CDRs (underlined in Table 6). Accordingly, the amino acid sequence has at least 80%, 85%, 90%, 91%, 92%, 93%, 94%, 95%, 96%, 97%, 98%, or 99% identity to the recited sequence with the proviso that the CDRs are not mutated.

Accordingly, in particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 265; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 266,
- with the proviso that the CDRs are selected from the following combinations:
   VH-CDR1, VH-CDR2 and VH-CDR3 comprising, or consisting of, amino acid sequence SEQ ID NO: 164, 165 and 166, respectively; and VL-CDR1 and VL-CDR3 comprising, or consisting of, amino acid sequence SEQ ID NO: 95 and 96, respectively, and VL-CDR2 comprising, or consisting of, amino acid sequence GAS.

Preferably, the antibody, or antigen-binding fragment thereof comprises:
- a heavy chain variable domain (VH) comprising, or consisting of, amino acid sequence SEQ ID NO: 265; and
- a light chain variable domain (VL) comprising, or consisting of, amino acid sequence SEQ ID NO: 266.

This preferred antibody has been named antibody C9P1.

### Family A'

According to another aspect of the invention, the human monoclonal antibody, or antigen-binding fragment thereof, that binds and optionally neutralizes at least one BK virus subtype, comprises a heavy chain variable domain (VH) and a light chain variable domain (VL) with the six following complementary-determining regions (CDRs):
- a VH-CDR1 comprising, or consisting of, amino acid sequence GFTFSTYT (SEQ ID NO: 167);
- a VH-CDR2 comprising, or consisting of, amino acid sequence ISSRSTSI (SEQ ID NO: 168);
- a VH-CDR3 comprising, or consisting of, amino acid sequence VRDRGYYGSNWFDS (SEQ ID NO: 169);
- a VL-CDR1 comprising, or consisting of, amino acid sequence SSDVGNYNY (SEQ ID NO: 170);
- a VL-CDR2 comprising, or consisting of, amino acid sequence DVT; and
- a VL-CDR3 comprising, or consisting of, amino acid sequence CAYAGSYTLV (SEQ ID NO: 171).

In a preferred aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 267; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 268.

A total of 1 to 10 amino acids may be optionally mutated in any one of SEQ ID NO: 267 or 268, such as by substitution, deletion and/or insertion, preferably by substitution, more preferably by conservative substitution. Said mutations, if any, preferably occur in regions outside the CDRs (underlined in Table 6). Accordingly, the amino acid sequence has at least 80%, 85%, 90%, 91%, 92%, 93%, 94%, 95%, 96%, 97%, 98%, or 99% identity to the recited sequence with the proviso that the CDRs are not mutated.

Accordingly, in a particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 267; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 268,
- with the proviso that the CDRs are selected from the following combinations:
   VH-CDR1, VH-CDR2 and VH-CDR3 comprising, or consisting of, amino acid sequence SEQ ID NO: 167, 168 and 169, respectively; and VL-CDR1 and VL-CDR3 comprising, or consisting of, amino acid sequence SEQ ID NO: 170 and 171, respectively, and VL-CDR2 comprising, or consisting of, amino acid sequence DVT.

Preferably, the antibody, or antigen-binding fragment thereof comprises:
- a heavy chain variable domain (VH) comprising, or consisting of, amino acid sequence SEQ ID NO: 267; and
- a light chain variable domain (VL) comprising, or consisting of, amino acid sequence SEQ ID NO: 268.

This preferred antibody has been named antibody B10P5.

### Family B'

According to another aspect of the invention, the human monoclonal antibody, or antigen-binding fragment thereof, that binds and optionally neutralizes at least one BK virus subtype, comprises a heavy chain variable domain (VH) and a light chain variable domain (VL) with the six following complementary-determining regions (CDRs):
- a VH-CDR1 comprising, or consisting of, amino acid sequence GFTVSTNC (SEQ ID NO: 42);
- a VH-CDR2 comprising, or consisting of, amino acid sequence IYSGGTT (SEQ ID NO: 43);
- a VH-CDR3 comprising, or consisting of, amino acid sequence ARSVDTYDIYALDV (SEQ ID NO: 44);
- a VL-CDR1 comprising, or consisting of, amino acid sequence QSISRW (SEQ ID NO: 45);
- a VL-CDR2 comprising, or consisting of, amino acid sequence KAS; and
- a VL-CDR3 comprising, or consisting of, amino acid sequence QQYNSYPWT (SEQ ID NO: 46).

In a preferred aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 205; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 206.

A total of 1 to 10 amino acids may be optionally mutated in any one of SEQ ID NO: 205 or 206, such as by substitution, deletion and/or insertion, preferably by substitution, more preferably by conservative substitution. Said mutations, if any, preferably occur in regions outside the CDRs (underlined in Table 6). Accordingly, the amino acid sequence has at least 80%, 85%, 90%, 91%, 92%, 93%, 94%, 95%, 96%, 97%, 98%, or 99% identity to the recited sequence with the proviso that the CDRs are not mutated.

Accordingly, in a particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 205; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 206,
- with the proviso that the CDRs are selected from the following combinations:
   VH-CDR1, VH-CDR2 and VH-CDR3 comprising, or consisting of, amino acid sequence SEQ ID NO: 42, 43 and 44, respectively; and VL-CDR1 and VL-CDR3 comprising, or consisting of, amino acid sequence SEQ ID NO: 45 and 46, respectively, and VL-CDR2 comprising, or consisting of, amino acid sequence KAS.

Preferably, the antibody, or antigen-binding fragment thereof comprises:
- a heavy chain variable domain (VH) comprising, or consisting of, amino acid sequence SEQ ID NO: 205; and
- a light chain variable domain (VL) comprising, or consisting of, amino acid sequence SEQ ID NO: 206.

This preferred antibody has been named antibody E2P6.

The human monoclonal antibodies, or antigen-binding fragments thereof, according to this aspect of the invention, exhibit a particularly good neutralization potency on several BKV subtypes, in particular on subtypes I, II and IV

### Family C'

According to another aspect of the invention, the human monoclonal antibody, or antigen-binding fragment thereof, that binds and optionally neutralizes at least one BK virus subtype, comprises a heavy chain variable domain and a light chain (LC) variable domain (VL) with the six following complementary-determining regions (CDRs):
- a VH-CDR1 comprising, or consisting of, amino acid sequence GGSIRNSY (SEQ ID NO: 23);
- a VH-CDR2 comprising, or consisting of, amino acid sequence IYYTGTT (SEQ ID NO: 28);
- a VH-CDR3 comprising, or consisting of, amino acid sequence AQGGGSGDLHY (SEQ ID NO: 25);
- a VL-CDR1 comprising, or consisting of, amino acid sequence QSVRSNY (SEQ ID NO: 4);
- a VL-CDR2 comprising, or consisting of, amino acid sequence GAS; and
- a VL-CDR3 comprising, or consisting of, amino acid sequence HQYASSPRT (SEQ ID NO: 11).

In a preferred aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 195; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 196.

A total of 1 to 10 amino acids may be optionally mutated in any one of SEQ ID NO: 195 or 196, such as by substitution, deletion and/or insertion, preferably by substitution, more preferably by conservative substitution. Said mutations, if any, preferably occur in regions outside the CDRs (underlined in Table 6). Accordingly, the amino acid sequence has at least 80%, 85%, 90%, 91%, 92%, 93%, 94%, 95%, 96%, 97%, 98%, or 99% identity to the recited sequence with the proviso that the CDRs are not mutated.

Accordingly, in a particular aspect, the antibody, or antigen-binding fragment thereof comprises:
- a heavy chain variable domain (VH) comprising, or consisting of, an amino acid sequence having at least 80%, 85%, 90%, 91%, 92%, 93%, 94%, 95%, 96%, 97%, 98%, or 99% sequence identity to SEQ ID NO: 195; and
- a light chain variable domain (VL) comprising, or consisting of, an amino acid sequence having at least 80%, 85%, 90%, 91%, 92%, 93%, 94%, 95%, 96%, 97%, 98%, or 99% sequence identity to SEQ ID NO: 196,
- with the proviso that the CDRs are selected from the following combinations:
   SEQ ID NO: 23, 28 and 25, respectively; and VL-CDR1 and VL-CDR3 comprising, or consisting of, amino acid sequence SEQ ID NO: 4 and 11, respectively, and VL-CDR2 comprising, or consisting of, amino acid sequence GAS.

Preferably, the antibody, or antigen-binding fragment thereof comprises:
- a heavy chain variable domain (VH) comprising, or consisting of, amino acid sequence SEQ ID NO: 195; and
- a light chain variable domain (VL) comprising, or consisting of, amino acid sequence SEQ ID NO: 196.

This preferred antibody has been named antibody B2P8.

**Table 5. CDRs of the antibodies, or antigen-binding fragments, according to the invention**

| | **VH CDR1** | | **VH CDR2** | | **VH CDR3** | | **VL CDR1** | | **VL CDR2** | **VL CDR3** | |
|---|---|---|---|---|---|---|---|---|---|---|---|
| **Name** | **Sequence** | **SEQ ID NO:** | **Sequence** | **SEQ ID NO:** | **Sequence** | **SEQ ID NO:** | **Sequence** | **SEQ ID NO:** | **Sequence** | **Sequence** | **SEQ ID NO:** |
| B9P6 | GFTFRSYT | 6 | ISGSSNHK | 7 | AREDDILTPLYGMDV | 3 | QSVRSNY | 4 | GAS | HQYGSSPRT | 8 |
| E9P7 | GFTFRSYT | 6 | ISGSSNHK | 7 | AREDDILTPLYGMDV | 3 | QSVRSNY | 4 | GAS | HQYGSSPRT | 8 |
| G11P1 | GFTFRSYS | 9 | ISGSSNQK | 10 | AREDDILTPLYGMDV | 3 | QSVRSNY | 4 | GAS | HQYASSPRT | 11 |
| E11P6 | GFTFRSYS | 9 | ISGSSNQK | 10 | AREDDILTPLYGMDV | 3 | QSVRSNY | 4 | GAS | HQYASSPRT | 11 |
| F11P5 | AYTFTNSA | 12 | INTNTGNP | 13 | TVPYNFGGDHNSYYFFGVDI | 17 | SSDVGSYNL | 18 | EGT | CSYAGSNTFV | 19 |
| D3P7 | AYTFTNSA | 12 | INTNTGNP | 13 | AVPYNFGGDHNSFHFFGMDV | 20 | SSDVGTYNF | 21 | EGT | SSYAGGNAFV | 22 |
| C11P1 | GGSIRNSY | 23 | IYYTGTT | 28 | AQGGGSGDLHY | 25 | RSDVGGYNY | 26 | DIS | ISFATGYTWV | 27 |
| G5P1 | GGSIRNSY | 23 | IYYTGTT | 28 | AQGGGSGDLHY | 25 | RSDVGGYNY | 26 | DIS | ISFATGYTWV | 27 |
| E7P5 | GGSIRNSY | 23 | IYYTGST | 29 | AQGGGSGDLHY | 25 | RSDVGGYNY | 26 | DIS | ISFATGYTWV | 27 |
| B2P8 | GGSIRNSY | 23 | IYYTGTT | 28 | AQGGGSGDLHY | 25 | QSVRSNY | 4 | GAS | HQYASSPRT | 11 |
| B8P7 | GGSISPYY | 30 | IYYSGNT | 31 | ARDSCSGTCYLGDNWFDP | 35 | SSNIGANND | 33 | GNN | QSYDSSLSTSKV | 34 |
| G6P7 | GGSISPYY | 30 | IYYSGNT | 31 | ARDSCSGTCYLGDNWFDP | 35 | SSNIGANND | 33 | GNN | QSYDSSLSTSKV | 34 |
| C4P6 | GGSISPYY | 30 | IYYSGNT | 31 | ARDSCSGTCYLGGNWFDP | 36 | SSNIGANND | 33 | GNN | QSYDSSLSTSKV | 34 |
| B3P3 | GFTFESYG | 37 | ISNDGSIE | 38 | AKDAVGITGGSLYYSYGMDV | 39 | QTIKNY | 40 | TAS | QQSYSAPYS | 41 |
| E2P6 | GFTVSTNC | 42 | IYSGGTT | 43 | ARSVDTYDIYALDV | 44 | QSISRW | 45 | KAS | QQYNSYPWT | as |
| E6P5 | GFTFDDHA | 47 | INWNSASI | 48 | AKDFGLWFGESRGIDA | 49 | QSLLHSNGYNY | 50 | LGS | MQALQTPIT | 51 |
| E4P2 | GGSISSGGYY | 52 | MYYSGST | 53 | ARDRRDYGDYYYFGMDV | 54 | SSNIGAGYD | 55 | ANS | QSYDSSLSVYW | 56 |
| B7P6 | GFTFTNYW | 57 | IYPDDSDA | 58 | ARGGFCSGSVCYNPAWFDL | 59 | QTINTY | 60 | DAS | QQSYSTPLT | 61 |
| F3P6 | GYTFTTHI | 62 | INAGNGNT | 63 | AREDQWLGRSPLEY | 64 | QSVSSSY | 65 | GAS | QQYGNSYPWT | 66 |
| G5P6 | GYTFTTHI | 62 | INAGNGNT | 63 | AREDQWLGRSPLEY | 64 | QSVSSSY | 65 | GAS | QQYGNSYPWT | 66 |
| B10P1 | GFTFSSYA | 72 | LTGSGGST | 73 | AKEGAYSYDFWYFDL | 74 | QTVSGNY | 75 | GAS | QQYGRPLT | 76 |
| G9P2 | GFTFSSYA | 72 | LTGSGGST | 73 | AKEGAYSYDFWYFDL | 74 | QTVSGNY | 75 | GAS | QQYGRPLT | 76 |
| C4P7 | GFTFSSYA | 72 | LTGSGGST | 73 | AKEGAYSYDFWYFDL | 74 | QTVSGNY | 75 | GAS | QQYGRPLT | 76 |
| D7P6 | GFPFSSYA | 77 | ITGSASGT | 78 | AKEGAYSYDYWYFDL | 79 | QSVSGSY | 80 | GAS | QQYGSLVT | 81 |
| B7P3 | GFTFSNFG | 82 | ISYDGSDK | 83 | AKDRVRTKYYYDHSGPFDY | 84 | NIGSKS | 85 | DDD | QVWMTNSDRPV | 86 |
| F4P5 | GFTFSNFG | 82 | ISYDGSDK | 83 | AKDRVRTKYYYDHSGPFDY | 84 | NIGSKS | 85 | DDD | QVWMTNSDRPV | 86 |
| B9P4 | GFTFSSYG | 87 | ISKDGSNK | 88 | AKIPLRRYSDSGDYSSGDF | 89 | ENLVYSDGNTY | 90 | KVS | MQTTHWPPLT | 91 |
| E5P6 | GFIFNNHG | 92 | ISYDGKKE | 93 | AKEAYGSGSPCDY | 94 | QRVSSNY | 95 | GAS | QQYGTSPRS | 96 |
| G7P3 | GFSFGSYS | 102 | ISSGSSSI | 98 | ARGWTGYDAFDI | 99 | QVISNY | 100 | AAS | QKYNNAPLT | 101 |
| E8P5 | GFRFGTYS | 103 | ISSGSSSI | 98 | ARGWTGYDAFDI | 99 | QVISNY | 100 | AAS | QKYNNAPLT | 101 |
| C11P7 | GFSLSNSE | 109 | VRSRANRHAT | 110 | TGVRGVISRYFAMDV | 106 | SSDVGAYNY | 111 | DVS | CSYAHRGTPL | 112 |
| F2P7 | GFSSSGSE | 113 | VRSTANRHAP | 114 | TGVRGVISRYFAMDV | 106 | NSDLGSYNY | 115 | DVT | CSYASVGSPI | 116 |

| | **VH CDR1** | | **VH CDR2** | | **VH CDR3** | | **VL CDR1** | | **VL CDR2** | **VL CDR3** | |
|---|---|---|---|---|---|---|---|---|---|---|---|
| **Name** | **Sequence** | **SEQ ID NO :** | **Sequence** | **SEQ ID NO :** | **Sequence** | **SEQ ID NO:** | **Sequence** | **SEQ ID NO:** | **Sequence** | **Sequence** | **SEQ ID NO:** |
| C6P8 | GFTFSGSE | 117 | VRSRANRHAT | 110 | TGVRGVISRYFAMDV | 106 | NIGSNS | 118 | DDS | QVWDRRGDLLV | 119 |
| G8P7 | GGYISTSSYY | 125 | IEYSGST | 126 | ASANSKYVDSGGYYAPGALDL | 127 | QSVSSH | 128 | AAS | QQYNNWPPGYS | 129 |
| D9P6 | GTYISTNSYY | 130 | IEYGGST | 131 | ASANSHYYDSGGYCAPSALDI | 132 | QSASSH | 133 | AAS | QQYNNWPPGT | 134 |
| E10P5 | GGSISSGNYY | 135 | IYTSGNT | 136 | ARVRRDGYNAFDY | 137 | NLGDKF | 138 | QDS | QAWDSDTLYV | 139 |
| G8P6 | GGSMTSGSYY | 140 | IYTSGRT | 141 | ARWGDSSAYYYVGDP | 142 | QNINTY | 143 | AAS | QQSFSLPHT | 144 |
| C6P7 | GGSMTSGSYY | 140 | IYTSGRT | 141 | ARWGDSSAYYYVGDP | 142 | QNINTY | 143 | AAS | QQSFSLPHT | 144 |
| G7P7 | GGSMTSGSYY | 140 | IYTSGRT | 141 | ARWGDSSAYYYVGDP | 142 | QNINTY | 143 | AAS | QQSFSLPHT | 144 |
| C10P7 | GETYVGHS | 145 | ISLHNGNV | 146 | ARVPMKIYGVIVFGEYYYDQLDV | 147 | GSDVGGYNY | 148 | EVS | SSYAGSKW | 149 |
| F3P7 | GYTFTSYS | 150 | INAGNGDT | 151 | ARSNSPTVTYNYYYYAMDV | 152 | QSVRSSY | 153 | DAS | QQYGNSPLT | 154 |
| B5P4 | GGRFSRYG | 155 | IIPLLGTA | 156 | ATARREYDRPGAEYFIQ | 157 | QSVSTN | 158 | DAF | QQYNYWPPLT | 159 |
| D11P7 | GFTFRSYT | 6 | ISGSSNHK | 7 | AREDTIILTPLYGMDV | 3 | SSDVGAYNY | 111 | DVS | CSYAHRGTPL | 112 |
| E10P2 | GFTFRSYA | 160 | LSSSGTTT | 161 | AKDRNAQWLGSEPLDP | 162 | QSVSSSY | 65 | GAS | QQAYTFPWT | 163 |
| C9P1 | GFTFSSYS | 164 | ISSSSRTI | 165 | ARQAVAHFDY | 166 | QRVSSNY | 95 | GAS | QQYGTSPRS | 96 |
| B10P5 | GFTFSTYT | 167 | ISSRSTSI | 168 | VRDRGYYGSNWFDS | 169 | SSDVGNYNY | 170 | DVT | CAYAGSYTLV | 171 |
| C10P8 | GGSISSGRYY | 172 | VYFSGST | 173 | ARAVRDYGDSYSFDY | 174 | SSDIGGYNY | 175 | DVV | CSYSIRGTPV | 176 |

**Table 6. Variable domains of the antibodies, or antigen-binding fragments, according to the invention (the sequences of the CDRs are underlined)**

| | **VH** | | **VL** | |
|---|---|---|---|---|
| **Name** | **Sequence** | **SEQ ID NO :** | **Sequence** | **SEQ ID NO :** |
| B9P6 | EVQLVESGGGLVQPGGSLRLSCAASGFTFRSYTMDWVRQAPGKGLEWV SCISGSSNHKYYADSVRGRFTISRDNAKNSLFLQMNSLRAEDSAVYYCAR EDDILTPLYGMDVWGPGTTVTVSS | 177 | EIVLTQSPDTLSLSPGARATLSCRASQSVRSNYLAWYQQKPGQAPRLLIYG ASSRATAIPDRFSGSGSGTDFTLTITRLEPEDFAVYYCHQYGSSPRTFGQG TKVEIK | 178 |
| E9P7 | EVQLVESGGGLVQPGGSLRLSCTASGFTFRSYTMDWVRQAPGKGLEWV SCISGSSNHKYYADSVRGRFTISRDNAKNSLLLQMNSLRAEDSAVYYCAR EDDILTPLYGMDVWGPGTTVTVSS | 179 | EIVLTQSPDTLSLSPGARATLSCRASQSVRSNYLAWYQQKPGQAPRLLIYG ASSRATAIPDRFSGSGSGTDFTLTITRLEPEDFAVYYCHQYGSSPRTFGQG TKVEIK | 180 |
| G11P1 | EVQLVESGGGLVQPGGSLRLSCAASGFTFRSYSMDWVRQAPGKGLEWV SYISGSSNQKYYTDSVRGRFTISRDNAKNSLFLQMNSLRAEDSAVYYCAR EDDILTPLYGMDVWGPGTTVTVSS | 181 | EIVLTQSPDTLSLSPGARATLSCRASQSVRSNYLAWYQQKPGQAPRLLIYG ASSRATAIPDRFSGSGSGTDFTLTITRLEPEDFAVYYCHQYASSPRTFGQG TKVEIK | 182 |
| E11P6 | EVQLVESGGGLVQPGGSLRLSCAASGFTFRSYSMDWVRQAPGKGLEWV SYISGSSNQKYYTDSVRGRFTISRDNAKNSLFLQMNSLRAEDSAVYYCAR EDDILTPLYGMDVWGPGTTVTVSS | 183 | EIVLTQSPDTLSLSPGARATLSCRASQSVRSNYLAWYQQKPGQAPRLLIYG ASSRATAIPDRFSGSGSGTDLTLTITRLEPEDFAVYYCHQYASSPRTFGQG TKVEIK | 184 |
| F11P5 | QVQLVQSGSELKKPGASVKVSCKASAYTFTNSAINWVRQAPGQGLEWMG WINTNTGNPAYAQGFRGRFVFSLDTSVSEAYLQISNLKAEDTATYYCTVPY NFGGDHNSYYFFGVDIWGQGTTVTVSS | 185 | QSALTQPASVSGSPGQSITISCTGSSSDVGSYNLVSWFQQHPAKAPKVMI YEGTKRPSGVSDRFSGSKSGNTASLTISGLQAEDEADYYCCSYAGSNTFV FGTGTKVSVL | 186 |
| D3P7 | QVQLVQSGSELKKPGASVRVSCKASAYTFTNSAINWVRQAPGQGLEWTG WINTNTGNPAYAQGFRGRFVFSLDTSVSEAYLQITNLKAEDTATYYCAVPY NFGGDHNSFHFFGMDVWGQGTTVTVSS | 187 | QSALTQPASVSGSPGQSITISCTGSSSDVGTYNFVSWYQQQPAKAPKVMI YEGTKRPSGISDRFSGSKSGNTASLTISGLQAEDEADYYCSSYAGGNAFV FGTGTRVSVL | 188 |
| C11P1 | QVQLQESGPGLVKPSETLSLTCTVSGGSIRNSYWSWIRQPPGKGLEWIGY IYYTGITTNSNPSLKSRATISVHMSQNQISLKLNSVTPADTAVYYCAQGGGS GDLHYWGQGTLVTVSS | 189 | NFMLTQPASVSGSPGQSITISCTGTRSDVGGYNYVSWYQQHPGKAPKLMI YDISSRPSGVSNRFSASKSGNTASLTISGLQAEDEADYYCISFATGYTWVF GGGTRLTVL | 190 |
| G5P1 | QVQLQESGPGLVKPSGTLSLTCTVSGGSIRNSYWSWIRQPPGKGLEWIGY IYYTGTTNSNPSLKSRVTISVHMSRNQISLKLNSVTPADTAVYYCAQGGGS GDLHYWGQGTLVTVSS | 191 | NFMLTQPASVSGSPGQSITISCTGTRSDVGGYNYVSWYQQHPGKAPKLMI YDISSRPSGVSNRFSASKSGNTASLTISGLQAEDEADYYCISFATGYTWVF GGGTRLTVL | 192 |
| E7P5 | QVQLQESGPGLVKPSETLSLTCTVSGGSIRNSYWSWIRQSPGKGLEWIGY IYYTGSTNSNPSLKSRLTVSVHMSQNQISLKLNSVTPADTAVYYCAQGGGS GDLHYWGQGTLVTVSS | 193 | QSALTQPASVSGSPGQSITISCTGTRSDVGGYNYVSWYQQHPGKAPKLIIY DISSRPSGVSNRFSASKSGNTASLTISGLQAEDEADYYCISFATGYTWVFG GGTSLTVL | 194 |
| B2P8 | QVQLQESGPGLVKPSETLSLTCTVSGGSIRNSYWSWIRQPPGKGLEWIGY IYYTGTTNSNPSLKSRVTISVHMSQNQISLKLNSVTPADTAVYYCAQGGGS GDLHYWGQGTLVTVSS | 195 | EIVLTQSPDTLSLSPGARATLSCRASQSVRSNYLAWYQQKPGQAPRLLIYG ASSRATAIPDRFSGSGSGTDFTLTITRLEPEDFAVYYCHQYASSPRTFGQG TKVEIK | 196 |
| B8P7 | QVQLQESGPGLVRPSETLSLTCTVSGGSISPYYWNWIRQPPGKGLEWIGY IYYSGNTKYNPSLKSRVTISVDTSKNQFSLKLSSVSAADTAVYYCARDSCS GTCYLGDNWFDPWGQGTLVTVSS | 197 | QSVLTQPPSVSGAPGQRVTISCTGSSSNIGANNDVHWYQHLPGTAPKLLIY GNNHRPSGVPDRFSGSKSGTSASLAITGLQAEDEADYYCQSYDSSLSTSK VFGGGTKLTVL | 198 |
| G6P7 | QVQLQESGPGLVRPSETLSLTCTVSGGSISPYYWNWIRQPPGKGLEWIGY IYYSGNTKYNPSLKSRVTISVDTSKNQFSLKLSSVSAADTAVYYCARDSCS GTCYLGDNWFDPWGQGTLVTVSS | 199 | QSVLTQPPPVSGAPGQRVTISCTGSSSNIGANNDVHWYQHLPGTAPKLLIY GNNHRPSGVPDRFSGSKSGTSASLAITGLQAEDEADYYCQSYDSSLSTSK VFGGGTKLTVL | 200 |
| C4P6 | QVQLQESGPGLVRPSETLSLTCTVSGGSISPYYWNWIRQPPGKGLEWIGY IYYSGNTKYNPSLKSRVTISVDTSKNQFSLKLSSVSAADTAVYYCARDSCS GTCYLGGNWFDPWGQGTLVTVSS | 201 | QSVLTQPPSVSGAPGQRVTISCTGSSSNIGANNDVHWYQHLPGTAPKLLIY GNNHRPSGVPDRFSGSKSGTSASLAITGLQAEDEADYYCQSYDSSLSTSK VFGGGTKLTVL | 202 |
| B3P3 | EVQLVESGGGVVQPGRSLRLSCVTSGFTFESYGIHWVRQAPGKGLEWVA VISNDGSIEDYAGSVKGRFTISGDKAKNTVYLQVNSLRPDDTAVYYCAKDA VGITGGSLYYSYGMDVWGQGTTVTVSS | 203 | DIQMTQSPSPLSAFIGGRVTITCRASQTIKNYLNWYQQKPGEAPKLLIYTAS TLQNGVPSRFSGSRSGTDFTLTITSLQPEDFATYYCQQSYSAPYSFGQGT KLEIK | 204 |
| E2P6 | EVQLVESGGGLIQPGGSLRLSCAASGFTVSTNCMSWVRQAPGTGLEWVS VIYSGGTTYYADSVKGRFTISRDNSRNTLYLQMNGLRAEDTAVYYCARSV DTYDIYALDVWGQGTTVTVSS | 205 | DIQMTQSPSTLSASVGDRVTITCRASQSISRWLAWYQQKPGKAPKLLIYKA SGLHSGAPSRFSGSGSGTEFTLTISGLQPDDFATYFCQQYNSYPWTFGQ GTKVEIK | 206 |
| E6P5 | EVQLVESGGGLVQPGRSLRLSCSASGFTFDDHAMHWVRQAPGKGLEWV AGINWNSASIVYADSVKGRFTISRDNVKNSLYLQMNSLRADDTALYYCAKD FGLWFGESRGIDAWGRGTTVTVSS | 207 | DIVMTQSPLSLPVTPGEPASISCRPSQSLLHSNGYNYLNWYLQKPGRSPQ LLIYLGSNRASGVPDRFSGSGSGTDFTLKISRVEAEDVGIYYCMQALQTPIT FGQGTRLEIK | 208 |
| | | | | |
| E4P2 | QVQLQESGPGLVKPSQTLSLTCTVSGGSISSGGYYWSWIRQHPGKGLEWI GYMYYSGSTNYNPSLKSRVTISVDTSKNQFSLKLTSVTAADTAVYYCARD RRDYGDYYYFGMDVWGQGTTVTVSS | 209 | QSVLTQPPSVSGAPGQRVTISCTGSSSNIGAGYDVHWYQQLPGTAPKLLI YANSNRPSGVPDRFSGSKSGTSASLAITGLQAEDEANYYCQSYDSSLSVY VVFGGGTKLTVL | 210 |
| B7P6 | EVQLVQSGAELKKPGESLKISCKGSGFTFTNYWIAWVRQMPGKGLEWMG IIYPDDSDARYSPSFEGQVTISVDKSISTAYLQWSSLKASDTAIYYCARGGF CSGSVCYNPAWFDLWGQGTLVTVSS | 211 | DIQMTQSPSSLSASVGDRVTITCRASQTINTYLNWYLQKPGKAPKLLISDAS SLETGVPSRFSGSGSGTDFTLTISSLQPDDFATYYCQQSYSTPLTFGGGTK VEIK | 212 |
| F3P6 | QVQLVQSGAEVKKPGASVKVSCKASGYTFTTHIIHWVRQAPGQRLEWMG WINAGNGNTHYSEKFQGRVTLTRDTSASTAYMELSSLRSEDTAIYYCARE DQWLGRSPLEYWGQGTLVTVSS | 213 | EIVLTQSPGTLSLSPGERATLSCRASQSVSSSYLAWYQHKPGQAPRLLIYG ASSRATGIPDRFSGSGSGTDFTLTISRLEPEDFAVYYCQQYGNSYPWTFG QGTKVEIK | 214 |
| G5P6 | QVQLVQSGAEVKKPGASVKVSCKASGYTFTTHIIHWVRQAPGQRLEWMG WINAGNGNTKYSEKFQGRVTLTRDTSASTAYMELSSLRSEDTAIYYCARE DQWLGRSPLEYWGQGTLVTVSS | 215 | EIVLTQSPGTLSLSPGERATLSCRASQSVSSSYLAWYQHKPGQAPRLLIYG ASSRATGIPDRFSGSGSGTDFTLTVSRLEPEDFAVYYCQQYGNSYPWTFG QGTKVEIK | 216 |
| B10P1 | EVQLSESGGGLVQPGGSLRLSCAASGFTFSSYAMSWVRQAPGKGLEWV SSLTGSGGSTYYRDSVKGRFTIFRDNSKKTLYMQMNSLRAEDTAVYYCAK EGAYSYDFWYFDLWGRGTLVTVSS | 217 | EIVLTQSPGTLSLSPGERATLSCRASQTVSGNYLAWYQQKPGQSPSLLIYG ASSRATGIPDRFTGSGSGTDFTLTISRLEPADFAVYYCQQYGRPLTFGGGT KVDIK | 218 |
| G9P2 | EVQLLESGGGLVQPGGSLRLSCAASGFTFSSYAMSWVRQAPGKGLEWV SSLTGSGGSTYYRDSVKGRFTIFRDNSKKTLYMQMNSLRAEDTAVYYCAK EGAYSYDFWYFDLWGRGTLVTVSS | 219 | EIVLTQSPGTLSLSPGERATLSCRASQTVSGNYLAWYQQKPGQSPSLLTY GASSRATGIPDRFTGSGSGTDFTLTISRLEPADFAVYYCQQYGRPLTFGG GTKVEIK | 220 |
| C4P7 | EVQLLESGGGLVQPGGSLRLSCAASGFTFSSYAMSWVRQAPGKGLEWV SSLTGSGGSTYYRDSVKGRFTIFRDNSKKTLYMQMNSLRAEDTAVYYCAK EGAYSYDFWYFDLWGRGTLVTVSS | 221 | EIVLTQSPGTLSLSPGERATLSCRASQTVSGNYLAWYQQKPGQSPSLLIYG ASSRATGIPDRFTGSGSGTDFTLTISRLEPADFAVYYCQQYGRPLTFGGGT KVEIK | 222 |
| D7P6 | EVQLLESGGGLVQPGGSLRLSCAASGFPFSSYAMSWVRQAPGKGLEWV SGITGSASGTYYADSVKGRFTISRDNSKNTLYLRMNSLRAEDTAVYYCAKE GAYSYDYWYFDLWGRGTLVTVSS | 223 | EIVLTQSPGTLSLSPGERATLSCRASQSVSGSYLAWYQQKPGQTPRLLIYG ASSRATGIPDRFSGSGSGTDFTLTINRLDPEDFGVYYCQQYGSLVTFGGG SKVEIK | 224 |
| B7P3 | EVQLVESGGGVVQPGRSLRLSCAASGFTFSNFGMHWVRQPPGKGLEWV AVISYDGSDKFYADSVKGRFTISRDNFKNTLYVQMNSLRAEDTAVYYCAKD RVRTKYYYDHSGPFDYWGRGTLVTVSS | 225 | SYVLTQPPSVSVAPGQTARIACGGNNIGSKSVHWYQQRPGQAPVLVVYD DDDRPSGIPERFSGSNSGNTATLTISRVEAGDEADYFCQVWMTNSDRPVF GGGTKLTVL | 226 |
| F4P5 | EVQLVESGGGVVQPGRSLRLSCAASGFTFSNFGMHWVRQPPGKGLEWV AVISYDGSDKFYADSVKGRFTISRDNFKNTLYVQMNSLRAEDTAVYYCAKD RVRTKYYYDHSGPFDYWGRGTLVTVSS | 227 | SYELTQPPSVSVAPGQTARIACGGNNIGSKSVHWYQQRPGQAPVLVVYD DDDRPSGIPERFSGSNSGNTATLTISRVEAGDEADYFCQVWMTNSDRPVF GGGTKLTVL | 228 |
| B9P4 | EVQLVESGGGVVQPGRSLRLSCAASGFTFSSYGMHWVRQAPGKGLEWV AVISKDGSNKYEADFVKGRFTISRDNSKNTLYLQMNSLRAEDTAVYYCAKI PLRRYSDSGDYSSGDFWGQGTLVTVSS | 229 | DVVMTQSPLSLTVTLGQPASISCRSSENLVYSDGNTYLSWFQQRPGQSPR RLIYKVSTRDSGVPDRFSGSGSGTDFTLKISRVEAEDVGVYYCMQTTHWP PLTFGGGTKVEIK | 230 |
| E5P6 | EVQLVESGGGVVQPGRSLRLSCAASGFIFNNHGMHWVRQAPGKGLEWV AGISYDGKKEYYIGSVKGRFTISRDNSRNTLYLHMNGLRPEDTAVYYCAKE AYGSGSPCDYWGQGTLVTVSS | 231 | EIVLTQSPGTLSLSPGERATLSCRASQRVSSNYLAWYQQKPGQAPRLLIYG ASSRATGIPDRFSGSGSGTDFTLTISRLEPEDFAVYYCQQYGTSPRSFGR GTKLEIK | 232 |
| G7P3 | EVQLVESGGGLVQPGGSLRLSCAASGFSFGSYSMNWVRQAPGKGLEWV SHISSGSSSIYYADSVKGRFTISRDSVKNSLYLQMNNLRAEDTAVYYCARG WTGYDAFDIWGQGTMVTVSS | 233 | DIQMTQSPSSLSASVGDRVTITCRASQVISNYLAWYQEKPGKVPKLLIYAA STLQSGVPSRFSGSGTGTDFTLTIRSLQPEDVATYYCQKYNNAPLTFGGG TKVEIK | 234 |
| E8P5 | EVQLVESGGGLVHPGGSLRLSCAASGFRFGTYSMNWVRQAPGKGLEWV SHISSGSSSIYYADSVKGRFTISRDSVKNSLYLQMNNLRAEDTAVYFCARG WTGYDAFDIWGQGTMVTVSS | 235 | DIQMTQSPSSLSASVGDRVTITCRASQVISNYLAWYQEKPGEVPKLLIYAA STLQSGVPSRFSGSGTGTDFTLTISSLQPEDVATYYCQKYNNAPLTFGGG TKVEIK | 236 |
| C11P7 | EVQLVESGGGLVQPGGSLKLSCAASGFSLSNSEVHWVRQASGKGLEWIG RVRSRANRHATGYTESVNGRFTVSRDDSKNTASLQLESLKIEDTAVYYCT GVRGVISRYFAMDVWGQGTTVTVSS | 237 | QSALTQPASVSGSPGQSITISCTGTSSDVGAYNYVSWYRLRPGKDPHLIIY DVSHRPAGVSNRFSGSKSGNTASLTISGLQAEDEDEYYCCSYAHRGTPLF GGGTKLTVL | 238 |
| F2P7 | EVQLVQSGGGLVQPGGSLKLSCAASGFSSSGSEIHWVRQASGRGLEWIG RVRSTANRHAPGYTESVRGTFTVSRDESKNTASLELKSLKIEDTAVYYCTG VRGVISRYFAMDVWGQGTTVTVSS | 236 | QSALTQPASVSGSPGQSITISCTGTNSDLGSYNYVSWYRLHPGKDPHLIIY DVTHRPSGVSSRFSGSKSGKTASLTISGLQSEDEAEYFCCSYASVGSPIFG GGTKVTVL | 240 |
| C6P8 | EVQLVESGGGLVQPGGSLKLSCAASGFTFSGSEIHWVRQASGKGLEWIG RVRSRANRHATGYSESVKGRFTISRDDSKNTASLQLNSLKIEDTAVYYCTG VRGVISRYFAMDVWGQGTTVTVSS | 241 | SYVLTQTPSVSVAPGQTARITCGGNNIGSNSVHWYQQKPGQAPILVVYDD SERPSGIPERFSGSNSGNTATLTISRVEGGDEADYQCQVWDRRGDLLVFG GGTKLTVL | 242 |
| C6P8 | EVQLVESGGGLVQPGGSLKLSCAASGFTFSGSEIHWVRQASGKGLEWIG RVRSRANRHATGYSESVKGRFTISRDDSKNTASLQLNSLKIEDTAVYYCTG VRGVISRYFAMDVWGQGTTVTVSS | 241 | SYVLTQTPSVSVAPGQTARITCGGNNIGSNSVHWYQQKPGQAPILVVYDD SERPSGIPERFSGSNSGNTATLTISRVEGGDEADYQCQVWDRRGDLLVFG GGTKLTVL | 242 |
| G8P7 | QLQLQESGPGLVQPSETLSLTCTVSGGYISTSSYYWGWIRQPPGKGLEWI GSIEYSGSTYYNPSLKSRVTISVDTSKNQFSLKLSSVTAADTAVYYCASAN SKYYDSGGYYAPGALDLWGQGTMVTVSS | 243 | EIVMTQSPATLSVSPGERATLSCRASQSVSSHLAWYQHKRGQAPRLLIYA ASSRATGIPARFSGRGSGTEFTLTISSLQSEDFAVYYCQQYNNWPPGYSF GQGTKLEIK | 244 |
| D9P6 | QLQLQESGPGLVKPSETLSLTCTVSGTYISTNSYYWGWIRQPPGKGLEWI GSIEYGGSTYYNPSLRSRLTISVDTSKNHFSLRLSSVTAADTAMYYCASAN SHYYDSGGYCAPSALDIWGQGTMVTVSS | 245 | EIVMTQSPATLSVSPGERVTLSCRASQSASSHLAWYQHKRGQAPRLLIYA ASSRATGIPARFSGRGSGTEFTLTISSLQSEDFAVYYCQQYNNWPPGYSF GQGTKLEIK | 246 |
| E10P5 | QVQLQESGPGLVKPSQTLSLTCSVSGGSISSGNYYWSWIRQPAGKGLEWI GRIYTSGNTNYNPSLKSRVTMSVDTSKNQFSLKLSSVTATDTAVYYCARV RRDGYNAFDYWGQGTLVTVSS | 247 | SYELTQPPSVSVSPGQTASIDCSGHNLGDKFTCWYQQKPGQSPVLVIYQD SKRPSGIPERFSGSNSGNTATLTISGTQAMDEADYYCQAWDSDTLYVFGT GTTVTVL | 248 |
| G8P6 | QVQLQESGPGLVRPSQTLSLTCTVSGGSMTSGSYYWSWIRQPAEKGLEW IGRIYTSGRTDYNPSLKSRVAMSIDTSKNQFSLRLTSVTAADTAVYYCARW GDSSAYYYVGDPWGRGTTVTVSS | 249 | DIQMTQSPSSLSASIGDRVTITCRASQNINTYLYWYQQKPGKAPQLLIYAAS NLQTGAPSRFSGSGSGTDFTLTISSLQPADFATYYCQQSFSLPHTFGQGT KLEIK | 250 |
| C6P7 | QVQLQESGPGLVRPSQTLSLTCTVSGGSMTSGSYYWSWIRQPAEKGLEW IGRIYTSGRTDYNPSLKSRVAMSIDTSKNQFSLRLTSVTAADTAVYYCARW GDSSAYYYVGDPWGRGTPVTVSS | 251 | DIQMTQSPSSLSASIGDRVTITCRASQNINTYLWYQQKPGKAPQLLIYAAS NLQTGAPSRFSGSGSGTDFTLTISSLQPADFATYYCQQSFSLPHTFGQGT KVEIK | 252 |
| G7P7 | QVQLQESGPGLVRPSQTLSLTCTVSGGSMTSGSYYWSWIRQPAEKGLEW IGRIYTSGRTDYNPSLKSRVAMSIDTSKNQFSLRLTSVTAADTAVYYCARW GDSSAYYYVGDPWGRGTPVTVSS | 253 | DIQMTQSPSSLSASIGDRVTITCRASQNINTYLWYQQKPGKAPQLLIYAAS NLQTGAPSRFSGSGSGTDFTLTISSLQPADFATYYCQQSFSLPHTFGQGT KLEIK | 254 |
| C10P7 | QVQLVQSGSEVKKPGTSVKVSCKASGETYVGHSISWVRQAPGAGLEWM GWISLHNGNVKYAQKLQGRVTMTTDTSTITAYMELRNLTSDDTAVYYCAR VPMKIYGVIVFGEYYYDQLDVWGQGTTVTVSS | 255 | QSALTQPPSASGSPGQSVTISCTGTGSDVGGYNYVSWYQQHPGKAPKLM IYEVSKRPSGVPDRFSGSKSGNTASLTVSGLQAEDEADYYCSSYAGSKVV FGGGTKLTVL | 256 |
| F3P7 | QVQLVQSGAEVKKPGASVKVSCKVSGYTFTSYSMHWLRQAPGQRLEWM GWINAGNGDTKYSQKFQGRITISRDTSARTTYMELSSLRSEDTAVFYCARS NSPTVTYNYYYYAMDVWGQGTTVTVSS | 257 | EIVLTQSPGTLSLSPGERATLSCRASQSVRSSYLAWYQQQAGQAPRLLIYD ASSRATGIPDRFSGSGSGTDFSLTISRLDPEDFAVYYCQQYGNSPLTFGG GTKVEIK | 258 |
| B5P4 | QVQLVQSGAEVKKPGSSVKVSCKASGGRFSRYGSSWVRQAPGQGLEW MGGIIPLLGTANYAQKFQDRVSITADESTSTVYMHLSSLRSEDTAVYYCAT ARREYDRPGAEYFIQWGQGTLVTVSS | 259 | EIVMTQSPATLSVSPGERATLSCRASQSVSTNLAWYQQKPGQAPRLLIYD AFLRATGIPARFSGSGSGTEFTLTISSLQPEDFAVYYCQQYNYWPPLTFGG GTKVEIK | 260 |
| D11P7 | EVQLVESGGGLVQPGGSLRLSCAASGFTFRSYTMDWVRQAPGKGLEWV SCISGSSNHKYYADSVRGRFTISRDNAKNSLFLQMNSLRAEDSAVYYCAR EDDILTPLYGMDVWGPGTTVTVSS | 261 | QSALTQPASVSGSPGQSITISCTGTSSDVGAYNYVSWYRLRPGKDPHLIIY DVSHRPAGVSNRFSGSKSGNTASLTISGLQAEDEDEYYCCSYAHRGTPLF GGGTKLTVL S | 262 |
| E10P2 | EVQLLESGGALVQPGGSLRLSCAASGFTFRSYAMNWVRQAPGKGLEWV SSLSSSGTTTYYADSVKGRFTISRDNSKNTMYLQMDSLRGEDTAVYYCAK DRNAQWLGSEPLDPWGQGTLVTVSS | 263 | EIVLTQSPGTLSLSPGERATLSCRASQSVSSSYLAWYQQKPGQAPRLLIYG ASSRATGIPDRFSGSGSGTDFTLTISSLQPDDFAIYYCQQAYTFPWTFGQG TKLEIK | 264 |
| C9P1 | EVQLLESGGGLVQPGGSLRLSCVTSGFTFSSYSMTWVRQAPGKGLEWVS YISSSSRTISYADSVQGRFTISRDNAKNVLYLQMNSLRAEDTAVYYCARQA VAHFDYWGQGTLVTVSS | 265 | EIVLTQSPGTLSLSPGERATLSCRASQRVSSNYLAWYQQKPGQAPRLLIYG ASSRATGIPDRFSGSGSGTDFTLTISRLEPEDFAVYYCQQYGTSPRSFGQ GTKVEIK | 266 |
| B10P5 | EVQLVESGGGLVQPGGSLRLSCSASGFTFSTYTMNWVRQAPGKGLDWV SYISSRSTSIYYADSVKGRFTISRDNAKNSLYLHMTSLRAEDTAVYYCVRD RGYYGSNWFDSWGQGTLVTVSS | 267 | QSALTQPRSVSGSPGQSVTISCTGTSSDVGNYNYVSWYQQHPGKAPRLM ISDVTKRPSGVPDRFSGSKSGNTASLTISGLQAEDEGDYYCCAYAGSYTLV FGGGTKLTVL | 268 |
| C10P8 | QVQLQESGPGLVKPSQTLSLTCTVSGGSISSGRYYWSWIRQPAGKGLEWI GRVYFSGSTNYNPSLKNRVTLSVDTSKNQFSLKLSSVIAADTAVYYCARAV RDYGDSYSFDYWGQGTLVTVSS | 269 | QSALTQPASVSGSPGQSITISCTGSSSDIGGYNYVSWYQVHPGKAPHLKIY DVVNRPSGVSNRFPGSKSGNTASLTISGLLADDEAYYYCCSYSIRGTPVFG GGTKLTVL | 270 |

### Competing antibodies and epitopes

In a further aspect, the invention provides an antibody, or antigen-binding fragment thereof, that binds and optionally neutralizes a plurality of BK virus subtypes including at least subtype I, II and IV, wherein said antibody (1) competes for binding to a BKV viral protein 1 (VP1), with a reference antibody of the invention as described above, such as antibodies E11P6 (VH: SEQ ID NO: 183; VL: SEQ ID NO: 184), G7P7 (VH: SEQ ID NO: 253; VL: SEQ ID NO: 254) and/or E6P5 (VH: SEQ ID NO: 207; VL: SEQ ID NO: 206).
Any BKV viral protein 1 (VP1) as described in above Table 1 may be used herein. A particularly preferred BKV viral protein 1 (VP1) is of SEQ ID NO: 271.

The competition for binding is herein preferably determined in a competition ELISA binding assay, also called cross-competition ELISA. Briefly, for such assay, ELISA plates can be pre-coated with VP1 protein (e.g. SEQ ID NO: 271), which may be in the form of VP1 pentamers and incubated with a labelled reference antibody (at a fixed concentration; the label may be biotin) in serially diluted solutions (e.g. three-fold dilution; preferably at a starting concentration of 100µg/mL) of an unlabeled antibody competitor in a suitable buffer (e.g. PBS). Antibody incubation step can then be performed, preferably for about 1h.

In a preferred embodiment, the antibody (1) inhibits at least 30%, preferably at least 50% or 60%, more preferably at least 70%, 75%, 80%, 85%, 90% or 95% of binding of at least one of the reference antibodies E11P6 (VH: SEQ ID NO: 183; VL: SEQ ID NO: 184), G8P6 (VH: SEQ ID NO: 249; VL: SEQ ID NO: 250) and/or E6P5 (VH: SEQ ID NO: 207; VL: SEQ ID NO: 206) to a BKV viral protein 1 (VP1) of SEQ ID NO: 271, as preferably determined in a competition ELISA binding assay as described herein.

In another aspect, the invention provides an antibody, or antigen-binding fragment thereof, that binds and optionally neutralizes a plurality of BK virus subtypes including at least subtype I, II and IV, wherein said antibody (2) binds to an epitope comprising one or more, preferably a plurality of, amino acids selected from D60, E61, N62, L68, K69, A72, E73, N74, D75, S77, S80, S133, K135, Y169, R170, K172, N273, S275 and/or T277 in a BKV viral protein 1 (VP1) of SEQ ID NO: 271.

It will be readily understood that any other BKV viral protein 1 (VP1) as described in above Table 1 may be used herein.

The epitope is herein preferably determined by Cryo-Electomicroscopy, preferably at a contact distance resolution ranging from 0 to 6 angström. Briefly, for such assay, the VP1 protein, which may be in the form of VP1 pentamers or VLPs, is incubated with the antibody, or antigen-binding fragment thereof, at an optimized ratio (e.g. two-fold molar ratio) to allow the formation of a stable complex. The complex is applied to an EM grid, which is then plunged into a cryogen. Automated image acquisition can be performed on a cryo-EM microscope equipped accordingly, and images processed to reconstruct 3D density map using a suitable algorithm. Atomic models of VP1 and antibody can be fitted into the 3D density map, to analyze the VP1 residues contacted by the antibody or antigen-binding fragment. A local refinement may be performed to improve resolution at the epitope interface. A more detailed protocol is provided in the below Examples, section 1.12.

In a preferred embodiment, the epitope of antibody (2) comprises one or more, preferably a plurality of, amino acids selected from D60, L68, A72, N74, S80, R170 and/or S275 in BKV viral protein 1 (VP1) of SEQ ID NO: 271, as preferably determined by Cryo-Electomicroscopy (cryo-EM) as described herein.

In another preferred embodiment, the epitope of antibody (2) comprises one or more, preferably a plurality of, amino acids selected from E61, N62, L68, K69, E73, N74, S77, S133, K135 and/or S275 in BKV viral protein 1 (VP1) of SEQ ID NO: 271, as preferably determined by Cryo-Electomicroscopy (cryo-EM) as described herein.

In another preferred embodiment, the epitope of antibody (2) comprises one or more, preferably a plurality of, amino acids selected from D60, D75, S77, Y169, R170, K172, N273, S275 and/or T277 in BKV viral protein 1 (VP1) of SEQ ID NO: 271, as preferably determined by Cryo-Electomicroscopy (cryo-EM) as described herein.

Binding to VP1 can be further analyzed in an ELISA binding assay, in which for example, multiple serial dilutions of those antibodies can be tested in ELISA plates precoated with VP1.

In a further aspect, the invention provides an antibody, or antigen-binding fragment thereof, that binds and optionally neutralizes a plurality of BK virus subtypes including at least subtype I, II and IV, wherein said antibody (3) has reduced binding or loses binding to a mutant BKV viral protein 1 (VP1), said mutant containing one or more, preferably a plurality of, of the following amino acid mutations in SEQ ID NO: 271: D60A, E61A, N62A, L68A, K69A, A72A, E73A, N74A, D75A, S77A, S80A, S133A, K135A, Y169A, R170A, K172A, N273A, S275A and/or T277A.

It will be readily understood that any other BKV viral protein 1 (VP1) as described in above Table 1 may be used herein.

Binding to VP1 mutants is herein preferably determined in an ELISA binding assay, in which for example, multiple serial dilutions of those antibodies can be tested in ELISA plates precoated with VP1 mutants and the naturally-occurring VP1. Briefly, for such assay, wells of ELISA plates can be pre-coated with either VP1 protein (e.g. SEQ ID NO: 271) or mutants thereof, preferably in the form of VP1 pentamers, or VLPs, and incubated with serial dilutions (e.g. threefold dilutions) of antibody (e.g. starting concentration of 50 µg/ml) in a suitable buffer (e.g. PBS). Antibody incubation step can then be performed, typically for about 1h.

In a preferred embodiment, the antibody (3) has a binding to those mutants that is reduced by greater than 30%, preferably greater than 40%, 50%, 60%, more preferably greater than 70%, 75%, 80%, 85%, 90% or 95% relative to the binding between the same antibody and naturally-occurring VP1 (SEQ ID NO: 271).

In a preferred embodiment, the antibody (3) has reduced binding or loses binding to a VP1 mutant containing one or more, preferably a plurality, of the following amino acid mutations in SEQ ID NO: 271: D60A, L68A, A72A, N74A, S80A, R170A and/or S275A, as preferably determined in an ELISA binding assay as described herein.

In another preferred embodiment, the antibody (3) has reduced binding or loses binding to a VP1 mutant containing one or more, preferably a plurality, of the following amino acid mutations in SEQ ID NO: 271: E61A, N62A, L68A, K69A, E73A, N74A, S77A, S133A, K135A and/or S275A, as preferably determined in an ELISA binding assay as described herein.

In another preferred embodiment, the antibody (3) has reduced binding or loses binding to a VP1 mutant containing one or more, preferably a plurality, of the following amino acid mutations in SEQ ID NO: 271: D60A, D75A, S77A, Y169A, R170A, K172A, N273A, S275A and/or T277A, as preferably determined in an ELISA binding assay as described herein.

In a preferred embodiment, the antibody (1), (2) or (3) as described above is a human antibody and/or a monoclonal antibody.

### Constant regions

The antibody constant regions, if present, may be selected in line with the intended use of the antibody, notably in line with the effector function(s) required for that use.

In the context of the present invention, constant regions are preferably those of a human antibody, such as a human IgA, IgD, IgE, IgG, or IgM, preferably a human IgG.

When the constant regions are those of a human IgG, the heavy chain (HC) constant region preferably contains a CH1 domain, a CH2 domain and a CH3 domain; and/or the light chain (LC) constant region contains a CL domain, either kappa or lambda.

Human IgG constant regions may be selected from any hIgG isotype, i.e. hIgG1, hIgG2, hIgG3 or hIgG4, depending on the intended use. These isotypes are indeed known to possess unique sequences and properties, with regard to their half-life and effector functions, the latter ranging from complement-dependent cytotoxicity (CDC), antibody-dependent cellular cytotoxicity (ADCC) to antibody-dependent cell phagocytosis (ADCP). These properties are mediated by the Fc region, i.e. by the CH2 and CH3 domains of the antibody's two heavy chains, and ultimately promote the killing of pathogen-infected cells and tumor cells.

For example, the half-life of hIgG3 (e.g. about 5-7.5 days) is typically much shorter than those of hIgG1, hIgG2, and hIgG4 (e.g. about 14-21 days). As another example, hIgG4 has reduced effector functions compared to hIgG1, hIgG2, and hIgG3. hIgG1 and hIgG4 are are also known to be more prone to proteolytic cleavage than hIgG2.

These difference in properties are essentially due to difference in binding affinity of the Fc region to receptors involved in effector functions, namely the Fcγ receptors (FcyRI, FcyRIIA, FcyRIIC and FcyRIIIA), the neonatal Fc receptor (FcRn), and the complement component 1q (C1q). The Fc region of IgG also typically contains a glycosylation site, which is believed to facilitate interaction with those receptors.

Thus, for a therapeutic use, one would favour using constant regions, especially the Fc region, that is best suited based on each isotype specific properties. In the context of the present invention, it shall be understood that selecting hIgG isotypes with strong effector functions is preferred.

These properties can nevertheless be optimized, by modulating for example antibody serum persistence (higher circulating levels, allowing less frequent administration and/or reduced doses), and/or pathogen clearance. Such enhancements may be achieved by introducing mutations and/or post-translational modifications, more particularly in the Fc region.

Thus, in a preferred embodiment, the antibody or antigen-binding fragment thereof according to the invention further comprises, especially in the Fc region, modifications which modulate Fc/FcRn binding, complement binding, complement-dependent cytotoxicity (CDC), antibody-dependent cellular toxicity (ADCC), antibody-dependent cell phagocytosis (ADCP), glycosylation and/or pharmacokinetics such as half-life.

The term *"modulate"* or *"modulation"* generally means inhibit(ion)/diminish or increase/augment(ation). In a preferred embodiment, modulate means increase.

Fc modification strategies and techniques are well-known in the art (Saunders, Front Immunol.. 2019; 10:1296, see e.g. Tables 1-3; Abdeldaim et al., Pharmaceutics 2023; 15(10): 2402; both reviews being incorporated herein by reference). One can, for example, engineer cross-isotype Fc regions (i.e. merge constant domains from different IgG isotypes to engage a wider range of Fc receptors), or introduce specific mutations in Fc regions to modulate effector functions and/or half-life. Sensitivity to proteolytic cleavage may also be abrogated.

Examples of human IgG engineered Fc regions that are suitable in the present invention are illustrated in Table 7 below.

**Table 7. Engineered human IgG Fc regions. Numbering scheme: EU. For correspondence with IMGT, see the IMGT Scientific chart as provided on the IMGT website (e.g. www.imgt.org/IMGTScientificChart/Numbering/Hu_IGHGnber.html#refs).**

| **Engineered Fc** | **Isotype** | **Mutations ["name" of the mutation]** | **FcR/C1q Binding** | **Effector Function** |
|---|---|---|---|---|
| hIgG1b12-Fc | IgG1 | M418L/N434S ["LS"] | Increased binding to FcRn | Increased half-life |
| hIaG1e1-Fc | IgG1 | T250Q/M428L | Increased binding to FcRn | Increased half-life |
| hIeG1e2-Fc | IgG1 | M252Y/S254T/T256E["YTE"] + H433K/N434F | Increased binding to FcRn | Increased half-life |
| hIeG1e4-Fc | IgG1 | E333A | Increased binding to FcγRIIIa | Increased ADCC and CDC |
| hIgG1e5-Fc | IgG1 | S239D/A330L/I332E ["DLE"] | Increased binding to FcγRIIIa | Increased ADCC |
| hIeG1e6-Fc | IgG1 | P257I/Q311 | Increased binding to FcRn | Unchanged half-life |
| hIeG1e7-Fc | IgG1 | K326W/E333S | Increased binding to C1q | Increased CDC |
| hIgG1e9-Fc | IgG1 | S239D/I332E/G236A ["DEA"] | Increased FcγRIIa/FcγRIIb ratio | Increased macrophage phagocytosis |
| hIgG4e1-Fc | IgG4 | L234A/L235A ["LALA"] | Increased binding to FcRn | Increased half-life |
| hIgG4e2-Fc | IgG4 | S228P + 252Y/S254T/T256E ["YTE"] | - Increased binding to FcRn | Reduced Fab-arm exchange Increased half-life |
| hIgG4e3-Fc | IgG4 | K444A | | Abolition of cleavage motif at the Cter of the antibody (hinge) |
| hIgG1e3-Fc | IgG1 | | | |

Post-translational modification of the Fc region by glycoengineering, chemical or enzymatic treatment may also be implemented. For example, galactosylation of IgG1 Fc region can increase C1q binding and CDC activity, as well as thermostability; this improvement is also applicable to IgG3. The single N-linked glycosylation site at position 297 within IgG1 Fc region can also be modified to improve ADCC activity, via e.g. hypofucosylation or afucosylation.

The type and extent of post-translation modification often depends on the host cell used to express the antibody as well as the culture conditions.

Other strategies conventionally used to extend half-life of protein therapeutics may also be applied herein. These include for example conjugation to polymers (e.g. PEGylation, polysialylation), carbohydrates, or serum albumin, to name a few.

### Antibody production

To produce an antibody, or antigen-binding fragment thereof, a nucleic acid or a group of nucleic acids encoding therefore are typically subcloned into one or more suitable vectors. Such vectors can be transfected into host cells, such as mammalian cells, which can then be cultured under appropriate conditions to produce the desired antibody or antigen-binding fragment thereof. Methods for producing antibodies and their antigen-binding fragments are well-known in the art (see e.g. Current protocols in Immunology, pp 10.19.1-10.19.11, Coligan et al. (eds.), Wiley Interscience, 1992; *"*Antibody engineering: a practical guide " by Borrebaeck Carl, W.H. Freeman and Company, New York, 1992; Immunobiology by Janeway et al., 5th edition, Garland publishing, 2001; Therapeutic Monoclonal Antibodies: From Bench to Clinic by Zhiqiang An, Wiley editions, 2009; Antibodies: A Laboratory Manual, by Harlow et al., Cold Spring Harbor Laboratory, New York, 1988; the relevant excerpts of each the above-mentioned manuals being hereby incorporated by reference in their entirety).

The present invention accordingly extends to a nucleic acid or set of nucleic acids encoding the antibody, or antigen-binding fragment thereof, as described above.

The terms *"nucleic acid*", *"nucleic acid molecule", "polynucleotide"* and *"nucleotide sequence"* are herein equivalent and refer to a polymer of nucleotides. These nucleotides may be natural (namely, A, T, G, C and U) or non-natural, preferably natural. These terms encompass a single-stranded or double-stranded DNA, as well as the transcription product of said DNA, such as an RNA. The nucleic acid is herein preferably a double-stranded DNA.

In a preferred embodiment, a nucleic acid may encode the heavy chain variable domain (VH) of the antibody, and another nucleic acid may encode the light chain variable domain (VL) of said antibody, said domains being as described above. More preferably, said nucleic acids are provided herein as a set of nucleic acids.

In a preferred embodiment, when the antibody contains constant regions, a nucleic acid may encode the heavy chain variable domain (VH) of the antibody, another nucleic acid the heavy chain constant domain (CH, which may comprise CH1, CH2 and CH3 domains) of said antibody, another nucleic acid the light chain variable domain (VL) of said antibody, and another nucleic acid the light chain constant domain (CL) of said antibody, said domains being as described above. More preferably, said nucleic acids are provided herein as a set of nucleic acids.

In another preferred embodiment, when the antibody contains constant regions, a nucleic acid may encode the heavy chain variable domain (VH) and the heavy chain constant domain (CH, which may comprise CH1, CH2 and CH3 domains) of the antibody, and another nucleic acid may encode the light chain variable domain (VL) and the light chain constant domain (CL) of said antibody, said domains being as described above. More preferably, said nucleic acids are provided herein as a set of nucleic acids.

The nucleic acid or set of nucleic acids according to the invention can be prepared by methods well-known in the art, including, but not limited to, any synthetic and/or recombinant method such as *de novo* solid phase DNA synthesis or by PCR mutagenesis of an existing anti-BKV antibody. It is within the skill of the person in the art to design the nucleotide sequence of a nucleic acid so as to allow an efficient production of the antibody, such as by codon-optimization based on the desired expression system (e.g. host cell).

The nucleic acid or set of nucleic acids according to the invention can be comprised in a vector capable of expressing the antibody, or antigen-binding fragment, in a suitable host cell. Said nucleic acids can thus be cloned either in separate vectors, or, in a single vector.

The invention therefore further relates to a vector, preferably an expression vector, which comprises the nucleic acid or set of nucleic acids as disclosed herein.

The term *"vector"* refers herein to a molecule, typically a nucleic acid molecule, used as a vehicle to transfer genetic material, such as a nucleic acid of interest, typically into a cell. Such vectors are very well known in the art, and include vectors capable of replication in order to amplify a nucleic acid of interest (i.e. a cloning vector), and/or to express the polypeptide encoded by said nucleic acid in a host cell (i.e. an expression vector). These types of vectors are publicly available and encompass for example plasmids, cosmids, artificial chromosomes (e.g. YACS, BACS), viral vectors (adenovirus, AAV, retrovirus such as lentivirus, EBV episome, etc.), and phage vectors. A vector is said to be recombinant when it is not found in nature, such as when it includes an heterologous nucleic acid of interest (i.e. it is not naturally-occurring). In general, vectors comprise at least an origin of replication, a multicloning site for incorporating a nucleic acid of interest, and one or more selection markers (e.g. antibiotic resistance), along with a larger sequence that serves as a backbone. Additional features of vectors typically include, without limitation, enhancers, promoters, protein purification tags, and/or a secretory signal peptide sequence (for the polypeptide of interest to be secreted into the culture medium). Should the nucleic acids coding for the heavy and light chains be included into a single vector, their sequences may be separated by a polyadenylation signal, and their transcription be driven by the same or different promoters, preferably by different promoters. The choice of the vector, and its features, will depend on the intended host cells in which it will be introduced.

The invention further pertains to a host cell expressing the antibody, or antigen binding fragment thereof, or comprising the nucleic acid, set of nucleic acids, or vector (or set of vectors) as disclosed herein.

The terms *"host cell", "cell"* and *"cell line"* can be used interchangeably to refer to a prokaryotic or eukaryotic cell in which a nucleic acid or vector can be introduced, such as for amplification of the nucleic acid of interest, and/or to express the polypeptide encoded by said nucleic acid. To this end, a host cell may be "*transfected*" or "*transformed*" by a process known in the art by which said nucleic acid or vector is transferred or introduced into the host cell. Examples of such methods include, without limitation, electroporation, lipofection, calcium phosphate transfection, transfection using DEAE dextran, microinjection, and the like. It should be understood that the term *"host cell"* also encompasses any progeny thereof which retains all the essential features of the parent host cell.

Techniques for inserting a nucleic acid into a vector and/or transferring a nucleic acid or vector into a host cell, are detailed in Molecular Cloning: A Laboratory Manual (4th edition) by Sambrook et al., Cold Spring Harbor Laboratory Press, 2012.

The skilled person will be able to choose the appropriate host cell among the many cell lines that are publicly available, notably among those suited for producing antibodies (see e.g. Yazaki et al. pages 255-268 in *Antibody Engineering: Methods and Protocols* by Benny K. C. Lo, pages 255-268, Humana Press, Totowa, NJ 2004). Eukaryotic cells are herein particularly preferred, and include mammalian cells, plant cells, insect cells or yeast cells. Mammalian cells are herein especially favored, for their capacity to provide suitable post-translational modifications such as glycosylation.

Non-limiting examples of mammalian host cells that can be used according to the invention include, without limitation, Chinese hamster ovary cells (CHO) and derivatives thereof (such as DHFR⁻ CHO cells), baby hamster kidney cells (BHK), human embryonic kidney cells (HEK 293), human embryonic retinal cells (PERC.6), human cervical carcinoma cells from Henrietta Lacks (HeLa), human lung cells (W138), human fetal lung fibroblast cells (MRC-5 cells), human liver cells (Hep G2), human foreskin cells (FSA), human myeloma cells (Y0, also referred as YB2/3.0Ag30), African green monkey kidney cells (Vero, such as VERO-76), monkey kidney cells (CV1) or derivatives thereof (COS cells, COS-7 cells), mouse Sertoli cells (TM4), mouse mammary tumor cells (MMT 060562), murine myeloma cells (NS0, Sp2/0), buffalo rat liver cells (BRL 3A), and canine kidney cells (MDCK).

Particularly preferred mammalian host cells according to the invention are CHO cells, HEK 293 cells, COS cells, or NS0 cells, more preferably CHO cells or HEK 293 cells, even more preferably CHO cells.

The present invention also provides a method for producing the antibody, or antigen-binding fragment thereof, as described above, said method comprising a) culturing the host cell under conditions allowing the expression of said antibody, or antigen-binding fragment thereof, and b) recovering said antibody, or antigen-binding fragment thereof.

In step a), the host cell may be cultured in any appropriate culture medium, which enables the expression of said antibody, or antigen-binding fragment thereof. Such culture media are well-known in the art, and need not be detailed herein.

In step b), the antibody, or antigen-binding fragment thereof, may be recovered from the host cell if said antibody, or antigen-binding fragment thereof, is expressed intracellularly.

Alternatively, in step b), the antibody, or antigen-binding fragment thereof, may be recovered from the culture medium in which the host cell is cultured if said antibody, or antigen-binding fragment thereof, is expressed extracellularly.

The antibody, or antigen-binding fragment thereof, recovered in step b) can advantageously be purified, in a further step of said method, defined as step c). Preferably, said purification step allows the obtention of a 100%-purified or almost 100%-purified antibody, or antigen-binding fragment thereof
The skilled person in the art may use any conventional method known in the art allowing the purification of a polypeptide, such as by chromatography.

### Composition, combination, kit and uses thereof

The invention also relates to a pharmaceutical or diagnostic composition comprising (1) at least one of the antibody, or antigen-binding fragment thereof, as described above, or any combination thereof; and (2) optionally at least one pharmaceutically or diagnostically acceptable excipient.

As used herein, the term a *"acceptable excipient"* means an inactive or inert, and therefore nontoxic compound. A *"pharmaceutically acceptable excipient"* is typically an acceptable excipient of pharmaceutical grade that is devoid of pharmacological action itself, and is accordingly physiologically compatible. A *"diagnostically acceptable excipient"* is typically an acceptable excipient that does not interfere in the signal to be detected in a diagnostic assay. Pharmaceutically or diagnostically acceptable excipients can be used to improve properties of a composition, such as shelf-life, stability, or retention time or consumer acceptance if administered to a subject, etc. Examples of pharmaceutically or diagnostically acceptable excipient include, without limitation, surfactants (cationic, anionic, or neutral); surface stabilizers; other enhancers, such as preservatives, wetting or emulsifying agents; solvents; buffers; salt solutions; dispersion medium; isotonic and absorption delaying agents, and the like.

It will be understood that the pharmaceutically or diagnostically acceptable excipient is preferably present in the composition in an amount effective to preserve the binding and neutralizing activities of the antibody or antigen-binding fragment thereof according to the invention.

The composition according to the invention may be in a solid form (such as in the form if a powder or tablet), or in a liquid form (such as in the form of a solution or aerosol).

The composition according the invention can be used in several industrial applications, for *in vivo* or *in vitro* use, more particularly in medical or diagnostic applications.

The pharmaceutical composition according the invention can notably be used to treat a BKV infection, or any related affection thereof.

The invention thus provides a pharmaceutical composition according to the invention for use as a medicament, preferably for the treatment of a BK virus (BKV) infection or any associated disorder thereof in a subject in need thereof, such as an immunocompromised subject, especially a graft-recipient such as a kidney-graft or bone marrow-graft recipient.

The invention is also directed to the use of the pharmaceutical composition according to the invention, for the preparation of a medicament, preferably for the treatment of a BK virus (BKV) infection or any associated disorder thereof in a subject in need thereof, such as an immunocompromised subject, especially a graft-recipient such as a kidney-graft or bone marrow-graft recipient.

The invention also provides a method for treating a BK virus (BKV) infection or any associated disorder thereof, in a subject in need thereof, such as an immunocompromised subject, especially a graft-recipient such as a kidney-graft or bone marrow-graft recipient said method comprising the administration to the subject of a therapeutically effective amount of the pharmaceutical composition according to the invention.

Typical examples of BKV-associated disorders are as described above. A particularly preferred BKV-associated disorder is a BKV-related leukodystrophy.

In the context of the present invention, it will be understood that the subject to be treated, such as an immunocompromised subject, especially a graft-recipient such as a kidney-graft or bone marrow-graft recipient, is preferably a human.

The invention further relates to a pharmaceutical composition as described herein, and at least one antibody reacting with a non-BKV virus, as a combined preparation for simultaneous, separate or sequential use as a medicament, the non-BKV virus being selected from the group consisting of a SARS-CoV-2 virus and other coronaviruses, a Respiratory Syncytial Virus (RSV), a Herpes Virus Simplex (HSV), an Epstein-Barr virus (EBV), a cytomegalovirus (CMV), an influenza virus, a parainfluenza virus, a metapneumovirus, and a JC virus (JCV).

It shall be understood that when a plurality of antibodies or antigen binding fragments thereof are to be administered to the subject, those may not necessarily be provided in a single pharmaceutical composition.

Accordingly, the invention further pertains to a combination of two or more antibodies or antigen binding fragments thereof, as described herein.

The invention is further directed to two or more antibodies or antigen-binding fragments thereof, as a combined preparation for simultaneous, separate or sequential use as a medicament, preferably in the treatment of a BK virus (BKV) infection or associated disorder thereof, in a subject in need thereof, such as an immunocompromised subject, especially a graft-recipient such as a kidney-graft or bone marrow-graft recipient.

The pharmaceutical composition or antibodies according to the invention can be administered to the subject in need using any suitable scheme of administration. For instance, the administration may be oral, intravenous, subcutaneous, dermal, intradermal, intramuscular, mucosal, parenteral, intraorgan, intralesional, intranasal, inhalation, intraocular, intramuscular, intravascular, intrathecal, intranodal, rectal, intraperitoneal, or any one or combination of a variety of well-known routes of administration, depending on the infection or associated disorder affecting the subject. The route of administration may also depend on whether local or systemic treatment is desired. The dose and/or scheme of administration can be easily determined and adapted by the skilled practitioner, in accordance with the age, weight and/or severity of the infection or associated disorder from which the subject suffers.

In a preferred embodiment, the pharmaceutical composition or antibodies are in a form suitable for a sustainable release, and/or for intravenous, intramuscular, subcutaneous, intranasal, or intrathecal administration, or for administration by infusion or by aerosol.

The diagnostic composition of the invention may be used to detect *in vitro* BKV, which may be particularly useful when performing a graft on a patient.

The invention accordingly provides an *in vitro* use of a diagnostic composition according to the invention, for detecting the presence of BKV in a biological sample.

A *"biological sample"* refers herein to a tissue or cell sample isolated from a subject to be tested, that is likely or suspected to be infected with BKV. In the context of a graft, the subject to be tested may be the graft-recipient and/or the graft-donor. Biological samples susceptible to contain BKV are typically renal samples (e.g. renal biopsies), urine samples, blood samples, or bone marrow samples.

The invention is also directed to a method for detecting the presence of BKV in a biological sample, said method comprising contacting *in vitro* the biological sample with the diagnostic composition according to the invention.

More precisely, the invention is directed to an *in vitro* method for detecting the presence of BKV in a biological sample, said method comprising: contacting said sample with the diagnostic composition, and detecting the binding of the antibody or antigen-binding fragment to a BK virus, and/or detecting neutralization of a BK virus by the antibody or antigen-binding fragment.

The invention is also directed to a method for diagnosing a BKV infection or associated disorder thereof in a subject, said method comprising contacting *in vitro* a biological sample of the subject with the diagnostic composition according to the invention.

More precisely, the invention is directed to an in vitro method for diagnosing a BKV infection or associated disorder thereof in a subject, said method comprising: contacting said sample with the diagnostic composition, and detecting the binding of the antibody or antigen-binding fragment to a BK virus, and/or detecting neutralization of a BK virus by the antibody or antigen-binding fragment.

The contacting step is preferably performed under conditions allowing the binding between VP1 and the antibody, or antigen-binding fragment thereof, that is contained in the composition. The detection of the binding is accordingly indicative of the presence of BKV in the biological sample, and/or that the subject is suffering from a BKV infection or associated disorder thereof. Alternatively, and/or additionally, the detection of neutralization is indicative of the presence of BKV in the biological sample and/or that the subject is suffering from a BKV infection or associated disorder thereof.

The binding can be detected by any methods well-known in the art, such as by Western-Blot, RIA, or ELISA, to name a few. To facilitate the detection of binding, the antibody, or antigen-binding fragment thereof, that is contained in the diagnostic composition may be labeled, notably with a detectable molecule, such as with a radiolabel, an enzyme label, a fluorescent label, a biotin-avidin label, a chemiluminescent label, and the like.

The neutralization can be detected by any methods well-known in the art, such as pseudotyped virus assay, microneutralization assay, pseudotyped virus assay, plaque reduction assay, rapid lateral flow, and the like.

The present invention also pertains to a kit for implementing any method as described herein, said kit comprising: (1) an antibody, or antigen-binding fragment thereof, or a pharmaceutical or diagnostic composition, or a nucleic acid, or a set of nucleic acids, or a vector (or set of vectors), or a host cell, as described above, or any combination thereof; and (2) instructions for implementing the method.

The term "instructions" refers to written, printed, or graphic displays generally provided on or alongside a container of a kit, for example written material presented on a bottle containing an active agent (herein (1)), such as details of its content and use thereof.

The present invention finally pertains to an *in vitro* use of an anti-idiotypic antibody that binds to the antibody or antigen-binding fragment thereof as described herein, for measuring the concentration of said antibody or fragment in a sample, preferably in a biological sample.

The present invention will be better understood in the light of the following detailed experiments. Nevertheless, the skilled artisan will appreciate that the present examples are not limitative and that various modifications, substitutions, omissions, and changes may be made without departing from the scope of the invention.

### EXAMPLES

In the present study, specific memory B cell sorting followed by single cell PCR were used to isolate anti-BKV human monoclonal antibodies from donors' blood. By using labelled antigens, specific single memory B-cells were isolated by flow-cytometry. Cloning and expression of human monoclonal antibodies were achieved through the recovery of immunoglobulin gene sequences from single B cells and by transfection of producer cells with the VH and VL chain genes. Recombinant BKV capsid protein VP1 of the three different BKV genotypes I, II and IV and of viral variants thereof, were used as antigen. Lead antibody candidates were selected for their binding and broad neutralization potency, and their respective epitopes characterized.

### Example 1

### 1. Materials & Methods

### 1.1. Donor material

Human peripheral bloods (PBMCs) were obtained and collected from 18 donors after receiving their written informed consent and approval by the Institutional Review Boards of the Strasbourg University Hospitals. PBMCs were isolated and stored in liquid nitrogen. Elite BKV neutralizers were identified as those having high serum titers (e.g. > 1/10⁵ IC₅₀) of anti-BKV Abs against all tested BKV genotypes.

### 1.2 Cell culture

BL21 Star strain of *Escherichia coli* cells (Invitrogen, #C6010) used for VP1 pentamers production, were cultured at 37°C and 26°C in LB medium supplemented with 50 µg/mL of ampicillin. Human monoclonal antibodies and Fabs were expressed in HEK293F cells cultured in FreeStyle 293 expression medium (Thermo-Fisher, #12338018) at 37°C under 5% CO2. 293TT cells (National Cancer Institute Tumor repository, Frederick, Maryland) were cultured as previously described by Solis et al. (J Am Soc Nephrol., 2018; 29(1):326-334).

### 1.3 VP1 pentamers

### Plasmids construction

A truncated form of VP1 from BKV genotype I was cloned into a pET-15b expression plasmid. This truncated form contains the amino acid residues 31 to 301, and is referred as the Dunlop sequence except for the cysteine residue at position 104 that is replaced by a serine (Neu et al. PLoS Pathog, 2013; 9: e1003688). VP1 of genotype II and IV were obtained by gene synthesis (Eurofins) and sub-cloned into a pESPRIT002 vector which contains a His-tag in N-Ter and a biotin acceptor peptide *(BAP)* in C-Ter. VP1 of genotype I was also subcloned into a pESPRIT002 plasmid.

### Production and purification of VP1 pentamers

VP1 pentamers were produced in *E. coli* and purified through a His tag on a nickel column. To do so, BL21 Star *E.coli* were firstly transformed with the pET15-b plasmid expressing the truncated VP1, and cultured until reaching an OD of ~0.6. After IPTG induction, cells were grown at 26 °C for 18 h. After centrifugation, the cell pellet was resuspended in buffer A (50 mM Tris-HCl, pH 7.5, 250 mM NaCl, 30 mM imidazole, 5% glycerol, complete EDTA - free protease inhibitor cocktail and sonicated. The bacterial lysate was pelleted by high speed centrifugation while the supernatant was filtered and applied onto a Ni-NTA resin (Ni-NTA superflow Qiagen, ref 30410), equilibrated with buffer A. Resin was washed with buffer A and VP1 pentamers were eluted in different fractions from the column with buffer B (50 mM Tris-HCl, pH 7.5, 250 mM NaCl, 500 mM imidazole, 5% glycerol). Fractions corresponding to pentamers, identified on Coomassie-stained SDS-PAGE, were pooled together and dialyzed. The sample was then concentrated using a 50,000 molecular weight cut-off (MWCO) Amicon concentrator and VP1 pentamers were purified using a Superdex increase S200 column (GE Healthcare) in 20 mM Hepes pH 7.5, 150 mM NaCl buffer, using an AKTA Purifier (GE Healthcare). Peak fractions were pooled and then concentrated with Amicon concentrator. Quantification of purified pentamers was determined by spectrophotometric absorbance at 280 nm. Pentamers were aliquoted and stored at -80 °C until use.

### Biotinylation of VP1 pentamers

VP1 pentamers were biotinylated using the Biotin-Protein Ligase-BirA-RT kit (Avidity, LLC), according to the manufacturer's instructions. Briefly, 100 µM of VP1, containing a Biotin Acceptor Peptide sequence, was incubated with the BirA enzyme in supermix buffer for 1h at room temperature. The biotinylated protein was then purified by size exclusion chromatography using a Superdex increase S200 column (GE Healthcare) in 20 mM Hepes pH 7.5, 150 mM NaCl buffer, using an AKTA Purifier (GE Healthcare).

### 1.4. Pseudovirions

### Reporter plasmids construction

Plasmids encoding synthetic codon-modified VP1, VP2 and VP3 capsid proteins of BK virus (BKV) were provided by Christopher B. Buck (National Cancer Institute, National Institute of Health, Bethesda, Maryland). The *Gaussia* luciferase (GLuc) reporter plasmid phGluc was constructed by transferring a fragment of the pCMV-Gluc 2 vector (New England BioLabs) into ph2m.

### Pseudovirion production

BKV pseudovirions were produced as previously described by Pastrana et al. (PLoS Pathog. 2012; 8(4): e1002650), for the three BKV genotypes Ia (BK-D; JF894228), BKV II (GBR-12; AB263920) and BKV IVc2 (A-66H; AB369093) as well as for single-or double-mutants thereof (BKV Ia: D60N, L68Q, A72VE73Q, E73Q, D75N, E82D; BKV IVc2: D77Q). Briefly, expression plasmids encoding the VP1, VP2 and VP3 capsid proteins, along with the reporter plasmid phGluc, were co-transfected into 293TT cells. Two days after transfection, cells were lysed and pseudovirions maturated overnight using Ambion^{®} RNase Cocktail^{™} (ThermoFischer Scientific). Pseudovirions were harvested and purified by ultracentrifugation through a iodixanol step gradient (Optiprep, Sigma-Aldrich). To minimize any variation, a single pseudovirion stock was produced for each genotype, aliquoted and used for all experiments.

### 1.5. Entire replicative virions

Entire replicative virions of BK virus genotype Ia (Dunlop strain; ATCC VR-837TM), and BKV genotype II and IV (patient's isolates) were produced.

### 1.6. Serum adsorptions for selection of elite BKV neutralizers

Serum adsorptions with VP1-coupled beads were performed using DynaBeads MyOne Tosylactivated kit (Invitrogen, # 65501) according to the manufacturer's instructions. Beads coupling was performed at a ratio of 0.5 mg of VP1 genotype I pentamer per 12.5 mg of beads. Briefly, VP1 pentamers were incubated with beads for 24h at 37 °C. Protein-coated beads were then collected by magnetic force and resuspended in a PBS blocking buffer for another 24h at 37°C. The magnetic beads were then washed 3 times in a PBS washing buffer, suspended in DMEM 10% FBS PSGNaPy and incubated 30 min at room temperature. Patients' sera or IgG (40 µl) were then added and incubated 30 min at room temperature. After 3 washes, the antibodies bound to beads were eluted by 3 cycles of glycine pH 2.7 followed by 3 cycles of glycine pH 2.2. Each eluted fraction was neutralized by Tris pH 9. Beads were then regenerated and three rounds of adsorption were performed to ensure complete removal of antigen-specific antibodies. The percentage of IgG titer in depleted fraction was calculated by the formula: (IgG titer-depleted/IgG titer-before) × 100%, where "IgG titer-depleted" represents the VP1-specific IgG titer of the patient's serum after the specific antibody depletion, and "IgG titer-before" represents the VP1-specific serum IgG titer before depletion. The percentage of depletion corresponds to: 100% - the percentage of IgG titer in depleted fraction.

### 1.7. Memory B cell sorting with VP1 pentamers

FACS sorting of cryopreserved PBMCs from donor 16 was performed on a BD FACSAria^{™} Fusion cytometer. PBMCs were stained with live/dead fixable Aqua Dead Cell Stain Kit (Thermofisher, #L34957). The following cocktail of antibodies from Miltenyi Biotec were also used to stain the cells prior to sorting by flow cytometry: anti-CD3-BV421, anti-CD19 (#130113649), anti-CD20-PECy7 (#130111345), anti-Human IgA-PE (#130113476), anti-IgM-PE (#130093075), anti-IgD-PE (#130110643). VP1 pentamers sorting baits were pre-complexed with a streptavidin fluorophore prior to addition to cells. Five fluorophores were used: Strep-APC (Miltenyi Biotec, #130-106-792) for genotype I, Strep-BUV 496 (BD Biosciences, #612961) for genotype II, =Strep-Vio 515 (Miltenyi Biotec, #130-107-459) for genotype IV, Strep-BUV 395 (BD Biosciences, #564176) for the S275A VP1 mutant, and Strep-BUV 737 (BD Biosciences, #612775) for the Y169A-R170A VP1 mutant. Live CD3/CD8-CD14- CD19+ IgG+VP1+ cells were sorted into individual wells of 96-well PCR plates (free of DNase and RNase, Bio-Rad) containing a lysis buffer. The plates were then stored at -80 °C for further processing.

### 1.8. Neutralization assays using pseudovirions or entire replicative virions

BKV pseudovirion neutralization was determined by analysis of luciferase reporter gene expression as previously described by Pastrana et al. (PLoS Pathog. 2012; 8(4): e1002650). Briefly, pseudovirions mixed with serially diluted sera or antibodies were added to 293TT cells for 72h at 37°C. Neutralization titers were determined by analysis of luciferase activity using the Gaussia Luciferase Glow Assay Kit (Thermo Fisher Scientific) and a luminescence reader (Victor Novo, Perkin Elmer).

For entire virions neutralization assay, BKV replicative virus was mixed with serially diluted antibodies and added to MRC5 cells for 72h at 37°C. Neutralization titers were determined by VP1 immunostaining using the M02 BKVP1 monoclonal antibody (clone 3B2; Abnova, # MAB3204-M02) and a peroxydase labeled anti-mouse IgG antibody (pi2000 Vector Laboratories) or by PCR quantification of BKV DNA (BKV R-gene^{™} kit, Biomérieux).

The neutralization titer was defined as the sample dilution that yielded 50% inhibition of pseudovirion or entire virion infectivity (IC₅₀) and was expressed as the log10 of the IC₅₀. IC₅₀ values were determined by Graphpad Prism using non-linear regression (curve fit) (GraphPad Software 10.0.2).

### 1.9. Antibodies

### Cloning of antibody variable regions

The RNA extracted from the sorted single B cells was converted into cDNA by RT-PCR using the SuperScript^{™} IV enzyme (Fischer Scientific, # 15317696) and random hexamers (Fischer Scientific, # 10580091). To efficiently and specifically amplify antibody genes from single B cells, two successive PCR reactions were run with different sets of primers. The first set of primers was designed to anneal upstream of the second set. After sequencing and lineage attribution by IMGT, the cDNAs of the first-round nested PCR were used to amplify the human antibody variable regions of heavy and kappa/lambda chains for subsequent cloning using appropriate primers with overlapping sequences. PCR products were purified and cloned by the overlapping sequences into human-IgG (Heavy, Kappa or Lambda) expression plasmids using the In-Fusion HD enzyme, according to the manufacturer's instructions (In-Fusion HD Cloning Plus kit, Takara Bio USA, # 638910).

### Monoclonal antibody production and purification

For 100 ml-scale monoclonal antibody production, heavy and light chain-encoded plasmids were re-amplified, and re-sequenced over the entire immunoglobulin Open Reading Frame. A total of 60 µg DNA (light and heavy chain plasmids) was transfected into HEK293F mammalian cells. Cells were cultured in suspension for several days at 37°C before supernatant harvesting and sterile filtration. Antibodies secreted in supernatant were purified via affinity chromatography onto protein A according to the manufacturer's instructions (rProtein A Sepharose^{™} Fast Flow Affinity Media, Cytiva, # 17127903).

### Fabs production and purification

The Fab part of the heavy chain of monoclonal antibodies was obtained after mutagenesis using the "In Fusion HD Cloning Plus" kit (In-Fusion HD Cloning Plus kit, Takara Bio USA, # 638910), according to manufacturer's instructions. The primers designed for the mutagenesis overlapped with the constant part of the heavy chain in order to create a stop codon at amino acid residue K222 corresponding to the Fab part of an antibody. Fabs were produced according to the same procedure as the monoclonal antibodies, albeit with a prior optimization of the HC/LC chains ratio for transfection. After several days of culture, the Fabs were purified on a Kappa or Lambda Select column (Cytiva, #17545811, # 17548211).

### 1.10. Antibody affinity

### Enzyme-linked immunosorbent assay (ELISA)

Purified VP1 pentamers of genotypes I, II or IV (100 ng/well) were coated on Nunc MaxiSorp 96-well plates (Thermo Scientific # 442404) at 4°C overnight in 150 mM NaCl, 20 mM Hepes pH 7.5 buffer. The following day, plates were washed 5 times with PBST (PBS + 0.1 % Tween 20) and incubated for 1h30 with blocking buffer (10 mM Tris pH7.4,150 mM NaCl,1 mM CaCl2, 4% BSA, 0.1% Tween 20) at 37°C. After removal of the blocking buffer, monoclonal antibodies were added at threefold dilutions (starting at 50 µg/mL) and incubated for 1h30 at 37°C. After washing, an Alkaline Phosphatase-AffiniPure Goat Anti-Human IgG, F(ab')2 Fragment Specific (Interchim, #**109-055-097**) was added at 1:10,000 dilution for an additional 1h at room temperature. After incubation and 5 wash cycles, the staining substrate (alkaline phosphatase substrate p-nitrophenyl phosphate, (Biorad, #BUF044B) was added for color development. Absorbance at 405 nm was measured after 1h using the TECAN microplate reader (SPARK 10M TECAN # 30086375). Results were analyzed with program GraphPad Prism v9.0 (GraphPad Software) to determine the EC₅₀.

### 1.11. Epitope binning

Competitive ELISA was used to identify epitopes overlap of the different generated monoclonal BKV- antibodies against VP1 pentamer **(****Figure 2****).** To do so, antibodies were biotinylated with the EZ-Link Micro Sulfo-NHS-LC-Biotinylation Kit according to the manufacturer's instructions (Thermoscientific, #21935). For this cross-competitive ELISA, the same protocol as described in section 1.10 titled "ELISA" was used, except for the incubation with the anti-BKV monoclonal antibodies. Briefly, after the blocking step, 50 µl of non-labeled antibodies, serially diluted (3 fold-dilution) in blocking buffer, were added into wells at a starting concentration of 100 µg/mL. After 45 min incubation at 37°C, biotinylated antibodies were added at the EC₇₀ concentration, previously determined, for 45 min more. Plates were then washed 5 times with PBST, and 100µl/well of Streptavidine-AP conjugate (Sigma Aldrich Ref: 11089161001) was added using a 1:10000 dilution (in PBS, 0.02%, Tween 20, 1% BSA) and incubated for 1h at room temperature. The absorbance was read after 1h at 405 nm by microplate reader (TECAN Spark 10M). To normalize the absorbance values, the detection signal was calculated by the formula: (OD value of mixture antibodies/OD value of biotinylated antibody alone at EC₇₀ concentration) x 100%. Monoclonal antibody VRCO1 (anti -HIV GP120) was used at negative control. IC₅₀ for each biotinylated antibody mixed with competitor antibody was determined using GraphPad Prism v9.0 (GraphPad Software, Inc.).

### 1.12. Electron microscopy

### Data collection and processing

To form antigen-antibody complexes, VP1 pentamers or VLPs (the latter being obtained from D.McIlroy, Nantes) were incubated with Fab at a two-fold molar ratio for 1h at room temperature. To eliminate free Fab, the complex was submitted to gel filtration onto S200 increase column. The peak fractions corresponding to the eluted complex were pooled, concentrated (1mg/mL) and applied onto a grid.

### Negative staining

4 µL of sample, diluted to approximately 0.05-0.1 mg/mL, were adsorbed onto the clean side of a carbon film that had been previously evaporated on mica. The sample was then stained with 2% w/v Sodium Silico Tungstate for 30 seconds. The sample and carbon film ensemble was transferred to an EM grid and air-dried. Images were acquired using a Tecnai 12 FEI electron microscope operated at 120 kV, with a Gatan ORIUS SC1000 camera (Gatan, Inc., Pleasanton, CA) at 30,000x nominal magnification.

### Cryo-EM

A 3.5 µL aliquot of the concentrated sample was applied to negatively glow-discharged R1.2/1.3 Quantifoil copper grids (Quantifoil Micro Tools) using a discharge of 25 mA for 40 seconds. Excess solution was blotted away with a Vitrobot Mark IV (FEI) at 20 °C and 100% humidity, with a blot time of 5.5 seconds and blot force set to 0, followed by rapid freezing in liquid ethane. Automated data collection was performed on a 200 kV Glacios cryo-TEM microscope (Thermo Fisher Scientific) equipped with a K2 direct electron detector (Gatan) using SerialEM software. Coma and astigmatism corrections were also performed via SerialEM. Movies consisting of 40 frames were collected in counting mode at a magnification of 36,000×, resulting in a pixel size of 1.145 Å. The defocus range was set between -1.0 and -2.5 µm using a multi-shot scheme (3 × 3 grid of holes without stage movement). The total exposure dose per movie was 40 e-/Å², and approximately 2500 images were obtained per sample.

### Image processing and 3D reconstruction

Movie drift correction and CTF determination were performed using Relion. Particle selection and analysis, including class averaging, initial 3D model generation, and 3D refinement with either I4 or C5 symmetry, were performed with Relion. The final reconstruction resolution was validated by Fourier Shell Correlation using the 0.143 threshold criterion.

### 2. Results

### 2.1. Selection of elite BKV neutralizers

In order to isolate BKV broadly neutralizing monoclonal antibodies, a cohort of kidney transplant patients with strong serum neutralizing activity against the three most prevalent BKV genotypes (I, II and IV) as described by Solis et al. (J Am Soc Nephrol., 2018; 29(1): 326-334) was investigated to select candidates for BKV neutralizing antibodies isolation. The target for BKV neutralizing antibodies is the VP1 capsid protein which forms the outer layer of the virus and whose external surface is accessible to antibodies. The VP1 protein assembles into a pentamer and 72 pentamers form a viral capsid. Recombinant soluble pentamers were used as baits to select specific B cells, in order to isolate neutralizing antibodies. Because such pentamers would not be effective for isolating VLP-specific potential quaternary neutralizing antibodies, serum antibodies were firstly absorbed onto VP1 pentamers from genotype I so as to verify that most neutralizing antibodies could be captured. This approach was used to determine whether VLP-specific quaternary antibodies constitute a significant proportion of the neutralizing activity in the donors, and whether donors with broad serum activity possessed broadly neutralizing antibodies rather than a combination of genotype-restricted antibodies. A loss of neutralization against all three genotypes after adsorption on VP1 from genotype 1 is indeed indicative of the presence of cross-reactive neutralizing antibodies.

In all donors tested (18/18), more than 98% of neutralizing activity against genotype 1 BKV was lost upon sera adsorption, showing that VLP-specific quaternary antibodies do not constitute a significant fraction of the serum neutralizing activity (data not shown). Adsorbed sera were further tested in neutralization against BKV from genotype II and IV, showing a similar loss of neutralizing activity (greater than 94%) in all donors except one (donor 2) (data not shown). Thus, while most donors possessed broadly neutralizing antibodies, the serum activity of some donors was actually due to antibodies that were genotype-specific. Donor 2 was therefore excluded for the isolation of BKV broadly neutralizing antibodies, and donor 16, who exhibited very strong neutralizing titers against the 3 genotypes, was selected for this purpose.

### 2.2. Single memory B cell sorting and production of VP1-specific monoclonal antibodies

Single live CD19+ memory B lymphocytes that bound labeled VP1 pentamers were sorted by FACS from the PBMCs of donor 16 as described above. A total of 600 of these B cells were obtained and seeded at one cell per well in 96-well plates. After cell lysis, RT-PCR, PCR and nested PCR, a total of 349 heavy chain immunoglobulin genes, 228 light kappa genes and 140 light lambda chain genes were recovered. Paired heavy and light chains originating from a same sorted cell corresponded to 239 potential monoclonal antibodies. After sequencing of PCR products, antibody gene usage was assigned using IMGT/V-QUEST (Brochet et al., Nucleic acids research, 2008; 36: W503-508).

The heavy and light chain genes of selected B cells were then further amplified using primers corresponding to the determined gene segment family usage and cloned into the appropriate expression vector (heavy, kappa, lambda). The plasmid DNA of each chain was recovered after transformation in *E. Coli* followed by DNA extraction and transfected in 293F cells. 53 unique monoclonal antibodies were produced, among which 33 had a kappa light chain and 20 had a lambda light chain.

### 2.3. Antibody affinity and germline lineage characterization

The 53 unique monoclonal antibodies that were produced and purified were tested in an initial screening to evaluate their binding to BKV VP1. The apparent affinity of each monoclonal antibody for VP1 pentamers of each genotype I, II and IV was assessed by ELISA, as described above.

Among these monoclonal antibodies, 47 antibodies were selected. These 47 monoclonal antibodies belong to 29 lineages originating from unrelated germlines, as illustrated by the identification of a variety of immunoglobulin gene families based on variable gene sequence analyses using IMGT database alignments **(Table 8** below).

**Table 8.Antibody lineage features**

| | | **HEAVY CHAIN** | | | | **LIGHT CHAIN** | | | |
|---|---|---|---|---|---|---|---|---|---|
| **family** | **mAbs** | **V gene** | **% identity V gene** | **J gene** | **% identity J gene** | **V gene** | **% identity V gene** | **J gene** | **% identity J gene** |
| **1** | **B9P6** | IGHV3-21 | 94,44 | IGHJ6 | 82.54 | IGKV3-20 | 95,74 | IGKJ1 | 94.74 |
| | **E9P7** | IGHV3-21 | 93,75 | IGHJ6 | 80.95 | IGKV3-20 | 95,74 | IGKJ1 | 94.74 |
| | **G11P1** | IGHV3-21 | 93,75 | IGHJ6 | 80.95 | IGKV3-20 | 95,39 | IGKJ1 | 94.74 |
| | **E11P6** | IGHV3-21 | 93,75 | IGHJ6 | 80.95 | IGKV3-20 | 95,04 | IGKJ1 | 94.74 |
| **2** | **C11P1** | IGHV4-59 | 93,68 | IGHJ4 | 91.67 | IGLV2-14 | 93,40 | IGLJ3 | 94.59 |
| | **G5P1** | IGHV4-59 | 93,33 | IGHJ4 | 91.67 | IGLV2-14 | 93,75 | IGLJ3 | 94.59 |
| | **E7P5** | IGHV4-59 | 92,63 | IGHJ4 | 91.67 | IGLV2-14 | 94,44 | IGLJ3 | 91.89 |
| **3** | **B8P7** | IGHV4-59 | 95,79 | IGHJ5 | 100.00 | IGLV 1-40 | 97,22 | IGJL3 | 92.11 |
| | **G6P7** | IGHV4-59 | 95,79 | IGHJ5 | 100.00 | IGLV1-40 | 96,88 | IGJL3 | 92.11 |
| | **C4P6** | IGHV4-59 | 95,79 | IGHJ5 | 98.04 | IGLV1-40 | 97,22 | IGJL3 | 92.11 |
| **4** | **G8P6** | IGHV4-61 | 93,47 | IGHJ5 | 80.39 | IGKV1-39 | 93,91 | IGKJ2 | 100.00 |
| | **C6P7** | IGHV4-61 | 93,47 | IGHJ5 | 82.35 | IGKV1-39 | 93,91 | IGKJ2 | 94.44 |
| | **G7P7** | IGHV4-61 | 93,47 | IGHJ5 | 82.35 | IGKV1-39 | 93,91 | IGKJ2 | 100.00 |
| **5** | **F3P6** | IGHV1-3 | 95,14 | IGHJ4 | 87.50 | IGKV3-20 | 98,58 | IGKJ1 | 100.00 |
| | G5P6 | IGHV1-3 | 96,18 | IGHJ4 | 87.50 | IGKV3-20 | 98,23 | IGKJ1 | 100.00 |
| **6** | **B7P6** | IGHV5-51 | 96,53 | IGHJ5 | 90.20 | IGKV1-39 | 94,62 | IGKJ4 | 97.37 |
| **7** | **E6P5** | IGHV3-9 | 96,53 | IGHJ6 | 73.02 | IGKV2-28 | 98,64 | IGKJ5 | 100.00 |
| **8** | **G7P3** | IGHV3-48 | 94,10 | IGHJ3 | 100.00 | IGKV1-27 | 96,77 | IGKJ4 | 97.37 |
| | **E8P5** | IGHV3-48 | 95,14 | IGHJ3 | 100.00 | IGKV1-27 | 96,77 | IGKJ4 | 94.74 |
| **9** | **B7P3** | IGHV3-30 | 96,53 | IGHJ4 | 89.58 | IGLV3-21 | 95,70 | IGLJ2 | 94.29 |
| | **F4P5** | IGHV3-30 | 96,53 | IGHJ4 | 89.58 | IGLV3-21 | 95,34 | IGLJ2 | 94.29 |
| **10** | **E10P5** | IGHV4-61 | 97,59 | IGHJ4 | 91.67 | IGLV3-1 | 95,34 | IGLJ1 | 97.37 |
| **11** | **B10P1** | IGHV3-23 | 94,44 | IGHJ2 | 94.34 | IGKV3-20 | 97,16 | IGKJ4 | 94.44 |
| | **G9P2** | IGHV3-23 | 94,79 | IGHJ2 | 94.34 | IGKV3-20 | 96,81 | IGKJ4 | 100.00 |
| | **C4P7** | IGHV3-23 | 94,79 | IGHJ2 | 94.34 | IGKV3-20 | 97,16 | IGKJ4 | 100.00 |
| | **D7P6** | IGHV3-23 | 96,53 | IGHJ2 | 96.23 | IGKV3-20 | 97,52 | IGKJ4 | 92.11 |
| **12** | **E4P2** | IGHV4-30 | 97,59 | IGHJ6 | 95.24 | IGLV1-40 | 98,96 | IGLJ2 | 100.00 |
| **13** | **F3P7** | IGHV1-3 | 95,83 | IGHJ6 | 96.83 | IGKV3-20 | 96,81 | IGKJ4 | 100.00 |
| **14** | **D11P7** | IGHV3-21 | 94,44 | IGHJ6 | 82.54 | IGLV2-14 | 90,63 | IGLJ2 | 86.11 |
| **15** | **F11P5** | IGHV7-4 | 94,79 | IGHJ6 | 90.48 | IGLV2-23 | 94,79 | IGLJ1 | 97.14 |
| | **D3P7** | IGHV7-4 | 93,75 | IGHJ6 | 90.48 | IGLV2-23 | 94,44 | IGLJ1 | 91.43 |
| **16** | **C11P7** | IGHV3-73 | 88,44 | IGHJ6 | 82.54 | IGLV2-14 | 90,28 | IGLJ2 | 86.11 |
| | **F2P7** | IGHV3-73 | 88,44 | IGHJ6 | 80.95 | IGLV2-14 | 89,24 | IGLJ2 | 83.33 |
| **17** | **C6P8** | IGHV3-73 | 93,88 | IGHJ6 | 84.13 | IGLV3-21 | 94,27 | IGLJ2 | 97.22 |
| **18** | **C10P7** | IGHV1-18 | 85,76 | IGHJ6 | 80.95 | IGLV2-8 | 99,31 | IGLJ2 | 97.37 |
| **19** | **E10P2** | IGHV3-23 | 92,71 | IGHJ5 | 86.27 | IGKV3-20 | 93,97 | IGKJ1 | 94.74 |
| **20** | **B9P4** | IGHV3-30 | 96,53 | IGHJ4 | 85.42 | IGKV2-30 | 96,94 | IGKJ4 | 97.37 |
| **21** | **C10P8** | IGHV4-61 | 97,59 | IGHJ4 | 97.92 | IGLV2-14 | 92,01 | IGLJ2 | 88.89 |
| **22** | **G8P7** | IGHV4-39 | 96,91 | IGHJ3 | 92.00 | IGKV3-15 | 96,77 | IGKJ2 | 97.44 |
| | **D9P6** | IGHV4-39 | 94,16 | IGHJ3 | 92.00 | IGKV3-15 | 95,70 | IGKJ2 | 97.44 |
| **23** | **B5P4** | IGHV1-69 | 92,71 | IGHJ1 | 92.31 | IGKV3-15 | 96,06 | IGKJ4 | 97.37 |
| **24** | **B3P3** | IGHV3-30 | 90,97 | IGHJ6 | 92.06 | IGKV1-39 | 93,19 | IGKJ2 | 100.00 |
| **25** | **E5P6** | IGHV3-30 | 91,32 | IGHJ4 | 87.50 | IGKV3-20 | 97,87 | IGKJ2 | 94.44 |
| **26** | **C9P1** | IGHV3-48 | 94,79 | IGHJ4 | 91.67 | IGKV3-20 | 97,87 | IGKJ2 | 94.44 |
| **27** | **B10P5** | IGHV3-48 | 95,83 | IGHJ5 | 94.12 | IGLV2-11 | 96,18 | IGLJ2 | 97.14 |
| **28** | **E2P6** | IGHV3-53 | 96,84 | IGHJ6 | 85.71 | IGKV1-5 | 96,42 | IGKJ1 | 97.30 |
| **29** | **B2P8** | IGHV4-59 | 94,04 | IGHJ4 | 91.67 | IGKV3-20 | 95,39 | IGKJ1 | 89.47 |

**Table 9** below summarizes the apparent affinity of each of the 47 monoclonal antibodies for VP1 pentamers of each genotype I, II and IV:
- Thirty-six monoclonal antibodies bound all three VP1 genotypes, some with a high affinity;
- Five monoclonal antibodies displayed high affinity for genotypes I and II but no binding to genotype IV: G7P3, E8P5, B7P3, E10P5, C4P7;
- Two monoclonal antibodies bound only to genotype I: D11P7 and E10P2. One monoclonal antibody bound to genotypes II and IV but not to genotype I: B10P5. One monoclonal antibody bound only to genotype IV: C10P8;
- Two monoclonal antibodies had no detectable affinity by ELISA (B5P4 and C9P1).

**Table 9. Antibody affinity (ELISA)**

| **Lineage** | | **EC50 (µg/mL)** | | |
|---|---|---|---|---|
| **Family** | **mAb** | **GI** | **GII** | **GIV** |
| **1** | **B9P6** | 0,12 | 0,035 | 0,12 |
| | **E9P7** | 1 | 0,1 | 0,2 |
| | **G11P1** | 0,7 | 0,055 | 0,035 |
| | **E11P6** | 0,6 | 0,09 | 0,12 |
| **2** | **C11P1** | 7 | 0,045 | 0,22 |
| | **GSP1** | 7 | 0,07 | 2,5 |
| | **E7P5** | 0,15 | 0,055 | 0,08 |
| **3** | **B8P7** | 0,05 | 0,04 | 3 |
| | **G6P7** | 0,07 | 0,2 | 2,5 |
| | **C4P6** | 1,5 | 2,5 | 2,25 |
| **4** | **G8P6** | 2 | 0,08 | 0,06 |
| | **C6P7** | 0,55 | 0,55 | 0,08 |
| | **G7P7** | 12 | 0,06 | 0,04 |
| **5** | **F3P6** | 2 | 0,15 | 1,25 |
| | **G5P6** | 4 | 0,28 | 0,7 |
| **6** | **B7P6** | 0,03 | 0,040 | 0,1 |
| **7** | **E6P5** | 0,075 | 0,6 | 3 |
| **8** | **G7P3** | 0,04 | 0,04 | negative |
| | **E8P5** | 0,06 | 0,08 | negative |
| **9** | **B7P3** | 0,02 | 5,5 | negative |
| | **F4P5** | 0,03 | 10 | 12 |
| **10** | **E10P5** | 0,1 | 0,065 | negative |
| **11** | **B10P1** | 7 | 2,8 | 15 |
| | **G9P2** | 5,5 | 1,5 | 10 |
| | **C4P7** | 5 | 5 | negative |
| | **D7P6** | 6 | 0,25 | 70 |
| **12** | **E4P2** | 0,45 | 0,025 | 0,18 |
| **13** | **F3P7** | 1,4 | 1,5 | 1,8 |
| **14** | **D11P7** | 2 | negative | negative |
| **15** | **F11P5** | 0,1 | 0,07 | 0,12 |
| | **D3P7** | 0,15 | 0,15 | 0,3 |
| **16** | **C11P7** | 6 | 6 | 4 |
| | **F2P7** | 5,5 | 10 | 4 |
| **17** | **C6P8** | 4,5 | 3 | 4 |
| **18** | **C10P7** | 8 | 8 | 18 |
| **19** | **E10P2** | 20 | negative | negative |
| **20** | **B9P4** | 2 | ≥ 7 | ≥ 10 |
| **21** | **C10P8** | negative | negative | 10 |
| **22** | **G8P7** | 10 | 25 | 5 |
| | **D9P6** | 8 | 9 | 4 |
| **23** | **B5P4** | negative | negative | negative |
| **24** | **B3P3** | 0,15 | 0,15 | 0,4 |
| **25** | **E5P6** | 6 | 6 | 5 |
| **26** | **C9P1** | negative | negative | negative |
| **27** | **B10P5** | negative | 20 | 25 |
| **28** | **E2P6** | 0,2 | 0,14 | 0,6 |
| **29** | **B2P8** | 10 | 5 | 12 |

### 2.4. Antibody neutralizing activity

The neutralizing activity of the 47 monoclonal antibodies was evaluated with a neutralization assay as described above. The potency of each monoclonal antibody was measured as IC₅₀ **(Table 10 below)**.

Ten monoclonal antibodies did not show any neutralizing activity against any of the 3 BKV genotypes even when tested at a high concentration of 50 µg/ml: B3P3, E5P6, C9P1, B10P5, E2P6, C11P7, F2P7, D9P6, B2P8, D3P7.

One antibody was tested only at a maximal concentration of 12.5 µg/ml and showed no neutralization of any genotype: C10P8.

Four antibodies showed no or poor neutralization (IC₅₀ greater than 3 µg/ml) against any of the 3 genotypes: C10P7, B5P4, G8P7, B9P4.

Twelve monoclonal antibodies showed neutralization of BKV genotypes I and II (IC50 < 3.5 µg/ml), and no or only very weak neutralization of genotype IV: B7P3, B10P1, D11P7, D7P6, E10P5, E8P5, F11P5, G7P3, G9P2, C4P7, E4P2, F4P5.

One antibody (E10P2) neutralized genotype I, but only very weakly genotype II, and had no activity against genotype IV

One monoclonal antibody showed specific neutralization of BKV genotypes II and IV: F3P7.

Finally, eighteen monoclonal antibodies showed inhibition of all BKV genotypes (F3P6, G5P6, B9P6, E9P7, G11P1, E11P6, C6P8, E6P5, C11P1, G5P1, E7P5, B8P7, G6P7, C4P6, G8P6,C6P7, G7P7, B7P6), including fourteen antibodies strongly neutralizing all 3 genotypes at an IC50s < 1 µg/ml. Among those, some monoclonal antibodies were remarkably potent against the 3 genotypes, in particular monoclonal antibodies of families 1, 2 and 3 (as numbered in Table 10 below), and more particularly monoclonal antibodies B9P6, E7P5 and B8P7.

**Table 10. Antibody potency (BKV pseudovirions)**

| | | | **Neutralization PSV** | | |
|---|---|---|---|---|---|
| **Family** | | | **IC50 (µg/mL)** | | |
| **Family** | **best mAb of the family** | **mAb** | **GI** | **GII** | **GIV** |
| **1** | **B9P6** | **B9P6** | 0,0371 | 0,0002 | 0,0176 |
| | | **E9P7** | 0,2770 | 0,0003 | 0,0333 |
| | | **G11P1** | 0,1120 | 0,0008 | 0,0200 |
| | | **E11P6** | 0,2163 | 0,0028 | 0,0452 |
| **2** | **E7P5** | **C11P1** | 0,3508 | 0,0015 | 0,0942 |
| | | **G5P1** | 0,5380 | 0,0016 | 0,1081 |
| | | **E7P5** | 0,0719 | 0,0002 | 0,0056 |
| **3** | **B8P7** | **B8P7** | 0,0019 | 0,0004 | 0,1644 |
| | | **G6P7** | 0,0038 | 0,0041 | 0,8826 |
| | | **C4P6** | 0,3199 | 0,1035 | 0,7256 |
| **4** | **C6P7** | **G8P6** | 6,444 | 0,0058 | 0,0582 |
| | | **C6P7** | 0,1038 | 0,0002 | 0,0053 |
| | | **G7P7** | 12,5353 | 0,0007 | 0,0036 |
| **5** | **G5P6** | **F3P6** | 0,3627 | 0,0062 | 0,2619 |
| | | **G5P6** | 0,2995 | 0,0082 | 0,0889 |
| **6** | **B7P6** | **B7P6** | 0,0479 | 0,0044 | 0,1215 |
| **7** | **E6P5** | **E6P5** | 0,0008 | 0,2315 | 1,1930 |
| **8** | **E8P5** | **G7P3** | 0,0008 | 0,0054 | >50 |
| | | **E8P5** | 0,0013 | 0,0040 | >50 |
| **9** | **F4P5** | **B7P3** | 0,0004 | 3,4259 | >50 |
| | | **F4P5** | 0,0008 | 0,4713 | 15,9300 |
| **10** | **E10P5** | **E10P5** | 0,0004 | 0,0433 | >50 |
| **11** | | **B10P1** | 1,2715 | 0,1068 | >50 |
| | | **G9P2** | 0,6827 | 0,0336 | >50 |
| | **D7P6** | **C4P7** | 0,4935 | 0,0609 | > 3,125 |
| | | **D7P6** | 0,0960 | 0,0103 | >50 |
| **12** | **E4P2** | **E4P2** | 0,0399 | 0,9792 | >50 |
| **13** | | **F3P7** | >50 | 0,0457 | 0,6140 |
| **14** | | **D11P7** | 0,2747 | 0,0901 | >50 |
| **15** | **F11P5** | **F11P5** | 0,4919 | 1,5630 | 46,4800 |
| | | **D3P7** | >50 | >50 | >50 |
| **16** | | **C11P7** | >50 | >50 | >50 |
| | | **F2P7** | >50 | >50 | >50 |
| **17** | **C6P8** | **C6P8** | 6,9215 | 0,4395 | 1,7395 |
| **18** | | **C10P7** | 6,5640 | 11,2200 | >50 |
| **19** | | **E10P2** | 1,6377 | 20,0800 | >50 |
| **20** | | **B9P4** | 4,956 | 16,3040 | >50 |
| **21** | | **C10P8** | >12,5 | >12,5 | >12,5 |
| **21** | | **G8P7** | >50 | 21,2947 | >50 |
| **22** | | **D9P6** | >50 | >50 | >50 |
| **23** | | **B5P4** | >3,125 | >50 | >50 |
| **24** | | **B3P3** | >50 | >50 | >50 |
| **25** | | **E5P6** | >50 | >50 | >50 |
| **26** | | **C9P1** | >50 | >50 | >50 |
| **27** | | **B10P5** | >50 | >50 | >50 |
| **28** | | **E2P6** | >50 | >50 | >50 |
| **29** | | **B2P8** | >50 | >50 | >50 |

### 2.4. Antibody epitopes

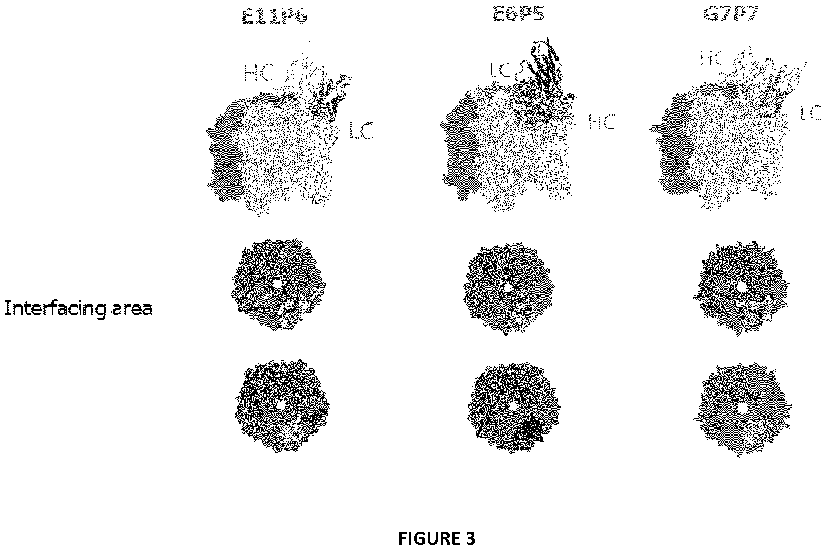

Monoclonal antibodies G7P7, E6P5 and E11P6 were selected for epitope determination using cryo-EM **(****Figures 2** **and** **3****)**. Fabs of the three monoclonal antibodies were produced and complexes with recombinant VP1 pentamers and/or VLPs were studied by cryo-EM. The three monoclonal antibodies bound VP1 interacted through their heavy and light chains with the accessible outer surface of the VP1 pentamer, as expected for neutralizing monoclonal antibodies. Both G7P7 and E11P6 mostly interacted with the BC loop while E6P5 made more distributed contacts across the BC, EF and HI loops.

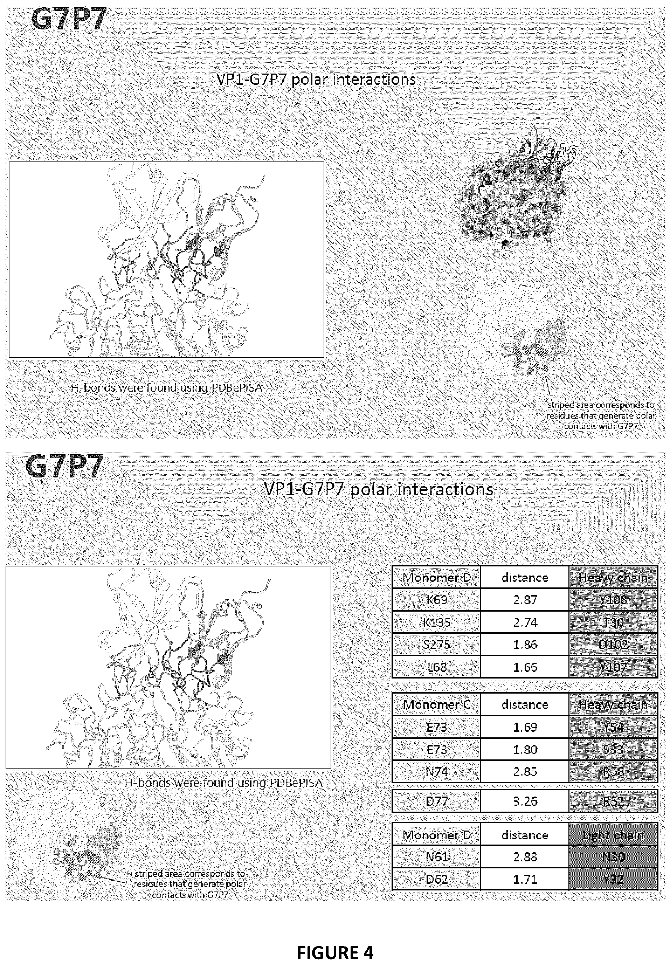

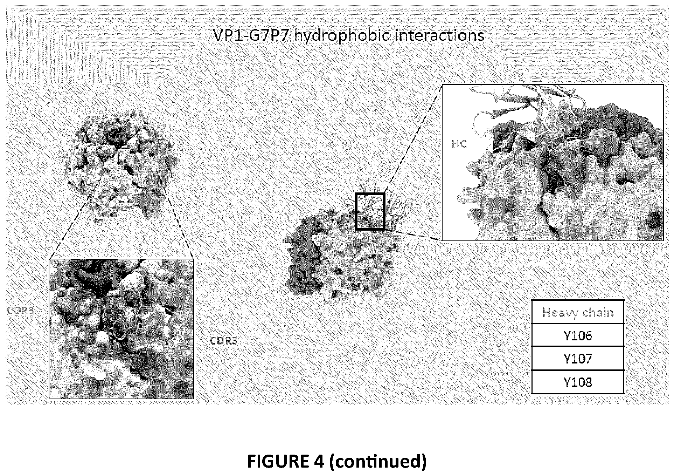

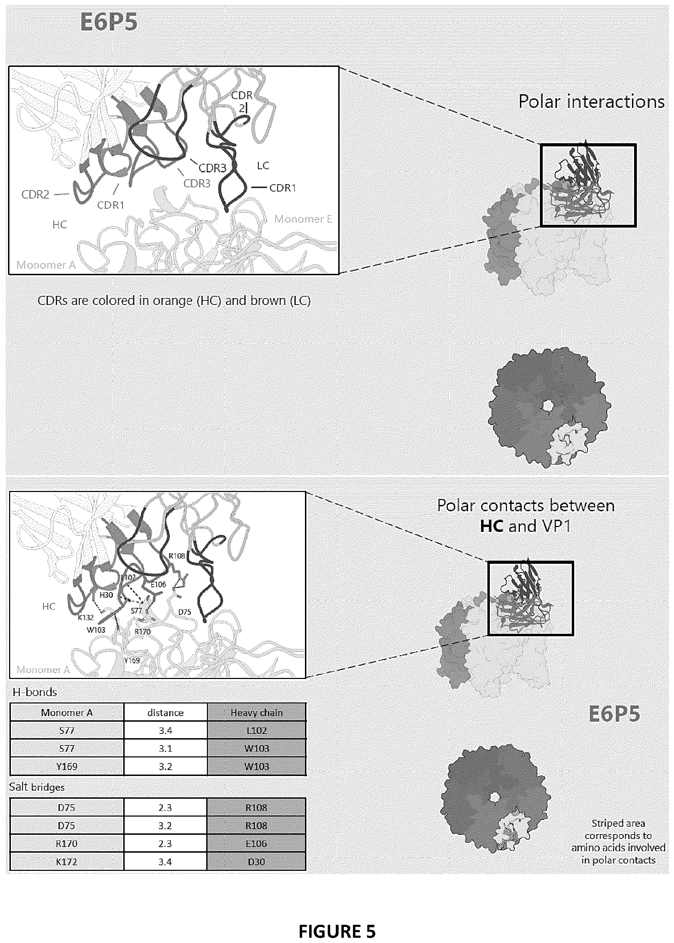

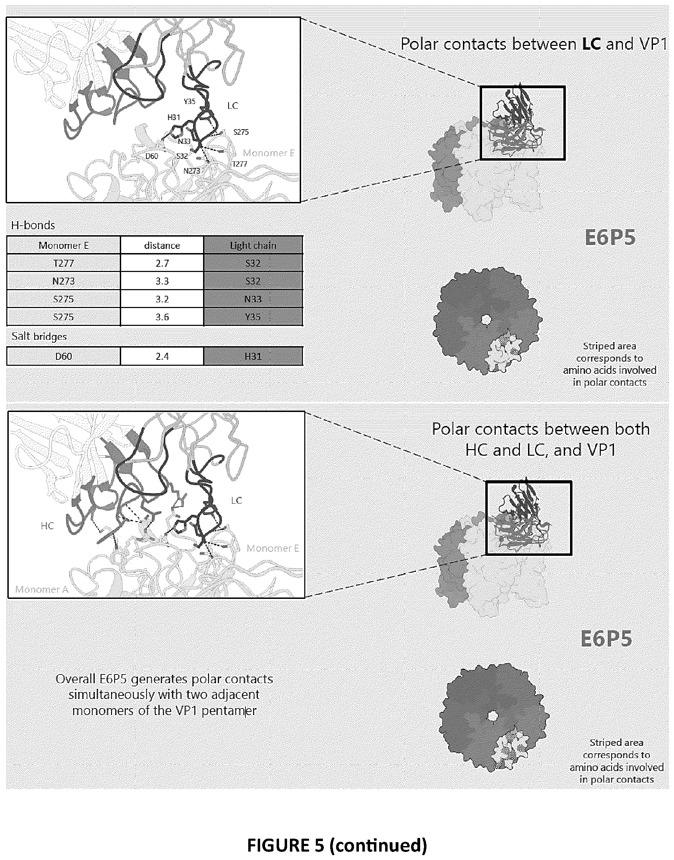

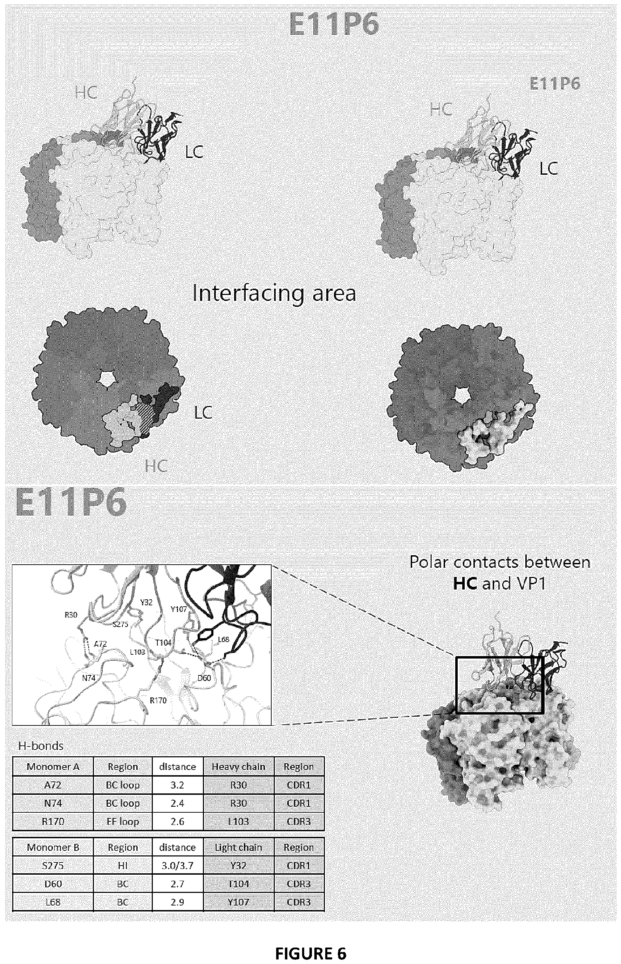

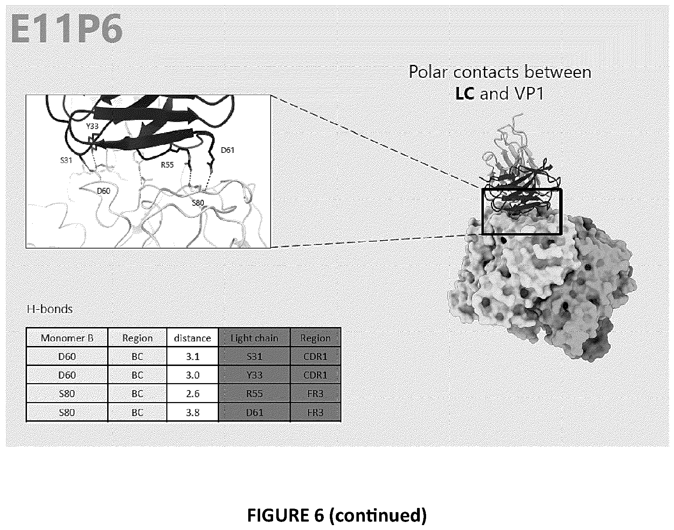

Details of interactions between antibodies and VP1 are shown in **Figures 4****,** **5** **and** **6****,** as well as in **Table 11** below.

**Table 11. G7P7, E6P5 and E11P6 epitopes as determined by cryo-EM**

| **VP1** | | | **Fab** | | | | |
|---|---|---|---|---|---|---|---|
| **VP1 pentamer residue** | **monomer involved** | **VP1 loop** | **Anti-VPI Fab residue** | **VP1_Fab distance** | **Fab chain** | **Interactions** | **Fab region** |
| **VP1 geno II pentamer_Fab G7P7** | | | | | | | |
| N61 | D | BC | N30 | 2,88 | LC | polar | CDR1 |
| D62 | D | BC | Y32 | 1,71 | LC | polar | CDR1 |
| L68 | D | BC | Y107 | 1,66 | HC | polar | CDR3 |
| K69 | D | BC | Y108 | 2,87 | HC | polar | CDR3 |
| E73 | C | BC | Y54 | 1,69 | HC | polar | CDR2 |
| E73 | C | BC | S33 | 1,80 | HC | polar | CDR1 |
| N74 | C | BC | R58 | 2,85 | HC | polar | CDR2 |
| D77 | C | BC | R52 | 3,26 | CDR2 | salt bridge | CDR2 |
| S133 | E | DE | it only intefaces the antibody,no polar bonds wi th it | | | | |
| K135 | D | DE | T30 | 2,74 | HC | polar | CDR1 |
| S275 | D | HI | D102 | 1,86 | HC | polar | CDR3 |

| **VP1 geno I pentamer_Fab E6P5** | | | | | | | |
|---|---|---|---|---|---|---|---|
| D60 | E | BC | H31 | 2,4 | LC | salt bridge | CDR1 |
| D75 | A | BC | R108 | 2,3/3,2 | HC | salt bridge | CDR3 |
| S77 | A | BC | L102 | 3,4 | HC | polar | CDR3 |
| S77 | A | BC | W103 | 3,1 | HC | polar | CDR3 |
| Y169 | A | EF | W103 | 3,2 | HC | polar | CDR3 |
| R170 | A | EF | E106 | 2,3 | HC | salt bridge | CDR3 |
| K172 | A | EF | D30 | 3,4 | HC | salt bridge | CDR1 |
| N273 | E | HI | S32 | 3,2 | LC | polar | CDR1 |
| S275 | E | HI | N33 | 3,3 | LC | polar | CDR1 |
| S275 | E | HI | Y35 | 3,6 | LC | polar | CDR1 |
| T277 | E | HI | S32 | 2,7 | LC | polar | CDR1 |

| **VP1 geno II pentamer_Fab E11P6** | | | | | | | |
|---|---|---|---|---|---|---|---|
| D60 | B | | T104 | 2,7 | HC | H-bond | CDR3 |
| D60 | B | BC | S31 | 3,1 | LC | H-bond | CDR1 |
| D60 | B | BC | Y33 | 3,0 | LC | H-bond | CDR1 |
| L68 | B | BC | Y107 | 2,9 | HC | H-bond | CDR3 |
| A72 | A | BC | R30 | 3,2 | HC | H-bond | CDR1 |
| N74 | A | BC | R30 | 2,4 | HC | H-bond | CDR1 |
| S80 | B | BC | R55 | 2,6 | LC | H-bond | FR3 |
| S80 | B | BC | D61 | 3,8 | LC | H-bond | FR3 |
| R170 | A | EF | L103 | 2,6 | HC | H-bond | CDR3 |
| S275 | B | HI | Y32 | 3/3,7 | HC | H-bond | CDR1 |

### 3. Conclusion

The generation of a panel of 47 BKV neutralizing human monoclonal antibodies obtained from the peripheral memory B cells of a kidney transplant patient is described herein. These monoclonal antibodies bound to the BKV VP1 capsid protein of different genotypes with various affinities and displayed various neutralization profiles.

Some monoclonal antibodies were specific of only certain genotypes and could be very useful as tools for diagnosis. A number of monoclonal antibodies cross-neutralized all 3 genotypes with high potency and represent strong candidates for the development of therapeutic and preventive approach of BKV reactivation in transplant patients.

## Claims

1. A monoclonal antibody, preferably human, or antigen-binding fragment or chain thereof, that binds and optionally neutralizes at least one BK virus subtype, said antibody comprising a heavy chain variable domain (VH) and/or a light chain variable domain (VL) with six complementary-determining regions (CDRs) selected from the following combinations:
a)
• a VH-CDR1 comprising, or consisting of, amino acid sequence GFTFRSYXₐ₁ (SEQ ID NO: 1), wherein Xₐ₁ is T or S;
• a VH-CDR2 comprising, or consisting of, amino acid sequence ISGSSNXₐ₂K (SEQ ID NO: 2), wherein Xₐ₂ is H or Q;
• a VH-CDR3 comprising, or consisting of, amino acid sequence AREDDII,TPLYGMDV (SEQ ID NO: 3);
• a VL-CDR1 comprising, or consisting of, amino acid sequence QSVRSNY (SEQ ID NO: 4);
• a VL-CDR2 comprising, or consisting of, amino acid sequence GAS; and
• a VL-CDR3 comprising, or consisting of, amino acid sequence HQYXₐ₃SSPRT (SEQ ID NO: 5), wherein Xₐ₃ is G or A; or
b)
• a VH-CDR1 comprising, or consisting of, amino acid sequence GGSIRNSY (SEQ ID NO: 23);
• a VH-CDR2 comprising, or consisting of, amino acid sequence IYYTGX_{c1}T (SEQ ID NO: 24), wherein X_{c1} is T or S;
• a VH-CDR3 comprising, or consisting of, amino acid sequence AQGGGSGDLHY (SEQ ID NO: 25);
• a VL-CDR1 comprising, or consisting of, amino acid sequence RSDVGGYNY (SEQ ID NO: 26);
• a VL-CDR2 comprising, or consisting of, amino acid sequence DIS; and
• a VL-CDR3 comprising, or consisting of, amino acid sequence ISFATGYTWV (SEQ ID NO: 27); or
c)
• a VH-CDR1 comprising, or consisting of, amino acid sequence GGSISPYY (SEQ ID NO: 30);
• a VH-CDR2 comprising, or consisting of, amino acid sequence IYYSGNT (SEQ ID NO: 31);
• a VH-CDR3 comprising, or consisting of, the amino acid sequence ARDSCSGTCYLGX_{d1}NWFDP (SEQ ID NO: 32), wherein X_{d1} is D or G;
• a VL-CDR1 comprising, or consisting of, amino acid sequence SSNIGANND (SEQ ID NO: 33);
• a VL-CDR2 comprising, or consisting of, amino acid sequence GNN; and
• a VL-CDR3 comprising, or consisting of, amino acid sequence QSYDSSLSTSKV (SEQ ID NO: 34); or
d)
• a VH-CDR1 comprising, or consisting of, amino acid sequence GGSMTSGSYY (SEQ ID NO: 140);
• a VH-CDR2 comprising, or consisting of, amino acid sequence IYTSGRT (SEQ ID NO: 141);
• a VH-CDR3 comprising, or consisting of, amino acid sequence ARWGDSSAYYYVGDP (SEQ ID NO: 142);
• a VL-CDR1 comprising, or consisting of, amino acid sequence QNINTY (SEQ ID NO: 143);
• a VL-CDR2 comprising, or consisting of, amino acid sequence AAS; and
• a VL-CDR3 comprising, or consisting of, amino acid sequence QQSFSLPHT (SEQ ID NO: 144); or
e)
• a VH-CDR1 comprising, or consisting of, amino acid sequence GYTFTTHI (SEQ ID NO: 62);
• a VH-CDR2 comprising, or consisting of, amino acid sequence INAGNGNT (SEQ ID NO: 63);
• a VH-CDR3 comprising, or consisting of, amino acid sequence AREDQWLGRSPLEY (SEQ ID NO: 64);
• a VL-CDR1 comprising, or consisting of, amino acid sequence QSVSSSY (SEQ ID NO: 65);
• a VL-CDR2 comprising, or consisting of, amino acid sequence GAS; and
• a VL-CDR3 comprising, or consisting of, amino acid sequence QQYGNSYPWT (SEQ ID NO: 66); or
f)
• a VH-CDR1 comprising, or consisting of, amino acid sequence GFTFTNYW (SEQ ID NO: 57);
• a VH-CDR2 comprising, or consisting of, amino acid sequence IYPDDSDA (SEQ ID NO: 58);
• a VH-CDR3 comprising, or consisting of, amino acid sequence ARGGFCSGSVCYNPAWFDL (SEQ ID NO: 59);
• a VL-CDR1 comprising, or consisting of, amino acid sequence QTINTY (SEQ ID NO: 60);
• a VL-CDR2 comprising, or consisting of, amino acid sequence DAS; and
• a VL-CDR3 comprising, or consisting of, amino acid sequence QQSYSTPLT (SEQ ID NO: 61); or
g)
• a VH-CDR1 comprising, or consisting of, amino acid sequence GFTFDDHA (SEQ ID NO: 47);
• a VH-CDR2 comprising, or consisting of, amino acid sequence INWNSASI (SEQ ID NO: 48);
• a VH-CDR3 comprising, or consisting of, amino acid sequence AKDFGLWFGESRGIDA (SEQ ID NO: 49);
• a VL-CDR1 comprising, or consisting of, amino acid sequence QSLLHSNGYNY (SEQ ID NO: 50);
• a VL-CDR2 comprising, or consisting of, amino acid sequence LGS; and
• a VL-CDR3 comprising, or consisting of, amino acid sequence MQALQTPIT (SEQ ID NO: 51); or
h)
• a VH-CDR1 comprising, or consisting of, amino acid sequence GFXₒ₁FGXₒ₂YS (SEQ ID NO: 97), wherein Xₒ₁ is S or R, Xₒ₂ is S or T;
• a VH-CDR2 comprising, or consisting of, amino acid sequence ISSGSSSI (SEQ ID NO: 98);
• a VH-CDR3 comprising, or consisting of, amino acid sequence ARGWTGYDAFDI (SEQ ID NO: 99);
• a VL-CDR1 comprising, or consisting of, amino acid sequence QVISNY (SEQ ID NO: 100);
• a VL-CDR2 comprising, or consisting of, amino acid sequence AAS; and
• a VL-CDR3 comprising, or consisting of, amino acid sequence QKYNNAPLT (SEQ ID NO: 101); or
i)
• a VH-CDR1 comprising, or consisting of, amino acid sequence GFTFSNFG (SEQ ID NO: 82);
• a VH-CDR2 comprising, or consisting of, amino acid sequence ISYDGSDK (SEQ ID NO: 83);
• a VH-CDR3 comprising, or consisting of, amino acid sequence AKDRVRTKYYYDHSGPFDY (SEQ ID NO: 84);
• a VL-CDR1 comprising, or consisting of, amino acid sequence NIGSKS (SEQ ID NO: 85);
• a VL-CDR2 comprising, or consisting of, amino acid sequence DDD; and
• a VL-CDR3 comprising, or consisting of, amino acid sequence QVWMTNSDRPV (SEQ ID NO: 86); or
j)
• a VH-CDR1 comprising, or consisting of, amino acid sequence GGSISSGNYY (SEQ ID NO: 135);
• a VH-CDR2 comprising, or consisting of, amino acid sequence IYTSGNT (SEQ ID NO: 136);
• a VH-CDR3 comprising, or consisting of, amino acid sequence ARVRRDGYNAFDY (SEQ ID NO: 137);
• a VL-CDR1 comprising, or consisting of, amino acid sequence NLGDKF (SEQ ID NO: 138);
• a VL-CDR2 comprising, or consisting of, amino acid sequence QDS; and
• a VL-CDR3 comprising, or consisting of, amino acid sequence QAWDSDTLYV (SEQ ID NO: 139); or
k)
• a VH-CDR1 comprising, or consisting of, amino acid sequence GFXₖ₁FSSYA (SEQ ID NO: 67), wherein Xₖ₁ is T or P;
• a VH-CDR2 comprising, or consisting of, amino acid sequence Xₖ₂TGSXₖ₃Xₖ₄Xₖ₅T (SEQ ID NO: 68), wherein Xₖ₂ is L or I, Xₖ₃ is G or A, Xₖ₄ is G or S, Xₖ₅ is S or G;
• a VH-CDR3 comprising, or consisting of, amino acid sequence AKEGAYSYDXₖ₆WYFDL (SEQ ID NO: 69), wherein Xₖ₆ is F or Y;
• a VL-CDR1 comprising, or consisting of, amino acid sequence QXₖ₇VSGXₖ₈Y (SEQ ID NO: 70), wherein Xₖ₇ is T or S and Xₖ₈ is N or S;
• a VL-CDR2 comprising, or consisting of, amino acid sequence GAS; and
• a VL-CDR3 comprising, or consisting of, amino acid sequence QQYGXₖ₉Xₖ₁₀Xₖ₁₁T (SEQ ID NO: 71), wherein Xₖ₉ is R or S, Xₖ₁₀ is P or L, Xₖ₁₁ is L or V; or
l)
• a VH-CDR1 comprising, or consisting of, amino acid sequence GGSISSGGYY (SEQ ID NO: 52);
• a VH-CDR2 comprising, or consisting of, amino acid sequence MYYSGST (SEQ ID NO: 53);
• a VH-CDR3 comprising, or consisting of, amino acid sequence ARDRRDYGDYYYFGMDV (SEQ ID NO: 54);
• a VL-CDR1 comprising, or consisting of, amino acid sequence SSNIGAGYD (SEQ ID NO: 55);
• a VL-CDR2 comprising, or consisting of, amino acid sequence ANS; and
• a VL-CDR3 comprising, or consisting of, amino acid sequence QSYDSSLSVYVV (SEQ ID NO: 56); or
m)
• a VH-CDR1 comprising, or consisting of, amino acid sequence GYTFTSYS (SEQ ID NO: 150);
• a VH-CDR2 comprising, or consisting of, amino acid sequence INAGNGDT (SEQ ID NO: 151);
• a VH-CDR3 comprising, or consisting of, amino acid sequence ARSNSPTVTYNYYYYAMDV (SEQ ID NO: 152);
• a VL-CDR1 comprising, or consisting of, amino acid sequence QSVRSSY (SEQ ID NO: 153);
• a VL-CDR2 comprising, or consisting of, amino acid sequence DAS; and
• a VL-CDR3 comprising, or consisting of, amino acid sequence QQYGNSPLT (SEQ ID NO: 154); or
n)
• a VH-CDR1 comprising, or consisting of, amino acid sequence GFTFRSYT (SEQ ID NO: 6);
• a VH-CDR2 comprising, or consisting of, amino acid sequence ISGSSNHK (SEQ ID NO: 7);
• a VH-CDR3 comprising, or consisting of, amino acid sequence AREDDII,TPLYGMDV (SEQ ID NO: 3);
• a VL-CDR1 comprising, or consisting of, amino acid sequence SSDVGAYNY (SEQ ID NO: 111);
• a VL-CDR2 comprising, or consisting of, amino acid sequence DVS; and
• a VL-CDR3 comprising, or consisting of, amino acid sequence CSYAHRGTPL (SEQ ID NO: 112); or
o)
• a VH-CDR1 comprising, or consisting of, amino acid sequence AYTFTNSA (SEQ ID NO: 12);
• a VH-CDR2 comprising, or consisting of, amino acid sequence INTNTGNP (SEQ ID NO: 13);
• a VH-CDR3 comprising, or consisting of, amino acid sequence X_{b1}VPYNFGGDHNSX_{b2}X_{b3}FFGX_{b4}DX_{b5} (SEQ ID NO: 14), wherein X_{b1} is T or A, X_{b2} is Y or F, X_{b3} is Y or H, X_{b4} is V or M and X_{b5} is I or V;
• a VL-CDR1 comprising, or consisting of, amino acid sequence SSDVGX_{b6}YNX_{b7} (SEQ ID NO: 15), wherein X_{b6} is S or T and X_{b7} is L or F;
• a VL-CDR2 comprising, or consisting of, amino acid sequence EGT; and
• a VL-CDR3 comprising, or consisting of, amino acid sequence X_{b8}SYAGX_{b9}NX_{b10}FV (SEQ ID NO: 16), wherein X_{b8} is C or S, X_{b9} is S or G and X_{b10} is T or A;
P)
• a VH-CDR1 comprising, or consisting of, amino acid sequence GFSXₚ₁SXₚ₂SE (SEQ ID NO: 104), wherein Xₚ₁ is L or S, Xₚ₂ is N or G;
• a VH-CDR2 comprising, or consisting of, amino acid sequence VRSXₒ₃ANRHAXₒ₄ (SEQ ID NO: 105), wherein Xₚ₃ is R or T, Xₚ₄ is T or P;
• a VH-CDR3 comprising, or consisting of, amino acid sequence TGVRGVISRYFAMDV (SEQ ID NO: 106);
• a VL-CDR1 comprising, or consisting of, amino acid sequence Xₚ₅SDXₚ₆GXₚ₇YNY (SEQ ID NO: 107), wherein Xₚ₅ is S or N, Xₚ₆ is V or L, Xₚ₇ is A or S;
• a VL-CDR2 comprising, or consisting of, amino acid sequence DVXₚ₈, wherein Xₚ₈ is S or T; and
• a VL-CDR3 comprising, or consisting of, amino acid sequence CSYAXₚ₉Xₚ₁₀GXₚ₁₁PXₚ₁₂ (SEQ ID NO: 108), wherein Xₚ₉ is H or S, Xₚ₁₀ is R or V, Xₚ₁₁ is T or S, Xₚ₁₂ is L or I; or
q)
• a VH-CDR1 comprising, or consisting of, amino acid sequence GFTFSGSE (SEQ ID NO: 117);
• a VH-CDR2 comprising, or consisting of, amino acid sequence VRSRANRHAT (SEQ ID NO: 110);
• a VH-CDR3 comprising, or consisting of, amino acid sequence TGVRGVISRYFAMDV (SEQ ID NO: 106);
• a VL-CDR1 comprising, or consisting of, amino acid sequence NIGSNS (SEQ ID NO: 118);
• a VL-CDR2 comprising, or consisting of, amino acid sequence DDS; and
• a VL-CDR3 comprising, or consisting of, amino acid sequence QVWDRRGDLLV (SEQ ID NO: 119); or
r)
• a VH-CDR1 comprising, or consisting of, amino acid sequence GETYVGHS (SEQ ID NO: 145);
• a VH-CDR2 comprising, or consisting of, amino acid sequence ISLHNGNV (SEQ ID NO: 146);
• a VH-CDR3 comprising, or consisting of, the amino acid sequence ARVPMKIYGVIVFGEYYYDQLDV (SEQ ID NO: 147);
• a VL-CDR1 comprising, or consisting of, amino acid sequence GSDVGGYNY (SEQ ID NO: 148);
• a VL-CDR2 comprising, or consisting of, amino acid sequence EVS; and
• a VL-CDR3 comprising, or consisting of, amino acid sequence SSYAGSKVV (SEQ ID NO: 149); or
s)
• a VH-CDR1 comprising, or consisting of, amino acid sequence GFTFRSYA (SEQ ID NO: 160);
• a VH-CDR2 comprising, or consisting of, amino acid sequence LSSSGTTT (SEQ ID NO: 161);
• a VH-CDR3 comprising, or consisting of, amino acid sequence AKDRNAQWLGSEPLDP (SEQ ID NO: 162);
• a VL-CDR1 comprising, or consisting of, amino acid sequence QSVSSSY (SEQ ID NO: 65);
• a VL-CDR2 comprising, or consisting of, amino acid sequence GAS; and
• a VL-CDR3 comprising, or consisting of, amino acid sequence QQAYTFPWT (SEQ ID NO: 163); or
t)
• a VH-CDR1 comprising, or consisting of, amino acid sequence GFTFSSYG (SEQ ID NO: 87);
• a VH-CDR2 comprising, or consisting of, amino acid sequence ISKDGSNK (SEQ ID NO: 88);
• a VH-CDR3 comprising, or consisting of, amino acid sequence AKIPLRRYSDSGDYSSGDF (SEQ ID NO: 89);
• a VL-CDR1 comprising, or consisting of, amino acid sequence ENLVYSDGNTY (SEQ ID NO: 90);
• a VL-CDR2 comprising, or consisting of, amino acid sequence KVS; and
• a VL-CDR3 comprising, or consisting of, amino acid sequence MQTTHWPPLT (SEQ ID NO: 91); or
u)
• a VH-CDR1 comprising, or consisting of, amino acid sequence GGSISSGRYY (SEQ ID NO: 172);
• a VH-CDR2 comprising, or consisting of, amino acid sequence VYFSGST (SEQ ID NO: 173);
• a VH-CDR3 comprising, or consisting of, amino acid sequence ARAVRDYGDSYSFDY (SEQ ID NO: 174);
• a VL-CDR1 comprising, or consisting of, amino acid sequence SSDIGGYNY (SEQ ID NO: 175;
• a VL-CDR2 comprising, or consisting of, amino acid sequence DVV; and
• a VL-CDR3 comprising, or consisting of, amino acid sequence CSYSIRGTPV (SEQ ID NO: 176);
v)
• a VH-CDR1 comprising, or consisting of, amino acid sequence GXᵣ₁YISTXᵣ₂SYY (SEQ ID NO: 120), wherein Xᵣ₁ is G or T, Xᵣ₂ is S or N;
• a VH-CDR2 comprising, or consisting of, amino acid sequence IEYXᵣ₃GST (SEQ ID NO: 121), wherein Xᵣ₃ is S or G;
• a VH-CDR3 comprising, or consisting of, amino acid sequence ASANSXᵣ₄YYDSGGYXᵣ₅APXᵣ₆ALDXᵣ₇ (SEQ ID NO: 122), wherein Xᵣ₄ is K or H, Xᵣ₅ is Y or C, Xᵣ₆ is G or S, Xᵣ₇ is L or I;
• a VL-CDR1 comprising, or consisting of, amino acid sequence QSXᵣ₈SSH (SEQ ID NO: 123), wherein Xᵣ₈ is V or A;
w)
• a VH-CDR1 comprising, or consisting of, amino acid sequence GGRFSRYG (SEQ ID NO: 155);
• a VH-CDR2 comprising, or consisting of, amino acid sequence IIPLLGTA (SEQ ID NO: 156);
• a VH-CDR3 comprising, or consisting of, amino acid sequence ATARREYDRPGAEYFIQ (SEQ ID NO: 157);
• a VL-CDR1 comprising, or consisting of, amino acid sequence QSVSTN (SEQ ID NO: 158);
• a VL-CDR2 comprising, or consisting of, amino acid sequence DAF; and
• a VL-CDR3 comprising, or consisting of, amino acid sequence QQYNYWPPLT (SEQ ID NO: 159); or
x)
• a VH-CDR1 comprising, or consisting of, amino acid sequence GFTFESYG (SEQ ID NO: 37);
• a VH-CDR2 comprising, or consisting of, amino acid sequence ISNDGSIE (SEQ ID NO: 38);
• a VH-CDR3 comprising, or consisting of, amino acid sequence AKDAVGITGGSLYYSYGMDV (SEQ ID NO: 39);
• a VL-CDR1 comprising, or consisting of, amino acid sequence QTIKNY (SEQ ID NO: 40);
• a VL-CDR2 comprising, or consisting of, amino acid sequence TAS; and
• a VL-CDR3 comprising, or consisting of, amino acid sequence QQSYSAPYS (SEQ ID NO: 41);
y)
• a VH-CDR1 comprising, or consisting of, amino acid sequence GFIFNNHG (SEQ ID NO: 92);
• a VH-CDR2 comprising, or consisting of, amino acid sequence ISYDGKKE (SEQ ID NO: 93);
• a VH-CDR3 comprising, or consisting of, amino acid sequence AKEAYGSGSPCDY (SEQ ID NO: 94);
• a VL-CDR1 comprising, or consisting of, amino acid sequence QRVSSNY (SEQ ID NO: 95);
• a VL-CDR2 comprising, or consisting of, amino acid sequence GAS; and
• a VL-CDR3 comprising, or consisting of, amino acid sequence QQYGTSPRS (SEQ ID NO: 96);
• a VL-CDR2 comprising, or consisting of, amino acid sequence AAS; and
• a VL-CDR3 comprising, or consisting of, amino acid sequence QQYNNWPPGXᵣ₉Xᵣ₁₀ (SEQ ID NO: 124), wherein Xᵣ₉ is Y or T, Xᵣ₁₀ is S or absent; or
z)
• a VH-CDR1 comprising, or consisting of, amino acid sequence GFTFSSYS (SEQ ID NO: 164);
• a VH-CDR2 comprising, or consisting of, amino acid sequence ISSSSRTI (SEQ ID NO: 165);
• a VH-CDR3 comprising, or consisting of, amino acid sequence ARQAVAHFDY (SEQ ID NO: 166);
• a VL-CDR1 comprising, or consisting of, amino acid sequence QRVSSNY (SEQ ID NO: 95);
• a VL-CDR2 comprising, or consisting of, amino acid sequence GAS; and
• a VL-CDR3 comprising, or consisting of, amino acid sequence QQYGTSPRS (SEQ ID NO: 96); or
a')
• a VH-CDR1 comprising, or consisting of, amino acid sequence GFTFSTYT (SEQ ID NO: 167);
• a VH-CDR2 comprising, or consisting of, amino acid sequence ISSRSTSI (SEQ ID NO: 168);
• a VH-CDR3 comprising, or consisting of, amino acid sequence VRDRGYYGSNWFDS (SEQ ID NO: 169);
• a VL-CDR1 comprising, or consisting of, amino acid sequence SSDVGNYNY (SEQ ID NO: 170);
• a VL-CDR2 comprising, or consisting of, amino acid sequence DVT; and
• a VL-CDR3 comprising, or consisting of, amino acid sequence CAYAGSYTLV (SEQ ID NO: 171); or
b')
• a VH-CDR1 comprising, or consisting of, amino acid sequence GFTVSTNC (SEQ ID NO: 42);
• a VH-CDR2 comprising, or consisting of, amino acid sequence IYSGGTT (SEQ ID NO: 43);
• a VH-CDR3 comprising, or consisting of, amino acid sequence ARSVDTYDIYALDV (SEQ ID NO: 44);
• a VL-CDR1 comprising, or consisting of, amino acid sequence QSISRW (SEQ ID NO: 45);
• a VL-CDR2 comprising, or consisting of, amino acid sequence KAS; and
• a VL-CDR3 comprising, or consisting of, amino acid sequence QQYNSYPWT (SEQ ID NO: 46); or
c')
• a VH-CDR1 comprising, or consisting of, amino acid sequence GGSIRNSY (SEQ ID NO: 23);
• a VH-CDR2 comprising, or consisting of, amino acid sequence IYYTGTT (SEQ ID NO: 28);
• a VH-CDR3 comprising, or consisting of, amino acid sequence AQGGGSGDLHY (SEQ ID NO: 25);
• a VL-CDR1 comprising, or consisting of, amino acid sequence QSVRSNY (SEQ ID NO: 4);
• a VL-CDR2 comprising, or consisting of, amino acid sequence GAS; and
• a VL-CDR3 comprising, or consisting of, amino acid sequence HQYASSPRT (SEQ ID NO: 11).

2. The antibody, or antigen-binding fragment thereof, according to claim 1, wherein:
i)
• a VH-CDR1 comprising, or consisting of, amino acid sequence SEQ ID NO: 6 or 9;
• a VH-CDR2 comprising, or consisting of, amino acid sequence SEQ ID NO: 7 or 10;
• a VH-CDR3 comprising, or consisting of, amino acid sequence SEQ ID NO: 3;
• a VL-CDR1 comprising, or consisting of, amino acid sequence SEQ ID NO: 4;
• a VL-CDR2 comprising, or consisting of, amino acid sequence GAS; and
• a VL-CDR3 comprising, or consisting of, amino acid sequence SEQ ID NO: 8 or 11; or
ii)
• a VH-CDR1 comprising, or consisting of, amino acid sequence SEQ ID NO: 125 or 130;
• a VH-CDR2 comprising, or consisting of, amino acid sequence SEQ ID NO: 126 or 131;
• a VH-CDR3 comprising, or consisting of, amino acid sequence SEQ ID NO: 127 or 132;
• a VL-CDR1 comprising, or consisting of, amino acid sequence SEQ ID NO: 128 or 133;
• a VL-CDR2 comprising, or consisting of, amino acid sequence AAS; and
• a VL-CDR3 comprising, or consisting of, amino acid sequence SEQ ID NO: 129 or 134;
or
iii)
• a VH-CDR1 comprising, or consisting of, amino acid sequence SEQ ID NO: 72 or 77;
• a VH-CDR2 comprising, or consisting of, amino acid sequence SEQ ID NO: 73 or 78;
• a VH-CDR3 comprising, or consisting of, amino acid sequence SEQ ID NO: 74 or 79;
• a VL-CDR1 comprising, or consisting of, amino acid sequence SEQ ID NO: 75 or 80;
• a VL-CDR2 comprising, or consisting of, amino acid sequence GAS; and
• a VL-CDR3 comprising, or consisting of, amino acid sequence SEQ ID NO: 76 or 81; or
iv)
• a VH-CDR1 comprising, or consisting of, amino acid sequence SEQ ID NO: 12;
• a VH-CDR2 comprising, or consisting of, amino acid sequence SEQ ID NO: 13;
• a VH-CDR3 comprising, or consisting of, amino acid sequence SEQ ID NO: 17 or 20;
• a VL-CDR1 comprising, or consisting of, amino acid sequence SEQ ID NO: 18 or 21;
• a VL-CDR2 comprising, or consisting of, amino acid sequence EGT; and
• a VL-CDR3 comprising, or consisting of, amino acid sequence SEQ ID NO: 19 or 22; or
v)
• a VH-CDR1 comprising, or consisting of, amino acid sequence SEQ ID NO: 109 or 113;
• a VH-CDR2 comprising, or consisting of, amino acid sequence SEQ ID NO: 110 or 114;
• a VH-CDR3 comprising, or consisting of, amino acid sequence SEQ ID NO: 106;
• a VL-CDR1 comprising, or consisting of, amino acid sequence SEQ ID NO: 111 or 115;
• a VL-CDR2 comprising, or consisting of, amino acid sequence DVS or DVT; and
• a VL-CDR3 comprising, or consisting of, amino acid sequence SEQ ID NO: 112 or 116.

3. The antibody, or antigen-binding fragment thereof, according to claim 1, wherein:
1) VH-CDR1, VH-CDR2 and VH-CDR3 comprising, or consisting of, amino acid sequence SEQ ID NO: 6, 7 and 3, respectively; and VL-CDR1 and VL-CDR3 comprising, or consisting of, amino acid sequence SEQ ID NO: 4 and 8, respectively, and VL-CDR2 comprising, or consisting of, amino acid sequence GAS; or
2) VH-CDR1, VH-CDR2 and VH-CDR3 comprising, or consisting of, amino acid sequence SEQ ID NO: 9, 10 and 3, respectively; and VL-CDR1 and VL-CDR3 comprising, or consisting of, amino acid sequence SEQ ID NO: 4 and 11, respectively, and VL-CDR2 comprising, or consisting of, amino acid sequence GAS; or
3) VH-CDR1, VH-CDR2 and VH-CDR3 comprising, or consisting of, amino acid sequence SEQ ID NO: 23, 28 and 25, respectively; and VL-CDR1 and VL-CDR3 comprising, or consisting of, amino acid sequence SEQ ID NO: 26 and 27, respectively and VL-CDR2 comprising, or consisting of, amino acid sequence DIS; or
4) VH-CDR1, VH-CDR2 and VH-CDR3 comprising, or consisting of, amino acid sequence SEQ ID NO: 23, 29 and 25, respectively; and VL-CDR1 and VL-CDR3 comprising, or consisting of, amino acid sequence SEQ ID NO: 26 and 27, respectively and VL-CDR2 comprising, or consisting of, amino acid sequence DIS; or
5) VH-CDR1, VH-CDR2 and VH-CDR3 comprising, or consisting of, amino acid sequence SEQ ID NO: 30, 31 and 35, respectively; and VL-CDR1 and VL-CDR3 comprising, or consisting of, amino acid sequence SEQ ID NO: 33 and 34, respectively, and VL-CDR2 comprising, or consisting of, amino acid sequence GNN; or
6) VH-CDR1, VH-CDR2 and VH-CDR3 comprising, or consisting of, amino acid sequence SEQ ID NO: 30, 31 and 36, respectively; and VL-CDR1 and VL-CDR3 comprising, or consisting of, amino acid sequence SEQ ID NO: 33 and 34, respectively, and VL-CDR2 comprising, or consisting of, amino acid sequence GNN; or
7) VH-CDR1, VH-CDR2 and VH-CDR3 comprising, or consisting of, amino acid sequence SEQ ID NO: 140, 141 and 142, respectively; and VL-CDR1 and VL-CDR3 comprising, or consisting of, amino acid sequence SEQ ID NO: 143 and 144, respectively, and VL-CDR2 comprising, or consisting of, amino acid sequence AAS; or
8) VH-CDR1, VH-CDR2 and VH-CDR3 comprising, or consisting of, amino acid sequence SEQ ID NO: 62, 63 and 64, respectively; and VL-CDR1 and VL-CDR3 comprising, or consisting of, amino acid sequence SEQ ID NO: 65 and 66, respectively, and VL-CDR2 comprising, or consisting of, amino acid sequence GAS; or
9) VH-CDR1, VH-CDR2 and VH-CDR3 comprising, or consisting of, amino acid sequence SEQ ID NO: 57, 58 and 59, respectively; and VL-CDR1 and VL-CDR3 comprising, or consisting of, amino acid sequence SEQ ID NO: 60 and 61, respectively, and VL-CDR2 comprising, or consisting of, amino acid sequence DAS; or
10) VH-CDR1, VH-CDR2 and VH-CDR3 comprising, or consisting of, amino acid sequence SEQ ID NO: 47, 48 and 49, respectively; and VL-CDR1 and VL-CDR3 comprising, or consisting of, amino acid sequence SEQ ID NO: 50 and 51, respectively, and VL-CDR2 comprising, or consisting of, amino acid sequence LGS; or
11) VH-CDR1, VH-CDR2 and VH-CDR3 comprising, or consisting of, amino acid sequence SEQ ID NO: 102, 98 and 99, respectively; and VL-CDR1 and VL-CDR3 comprising, or consisting of, amino acid sequence SEQ ID NO: 100 and 101, respectively, and VL-CDR2 comprising, or consisting of, amino acid sequence AAS; or
12) VH-CDR1, VH-CDR2 and VH-CDR3 comprising, or consisting of, amino acid sequence SEQ ID NO: 103, 98 and 99, respectively; and VL-CDR1 and VL-CDR3 comprising, or consisting of, amino acid sequence SEQ ID NO: 100 and 101, respectively, and VL-CDR2 comprising, or consisting of, amino acid sequence AAS; or
13) VH-CDR1, VH-CDR2 and VH-CDR3 comprising, or consisting of, amino acid sequence SEQ ID NO: 82, 83 and 84, respectively; and VL-CDR1, VL-CDR2 and VL-CDR3 comprising, or consisting of, amino acid sequence SEQ ID NO: 85 and 86, respectively, and VL-CDR2 comprising, or consisting of, amino acid sequence DDD; or
14) VH-CDR1, VH-CDR2 and VH-CDR3 comprising, or consisting of, amino acid sequence SEQ ID NO: 135, 136 and 137, respectively; and VL-CDR1 and VL-CDR3 comprising, or consisting of, amino acid sequence SEQ ID NO: 138 and 139, respectively, and VL-CDR2 comprising, or consisting of, amino acid sequence QDS; or
15) VH-CDR1, VH-CDR2 and VH-CDR3 comprising, or consisting of, amino acid sequence SEQ ID NO: 72, 73 and 74, respectively; and VL-CDR1, VL-CDR2 and VL-CDR3 comprising, or consisting of, amino acid sequence SEQ ID NO: 75 and 76, respectively, and VL-CDR2 comprising, or consisting of, amino acid sequence GAS; or
16) VH-CDR1, VH-CDR2 and VH-CDR3 comprising, or consisting of, amino acid sequence SEQ ID NO: 77, 78 and 79, respectively; and VL-CDR1, VL-CDR2 and VL-CDR3 comprising, or consisting of, amino acid sequence SEQ ID NO:80 and 81, respectively, and VL-CDR2 comprising, or consisting of, amino acid sequence GAS; or
17) VH-CDR1, VH-CDR2 and VH-CDR3 comprising, or consisting of, amino acid sequence SEQ ID NO: 52, 53 and 54, respectively; and VL-CDR1 and VL-CDR3 comprising, or consisting of, amino acid sequence SEQ ID NO: 55 and 56, respectively, and VL-CDR2 comprising, or consisting of, amino acid sequence ANS; or
18) VH-CDR1, VH-CDR2 and VH-CDR3 comprising, or consisting of, amino acid sequence SEQ ID NO: 150, 151 and 152, respectively; and VL-CDR1 and VL-CDR3 comprising, or consisting of, amino acid sequence SEQ ID NO: 153 and 154, respectively, and VL-CDR2 comprising, or consisting of, amino acid sequence DAS; or
19) VH-CDR1, VH-CDR2 and VH-CDR3 comprising, or consisting of, amino acid sequence SEQ ID NO: 6, 7 and 3, respectively; and VL-CDR1 and VL-CDR3 comprising, or consisting of, amino acid sequence SEQ ID NO: 111 and 112, respectively, and VL-CDR2 comprising, or consisting of, amino acid sequence DVS; or
20) VH-CDR1, VH-CDR2 and VH-CDR3 comprising, or consisting of, amino acid sequence SEQ ID NO: 12, 13 and 17, respectively; and VL-CDR1 and VL-CDR3 comprising, or consisting of, amino acid sequence SEQ ID NO: 18 and 19, respectively and VL-CDR2 comprising, or consisting of, amino acid sequence EGT; or
21) VH-CDR1, VH-CDR2 and VH-CDR3 comprising, or consisting of, amino acid sequence SEQ ID NO: 12, 13 and 20, respectively; and VL-CDR1 and VL-CDR3 comprising, or consisting of, amino acid sequence SEQ ID NO: 21 and 22, respectively and VL-CDR2 comprising, or consisting of, amino acid sequence EGT; or
22) VH-CDR1, VH-CDR2 and VH-CDR3 comprising, or consisting of, amino acid sequence SEQ ID NO: 109, 110 and 106, respectively; and VL-CDR1 and VL-CDR3 comprising, or consisting of, amino acid sequence SEQ ID NO: 111 and 112, respectively, and VL-CDR2 comprising, or consisting of, amino acid sequence DVS; or
23) VH-CDR1, VH-CDR2 and VH-CDR3 comprising, or consisting of, amino acid sequence SEQ ID NO: 113, 114 and 106, respectively; and VL-CDR1 and VL-CDR3 comprising, or consisting of, amino acid sequence SEQ ID NO: 115 and 116, respectively, and VL-CDR2 comprising, or consisting of, amino acid sequence DVT; or
24) VH-CDR1, VH-CDR2 and VH-CDR3 comprising, or consisting of, amino acid sequence SEQ ID NO: 117, 110 and 106, respectively; and VL-CDR1 and VL-CDR3 comprising, or consisting of, amino acid sequence SEQ ID NO: 118 and 119, respectively, and VL-CDR2 comprising, or consisting of, amino acid sequence DDS; or
25) VH-CDR1, VH-CDR2 and VH-CDR3 comprising, or consisting of, amino acid sequence SEQ ID NO: 145, 146 and 147, respectively; and VL-CDR1 and VL-CDR3 comprising, or consisting of, amino acid sequence SEQ ID NO: 148 and 149, respectively, and VL-CDR2 comprising, or consisting of, amino acid sequence EVS; or
26) VH-CDR1, VH-CDR2 and VH-CDR3 comprising, or consisting of, amino acid sequence SEQ ID NO: 160, 161 and 162, respectively; and VL-CDR1 and VL-CDR3 comprising, or consisting of, amino acid sequence SEQ ID NO: 65 and 163, respectively, and VL-CDR2 comprising, or consisting of, amino acid sequence GAS; or
27) VH-CDR1, VH-CDR2 and VH-CDR3 comprising, or consisting of, amino acid sequence SEQ ID NO: 87, 88 and 89, respectively; and VL-CDR1, VL-CDR2 and VL-CDR3 comprising, or consisting of, amino acid sequence SEQ ID NO: 90 and 91, respectively, and VL-CDR2 comprising, or consisting of, amino acid sequence KVS; or
28) VH-CDR1, VH-CDR2 and VH-CDR3 comprising, or consisting of, amino acid sequence SEQ ID NO: 172, 173 and 174, respectively; and VL-CDR1 and VL-CDR3 comprising, or consisting of, amino acid sequence SEQ ID NO: 175 and 176, respectively, and VL-CDR2 comprising, or consisting of, amino acid sequence DVV; or
29) VH-CDR1, VH-CDR2 and VH-CDR3 comprising, or consisting of, amino acid sequence SEQ ID NO: 125, 126 and 127, respectively; and VL-CDR1 and VL-CDR3 comprising, or consisting of, amino acid sequence SEQ ID NO: 128 and 129, respectively, and VL-CDR2 comprising, or consisting of, amino acid sequence AAS; or
30) VH-CDR1, VH-CDR2 and VH-CDR3 comprising, or consisting of, amino acid sequence SEQ ID NO: 130, 131 and 132, respectively; and VL-CDR1 and VL-CDR3 comprising, or consisting of, amino acid sequence SEQ ID NO: 133 and 134, respectively, and VL-CDR2 comprising, or consisting of, amino acid sequence AAS; or
31) VH-CDR1, VH-CDR2 and VH-CDR3 comprising, or consisting of, amino acid sequence SEQ ID NO: 155, 156 and 157, respectively; and VL-CDR1 and VL-CDR3 comprising, or consisting of, amino acid sequence SEQ ID NO: 158 and 159, respectively, and VL-CDR2 comprising, or consisting of, amino acid sequence DAF; or
32) VH-CDR1, VH-CDR2 and VH-CDR3 comprising, or consisting of, amino acid sequence SEQ ID NO: 37, 38 and 39, respectively; and VL-CDR1 and VL-CDR3 comprising, or consisting of, amino acid sequence SEQ ID NO: 40 and 41, respectively, and VL-CDR2 comprising, or consisting of, amino acid sequence TAS; or
33) VH-CDR1, VH-CDR2 and VH-CDR3 comprising, or consisting of, amino acid sequence SEQ ID NO: 92, 93 and 94, respectively; and VL-CDR1, VL-CDR2 and VL-CDR3 comprising, or consisting of, amino acid sequence SEQ ID NO: 95 and 96, respectively, and VL-CDR2 comprising, or consisting of, amino acid sequence GAS; or
34) VH-CDR1, VH-CDR2 and VH-CDR3 comprising, or consisting of, amino acid sequence SEQ ID NO: 164, 165 and 166, respectively; and VL-CDR1 and VL-CDR3 comprising, or consisting of, amino acid sequence SEQ ID NO: 95 and 96, respectively, and VL-CDR2 comprising, or consisting of, amino acid sequence GAS; or
35) VH-CDR1, VH-CDR2 and VH-CDR3 comprising, or consisting of, amino acid sequence SEQ ID NO: 167, 168 and 169, respectively; and VL-CDR1 and VL-CDR3 comprising, or consisting of, amino acid sequence SEQ ID NO: 170 and 171, respectively, and VL-CDR2 comprising, or consisting of, amino acid sequence DVT; or
36) VH-CDR1, VH-CDR2 and VH-CDR3 comprising, or consisting of, amino acid sequence SEQ ID NO: 42, 43 and 44, respectively; and VL-CDR1 and VL-CDR3 comprising, or consisting of, amino acid sequence SEQ ID NO: 45 and 46, respectively, and VL-CDR2 comprising, or consisting of, amino acid sequence KAS; or
37) VH-CDR1, VH-CDR2 and VH-CDR3 comprising, or consisting of, amino acid sequence SEQ ID NO: 23, 28 and 25, respectively; and VL-CDR1 and VL-CDR3 comprising, or consisting of, amino acid sequence SEQ ID NO: 4 and 11, respectively, and VL-CDR2 comprising, or consisting of, amino acid sequence GAS.

4. The antibody, or antigen-binding fragment thereof, according to any one to claims 1 or 3, wherein:
I) • the heavy chain variable domain (VH) comprises, or consists of, an amino acid sequence having at least 80% sequence identity to SEQ ID NO: 177, 179, 181 or 183; and
• the light chain variable domain (VL) comprises, or consists of, an amino acid sequence having at least 80 % sequence identity to SEQ ID NO: 178, 180, 182 or 184,
with the proviso that the CDRs are as defined in claim 1a); or
II) • the heavy chain variable domain (VH) comprises, or consists of, an amino acid sequence having at least 80% sequence identity to SEQ ID NO: 189, 191, 193, or 195; and
• the light chain variable domain (VL) comprises, or consists of, an amino acid sequence having at least 80 % sequence identity to SEQ ID NO: 190, 192, 194 or 196,
with the proviso that the CDRs are as defined in claim 1b) or 1c'); or
III) • the heavy chain variable domain (VH) comprises, or consists of, an amino acid sequence having at least 80% sequence identity to SEQ ID NO: 197, 199 or 201; and
• the light chain variable domain (VL) comprises, or consists of, an amino acid sequence having at least 80 % sequence identity to SEQ ID NO: 198, 200 or 202,
with the proviso that the CDRs are as defined in claim 1c); or
IV) • the heavy chain variable domain (VH) comprises, or consists of, an amino acid sequence having at least 80% sequence identity to SEQ ID NO: 249, 251 or 243; and
• the light chain variable domain (VL) comprises, or consists of, an amino acid sequence having at least 80 % sequence identity to SEQ ID NO: 250, 252 or 254,
with the proviso that the CDRs are as defined in claim 1d); or
V) • the heavy chain variable domain (VH) comprises, or consists of, an amino acid sequence having at least 80% sequence identity to SEQ ID NO: 213 or 215; and
• the light chain variable domain (VL) comprises, or consists of, an amino acid sequence having at least 80 % sequence identity to SEQ ID NO: 214 or 216,
with the proviso that the CDRs are as defined in claim 1e); or
VI) • the heavy chain variable domain (VH) comprises, or consists of, an amino acid sequence having at least 80% sequence identity to SEQ ID NO: 211; and
• the light chain variable domain (VL) comprises, or consists of, an amino acid sequence having at least 80 % sequence identity to SEQ ID NO: 212,
with the proviso that the CDRs are as defined in claim 1f); or
VII) • the heavy chain variable domain (VH) comprises, or consists of, an amino acid sequence having at least 80% sequence identity to SEQ ID NO: 207; and
• the light chain variable domain (VL) comprises, or consists of, an amino acid sequence having at least 80 % sequence identity to SEQ ID NO: 208,
with the proviso that the CDRs are as defined in claim 1g); or
VIII) • the heavy chain variable domain (VH) comprises, or consists of, an amino acid sequence having at least 80% sequence identity to SEQ ID NO: 233 or 235; and
• the light chain variable domain (VL) comprises, or consists of, an amino acid sequence having at least 80 % sequence identity to SEQ ID NO: 234 or 236,
with the proviso that the CDRs are as defined in claim 1h); or
IX) • the heavy chain variable domain (VH) comprises, or consists of, an amino acid sequence having at least 80% sequence identity to SEQ ID NO: 225 or 227; and
• the light chain variable domain (VL) comprises, or consists of, an amino acid sequence having at least 80 % sequence identity to SEQ ID NO: 226 or 228,
with the proviso that the CDRs are as defined in claim 1i); or
X) • the heavy chain variable domain (VH) comprises, or consists of, an amino acid sequence having at least 80% sequence identity to SEQ ID NO: 247; and
• the light chain variable domain (VL) comprises, or consists of, an amino acid sequence having at least 80 % sequence identity to SEQ ID NO: 248,
with the proviso that the CDRs are as defined in claim 1j); or
XI) • the heavy chain variable domain (VH) comprises, or consists of, an amino acid sequence having at least 80% sequence identity to SEQ ID NO: 217, 219, 221 or 223; and
• the light chain variable domain (VL) comprises, or consists of, an amino acid sequence having at least 80 % sequence identity to SEQ ID NO: 218, 220, 222 or 224,
with the proviso that the CDRs are as defined in claim 1k); or
XII) • the heavy chain variable domain (VH) comprises, or consists of, an amino acid sequence having at least 80% sequence identity to SEQ ID NO: 209; and
• the light chain variable domain (VL) comprises, or consists of, an amino acid sequence having at least 80 % sequence identity to SEQ ID NO: 210,
with the proviso that the CDRs are as defined in claim 11); or
XIII) • the heavy chain variable domain (VH) comprises, or consists of, an amino acid sequence having at least 80% sequence identity to SEQ ID NO: 257; and
• the light chain variable domain (VL) comprises, or consists of, an amino acid sequence having at least 80 % sequence identity to SEQ ID NO: 258,
with the proviso that the CDRs are as defined in claim 1m); or
XIV) • the heavy chain variable domain (VH) comprises, or consists of, an amino acid sequence having at least 80% sequence identity to SEQ ID NO: 261; and
• the light chain variable domain (VL) comprises, or consists of, an amino acid sequence having at least 80 % sequence identity to SEQ ID NO: 262,
with the proviso that the CDRs are as defined in claim 1n); or
XV) • the heavy chain variable domain (VH) comprises, or consists of, an amino acid sequence having at least 80% sequence identity to SEQ ID NO: 185 or 187; and
• the light chain variable domain (VL) comprises, or consists of, an amino acid sequence having at least 80 % sequence identity to SEQ ID NO: 186 or 188,
with the proviso that the CDRs are as defined in claim 1o); or
XVI) • the heavy chain variable domain (VH) comprises, or consists of, an amino acid sequence having at least 80% sequence identity to SEQ ID NO: 237, 239 or 241; and
• the light chain variable domain (VL) comprises, or consists of, an amino acid sequence having at least 80 % sequence identity to SEQ ID NO: 238, 240 or 242,
with the proviso that the CDRs are as defined in claim 1p) or 1q); or
XVII) • the heavy chain variable domain (VH) comprises, or consists of, an amino acid sequence having at least 80% sequence identity to SEQ ID NO: 237, 239 or 241; and
• the light chain variable domain (VL) comprises, or consists of, an amino acid sequence having at least 80 % sequence identity to SEQ ID NO: 238, 240 or 242,
with the proviso that the CDRs are as defined in claim 1q); or
XVIII) • the heavy chain variable domain (VH) comprises, or consists of, an amino acid sequence having at least 80% sequence identity to SEQ ID NO: 255; and
• the light chain variable domain (VL) comprises, or consists of, an amino acid sequence having at least 80 % sequence identity to SEQ ID NO: 256,
with the proviso that the CDRs are as defined in claim Ir); or
XIX) • the heavy chain variable domain (VH) comprises, or consists of, an amino acid sequence having at least 80% sequence identity to SEQ ID NO: 263; and
• the light chain variable domain (VL) comprises, or consists of, an amino acid sequence having at least 80 % sequence identity to SEQ ID NO: 264,
with the proviso that the CDRs are as defined in claim 1s); or
XX) • the heavy chain variable domain (VH) comprises, or consists of, an amino acid sequence having at least 80% sequence identity to SEQ ID NO: 229; and
• the light chain variable domain (VL) comprises, or consists of, an amino acid sequence having at least 80 % sequence identity to SEQ ID NO: 230,
with the proviso that the CDRs are as defined in claim 1t); or
XXI) • the heavy chain variable domain (VH) comprises, or consists of, an amino acid sequence having at least 80% sequence identity to SEQ ID NO: 269; and
• the light chain variable domain (VL) comprises, or consists of, an amino acid sequence having at least 80 % sequence identity to SEQ ID NO: 270,
with the proviso that the CDRs are as defined in claim 1u); or
XXII) • the heavy chain variable domain (VH) comprises, or consists of, an amino acid sequence having at least 80% sequence identity to SEQ ID NO: 243 or 245; and
• the light chain variable domain (VL) comprises, or consists of, an amino acid sequence having at least 80 % sequence identity to SEQ ID NO: 244 or 246,
with the proviso that the CDRs are as defined in claim 1v); or
XXIII) • the heavy chain variable domain (VH) comprises, or consists of, an amino acid sequence having at least 80% sequence identity to SEQ ID NO: 259; and
• the light chain variable domain (VL) comprises, or consists of, an amino acid sequence having at least 80 % sequence identity to SEQ ID NO: 260,
with the proviso that the CDRs are as defined in claim 1w); or
XXIV) • the heavy chain variable domain (VH) comprises, or consists of, an amino acid sequence having at least 80% sequence identity to SEQ ID NO: 203; and
• the light chain variable domain (VL) comprises, or consists of, an amino acid sequence having at least 80 % sequence identity to SEQ ID NO: 204,
with the proviso that the CDRs are as defined in claim 1x); or
XXV) • the heavy chain variable domain (VH) comprises, or consists of, an amino acid sequence having at least 80% sequence identity to SEQ ID NO: 231; and
• the light chain variable domain (VL) comprises, or consists of, an amino acid sequence having at least 80 % sequence identity to SEQ ID NO: 232,
with the proviso that the CDRs are as defined in claim 1y); or
XXVI) • the heavy chain variable domain (VH) comprises, or consists of, an amino acid sequence having at least 80% sequence identity to SEQ ID NO: 265; and
• the light chain variable domain (VL) comprises, or consists of, an amino acid sequence having at least 80 % sequence identity to SEQ ID NO: 266,
with the proviso that the CDRs are as defined in claim 1z); or
XXVII) • the heavy chain variable domain (VH) comprises, or consists of, an amino acid sequence having at least 80% sequence identity to SEQ ID NO: 267; and
• the light chain variable domain (VL) comprises, or consists of, an amino acid sequence having at least 80 % sequence identity to SEQ ID NO: 268,
with the proviso that the CDRs are as defined in claim 1a'); or
XXVIII) • the heavy chain variable domain (VH) comprises, or consists of, an amino acid sequence having at least 80% sequence identity to SEQ ID NO: 205; and
• the light chain variable domain (VL) comprises, or consists of, an amino acid sequence having at least 80 % sequence identity to SEQ ID NO: 206,
with the proviso that the CDRs are as defined in claim 1b').

5. The antibody, or antigen-binding fragment thereof, according to claim 3, wherein:
• the heavy chain variable domain (VH) comprises, or consists of, an amino acid sequence having at least 80% sequence identity to SEQ ID NO: 177; and
the light chain variable domain (VL) comprises, or consists of, an amino acid sequence having at least 80 % sequence identity to SEQ ID NO: 178,
with the proviso that the CDRs are as defined in claim 3.1); or
• the heavy chain variable domain (VH) comprises, or consists of, an amino acid sequence having at least 80% sequence identity to SEQ ID NO: 179; and
the light chain variable domain (VL) comprises, or consists of, an amino acid sequence having at least 80 % sequence identity to SEQ ID NO: 180,
with the proviso that the CDRs are as defined in claim 3.1); or
• the heavy chain variable domain (VH) comprises, or consists of, an amino acid sequence having at least 80% sequence identity to SEQ ID NO: 181; and
the light chain variable domain (VL) comprises, or consists of, an amino acid sequence having at least 80 % sequence identity to SEQ ID NO: 182,
with the proviso that the CDRs are as defined in claim 3.2); or
• the heavy chain variable domain (VH) comprises, or consists of, an amino acid sequence having at least 80% sequence identity to SEQ ID NO: 183; and
the light chain variable domain (VL) comprises, or consists of, an amino acid sequence having at least 80 % sequence identity to SEQ ID NO: 184,
with the proviso that the CDRs are as defined in claim 3.2); or
• the heavy chain variable domain (VH) comprises, or consists of, an amino acid sequence having at least 80% sequence identity to SEQ ID NO: 189; and
the light chain variable domain (VL) comprises, or consists of, an amino acid sequence having at least 80 % sequence identity to SEQ ID NO: 190,
with the proviso that the CDRs are as defined in claim 3.3); or
• the heavy chain variable domain (VH) comprises, or consists of, an amino acid sequence having at least 80% sequence identity to SEQ ID NO: 191; and
the light chain variable domain (VL) comprises, or consists of, an amino acid sequence having at least 80 % sequence identity to SEQ ID NO: 192,
with the proviso that the CDRs are as defined in claim 3.3); or
• the heavy chain variable domain (VH) comprises, or consists of, an amino acid sequence having at least 80% sequence identity to SEQ ID NO: 193; and
the light chain variable domain (VL) comprises, or consists of, an amino acid sequence having at least 80 % sequence identity to SEQ ID NO: 194,
with the proviso that the CDRs are as defined in claim 3.4); or
• the heavy chain variable domain (VH) comprises, or consists of, an amino acid sequence having at least 80% sequence identity to SEQ ID NO: 197; and
the light chain variable domain (VL) comprises, or consists of, an amino acid sequence having at least 80 % sequence identity to SEQ ID NO: 198,
with the proviso that the CDRs are as defined in claim 3.5); or
• the heavy chain variable domain (VH) comprises, or consists of, an amino acid sequence having at least 80% sequence identity to SEQ ID NO: 199; and
the light chain variable domain (VL) comprises, or consists of, an amino acid sequence having at least 80 % sequence identity to SEQ ID NO: 200,
with the proviso that the CDRs are as defined in claim 3.5); or
• the heavy chain variable domain (VH) comprises, or consists of, an amino acid sequence having at least 80% sequence identity to SEQ ID NO: 201; and
the light chain variable domain (VL) comprises, or consists of, an amino acid sequence having at least 80 % sequence identity to SEQ ID NO: 202,
with the proviso that the CDRs are as defined in claim 3.6); or
• ) the heavy chain variable domain (VH) comprises, or consists of, an amino acid sequence having at least 80% sequence identity to SEQ ID NO: 249; and
the light chain variable domain (VL) comprises, or consists of, an amino acid sequence having at least 80 % sequence identity to SEQ ID NO: 250,
with the proviso that the CDRs are as defined in claim 3.7); or
• the heavy chain variable domain (VH) comprises, or consists of, an amino acid sequence having at least 80% sequence identity to SEQ ID NO: 251; and
the light chain variable domain (VL) comprises, or consists of, an amino acid sequence having at least 80 % sequence identity to SEQ ID NO: 252,
with the proviso that the CDRs are as defined in claim 3.7); or
• the heavy chain variable domain (VH) comprises, or consists of, an amino acid sequence having at least 80% sequence identity to SEQ ID NO: 253; and
the light chain variable domain (VL) comprises, or consists of, an amino acid sequence having at least 80 % sequence identity to SEQ ID NO: 254,
with the proviso that the CDRs are as defined in claim 3.7); or
• the heavy chain variable domain (VH) comprises, or consists of, an amino acid sequence having at least 80% sequence identity to SEQ ID NO: 213; and
the light chain variable domain (VL) comprises, or consists of, an amino acid sequence having at least 80 % sequence identity to SEQ ID NO: 214,
with the proviso that the CDRs are as defined in claim 3.8); or
• the heavy chain variable domain (VH) comprises, or consists of, an amino acid sequence having at least 80% sequence identity to SEQ ID NO: 215; and
the light chain variable domain (VL) comprises, or consists of, an amino acid sequence having at least 80 % sequence identity to SEQ ID NO: 216,
with the proviso that the CDRs are as defined in claim 3.8); or
• the heavy chain variable domain (VH) comprises, or consists of, an amino acid sequence having at least 80% sequence identity to SEQ ID NO: 211; and
the light chain variable domain (VL) comprises, or consists of, an amino acid sequence having at least 80 % sequence identity to SEQ ID NO: 212,
with the proviso that the CDRs are as defined in claim 3.9); or
• the heavy chain variable domain (VH) comprises, or consists of, an amino acid sequence having at least 80% sequence identity to SEQ ID NO: 207; and
the light chain variable domain (VL) comprises, or consists of, an amino acid sequence having at least 80 % sequence identity to SEQ ID NO: 208,
with the proviso that the CDRs are as defined in claim 3.10); or
• ) the heavy chain variable domain (VH) comprises, or consists of, an amino acid sequence having at least 80% sequence identity to SEQ ID NO: 233; and
the light chain variable domain (VL) comprises, or consists of, an amino acid sequence having at least 80 % sequence identity to SEQ ID NO: 234,
with the proviso that the CDRs are as defined in claim 3.11); or
• the heavy chain variable domain (VH) comprises, or consists of, an amino acid sequence having at least 80% sequence identity to SEQ ID NO: 235; and
the light chain variable domain (VL) comprises, or consists of, an amino acid sequence having at least 80 % sequence identity to SEQ ID NO: 236,
with the proviso that the CDRs are as defined in claim 3.12); or
• the heavy chain variable domain (VH) comprises, or consists of, an amino acid sequence having at least 80% sequence identity to SEQ ID NO: 225; and
the light chain variable domain (VL) comprises, or consists of, an amino acid sequence having at least 80 % sequence identity to SEQ ID NO: 226,
with the proviso that the CDRs are as defined in claim 3.13); or
• the heavy chain variable domain (VH) comprises, or consists of, an amino acid sequence having at least 80% sequence identity to SEQ ID NO: 227; and
the light chain variable domain (VL) comprises, or consists of, an amino acid sequence having at least 80 % sequence identity to SEQ ID NO: 228,
with the proviso that the CDRs are as defined in claim 3.13); or
• ) the heavy chain variable domain (VH) comprises, or consists of, an amino acid sequence having at least 80% sequence identity to SEQ ID NO: 247; and
the light chain variable domain (VL) comprises, or consists of, an amino acid sequence having at least 80 % sequence identity to SEQ ID NO: 248,
with the proviso that the CDRs are as defined in claim 3.14); or
• the heavy chain variable domain (VH) comprises, or consists of, an amino acid sequence having at least 80% sequence identity to SEQ ID NO: 217; and
the light chain variable domain (VL) comprises, or consists of, an amino acid sequence having at least 80 % sequence identity to SEQ ID NO: 218,
with the proviso that the CDRs are as defined in claim 3.15); or
• the heavy chain variable domain (VH) comprises, or consists of, an amino acid sequence having at least 80% sequence identity to SEQ ID NO: 219; and
the light chain variable domain (VL) comprises, or consists of, an amino acid sequence having at least 80 % sequence identity to SEQ ID NO: 220,
with the proviso that the CDRs are as defined in claim 3.15); or
• the heavy chain variable domain (VH) comprises, or consists of, an amino acid sequence having at least 80% sequence identity to SEQ ID NO: 221; and
the light chain variable domain (VL) comprises, or consists of, an amino acid sequence having at least 80 % sequence identity to SEQ ID NO: 222,
with the proviso that the CDRs are as defined in claim 3.15); or
• the heavy chain variable domain (VH) comprises, or consists of, an amino acid sequence having at least 80% sequence identity to SEQ ID NO: 223; and
the light chain variable domain (VL) comprises, or consists of, an amino acid sequence having at least 80 % sequence identity to SEQ ID NO: 224,
with the proviso that the CDRs are as defined in claim 3.16); or
• the heavy chain variable domain (VH) comprises, or consists of, an amino acid sequence having at least 80% sequence identity to SEQ ID NO: 209; and
the light chain variable domain (VL) comprises, or consists of, an amino acid sequence having at least 80 % sequence identity to SEQ ID NO: 210,
with the proviso that the CDRs are as defined in claim 3.17); or
• the heavy chain variable domain (VH) comprises, or consists of, an amino acid sequence having at least 80% sequence identity to SEQ ID NO: 257; and
the light chain variable domain (VL) comprises, or consists of, an amino acid sequence having at least 80 % sequence identity to SEQ ID NO: 258,
with the proviso that the CDRs are as defined in claim 3.18); or
• the heavy chain variable domain (VH) comprises, or consists of, an amino acid sequence having at least 80% sequence identity to SEQ ID NO: 261; and
the light chain variable domain (VL) comprises, or consists of, an amino acid sequence having at least 80 % sequence identity to SEQ ID NO: 262,
with the proviso that the CDRs are as defined in claim 3.19); or
• the heavy chain variable domain (VH) comprises, or consists of, an amino acid sequence having at least 80% sequence identity to SEQ ID NO: 185; and
the light chain variable domain (VL) comprises, or consists of, an amino acid sequence having at least 80 % sequence identity to SEQ ID NO: 186,
with the proviso that the CDRs are as defined in claim 3.20); or
• the heavy chain variable domain (VH) comprises, or consists of, an amino acid sequence having at least 80% sequence identity to SEQ ID NO: 187; and
the light chain variable domain (VL) comprises, or consists of, an amino acid sequence having at least 80 % sequence identity to SEQ ID NO: 188,
with the proviso that the CDRs are as defined in claim 3.21); or
• the heavy chain variable domain (VH) comprises, or consists of, an amino acid sequence having at least 80% sequence identity to SEQ ID NO: 237; and
the light chain variable domain (VL) comprises, or consists of, an amino acid sequence having at least 80 % sequence identity to SEQ ID NO: 238,
with the proviso that the CDRs are as defined in claim 3.22); or
• the heavy chain variable domain (VH) comprises, or consists of, an amino acid sequence having at least 80% sequence identity to SEQ ID NO: 239; and
the light chain variable domain (VL) comprises, or consists of, an amino acid sequence having at least 80 % sequence identity to SEQ ID NO: 240,
with the proviso that the CDRs are as defined in claim 3.23); or
• the heavy chain variable domain (VH) comprises, or consists of, an amino acid sequence having at least 80% sequence identity to SEQ ID NO: 241; and
the light chain variable domain (VL) comprises, or consists of, an amino acid sequence having at least 80 % sequence identity to SEQ ID NO: 242,
with the proviso that the CDRs are as defined in claim 3.24); or
• ) the heavy chain variable domain (VH) comprises, or consists of, an amino acid sequence having at least 80% sequence identity to SEQ ID NO: 255; and
the light chain variable domain (VL) comprises, or consists of, an amino acid sequence having at least 80 % sequence identity to SEQ ID NO: 256,
with the proviso that the CDRs are as defined in claim 3.25); or
• the heavy chain variable domain (VH) comprises, or consists of, an amino acid sequence having at least 80% sequence identity to SEQ ID NO: 263; and
the light chain variable domain (VL) comprises, or consists of, an amino acid sequence having at least 80 % sequence identity to SEQ ID NO: 264,
with the proviso that the CDRs are as defined in claim 3.26); or
• ) the heavy chain variable domain (VH) comprises, or consists of, an amino acid sequence having at least 80% sequence identity to SEQ ID NO: 229; and
the light chain variable domain (VL) comprises, or consists of, an amino acid sequence having at least 80 % sequence identity to SEQ ID NO: 230,
with the proviso that the CDRs are as defined in claim 3.27); or
• the heavy chain variable domain (VH) comprises, or consists of, an amino acid sequence having at least 80% sequence identity to SEQ ID NO: 269; and
the light chain variable domain (VL) comprises, or consists of, an amino acid sequence having at least 80 % sequence identity to SEQ ID NO: 270,
with the proviso that the CDRs are as defined in claim 3.28); or
• the heavy chain variable domain (VH) comprises, or consists of, an amino acid sequence having at least 80% sequence identity to SEQ ID NO: 243; and
the light chain variable domain (VL) comprises, or consists of, an amino acid sequence having at least 80 % sequence identity to SEQ ID NO: 244,
with the proviso that the CDRs are as defined in claim 3.29); or
• the heavy chain variable domain (VH) comprises, or consists of, an amino acid sequence having at least 80% sequence identity to SEQ ID NO: 245; and
the light chain variable domain (VL) comprises, or consists of, an amino acid sequence having at least 80 % sequence identity to SEQ ID NO: 246,
with the proviso that the CDRs are as defined in claim 3.30); or
• the heavy chain variable domain (VH) comprises, or consists of, an amino acid sequence having at least 80% sequence identity to SEQ ID NO: 259; and
the light chain variable domain (VL) comprises, or consists of, an amino acid sequence having at least 80 % sequence identity to SEQ ID NO: 260,
with the proviso that the CDRs are as defined in claim 3.31); or
• the heavy chain variable domain (VH) comprises, or consists of, an amino acid sequence having at least 80% sequence identity to SEQ ID NO: 203; and
the light chain variable domain (VL) comprises, or consists of, an amino acid sequence having at least 80 % sequence identity to SEQ ID NO: 204,
with the proviso that the CDRs are as defined in claim 3.32); or
• the heavy chain variable domain (VH) comprises, or consists of, an amino acid sequence having at least 80% sequence identity to SEQ ID NO: 231; and
the light chain variable domain (VL) comprises, or consists of, an amino acid sequence having at least 80 % sequence identity to SEQ ID NO: 232,
with the proviso that the CDRs are as defined in claim 3.33); or
• the heavy chain variable domain (VH) comprises, or consists of, an amino acid sequence having at least 80% sequence identity to SEQ ID NO: 265; and
the light chain variable domain (VL) comprises, or consists of, an amino acid sequence having at least 80 % sequence identity to SEQ ID NO: 266,
with the proviso that the CDRs are as defined in claim 3.34); or
• the heavy chain variable domain (VH) comprises, or consists of, an amino acid sequence having at least 80% sequence identity to SEQ ID NO: 267; and
the light chain variable domain (VL) comprises, or consists of, an amino acid sequence having at least 80 % sequence identity to SEQ ID NO: 268,
with the proviso that the CDRs are as defined in claim 3.35); or
• the heavy chain variable domain (VH) comprises, or consists of, an amino acid sequence having at least 80% sequence identity to SEQ ID NO: 205; and
the light chain variable domain (VL) comprises, or consists of, an amino acid sequence having at least 80 % sequence identity to SEQ ID NO: 206,
with the proviso that the CDRs are as defined in claim 3.36); or
• the heavy chain variable domain (VH) comprises, or consists of, an amino acid sequence having at least 80% sequence identity to SEQ ID NO: 195; and
the light chain variable domain (VL) comprises, or consists of, an amino acid sequence having at least 80 % sequence identity to SEQ ID NO: 196,
with the proviso that the CDRs are as defined in claim 3.37).

6. An antibody, or antigen-binding fragment thereof, that binds and optionally neutralizes a plurality of BK virus subtypes including at least subtype I, II and IV, wherein said antibody has one or more of the following characteristics:
(1) competes for binding to a BKV viral protein 1 (VP1), with a reference antibody as in defined in claims 1, 3 or 5, such as antibody E11P6 (VH: SEQ ID NO: 183; VL: SEQ ID NO: 184); and/or
(2) binds to an epitope comprising one or more amino acids selected from D60, L68, A72, N74, S80, R170 and/or S275 in BKV viral protein 1 (VP1) of SEQ ID NO: 271; and/or
(3) has reduced binding or loses binding to a mutant BKV viral protein 1 (VP1), said mutant containing one or more of the following amino acid mutations in SEQ ID NO: 271: D60A, L68A, A72A, N74A, S80A, R170A and/or S275A.

7. An antibody, or antigen-binding fragment thereof, that binds and optionally neutralizes a plurality of BK virus subtypes including at least subtype I, II and IV, wherein said antibody has one or more of the following characteristics:
(1) competes for binding to a BKV viral protein 1 (VP1), with a reference antibody as in defined in claims 1, 3 or 5, such as antibody G7P7 (VH: SEQ ID NO: 253; VL: SEQ ID NO: 254); and/or
(2) binds to an epitope comprising one or more amino acids selected from E61, N62, L68, K69, E73, N74, S77, S133, K135 and/or S275 in BKV viral protein 1 (VP1) of SEQ ID NO: 271; and/or
(3) has reduced binding or loses binding to a mutant BKV viral protein 1 (VP1), said mutant containing one or more of the following amino acid mutations in SEQ ID NO: 271: E61A, N62A, L68A, K69A, E73A, N74A, S77A, S133A, K135A and/or S275A.

8. An antibody, or antigen-binding fragment thereof, that binds and optionally neutralizes a plurality of BK virus subtypes including at least subtype I, II and IV, wherein said antibody has one or more of the following characteristics:
(1) competes for binding to a BKV viral protein 1 (VP1), with a reference antibody as in defined in claims 1, 3 or 5, such as antibody E6P5 (VH: SEQ ID NO: 207; VL: SEQ ID NO: 206); and/or
(2) binds to an epitope comprising one or more amino acids selected from D60, D75, S77, Y169, R170, K172, N273, S275 and/or T277 in BKV viral protein 1 (VP1) of SEQ ID NO: 271; and/or
(3) has reduced binding or loses binding to a mutant BKV viral protein 1 (VP1), said mutant containing one or more of the following amino acid mutations in SEQ ID NO: 271: D60A, D75A, S77A, Y169A, R170A, K172A, N273A, S275A and/or T277A.

9. The antibody, or antigen-binding fragment thereof, according to any one of the preceding claims, which is an IgG, an IgA, an sIgA, an IgM or a nanobody, preferably an IgG, said IgG being preferably an IgG1, IgG2, IgG3 or IgG4.

10. The antibody, or antigen-binding fragment thereof, according to any one of the preceding claims, further comprising, especially in the Fc region, mutations or modifications which modulate Fc/FcRn binding, complement binding, antibody-dependent cellular toxicity (ADCC), antibody-dependent cell phagocytosis (ADCP), glycosylation and/or pharmacokinetics such as half-life.

11. The antibody, or antigen-binding fragment thereof, according to any one of the preceding claims, which comprises one or more non-natural amino acids.

12. The antibody, or antigen-binding fragment thereof, according to any one of the preceding claims, which is comprised in an immunoconjugate, such as an immunoconjugate wherein the antibody or antigen-binding fragment is conjugated with another moiety, such as another moiety selected from another antibody, including another anti-BKV antibody, a cytotoxic moiety (to form an ADC), a cell-penetrating compound or a tissue-penetrating compound.

13. The antibody, or antigen-binding fragment thereof, according to any one of claims 1 to 12, which is labeled with a detectable molecule.

14. A combination of two or more antibodies, or antigen-binding fragments thereof, as defined in any one of claims 1 to 13.

15. A set of nucleic acids encoding the antibody, or antigen-binding fragment thereof, as defined in any one of claims 1 to 13, or a vector comprising said set of nucleic acids.

16. A host cell expressing the antibody, or antigen-binding fragment thereof, as defined in any one of claims 1 to 13, or comprising the set of nucleic acids or the vector as defined in claim 15.

17. A pharmaceutical or diagnostic composition comprising:
(1) at least one of the antibody, or antigen-binding fragment thereof, as defined in any one of claims 1 to 13, or any combination thereof; and
(2) optionally at least one pharmaceutically or diagnostically acceptable carrier.

18. A pharmaceutical composition according to claim 17, which in a form suitable for a sustainable release, and/or for intravenous, intramuscular, subcutaneous intranasal, or intrathecal administration, or for administration by infusion or by aerosol.

19. A pharmaceutical composition as defined in claim 17 or 18, for use as a medicament.

20. The pharmaceutical composition for use according to claim 19, in the prevention or treatment of a BK virus (BKV) infection or associated disorder thereof, in a subject in need thereof.

21. The pharmaceutical composition for use according to claim 20, wherein the subject is an immunocompromised subject, especially a graft-recipient such as a kidney-graft or bone-marrow-graft recipient.

22. The pharmaceutical composition for use according to claim 20 or 21, wherein the disorder associated with a BK virus (BKV) infection is a BKV-related leukodystrophy.

23. A pharmaceutical composition as defined in claim 17 or 18, and at least one antibody reacting with a non-BKV virus, as a combined preparation for simultaneous, separate or sequential use as a medicament, the non-BKV virus being selected from the group consisting of a SARS-CoV-2 virus and other coronaviruses, a Respiratory Syncytial Virus (RSV), a Herpes Virus Simplex (HSV), an Epstein-Barr virus (EBV), a cytomegalovirus (CMV), an influenza virus, a parainfluenza virus, a metapneumovirus, and a JC virus (JCV).

24. Two or more antibodies or antigen-binding fragments thereof as defined in any one of claims 1 to 13, as a combined preparation for simultaneous, separate or sequential use as a medicament, preferably in the treatment of a BK virus (BKV) infection or associated disorder thereof, in a subject in need thereof.

25. An *in vitro* use of the diagnostic composition as defined in claim 17, for detecting the presence of BKV in a sample, preferably in a biological sample.

26. An *in vitro* use of an anti-idiotypic antibody that binds to the antibody or antigen-binding fragment thereof as defined in any one of claims 1 to 13, for measuring the concentration of said antibody or fragment in a sample, preferably in a biological sample.
